# EUROPEAN PATENT APPLICATION

(11) **EP 3 323 893 A1**
(43) Date of publication of application: **23.05.2018**
(21) Application number: 16824555.3
(22) Date of filing: 15.07.2016
(51) Int. Cl.: C12N 15/113, A61K 31/713, A61K 48/00, A61P 7/02, A61P 37/02, C07H 21/02

(54) **BETA2GPI GENE EXPRESSION INHIBITING NUCLEIC ACID COMPLEX**

(30) Priority: 16.07.2015 JP 2015142522
(71) Applicant: Kyowa Hakko Kirin Co., Ltd., Tokyo 100-8185 (JP)
(72) Inventor: NAGATOMO, Kazutaka, Tokyo 100-8185 (JP); MASUDA, Kazuhiro, Tokyo 100-8185 (JP); KANDA, Minako, Tokyo 100-8185 (JP); YAMADA, Yoji, Tokyo 100-8185 (JP); IWAI, Hiroto, Tokyo 100-8185 (JP); MAKINO, Asana, Tokyo 100-8185 (JP); HOSOE, Shintaro, Tokyo 100-8185 (JP); KUBOYAMA, Takeshi, Tokyo 100-8185 (JP); UEHARA, Keiji, Tokyo 100-8185 (JP); SUZUKI, Yasuhiro, Tokyo 100-8185 (JP)
(74) Representative: D Young & Co LLP
(86) International application number: PCT/JP2016/071087
(87) International publication number: WO 2017/010575

(57) **Abstract**

The present invention provides a nucleic acid conjugate comprising: a double-stranded nucleic acid consisting of a sense strand and an antisense strand and comprising a duplex region of at least 11 base pairs; and a ligand part, wherein an oligonucleotide with a strand length of 17 to 30 nucleotides in the antisense strand is complementary to a target β2GPI mRNA sequence, and the 3' end or 5' end of the sense strand has a ligand part represented by following formula (I), formula (II), formula (III), formula (IV) or formula (V).

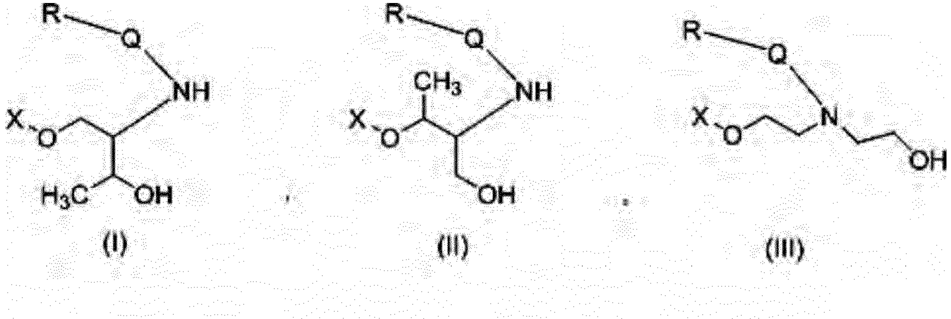

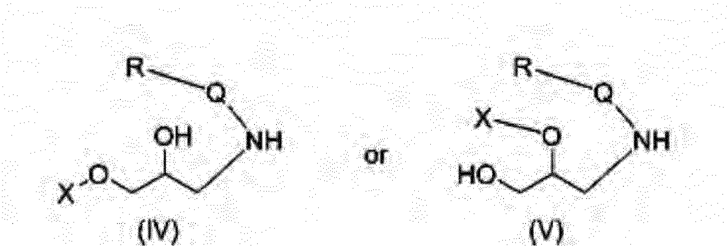

## Description

### Technical Field

The present invention relates to a nucleic acid conjugate that suppresses β2GPI gene expression, a composition containing the conjugate, a medicine containing the composition and the like.

### Background Art

β2-Glycoprotein 1 (β2GPI, also called apolipoprotein H (apoH)) is a soluble glycoprotein composed of 326 amino acid residues, which is principally produced in the liver (International Journal of Clinical and Laboratory Research, 1992, Vol. 21, pp. 256-263). β2GPI is thought to have various physiological actions, and is reportedly associated with platelet aggregation reactions, coagulation and fibrinolysis reactions, and uptake of oxidized LDL by macrophages (Non-Patent Document 1).

In terms of an association with diseases, β2GPI is known as a principal antigen corresponding to antiphospholipid antibodies, which occur in autoimmune disorders such as antiphospholipid syndrome (APS) and systemic lupus erythematosus (SLE) (Non-Patent Document 2). Anti-β2GPI antibodies are also deeply involved in disease pathogenesis: it has been shown from clinical research and studies using animal models that complexes formed from β2GPI and anti-β2GPI antibodies generate activation signals on membrane receptors of various cells such as vascular endothelial cells, monocytes, platelets and trophoblasts, leading to pathologies characteristic of APS, such as thrombosis and abnormal pregnancy (Non-Patent Document 3). It is expected that such diseases can be prevented or treated by specifically inhibiting the formation of immune complexes consisting of β2GPI and anti-β2GPI antibodies. However, since β2GPI is present in blood at a relatively high concentration of 50 to 500 µg/mL, it is difficult to continuously inhibit all of β2GPI with, for example, an ordinary antibody medicine (Non-Patent Document 4).

On the other hand, methods using RNA interference (hereinafter referred to as "RNAi") are known as methods for suppressing expression of target genes. Specifically, it has been reported that expression of a target gene has been specifically inhibited by introducing a double-stranded RNA having a sequence identical to the target gene in nematodes (Nature, 1998, Vol. 391, No. 6669, pp. 806-811). It has also been discovered that expression of a target gene can be suppressed in Drosophila by introducing a double-stranded RNA having a length of 21 to 23 bases instead of a longer double-stranded RNA. This is called short interfering RNA (siRNA) (WO 01/75164).

RNAi has also been verified in many in vivo tests, and it has been reported that siRNA having a length of 50 base pairs or less provides effects in fetal animals (see the specification of US Patent Publication No. 2002/132788) and effects in adult mice (WO 03/10180) by. It has also been confirmed that intravenous administration of siRNA to fetal mice suppresses the expression of specific genes in the kidney, spleen, lungs, pancreas and liver (Nature Genetics, 2002, Vol. 32, No. 1, pp. 107-108). Furthermore, it has also been reported that direct administration of siRNA to brain cells suppresses the expression of a specific gene (Nature Biotechnology, 2002, Vol. 20, No. 10, pp. 1006-1010).

One method that has been reported to be effective for delivering medicines (especially nucleic acids) in vivo is use of a nucleic acid complex (conjugate) having a targeting compound and a nucleic acid (especially siRNA). The targeting compound may be a ligand capable of binding to an expressed extracellular receptor. In particular, there have been many reports of nucleic acid conjugates using ligands such as N-acetyl-D-galactosamine (GalNAc), which can bind to the asialoglycoprotein receptor (ASGPR), which is highly expressed in liver cells. Recently, it was reported that nucleic acid conjugates having siRNA bound to such ligands can be effectively delivered to liver cells (Non-Patent Document 5).

Complexes of targeting compounds and siRNA are described for example in Patent Documents 1 to 4.

For example, Patent Documents 1 and 2 disclose the following nucleic acid conjugate. 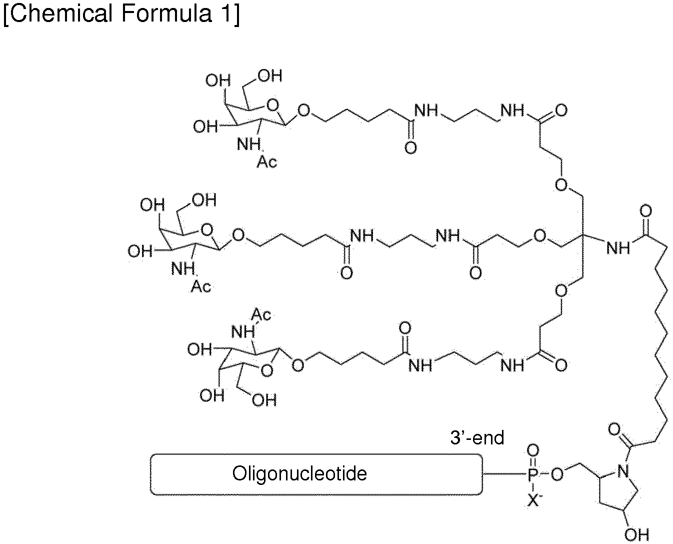 (In this formula and below, Ac represents acetyl group).

Patent Document 3 discloses the following nucleic acid conjugate for example.

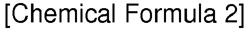

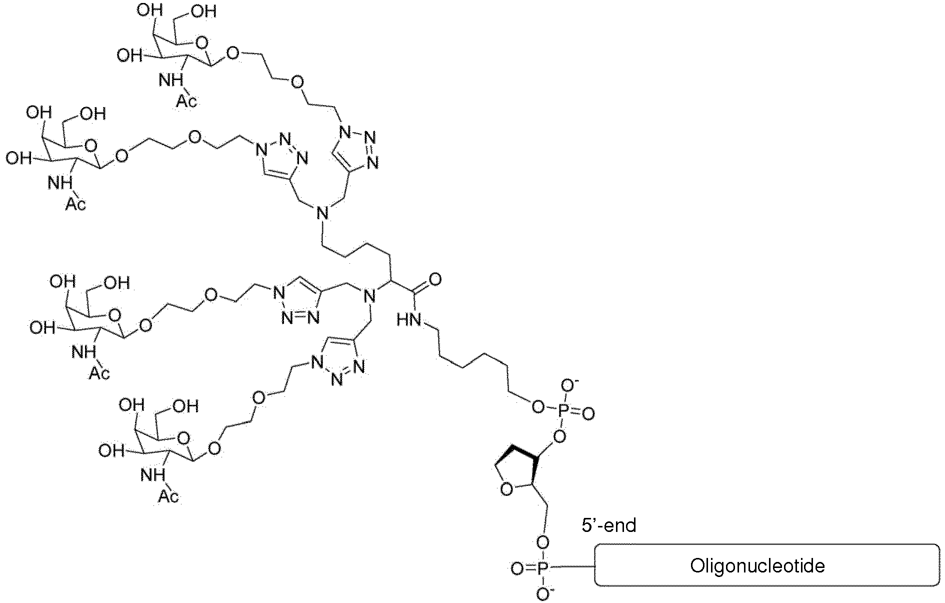

Patent Document 4 also discloses similar nucleic acid conjugates.

Although Patent Documents 5 and 6 disclose a part of a siRNA sequence targeting the human β2GPI gene, they do not disclose that this siRNA sequence suppresses expression of the human β2GPI gene.

### Citation List

### Patent Documents

Patent Document 1: WO 2009/073809
Patent Document 2: WO 2013/075035
Patent Document 3: WO 2013/166121
Patent Document 4: WO 2011/104169
Patent Document 5: WO 2005/116204
Patent Document 6: WO 2008/043561

### Non-Patent Documents

Non-Patent Document 1: Annals of the New York Academy of Sciences, 2013, Vol. 1285, pp. 44-58
Non-Patent Document 2: Nature Review Rheumatology, 2011, Vol. 7, No. 6, pp. 330-339
Non-Patent Document 3: The New England Journal of Medicine, 2013, Vol. 368, No. 11, pp. 1033-1044
Non-Patent Document 4: Journal of Thrombosis and Haemostasis, 2011, Vol. 9, No. 7, pp. 1275-1284
Non-Patent Document 5: Journal of American Chemical Society, 2014, Vol. 136, pp. 16958-16961

### Summary of Invention

### Problem to be solved be the invention

An object of the present invention is to provide a nucleic acid conjugate that suppresses β2GPI gene expression, as well as a composition containing the conjugate, a medicine containing the composition and the like.

### Means for solving the Problem

The present invention relates to (1) to (30) below.
(1) A nucleic acid conjugate comprising: a double-stranded nucleic acid consisting of a sense strand and an antisense strand and comprising a duplex region of at least 11 base pairs; and a ligand part,
   wherein an oligonucleotide strand with a strand length of 17 to 30 nucleotides in the antisense strand is complementary to a target β2GPI mRNA sequence selected from the group described in Tables 4-1 to 4-16, and
   the 3' end or 5' end of the sense strand has the ligand part represented by formula (I), formula (II), formula (III), formula (IV) or formula (V): 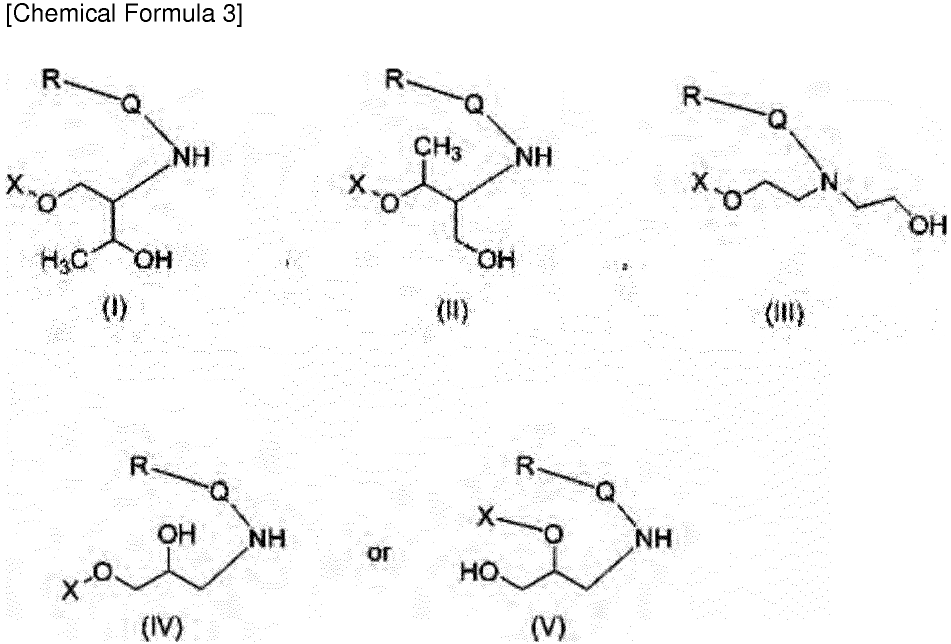 (wherein,
   X is oligonucleotide-P(Z1)(Z2)-, where the oligonucleotide is the sense strand,
   Q is absent or -T4-[Q4-P4]q4-,
   R is the following structure

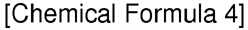

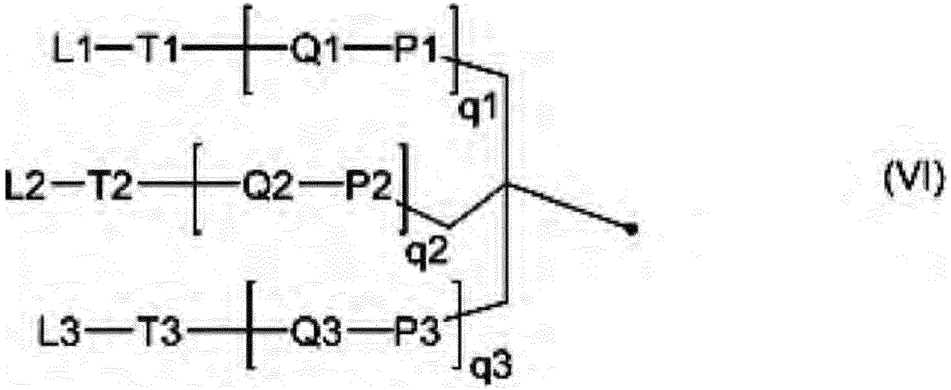

   Z1 and Z2 are, independently of each other, O or S;
   q1, q2, q3 and q4 are, independently of each other, an integer from 0 to 20;
   P1, P2, P3 and P4 and T1, T2, T3 and T4 are, independently of each other, absent or -CO-, -NH-, -O-, -S-, -O-CO-, -NH-CO-, -CO-O- or CO-NH-, respectively, provided that when each of q1, q2, q3 and q4 is an integer from 2 to 20, each of P1, P2, P3 and P4 may be the same or different,
   Q1, Q2, Q3 and Q4 are, independently of each other, absent or a substituted or unsubstituted C₁₋₁₄ alkylene, provided that when each of q1, q2, q3 and q4 is an integer from 2 to 20, each of Q1, Q2, Q3 and Q4 may be the same or different,
   L1, L2 and L3 are, independently of each other, a sugar ligand.)
(2) The nucleic acid conjugate according to (1) above, wherein one or more of L1, L2 and L3 are a sugar ligand represented by the following structure 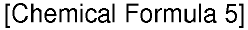 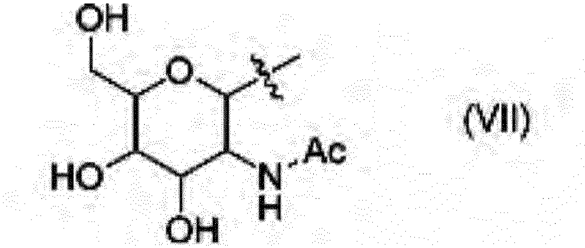 (wherein, Ac represents acetyl group).
(3) The nucleic acid conjugate according to (1) or (2) above, wherein L1, L2 and L3 are identical.
(4) The nucleic acid conjugate according to any of (1) to (3) above, wherein T1, T2 and T3 are identical, q1, q2 and q3 are identical, Q1, Q2 and Q3 are identical, and P1, P2 and P3 are identical.
(5) The nucleic acid conjugate according to any of (1) to (4) above, wherein R is 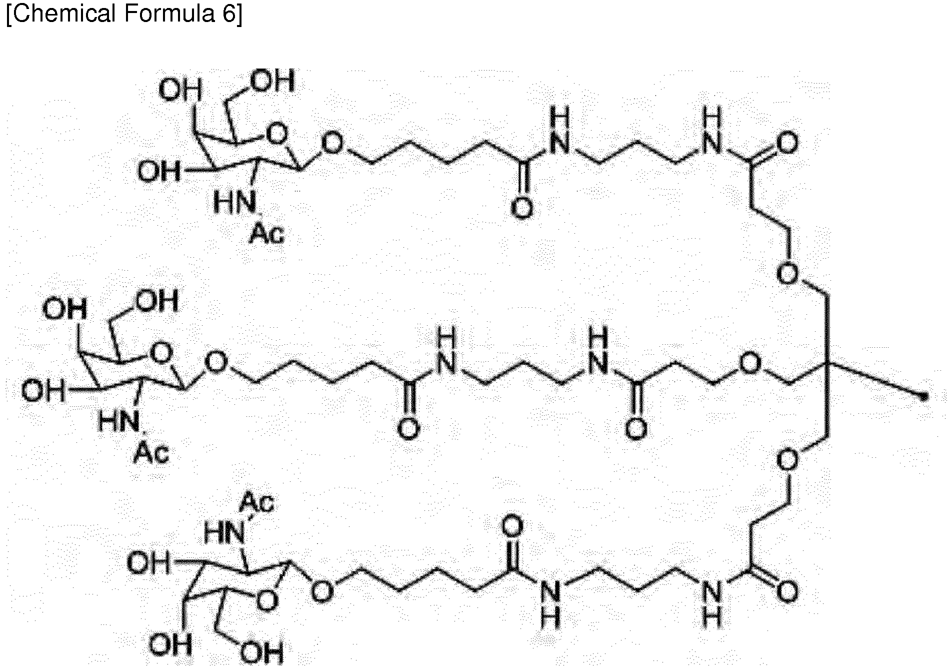 (wherein, Ac represents acetyl group).
(6) The nucleic acid conjugate according to any of (1) to (5) above, wherein R-Q- is 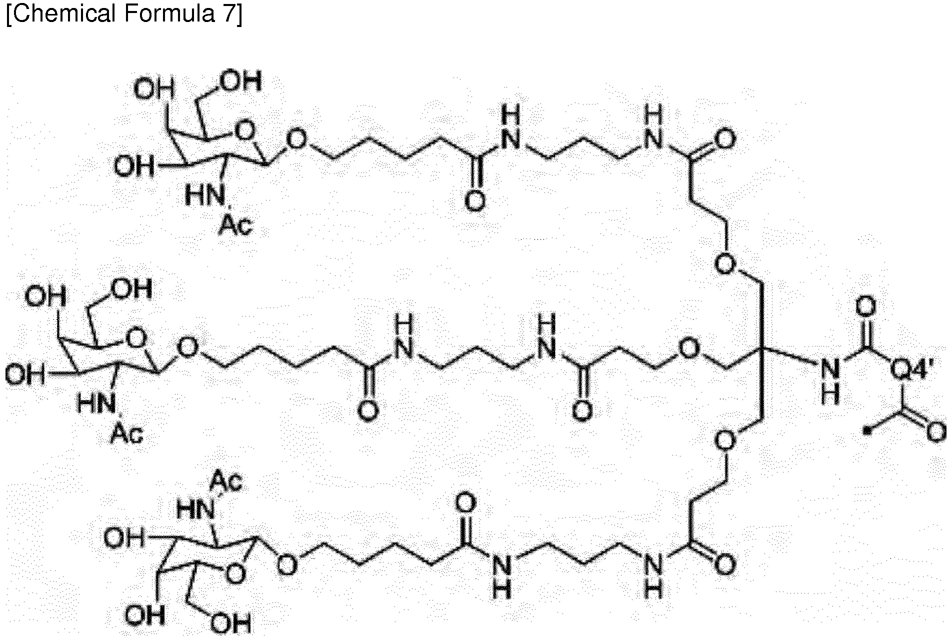 (wherein, Ac represents acetyl group, and Q4' is a substituted or unsubstituted C₁₋₁₄ alkylene).
(7) The nucleic acid conjugate according to any of (1) to (6) above, comprising a ligand part represented by any of formulae (I) to (V) at the 3' end of the sense strand of the double-stranded nucleic acid.
(8) The nucleic acid conjugate according to any of (1) to (6) above, comprising a ligand part represented by any of formulae (I) to (V) at the 5' end of the sense strand of the double-stranded nucleic acid.
(9) The nucleic acid conjugate according to any of (1) to (8) above, wherein
   the duplex region comprises 11 to 27 base pairs, and
   the second nucleotide from the 5' end of the antisense strand, which is complementary to the target β2GPI mRNA sequence selected from the group described in Tables 4-1 to 4-16, is complementary to the second ribonucleotide from the 3' end of the target β2GPI mRNA sequence.
(10) The nucleic acid conjugate according to any of (1) to (9) above, wherein the sense strand has a strand length of 21 to 25 nucleotides, and the antisense strand has a strand length of 21 to 25 nucleotides.
(11) The nucleic acid conjugate according to any of (1) to (10) above, wherein the double-stranded nucleic acid in which the sense strand has a strand length of 21 to 25 nucleotides and the antisense strand has a strand length of 21 to 25 nucleotides comprises a duplex region of 19 to 21 base pairs.
(12) The nucleic acid conjugate according to any of (1) to (9) above, wherein the 3' end of the sense strand and the 5' end of the antisense strand form a blunt end.
(13) The nucleic acid conjugate according to any of (1) to (12) above, wherein the double-stranded nucleic acid comprises a 2'-modified nucleotide.
(14) The nucleic acid conjugate according to (13) above, wherein 50% to 100% of the nucleotides in the duplex region is the 2'-modified nucleotide..
(15) The nucleic acid conjugate according to (13) or (14) above, wherein the 2'-modified nucleotide is 2'-O-methyl modified nucleotide, 2'-F modified nucleotide or 2'-H modified nucleotide.
(16) The nucleic acid conjugate according to any of (1) to (15) above, wherein the antisense strand comprises a sequence selected from the group of antisense strands described in Tables 2-1 to 2-18, Tables 3-1 to 3-18 or Tables 4-1 to 4-16.
(17) The nucleic acid conjugate according to any of (1) to (15) above, wherein the sense strand comprises a sequence selected from the group of sense strands described in Tables 2-1 to 2-18, Tables 3-1 to 3-18 or Tables 4-1 to 4-16.
(18) The nucleic acid conjugate according to any of (1) to (8) above, comprising sequences of a pair of sense/antisense strands selected from the group of sense/antisense strands described in Tables 2-1 to 2-18, Tables 3-1 to 3-18 or Tables 4-1 to 4-16.
(19) A nucleic acid conjugate-comprising composition, comprising the nucleic acid conjugate according to any of (1) to (18) above.
(20) A method for suppressing expression of a β2GPI gene, comprising introducing a double-stranded nucleic acid into cells by using the composition according to (19) above.
(21) The method according to (20) above, wherein the cells are cells in the liver of a mammal.
(22) The method according to (20) or (21) above, wherein the method of introduction into cells is a method of introduction into cells by intravenous administration or subcutaneous administration.
(23) A method for treating a β2GPI-associated disease, comprising administrating a composition according to (19) above to a mammal.
(24) The method according to (23) above, wherein the β2GPI-associated disease is an autoimmune disease or thrombosis.
(25) The method according to (23) or (24) above, wherein the method of administration is intravenous administration or subcutaneous administration.
(26) A medicine for use in the treatment of a β2GPI-associated disease, comprising the composition according to (19) above.
(27) The medicine according to (26) above, wherein the β2GPI-associated disease is an autoimmune disease or thrombosis.
(28) The medicine according to (26) or (27) above, for intravenous administration or subcutaneous administration.
(29) An agent for treating an autoimmune disease or thrombosis, comprising the composition according to (19) above.
(30) The agent for treating an autoimmune disease or thrombosis according to (29) above, for intravenous administration or subcutaneous administration.

### Advantageous Effects of Invention

For example, an administration of a composition containing the nucleic acid conjugate of the present invention to a mammal can suppress β2GPI gene expression, thereby treating a β2GPI-associated disease in vivo.

### Description of Embodiments

An example of a β2GPI gene (gene encoding β2GPI) targeted by the nucleic acid of the invention includes a gene corresponding to β2GPI cDNA (SEQ ID NO: 3541) recorded under Genbank Accession No. NM_000042, which produces full-length β2GPI mRNA.

The present invention provides, as a medicine, a nucleic acid conjugate comprising a double-stranded nucleic acid capable of reducing or stopping β2GPI gene expression, and a ligand part.

It also provides a method for treating a β2GPI-associated disease in vivo through suppression of β2GPI gene expression, by administering a nucleic acid conjugate-containing composition containing the conjugate to a mammal.

Moreover, the present invention also provides a method for treating or preventing disorders mediated by anti-β2GPI antibodies.

The nucleic acid conjugate of the present invention is a nucleic acid conjugate having a ligand part represented by formula (I), formula (II), formula (III), formula (IV) or formula (V) at the 3' end or 5' end of a sense strand constituting the double-stranded nucleic acid. 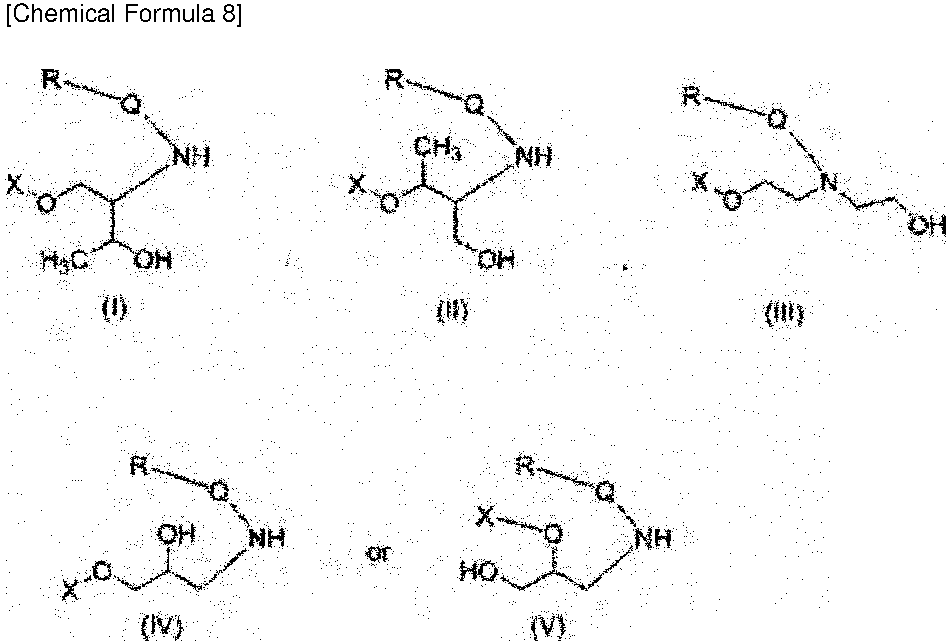 (wherein,
X is oligonucleotide-P(Z1)(Z2)-, wherein the oligonucleotide is the sense strand,
Q is absent or -T4-[Q4-P4]q4-,
R is the following structure

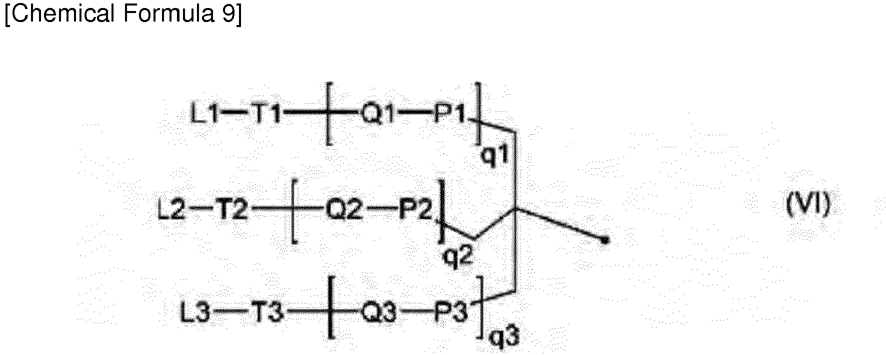

Z1 and Z2 are, independently of each other, O or S;
q1, q2, q3 and q4 are, independently of each other, an integer from 0 to 20;
P1, P2, P3 and P4 and T1, T2, T3 and T4 are, independently of each other, absent or -CO-, -NH-, -O-, -S-, -O-CO-, -NH-CO-, -CO-O- or CO-NH-, respectively, provided that when each of q1, q2, q3 and q4 is an integer from 2 to 20, each of P1, P2, P3 and P4 may be the same or different,
Q1, Q2, Q3 and Q4 are, independently of each other, absent or a substituted or unsubstituted C₁₋₁₄ alkylene, provided that when each of q1, q2, q3 and q4 is an integer from 2 to 20, each of Q1, Q2, Q3 and Q4 may be the same or different,
L1, L2 and L3 are, independently of each other, a sugar ligand.)

In formulae (I) to (V) above, X is oligonucleotide-P(Z1)(Z2)-, in which the oligonucleotide is the sense strand of the double-stranded nucleic acid in the nucleic acid conjugate of the present invention.

Z1 and Z2 above are, independently of each other, O or S, preferably Z1 is O and Z2 is S or O, and more preferably both Z1 and Z2 are O.

The sense strand constituting the oligonucleotide includes those described below, and preferred sense strands are also described below.

Q in formulae (I) to (V) above is either absent or -T4-[Q4-P4]q4-, preferably - T4-Q4-P4-, and more preferably -NH-CO-Q4-CO- (Q4 is defined as above).

T4 is either absent, or -CO-, -NH-, -O-, -S-, -O-CO-, -NH-CO-, -CO-O- or -CO-NH-, preferably -NH, -NH-CO- or -CO-NH-, and more preferably -NH-CO-.

Q4 above is either absent, or a substituted or unsubstituted C₁₋₁₄ alkylene, preferably a C₄₋₁₄ alkylene, more preferably a C₆₋₁₂ alkylene, and still more preferably a C₈₋₁₀ alkylene. When there are 2 or more Q4s (when q4 in the above formula is an integer from 2 to 20), Q4s may be the same or different.

Q4' above is a substituted or unsubstituted C₁₋₁₄ alkylene, preferably a C₄₋₁₄ alkylene, more preferably a C₆₋₁₂ alkylene, and still more preferably a C₈₋₁₀ alkylene.

P4 above is either absent, or -CO-, -NH-, -O-, -S-, -O-CO-, -NH-CO-, -CO-O- or -CO-NH-, preferably -CO-, -O-, -O-CO-, -NH-CO-, -CO-O- or -CO-NH-, and more preferably -CO-, -O-, -NH-CO-or -CO-NH-. When there are 2 or more P4s (when q4 in the above formula is an integer from 2 to 20), P4s may be the same or different. When there is one P4 (when q4 in the formula is 1), P4 is preferably -CO-.

q4 above is an integer from 0 to 20, preferably 0 to 4, more preferably 1 to 3, and still more preferably 1 to 2. When q4 in the above formula is an integer from 2 to 20, Q4s and P4s may be the same or different.

R in formulae (I) to (V) above is a structure represented by formula (VI) above.

q1, q2 and q3 in the formula (VI) are, independently of each other, an integer from 0 to 20, preferably q1, q2 and q3 are 2 to 4 and may be the same or different, more preferably are 2 to 4 and are all the same, and still more preferably are all 3.

Q1, Q2 and Q3 in the formula (VI) are, independently of each other, absent or a substituted or unsubstituted C₁₋₁₄ alkylene, preferably each is independently a C₁₋₅ alkylene, and more preferably a C₂₋₄ alkylene. When q1, q2 and q3 in the formula (VI) are integers from 2 to 20, Q1, Q2 and Q3 may be the same or different.

The alkylene part of the substituted or unsubstituted C₁₋₁₄ alkylenes of Q1, Q2, Q3 and Q4 in the formulae (I) to (VI) may be any C₁₋₁₄ alkylenes. Examples of such C₁₋₁₄ alkylenes include methylene, ethylene, propylene, butylene, pentylene, hexylene, heptylene, octylene, nonylene, decylene, undecylene, dodecylene, tridecylene and tetradecylene.

In the definitions of Q1, Q2, Q3 and Q4 above, examples of the substituents of the substituted or unsubstituted C₁₋₁₄ alkylenes include alkyl groups such as methyl, ethyl, propyl, isopropyl, butyl, isobutyl and tert-butyl groups, hydroxyl group, C₁₋₄ linear or branched alkoxy groups such as methoxy, ethoxy, propoxy and butoxy groups, cyano group, and halogen groups such as fluoro, chloro and bromo groups and the like.

L1, L2 and L3 in formula (VI) above are, independently of each other, a sugar ligand.

In the present invention, a sugar ligand is a group that derives from a saccharide (monosaccharide, disaccharide, trisaccharide, polysaccharide or the like) and that is capable of binding to a receptor expressed in a target cell. In the present invention, when a sugar ligand is bound by a glycoside bond for example, the sugar ligand as a group derived from a saccharide is considered to be a part excluding a hydroxyl group involved in binding of the saccharides constituting the sugar ligand. Moreover, when bound by a glycoside bond for example, the sugar ligand as a group derived from a saccharide is considered to be a part excluding a hydrogen atom of a hydroxyl group involved in binding of the saccharides constituting the sugar ligand.

In the present invention, the sugar ligands may be selected according to the target cell of the double-stranded nucleic acid.

Examples of monosaccharides include allose, altose, arabinose, cladinose, erythrose, erythrulose, fructose, D-fucitol, L-fucitol, fucosamine, fucose, fuculose, galactosamine, D-galactosaminitol, N-acetyl-galactosamine, galactose, glucosamine, N-acetyl-glucosamine, glucosaminitol, glucose, glucose-6-phosphate, gulose, glyceraldehyde, L-glycero-D-mannoheptose, glycerol, glycerone, gulose, idose, lyxose, mannosamine, mannose, mannose-6-phosphate, psicose, quinovose, quinovosamine, rhamnitol, rhamnosamine, rhamnose, ribose, ribulose, sedoheptulose, sorbose, tagatose, talose, tartaric acid, threose, xylose and xylulose.

Examples of disaccharides, trisaccharides and polysaccharides include abequose, acarbose, amicetose, amylopectin, amylose, apiose, arcanose, ascarylose, ascorbic acid, boivinose, cellobiose, cellotriose, cellulose, chacotriose, chalcose, chitin, colitose, cyclodextrin, cymarose, dextrin, 2-deoxyribose, 2-deoxyglucose, diginose, digitalose, digitoxose, evalose, evemitrose, fructo-oligosaccharide, galto-oligosaccharide, gentianose, gentiobiose, glucan, glucogen, glycogen, hamamelose, heparin, inulin, isolevoglucosenone, isomaltose, isomaltotriose, isopanose, kojibiose, lactose, lactosamine, lactosediamine, laminarabiose, levoglucosan, levoglucosenone, β-maltose, maltriose, mannan-oligosaccharide, manninotriose, melezitose, melibiose, muramic acid, mycarose, mycinose, neuraminic acid, nigerose, nojirimycin, noviose, oleandrose, panose, paratose, planteose, primeverose, raffinose, rhodinose, rutinose, sarmentose, sedoheptulose, sedoheptulosan, solatriose, sophorose, stachyose, streptose, sucrose, α,α-trehalose, trehalosamine, turanose, tyvelose, xylobiose and umbelliferose.

Each monosaccharide in the saccharides may be in D-form or L-form, and may also be a mixture of D- and L-forms in any proportions.

The saccharide may also include a deoxysaccharide (one in which an alcoholic hydroxyl group has been replaced by a hydrogen atom), an aminosaccharide (one in which an alcoholic hydroxyl group has been replaced by an amino group), a thiosaccharide (one in which an alcoholic hydroxyl group has been replaced by a thiol group, or C=O has been replaced by C=S, or a ring oxygen has been replaced by sulfur), a selenosaccharide, a tellurosaccharide, an azasaccharide (one in which a ring carbon has been replaced by nitrogen), an iminosaccharide (one in which a ring oxygen has been replaced by nitrogen), a phosphanosaccharide (one in which a ring oxygen has been replaced by phosphorus), a phosphasaccharide (one in which a ring carbon has been replaced by phosphorus), a C-substituted monosaccharide (one in which a hydrogen at a non-terminal carbon atom is replaced by a carbon atom), an unsaturated monosaccharide, an alditol (one in which a carbonyl group is replaced by a CHOH group), an aldonic acid (one in which an aldehyde group is replaced by a carboxyl group), a ketoaldonic acid, a uronic acid or an aldaric acid.

Regarding aminosaccharides, examples of amino monosaccharides in the saccharide include galactosamine, glucosamine, mannosamine, fucosamine, quinovosamine, neuraminic acid, muramic acid, lactose diamine, acosamine, bacillosamine, daunosamine, desosamine, forosamine, gallosamine, kanosamine, kansosamine, mycaminose, mycosamine, perosamine, pneumosamine, purpurosamine and rhodosamine. An amino group of the aminosaccharide may also be replaced with an acetyl group or the like.

Examples of sialic acid-containing sugar chains include sugar chains containing NeuAc at the non-reducing end of the sugar chain, such as sugar chains containing NeuAc-Gal-GlcNAc, and Neu5Acα(2-6)Galβ(1-3)GlcNAc.

Each monosaccharide in the saccharide may be substituted with a substituent as long as it can bind to a receptor expressed on a target cell. For example, a hydroxyl group may be substituted, and one or more hydrogen atoms in each monosaccharide may be substituted with azide and/or an optionally substituted aryl group.

Regarding the sugar ligand of the nucleic acid conjugate of the present invention, a sugar ligand that binds to a receptor expressed on the surface of a target cell is preferably selected to target each organ. When the targeted cell is a liver cell for example, a sugar ligand targeting a receptor expressed on the surface of liver cells is preferred, and a sugar ligand targeting asialoglycoprotein receptor (ASGPR) is more preferred.

Mannose or N-acetylgalactosamine is preferred as the sugar ligand targeting ASGPR, and N-acetylgalactosamine is more preferred.

The sugar derivatives described in Bioorganic Medicinal Chemistry, Vol. 7, pp. 7254-7264, 2009 and Journal of the American Chemical Society, Vol. 134, pp. 1978-1981, 2012 are known as sugar ligands with high affinity for ASGPR, and these may be used.

The nucleic acid conjugate of the present invention may also form a salt with a pharmaceutically acceptable anion when a hydrogen ion is coordinated to a lone electron pair on any nitrogen atom.

In the present invention, examples of pharmaceutically acceptable anions include inorganic ions such as chloride ion, bromide ion, nitrate ion, sulfate ion and phosphate ion, and organic acid ions such as acetate ion, oxalate ion, maleate ion, fumarate ion, citrate ion, benzoate ion and methanesulfonate ion.

The method for producing the nucleic acid conjugate of the invention is explained here. In the production method given below, when a defined group changes under the conditions of the production method or is unsuited to implementing the production method, the target compound can be produced by applying methods ordinarily used in organic synthetic chemistry to introduce and remove protective groups, such as the methods described in Protective Groups in Organic Synthesis, Third Edition, T.W. Greene Ed., John Wiley & Sons Inc. (1999). Moreover, the order of reaction steps such as substituent introduction may also be changed as necessary.

### Production method 1

Of the nucleic acid conjugates in which the 3' end of the sense strand of the double-stranded nucleic acid has a ligand part represented by formula (I), a sense strand in which the functional group at the right end of Q is -CO- (a sense strand in which the 3' end of the oligonucleotide is represented by formula (I')) can be produced by the following method. The phrase "the functional group at the right end of Q is -CO-" means that when Q in formula (I) is -T4-[Q4-P4]q4-, q4 is 0 and T4 is - CO-, or q4 is 1 or more and P4 in the -Q4-P- part of Q that binds directly to the nitrogen atom in formula (I) is -CO-. 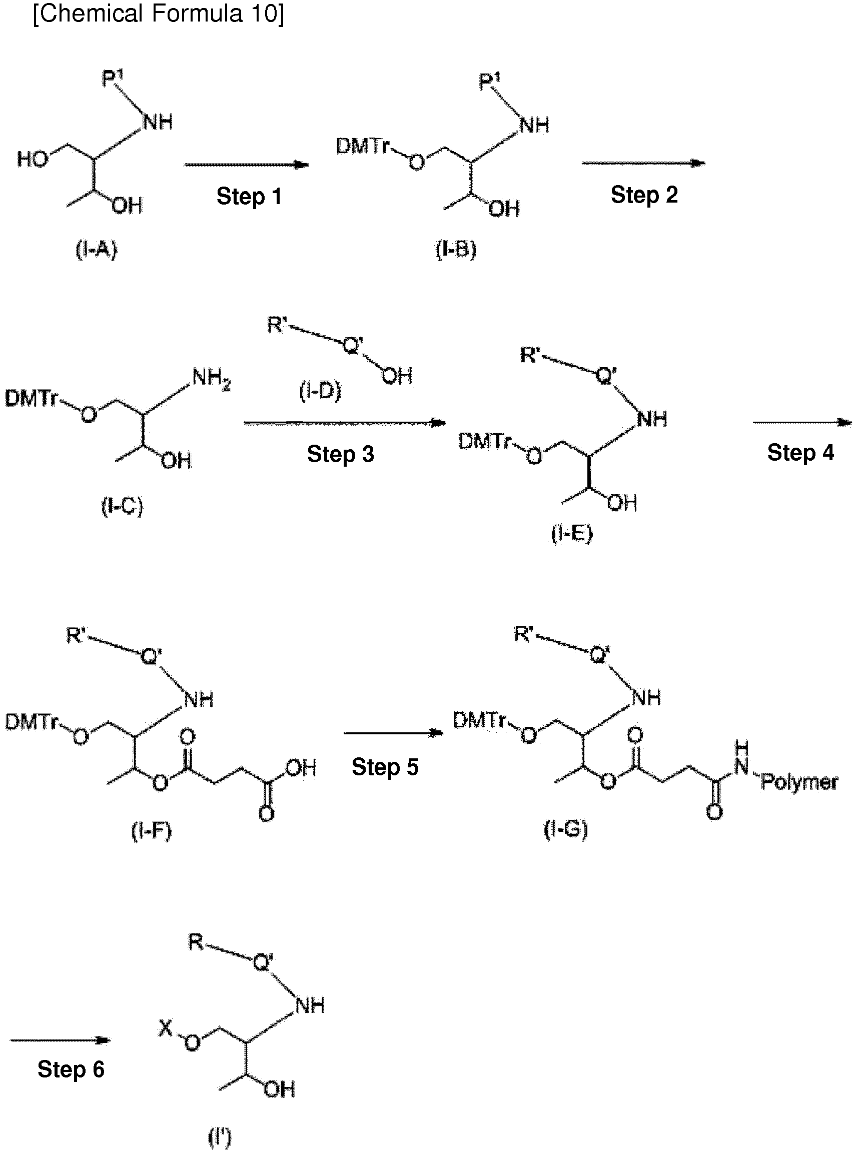 (wherein, P1 is a protective group that can be deprotected with a base such as Fmoc, DMTr is p,p'-dimethoxytrityl group, R' is R group in which each hydroxyl group is protected by a protective group such as acetyl group that can be deprotected with a base, Polymer is a solid-phase carrier, and Q' is Q in which the functional group at the right end is -CO-.)

### Step 1

Compound (I-B) can be produced by reacting compound (I-A) and p,p'-dimethoxytrityl chloride for 5 minutes to 100 hours at a temperature between 0°C and 100°C in a solvent such as pyridine in the presence of a co-solvent as necessary.

Examples of the co-solvents include methanol, ethanol, dichloromethane, chloroform, 1,2-dichloroethane, toluene, ethyl acetate, acetonitrile, diethyl ether, tetrahydrofuran, 1,2-dimethoxyethane, dioxane, N,N'-dimethylformamide (DMF), N,N-dimethylacetamide, N-methylpyrrolidone, pyridine and water, and these may be used alone or as a mixture.

### Step 2

Compound (I-C) can be produced by reacting compound (I-B) for 5 minutes to 100 hours at a temperature between room temperature and 200°C with or without a solvent in the presence of 1 to 1,000 equivalents of a secondary amine.

Examples of the solvent include methanol, ethanol, dichloromethane, chloroform, 1,2-dichloroethane, toluene, ethyl acetate, acetonitrile, diethyl ether, tetrahydrofuran, 1,2-dimethoxyethane, dioxane, N,N'-dimethylformamide (DMF), N,N-dimethylacetamide, N-methylpyrrolidone, pyridine and water, and these may be used alone or as a mixture.

Examples of the secondary amine include diethylamine and piperidine.

### Step 3

Compound (1-E) can be produced by reacting compounds (I-C) and (I-D) for 5 minutes to 100 hours at a temperature between room temperature and 200°C with or without a solvent in the presence of 1 to 30 equivalents of a base, a condensing agent, and 0.01 to 30 equivalents of an additive as necessary.

Examples of the solvent include those given as examples in step 2.

Examples of the base include cesium carbonate, potassium carbonate, potassium hydroxide, sodium hydroxide, sodium methoxide, potassium tert-butoxide, triethylamine, diisopropylethylamine, N-methylmorpholine, pyridine, 1,8-diazabicyclo[5.4.0]-7-undecene (DBU) and N,N-dimethyl-4-aminopyridine (DMAP).

Examples of the condensing agent include 1,3-dicyclohexane carbodiimide, 1-ethyl-3-(3-dimethylaminopropyl)carbodiimide-hydrochloride (EDC), carbonyldiimidazole, benzotriazol-1-yloxytris(dimethylamino)phosphonium hexafluorophosphate, (benzotriazol-1-yloxy)tripyrrolidinophosphonium hexafluorophosphate, O-(7-azabenzotriazol-1-yl)-N,N,N',N'-tetramethyluronium hexafluorophosphate (HATU), O-(benzotriazol-1-yl)-N,N,N',N'-tetramethyluronium hexafluorophosphate (HBTU) and 2-chloro-1-methylpyridinium iodide.

Examples of the additive include 1-hydroxybenzotriazole (HOBt) and 4-dimethylaminopyridine (DMAP).

Compound (I-D) can be obtained by known methods (see for example Journal of the American Chemical Society, Vol. 136, 16958-16961, 2014) or similar methods.

### Step 4

Compound (I-F) can be produced by reacting compound (I-E) with succinic anhydride for 5 minutes to 100 hours at a temperature between room temperature and 200°C in a solvent in the presence of 1 to 30 equivalents of a base.

Examples of the solvent include those listed in step 2.

Examples of the base include those listed in step 3.

### Step 5

Compound (I-G) can be produced by reacting compound (I-F) with a terminally aminated solid-phase carrier for 5 minutes to 100 hours at a temperature between room temperature and 200°C with or without a solvent in the presence of 1 to 30 equivalents of a base, a condensing agent, and 0.01 to 30 equivalents of an additive as necessary, and then further reacting with an acetic anhydride/pyridine solution for 5 minutes to 100 hours at a temperature between room temperature and 200°C.

Examples of the solvent include those listed in step 2.

Examples of the base, the condensing agent and the additive include those listed in step 3.

The aminated solid-phase carrier may be a long-chain alkylamine-controlled pore glass (LCAA-CPG) or the like, and may be obtained as a commercial product.

### Step 6

A nucleic acid conjugate in which the 3' end of the sense strand constituting the double-stranded nucleic acid has a ligand part represented by formula (I) can be produced by elongating a corresponding nucleotide strand by known oligonucleotide chemical synthesis methods, using compound (I-G), and then performing desorption from the solid phase, deprotection of the protecting group and purification.

Examples of known oligonucleotide chemical synthesis methods include phosphoramidite method, phosphorothioate method, phosphotriester method and CEM method (Nucleic Acids Research, Vol. 35, page 3287, 2007), and synthesis may be performed using an ABI3900 high throughput nucleic acid synthesizer (Applied Biosystems, Inc.).

Desorption from the solid phase and deprotection can be performed following the oligonucleotide chemical synthesis by treating the product for 10 seconds to 72 hours with a base at a temperature of -80°C to 200°C with or without a solvent.

Examples of the base include ammonia, methylamine, dimethylamine, ethylamine, diethylamine, isopropylamine, diisopropylamine, piperidine, triethylamine, ethylenediamine, 1,8-diazabicyclo[5.4.0]-7-undecene (DBU) and potassium carbonate.

Examples of the solvent include water, methanol, ethanol and THF.

The oligonucleotide may be purified by a C18 reverse-phase column or anion exchange column, or preferably by a combination of these two methods. The purity of the nucleic acid conjugate after purification is preferably 90% or more, and more preferably 95% or more.

In step 3 above, as necessary, compound (I-D) may be divided into two units and condensed with the compound (I-C) in two stages. Specifically, when Q' is -NH-CO-Q4'-CO- for example (Q4' is a substituted or unsubstituted C₁₋₁₄ alkylene), in step 3, the compound (I-C) can be condensed with CH₃CH₂-O-CO-Q4'-CO-OH (Q4' is defined as above) by the same methods as in step 3, and the resulting ethyl ester compound can be hydrolyzed with a base such as lithium hydroxide in a solvent such as ethanol or water, and then condensed with R'-NH₂ (R' is defined as above) to obtain the target compound. CH₃CH₂-O-CO-Q4'-CO-OH (Q4' is defined as above) and R'-NH₂ (R' is defined as above) can be obtained by known methods (see for example Journal of the American Chemical Society, Vol. 136, pp. 16958-16961, 2014) or similar methods. In Q4', the substituent and alkylene part of the substituted or unsubstituted C₁₋₁₄ alkylene are the same as above.

Of the nucleic acid conjugates in which the 3' end of the sense strand of the double-stranded nucleic acid has a ligand part represented by formula (I), a sense strand in which the functional group at the right end of Q is not -CO- can be prepared by methods similar to those described above, known methods or a combination thereof under reaction conditions optimized according to the structure of Q. The phrase "sense strand in which the functional group at the right end of Q is not -CO-" refers to sense strands of the nucleic acid conjugates having a ligand part represented by formula (I) other than the sense strands in which the functional group at the right end of Q is -CO-.

### Production method 2

Of the nucleic acid conjugates in which the 3' end of the sense strand of the double-stranded nucleic acid has a ligand part represented by formula (II), a sense strand in which the functional group at the right end of Q is -CO- (a sense strand in which the 3' end of the oligonucleotide is represented by formula (II')) can be produced by the following method. 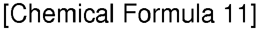 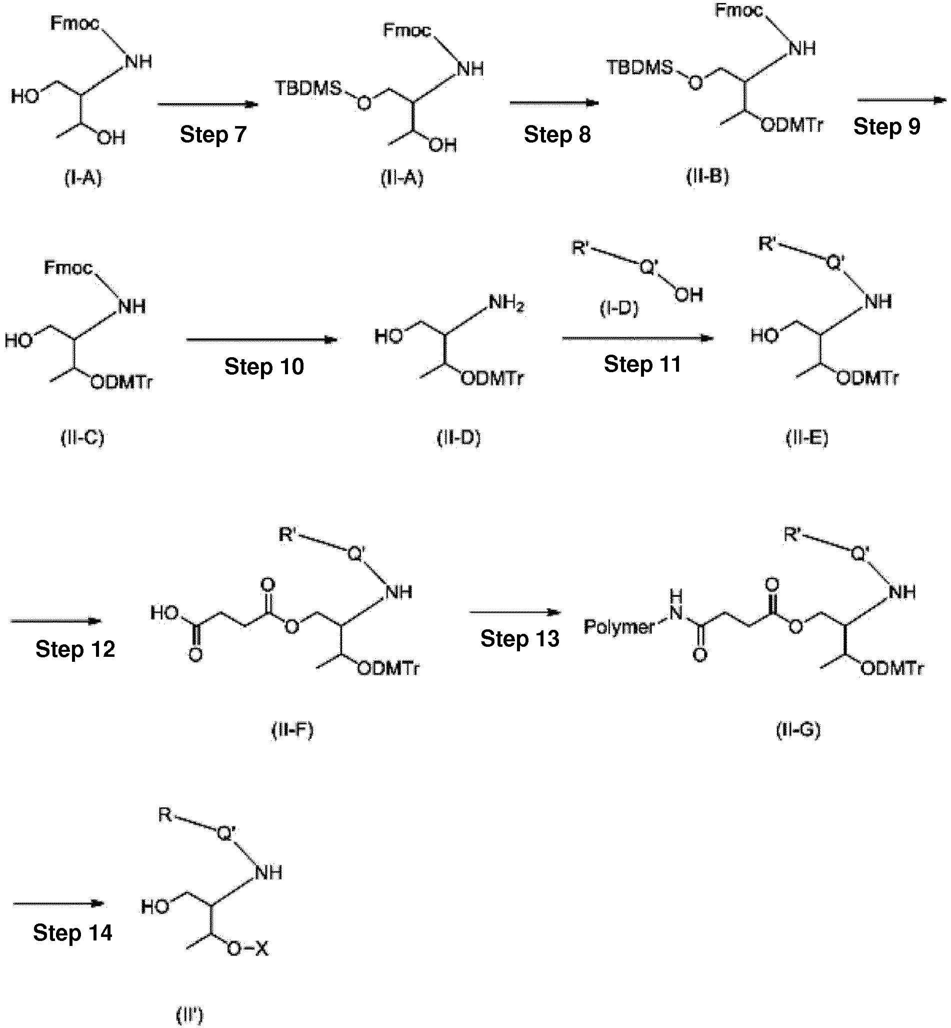 (wherein, DMTr, R, R', X, Q' and Polymer are defined as above. TBDMS represents t-butyldimethylsilyl group, and Fmoc represents 9-fluorenylmethyloxycarbonyl group.)

### Step 7

Compound (II-A) can be produced by reacting compound (I-A), t-butyldimethylsilyl chloride and dimethylaminopyridine in a solvent such as N,N-dimethylformamide (DMF) for 5 minutes to 100 hours at a temperature between 0°C and 100°C, preferably in the presence of 2 equivalents of a base.

Examples of the base include those listed in step 3 of Production method 1.

### Step 8

Compound (II-B) can be produced using compound (II-A) under conditions similar to those of step 1 of Production method 1.

### Step 9

Compound (II-C) can be produced by reacting compound (II-B) and n-tetrabutylammonium fluoride (TBAF) for 5 minutes to 100 hours at a temperature between room temperature and 200°C in a solvent.

Examples of the solvent include those listed in step 2.

### Step 10

Compound (II-D) can be produced using compound (II-C) under conditions similar to those of step 2 of Production method 1.

### Step 11

Compound (II-E) can be produced using compound (II-D) and compound (I-D) under conditions similar to those of step 3 of Production method 1.

### Steps 12 to 14

Compound (II') can be produced using compound (II-E) under conditions similar to those of steps 4 to 6 of Production method 1.

In step 11 above, as necessary, compound (I-D) may be divided into two units and condensed with compound (II-C) in two stages. Specifically, when Q' is -NH-CO-Q4'-CO- for example (Q4' is a substituted or unsubstituted C₁₋₁₄ alkylene), in step 11, compound (II-C) can be condensed with CH₃CH₂-O-CO-Q4'-CO-OH (Q4' is defined as above) by the same methods as in step 11, and the resulting ethyl ester compound can be hydrolyzed with a base such as lithium hydroxide in a solvent such as ethanol or water, and then condensed with R'-NH₂ (R' is defined as above) to obtain the target compound. CH₃CH₂-O-CO-Q4'-CO-OH (Q4' is defined as above) and R'-NH₂ (R' is defined as above) can be obtained by known methods (see for example Journal of the American Chemical Society, Vol. 136, pp. 16958-16961, 2014) or similar methods. In Q4', the substituent and alkylene part in the substituted or unsubstituted C₁₋₁₄ alkylene are the same as above.

Of the nucleic acid conjugates in which the 3' end of the sense strand of the double-stranded nucleic acid has a ligand part represented by formula (II), a sense strand in which the functional group at the right end of Q is not -CO- can be prepared by methods similar to those described above, known methods or a combination thereof under reaction conditions optimized according to the structure of Q.

### Production method 3

Of the nucleic acid conjugates in which the 3' end of the sense strand of the double-stranded nucleic acid has a ligand part represented by formula (III), a sense strand in which the functional group at the right end of Q is -CO- (a sense strand in which the 3' end of the oligonucleotide is represented by formula (III')) can be produced by the following method. 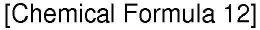 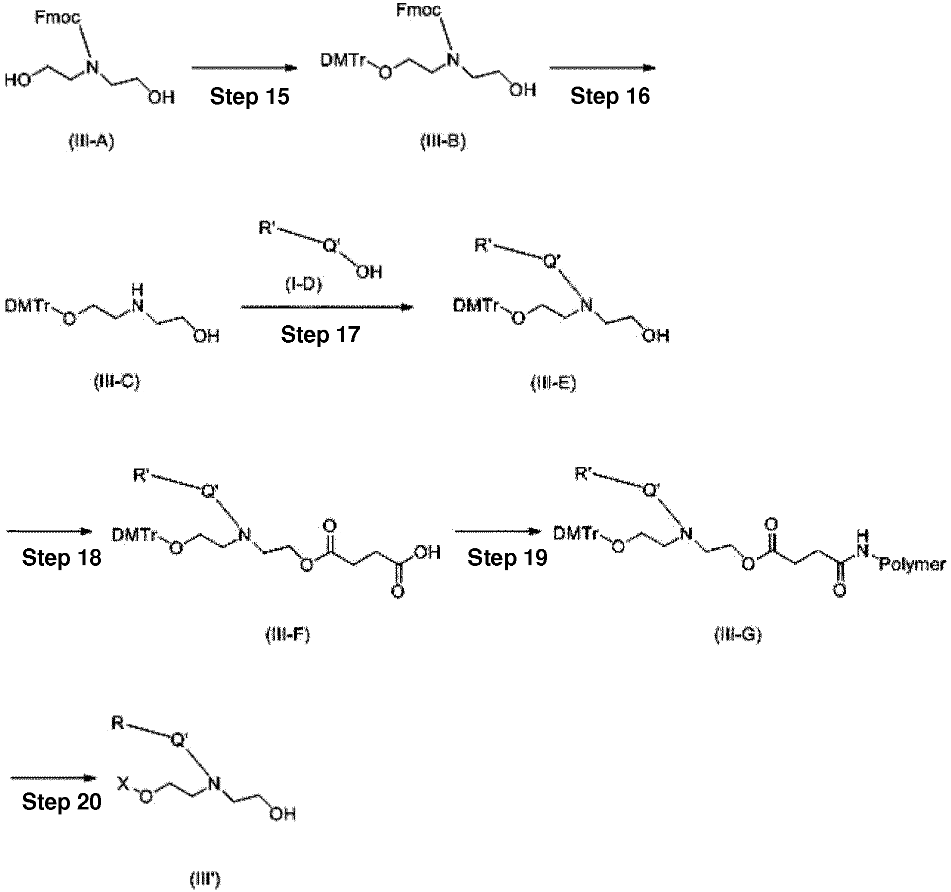 (wherein, DMTr, Fmoc, R, R', Q', X and Polymer are defined as above.)

Compound (III') can be produced using compound (III-A) under conditions similar to those of steps 1 to 6 of Production method 1. Compound (III-A) can be obtained as a commercial product.

### Step 15

Compound (III-B) can be produced using compound (III-A) under conditions similar to those of step 1 of Production method 1.

Compound (III-A) can be purchased as a commercial product.

### Step 16

Compound (III-C) can be produced using compound (III-B) under conditions similar to those of step 2 of Production method 1.

### Step 17

Compound (III-E) can be produced using compound (III-C) under conditions similar to those of step 3 of Production method 1.

### Steps 18 to 20

Compound (III') can be produced using compound (III-E) under conditions similar to those of steps 4 to 6 of Production method 1.

In step 17 above, as necessary, compound (I-D) may be divided into two units and condensed with compound (III-C) in two stages. Specifically, when Q' is -NH-CO-Q4'-CO- for example (Q4' is a substituted or unsubstituted C₁₋₁₄ alkylene), in step 17, compound (III-C) can be condensed with CH₃CH₂-O-CO-Q4'-CO-OH (Q4' is defined as above) by the same methods as in step 17, and the resulting ethyl ester compound can be hydrolyzed with a base such as lithium hydroxide in a solvent such as ethanol or water, and then condensed with R'-NH₂ (R' is defined as above) to obtain the target compound. CH₃CH₂-O-CO-Q4'-CO-OH (Q4' is defined as above) and R'-NH₂ (R' is defined as above) can be obtained by known methods (see for example Journal of the American Chemical Society, Vol. 136, pp. 16958-16961, 2014) or similar methods. In Q4', the substituent and alkylene part of the substituted or unsubstituted C₁₋₁₄ alkylene are the same as above.

Of the nucleic acid conjugates in which the 3' end of the sense strand of the double-stranded nucleic acid has a ligand part represented by formula (III), a sense strand in which the functional group at the right end of Q is not -CO- can be prepared by methods similar to those described above, known methods or a combination thereof under reaction conditions optimized according to the structure of Q.

### Production method 4

Of the nucleic acid conjugates in which the 3' end of the sense strand of the double-stranded nucleic acid has a ligand part represented by formula (IV), a sense strand in which the functional group at the right end of Q is -CO- (a sense strand in which the 3' end of the oligonucleotide is represented by formula (IV')) can be produced by the following method. 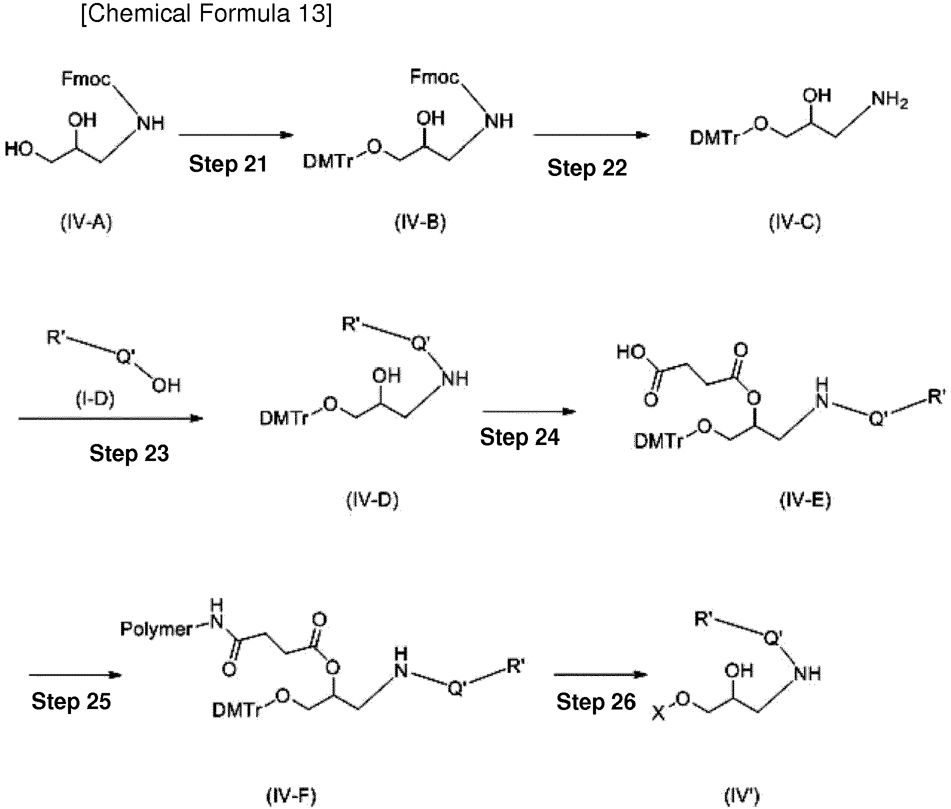 (wherein, DMTr, Fmoc, R', Q', X and Polymer are defined as above.)

Compound (IV') can be produced using compound (IV-A) under conditions similar to those of steps 1 to 6 of Production method 1. Compound (IV-A) may be obtained as a commercial product.

In step 23 above, as necessary, compound (I-D) may be divided into two units and condensed with compound (IV-C) in two stages. Specifically, when Q' is -NH-CO-Q4'-CO- for example (Q4' is a substituted or unsubstituted C₁₋₁₄ alkylene), in step 23, compound (IV-C) can be condensed with CH₃CH₂-O-CO-Q4'-CO-OH (Q4' is defined as above) by the same methods as in step 23, and the resulting ethyl ester compound can be hydrolyzed with a base such as lithium hydroxide in a solvent such as ethanol or water, and then condensed with R'-NH₂ (R' is defined as above) to obtain the target compound. CH₃CH₂-O-CO-Q4'-CO-OH (Q4' is defined as above) and R'-NH₂ (R' is defined as above) can be obtained by known methods (see for example Journal of the American Chemical Society, Vol. 136, pp. 16958-16961, 2014) or similar methods. In Q4', the substituent and alkylene part of the substituted or unsubstituted C₁₋₁₄ alkylene are the same as above.

Of the nucleic acid conjugates in which the 3' end of the sense strand of the double-stranded nucleic acid has a ligand part represented by formula (IV), a sense strand in which the functional group at the right end of Q is not -CO- can be prepared by methods similar to those described above, known methods or a combination thereof under reaction conditions optimized according to the structure of Q.

### Production method 5

Of the sense strands in which the 3' end of the oligonucleotide is represented by formula (V), a sense strand in which the functional group at the right end of Q is - CO- (a sense strand in which the 3' end of the oligonucleotide is represented by formula (V')) can be produced by the following Production method 5. 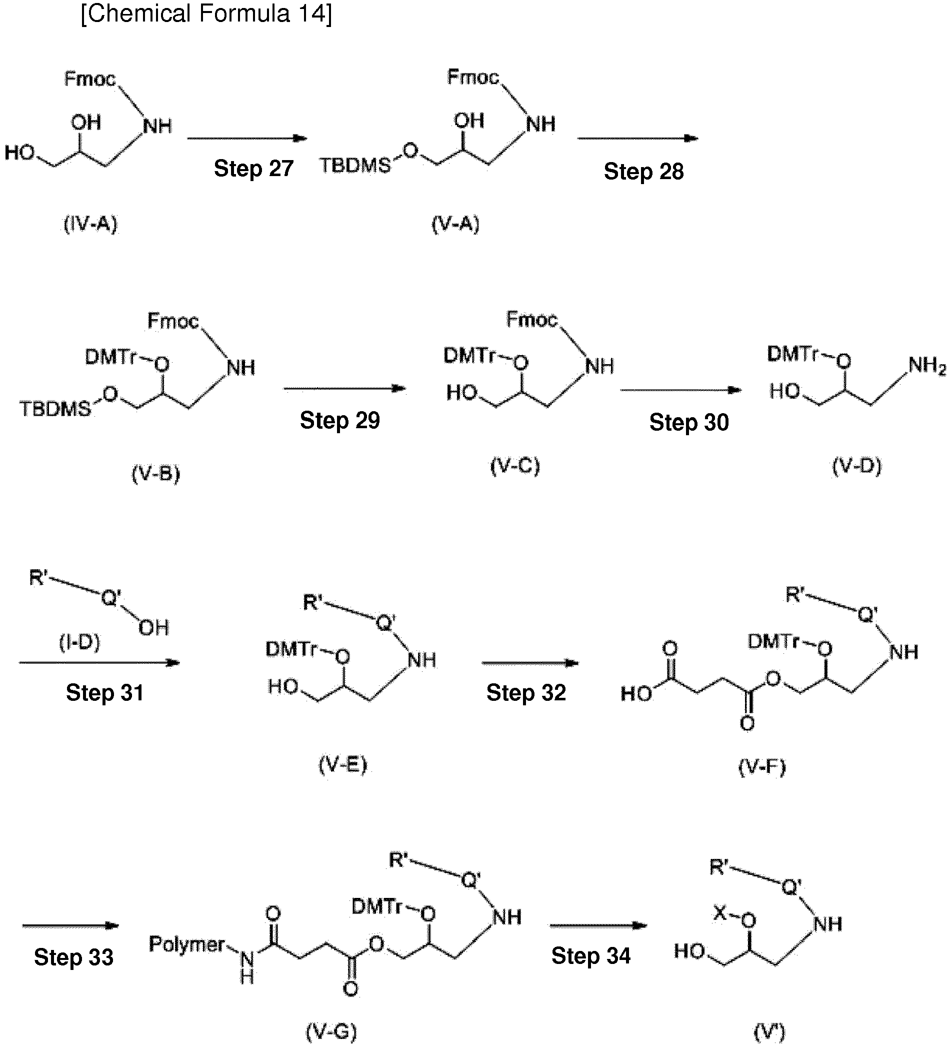 (wherein, DMTr, R', X, Q', TBDMS, Fmoc and Polymer are defined as above.)

Compound (V') can be produced using compound (IV-A) under conditions similar to those of steps 1 to 7 in Production method 2. Compound (IV-A) can be obtained as a commercial product.

In step 31 above, as necessary, compound (I-D) may be divided into two units and condensed with compound (V-D) in two stages. Specifically, when Q' is -NH-CO-Q4'-CO- for example (Q4' is a substituted or unsubstituted C₁₋₁₄ alkylene), in step 31, compound (V-D) can be condensed with CH₃CH₂-O-CO-Q4'-CO-OH (Q4' is defined as above) by the same methods as in step 31, and the resulting ethyl ester compound can be hydrolyzed with a base such as lithium hydroxide in a solvent such as ethanol or water, and then condensed with R'-NH₂ (R' is defined as above) to obtain the target compound. CH₃CH₂-O-CO-Q4'-CO-OH (Q4' is defined as above) and R'-NH₂ (R' is defined as above) can be obtained by known methods (see for example Journal of the American Chemical Society, Vol. 136, pp. 16958-16961, 2014) or similar methods. In Q4', the substituent and alkylene part of the substituted or unsubstituted C₁₋₁₄ alkylene are the same as above.

Of the nucleic acid conjugates in which the 3' end of the sense strand of the double-stranded nucleic acid has a ligand part represented by formula (V), a sense strand in which the functional group at the right end of Q is not -CO- can be prepared by methods similar to those described above, known methods or a combination thereof under reaction conditions optimized according to the structure of Q.

### Production method 6

Of the sense strands in which the 5' end of the oligonucleotide is represented by formula (I), a sense strand in which the functional group at the right end of Q is - CO- (a sense strand in which the 5' end of the oligonucleotide is represented by formula (I")) can be produced by following Production method 6. 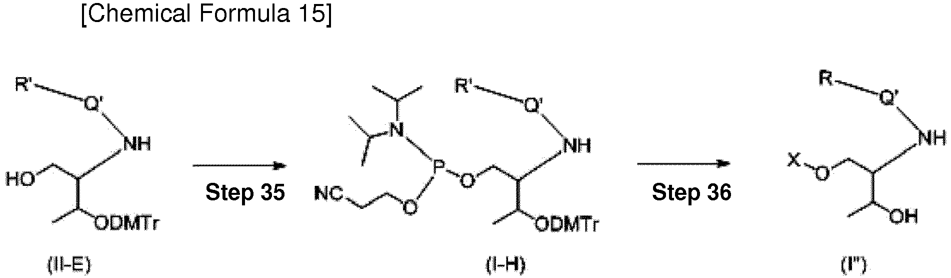 (wherein, R, R', Q', DMTr and X are defined as above.)

### Step 35

Compound (I-H) can be produced by reacting compound (II-E) with 2-cyanoethyl-N,N,N',N'-tetraisopropylphosphodiamidite for 5 minutes to 100 hours at a temperature between room temperature and 200°C with or without a solvent in the presence of a base and a reaction accelerator.

Examples of the solvent include those listed in step 2 of Production method 1.

Examples of the base include those listed in step 3 of Production method 1.

The reaction accelerator may be 1H-tetrazole, 4,5-dicyanoimidazole, 5-ethylthiotetrazole or 5-benzylthiotetrazole for example, and may be purchased as a commercial product.

### Step 36

A sense strand in which the 5' end of the oligonucleotide is represented by formula (I) can be produced by elongating a oligonucleotide strand, using compound (I-H) and then finally performing desorption from the solid phase, deprotection of the protecting group and purification. Desorption from the solid phase, deprotection of the protecting group and purification may each be performed as in step 6 of Production method 1.

Furthermore, as in Production method 1, of the nucleic acid conjugates in which the 5' end of the sense strand of the double-stranded nucleic acid has a ligand part represented by formula (I), a sense strand in which the functional group at the right end of Q is not -CO- can be prepared by methods similar to those described above, known methods or a combination thereof under reaction conditions optimized according to the structure of Q.

### Production method 7

Of the sense strands in which the 5' end of the oligonucleotide is represented by formula (II), a sense strand in which the functional group at the right end of Q is - CO- (a sense strand in which the 5' end of the oligonucleotide is represented by formula (II")) can be produced under conditions similar to those of steps 35 and 36 of Production method 6. 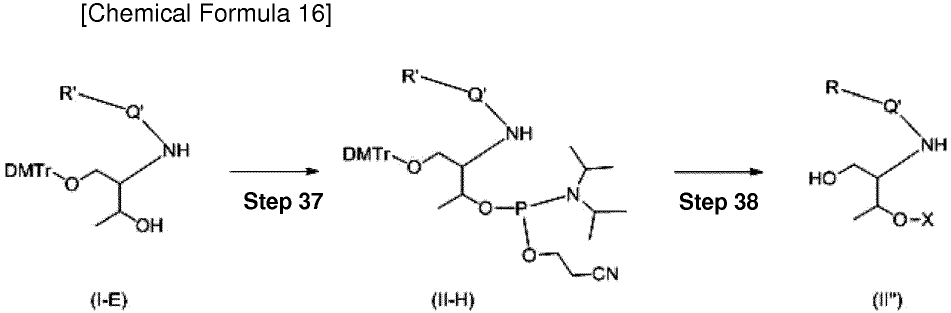 (wherein, R, R', Q', DMTr and X are as defined above.)

Moreover, as in Production method 2, of the nucleic acid conjugates in which the 5' end of the sense strand of the double-stranded nucleic acid has a ligand part represented by formula (II), a sense strand in which the functional group at the right end of Q is not -CO- can be prepared by methods similar to those described above, known methods or a combination thereof under reaction conditions optimized according to the structure of Q.

### Production method 8

Of the sense strands in which the 5' end of the oligonucleotide is represented by formula (III), a sense strand in which the functional group at the right end of Q is - CO- (a sense strand in which the 5' end of the oligonucleotide is represented by formula (III')) can be produced under conditions similar to those of steps 35 and 36 of Production method 6. 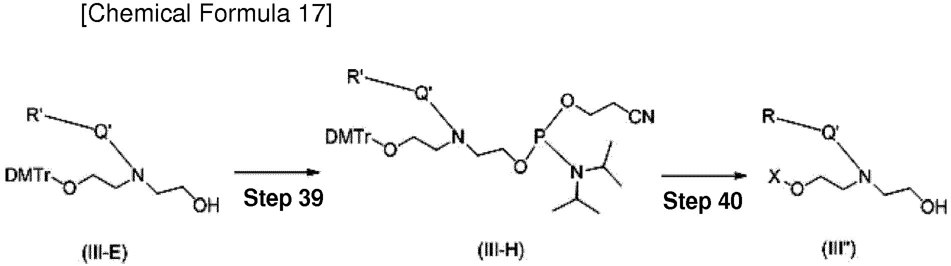 (wherein, R, R', Q', DMTr and X are as defined above.)

As in Production method 3, of the nucleic acid conjugates in which the 5' end of the sense strand of the double-stranded nucleic acid has a ligand part represented by formula (III), a sense strand in which the functional group at the right end of Q is not -CO- can be prepared by methods similar to those described above, known methods or a combination thereof under the reaction conditions optimized according to the structure of Q.

### Production method 9

Of the sense strands in which the 5' end of the oligonucleotide is represented by formula (IV), a sense strand in which the functional group at the right end of Q is - CO- (a sense strand in which the 5' end of the oligonucleotide is represented by formula (IV')) can be produced under conditions similar to those of steps 35 and 36 of Production method 6. 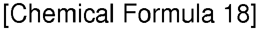 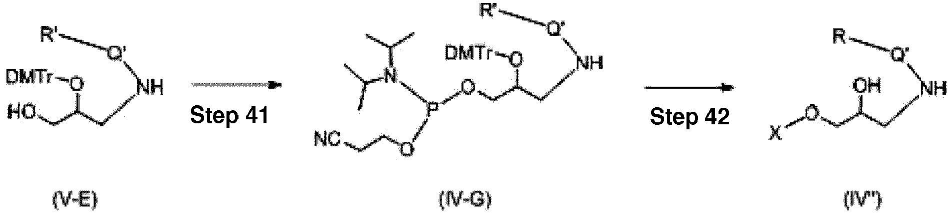 (wherein, R, R', Q', DMTr and X are as defined above.)

As in Production method 4, of the nucleic acid conjugates in which the 5' end of the sense strand of the double-stranded nucleic acid has a ligand part represented by formula (IV), a sense strand in which the functional group at the right end of Q is not -CO- can be prepared by methods similar to those described above, known methods or a combination thereof under reaction conditions optimized according to the structure of Q.

### Production method 10

Of the sense strands in which the 5' end of the oligonucleotide is represented by formula (V), a sense strand in which the functional group at the right end of Q is - CO- (a sense strand in which the 5' end of the oligonucleotide is represented by formula (V')) can be produced under conditions similar to those of steps 35 and 36 of Production method 6. 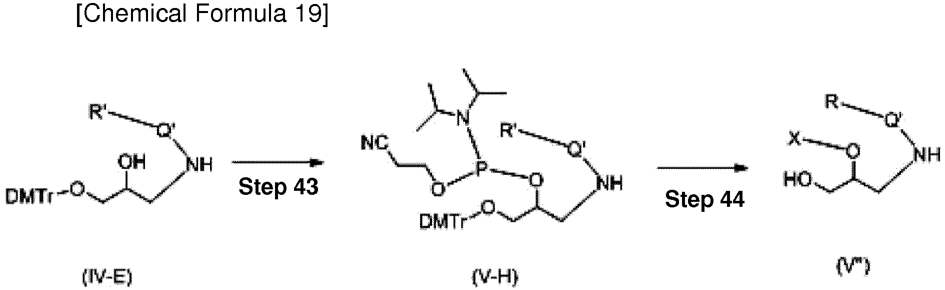 (wherein, R, R', Q', DMTr and X are as defined above.)

As in Production method 5, of the nucleic acid conjugates in which the 5' end of the sense strand of the double-stranded nucleic acid has a ligand part represented by formula (V), a sense strand in which the functional group at the right end of Q is not -CO- can be prepared by methods similar to those described above, known methods or a combination thereof under reaction conditions optimized according to the structure of Q.

### Production method 11

The sense strand of a double-stranded nucleic acid in which the 3' end or 5' end of the sense strand has a ligand part represented by formula (I) to (V) and the antisense strand of the double-stranded nucleic acid can be dissolved in water or a suitable buffer, and mixed to obtain a nucleic acid conjugate in which the 3' or 5' end of the sense strand of the double-stranded nucleic acid is represented by formula (I).

Examples of the buffer include acetic acid buffer, tris buffer, citric acid buffer, phosphoric acid buffer and water, and these may be used alone or in a mixture.

The mixing ratio of the sense strand and the antisense strand is preferably 0.5 to 2 equivalents, more preferably 0.9 to 1.1 equivalents, or still more preferably 0.95 to 1.05 equivalents of the antisense strand per 1 equivalent of the sense strand.

After the sense strand and the antisense strand are mixed, suitable annealing may be performed. Annealing is performed by first heating the mixture of the sense strand and the antisense strand to a temperature of preferably 50°C to 100°C, more preferably 60°C to 100°C, or still more preferably 80°C to 100°C, and then gradually cooling to room temperature.

The antisense strand can be obtained by the known oligonucleotide synthesis methods described above.

The nucleic acid conjugate described herein can be obtained in the form of a salt such as an acid addition salt, metal salt, ammonium salt, organic amine addition salt, amino acid addition salt or the like .

The acid addition salt may be an inorganic acid salt such as hydrochloride salt, sulfate salt or phosphate salt, or an organic acid salt such as acetate salt, maleate salt, fumarate salt, citrate salt or methanesulfonate salt. The metal salt may be an alkali metal salt such as sodium salt or potassium salt, an alkali earth metal salt such as magnesium salt or calcium salt, or an aluminum salt, zinc salt or the like. The ammonium salt may be a salt of ammonium, tetramethylammonium or the like. The organic amine addition salt may be an addition salt of morpholine, piperidine or the like. The amino acid addition salt may be an addition salt of lysine, glycine, phenylalanine or the like.

If a salt of the nucleic acid conjugate described herein is desired, when the conjugate is obtained in the form of a desired salt, it may be purified as it is, while when it is obtained in a free form, the conjugate may be dissolved or suspended in a suitable solvent, to which a corresponding acid or base is added, and the conjugate is then isolated and purified. In order to substitute the counter-ion forming the conjugate salt with another counter-ion, the conjugate salt may be dissolved or suspended in a suitable solvent, to which an acid, base and/or salt (for example, inorganic salts such as sodium chloride and ammonium chloride) is added in an amount of several equivalents to a large excess, and the conjugate is then isolated and purified.

Stereoisomers such as geometric isomers and optical isomers, tautomers and the like may be present in the nucleic acid conjugate described herein. All possible isomers and mixtures thereof are encompassed in the present invention.

The nucleic acid conjugate described herein may also exist in the form of an adduct with water or various solvents. These adducts are also encompassed in the present invention.

Moreover, the nucleic acid conjugate of the present invention also encompasses those in which a part or all of the atoms in the molecule have been replaced with atoms with a different mass number (isotopes) (such as deuterium).

Specific examples of the nucleic acid conjugate of the present invention are described in Table 1. However, the nucleic acid conjugate of the present invention is not limited to these.

### [Table 1]

**Table 1**

| No. | Structural formula |
|---|---|
| I | 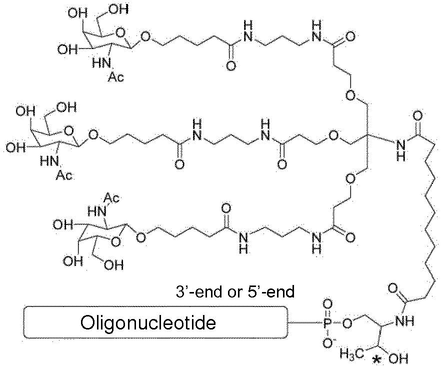 |
| II | 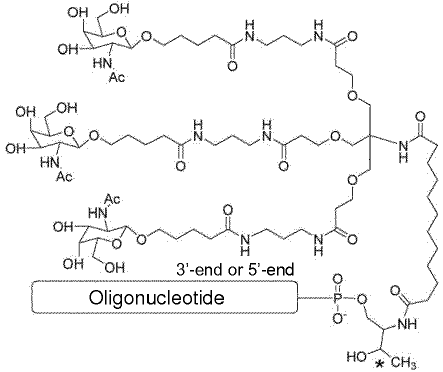 |
| III | 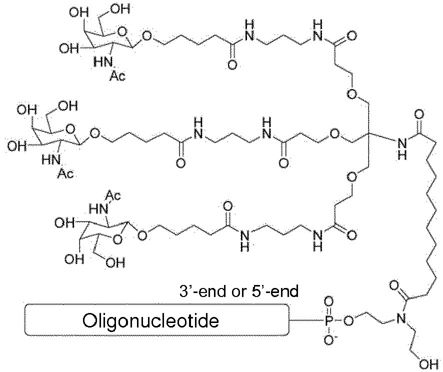 |

### [Table 2]

**Table 1 (continued)**

| No. | Structural formula |
|---|---|
| IV | 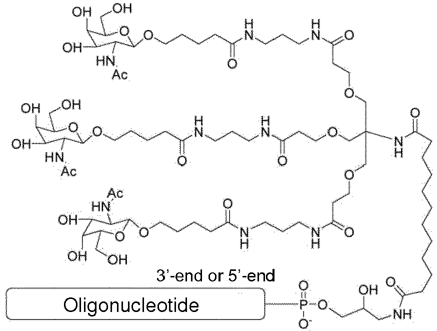 |
| V | 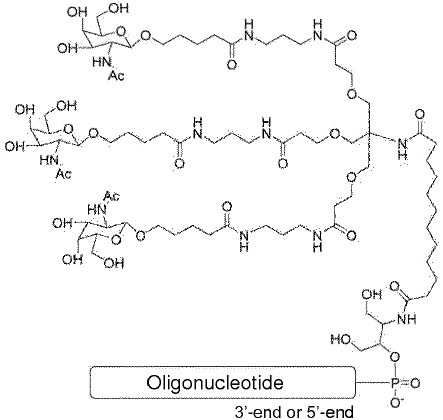 |

In Table 1, the oligonucleotide part in nucleic acid conjugates I to V represents the sense strand of the double-stranded nucleic acid, examples of which include the sense strands represented by the sense strand sequence in Tables 2-1 to 2-18, Tables 3-1 to 3-18 or Tables 4-1 to 4-16 below.

In the present invention, a nucleic acid containing a base sequence complementary to β2GPI mRNA is called an antisense strand nucleic acid, and a nucleic acid containing a base sequence complementary to the base sequence of the antisense strand nucleic acid is also a sense strand nucleic acid.

The double-stranded nucleic acid constituting the nucleic acid conjugate in the present invention is a double-stranded nucleic acid having the ability to reduce or stop expression of the β2GPI gene when introduced into mammalian cells, and is a double-stranded nucleic acid having a sense strand and an antisense strand. The sense strand and the antisense strand comprise at least 11 base pairs, and at least 17 and not more than 30 nucleotides in the antisense strand (in other words, an oligonucleotide strand with a strand length of 17 to 30 nucleotides) are complementary to a target β2GPI mRNA sequence selected from the group described in Tables 4-1 to 4-16.

The double-stranded nucleic acid constituting the nucleic acid conjugate of the present invention may be any molecule that is a polymer of nucleotides or molecules having a function equivalent to that of the nucleotide. Examples thereof include RNA (a polymer of ribonucleotides), DNA (a polymer of deoxyribonucleotides), chimera nucleic acids consisting of RNA and DNA, and nucleotide polymers in which at least one nucleotide in these nucleic acids has been replaced with a molecule having a function equivalent to that of the nucleotide. Derivatives containing at least one molecule having a function equivalent to that of the nucleotide in these nucleic acids are also encompassed in the double-stranded nucleic acid of the present invention, which is used as a medicine. Moreover, uracil (U) may be unambiguously replaced with thymine (T).

Examples of the molecules having a function equivalent to a nucleotide include nucleotide derivatives. The nucleotide derivative may be any molecule obtained by modifying a nucleotide, and is preferably a molecule obtained by modifying a ribonucleotide or deoxyribonucleotide in order to increase or stabilize nuclease resistance, increase affinity for a complement strand nucleic acid, increase cell permeability or achieve visibility, in comparison with RNA or DNA.

Examples of the molecules obtained by modifying nucleotides include sugar-modified nucleotides, phosphate diester bond-modified nucleotides, base-modified nucleotides, and nucleotides in which at least one of the sugar part, phosphate diester bond and base has been modified.

The sugar-modified nucleotide may be a nucleotide in which all or part of the chemical structure of the sugar in the nucleotide has been modified or replaced with any substituent, or replaced with any atom, and is preferably a 2'-modified nucleotide.

The 2'-modified nucleotide may be a nucleotide in which the 2'-OH group of ribose for example has been replaced with a substituent selected from the group consisting of H, OR, R, R'OR, SH, SR, NH₂, NHR, NR₂, N₃, CN, F, Cl, Br and I (where, R is alkyl or aryl, preferably C₁₋₆ alkyl, and R' is alkylene, preferably C₁₋₆ alkylene), preferably a nucleotide in which the 2'-OH group has been replaced with H, F or methoxy group, more preferably a nucleotide in which the 2'-OH group has been replaced with F or methoxy group. It may also be a nucleotide in which the 2'-OH group has been replaced with a substituent selected from the group consisting of 2-(methoxy)ethoxy, 3-aminopropoxy, 2-[(N,N-dimethylamino)oxy]ethoxy, 3-(N,N-dimethylamino)propoxy, 2-[2-(N,N-dimethylamino)ethoxy]ethoxy, 2-(methylamino)-2-oxoethoxy, 2-(N-methylcarbamoyl)etoxy and 2-cyanoetoxy.

The 2'-modified nucleotides constitute preferably 50% to 100%, more preferably 70% to 100%, or still more preferably 90% to 100% of the nucleotides in the double-stranded nucleic acid region. Moreover, the 2'-modified nucleotides constitute preferably 20% to 40%, more preferably 40% to 60%, or still more preferably 60% to 100% of the nucleotides of the sense strand. Furthermore, the 2'-O-methyl-modified nucleotides constitute preferably 0% to 40%, more preferably 10% to 20%, or still more preferably 20% to 40% of the nucleotides of the antisense strand.

The phosphate diester bond-modified nucleotide may be a nucleotide in which all or part of the chemical structure of the phosphate diester bond of the nucleotide has been modified or replaced with any substituent, or replaced with any atom. Examples thereof include nucleotides in which the phosphate diester bond has been replaced with a phosphorothioate bond, nucleotides in which the phosphate diester bond has been replaced with a phosphorodithioate bond, nucleotides in which the phosphate diester bond has been replaced with an alkylphosphonate bond, and nucleotides in which the phosphate diester bond has been replaced with a phosphoramidate bond

The base-modified nucleotide may be a nucleotide in which all or part of the chemical structure of the base of the nucleotide has been modified or replaced with any substituent, or replaced with any atom. Examples thereof include those in which an oxygen atom in the base has been replaced with a sulfur atom, those in which a hydrogen atom has been replaced with a C₁₋₆ alkyl group, halogen or the like, those in which a methyl group has been replaced with hydrogen, hydroxymethyl or C₂₋₆ alkyl group or the like, and those in which an amino group has been replaced with a C₁₋₆ alkyl group, C₁₋₆ alkanoyl group, oxo group, hydroxy group or the like.

Examples of the nucleotide derivatives also include those obtained by adding another chemical substance such as a peptide, a protein, a sugar, a lipid, a phospholipid, phenazine, folate, phenanthrizine, anthraquinone, acridine, fluorescein, rhodamine, coumarin and a dye, either directly or via a linker, to a nucleotide or a nucleotide in which at least one of the sugar part, phosphate diester bond and base is modified. Specific examples include 5'-polyamine addition nucleotide derivatives, cholesterol addition nucleotide derivatives, steroid addition nucleotide derivatives, bile acid addition nucleotide derivatives, vitamin addition nucleotide derivatives, Cy5 addition nucleotide derivatives, Cy3 addition nucleotide derivatives, 6-FAM addition nucleotide derivatives and biotin addition nucleotide derivatives.

The nucleotide derivative may also form, together with another nucleotide or nucleotide derivative in the nucleic acid, a crosslinked structure such as an alkylene structure, peptide structure, nucleotide structure, ether structure, ester structure and a combination of at least one of these structures.

The term "complementation" described herein means a relationship in which a base pair can be formed between two bases, such as relationships between adenine and thymine or uracil and between guanine and cytosine, which form mild hydrogen bonds to make the whole duplex region double-helical.

The term "complementary" described herein means not only the case in which two nucleotide sequences are completely complementary to each other, but also the case in which two nucleotide sequences have 0% to 30%, 0% to 20% or 0% to 10% mismatched nucleotides. For example, this means that an antisense strand complementary to β2GPI mRNA may have one or more substituted nucleotides in a base sequence completely complementary to the partial base sequence of that mRNA. Specifically, the antisense strand may have 1 to 8, preferably 1 to 6, 1 to 4, 1 to 3, or especially 2 or 1 mismatched nucleotide in relation to the target sequence of the target gene. If the antisense strand is 21 nucleotides in length for example, it may have 6, 5, 4, 3, 2, or 1 mismatched nucleotide in relation to the target sequence of the target gene. The mismatches may be located at the 5' end or the 3' end of the either sequence.

The term "complementary" also encompasses the case in which one nucleotide sequence which is completely complementary to another nucleotide sequence has one or more added and/or deleted nucleotides. For example, β2GPI mRNA and the antisense strand nucleic acid of the present invention may have one or two bulge bases in the antisense strand and/or the target β2GPI mRNA region due to addition and/or deletion of a base in the antisense strand.

The double-stranded nucleic acid of the present invention used as a medicine may be composed of any nucleotides or derivatives thereof as long as it is a nucleic acid containing a base sequence complementary to a partial base sequence of β2GPI mRNA, and/or a nucleic acid containing a base sequence complementary to the base sequence of said nucleic acid. In the double-stranded nucleic acid of the present invention, the nucleic acid containing a base sequence complementary to the target β2GPI mRNA sequence and the nucleic acid containing a base sequence complementary to the nucleotide sequence of said nucleic acid may be of any length as long as they can form a duplex strand of at least 11 base pairs. The length of the sequence capable of forming the duplex strand is normally 11 to 35 bases, preferably 15 to 30 bases, more preferably 17 to 25 bases, still more preferably 17 to 23 bases, and yet more preferably 19 to 23 bases.

A nucleic acid containing a base sequence complementary to the target β2GPI mRNA sequence may be used as the antisense strand in the nucleic acid conjugate of the present invention. It is also possible to use a nucleic acid obtained by deleting, substituting or adding 1 to 3, preferably 1 to 2, and more preferably 1 base in said nucleic acid

Either a single-stranded nucleic acid that contains a base sequence complementary to the target β2GPI mRNA sequence and that suppresses β2GPI expression, or a double-stranded nucleic acid that has a nucleic acid containing a base sequence complementary to the target β2GPI mRNA sequence and a nucleic acid containing a base sequence complementary to the base sequence of said nucleic acid and that suppresses β2GPI expression, may be used favorably as the nucleic acid that suppresses β2GPI expression.

The double-stranded nucleic acid of the present invention is a nucleic acid having two nucleotide strands which are paired and having a duplex region of at least 11 base pairs. The duplex region is a region in which nucleotides or derivatives thereof constituting the double-stranded nucleic acid form base pairs, leading to a double strand. The duplex region consists of normally 11 to 27 base pairs, preferably 15 to 25 base pairs, more preferably 15 to 23 base pairs, and still more preferably 17 to 21 base pairs.

The single-stranded nucleic acid constituting the double-stranded nucleic consists of normally 11 to 30 bases, preferably 15 to 29 bases, more preferably 15 to 27 bases, still more preferably 15 to 25 bases, and yet more preferably 17 to 23 bases.

When the double-stranded nucleic acid of the present invention used as a medicine has an additional nucleotide or nucleotide derivative not forming a double strand at the 3' side or the 5' side following the duplex region, this is called an overhang. When there is an overhang, the nucleotide constituting the overhang may be a ribonucleotide, a deoxyribonucleotide or a derivatives thereof.

A double-stranded nucleic acid having an overhang may be one having an overhang consisting of 1 to 6 bases, normally 1 to 3 bases, or preferably 2 bases at the 3' end or the 5' end of at least one strand. Examples thereof include those having an overhang consisting of dTdT (where, dT represents deoxythymidine) or UU (where, U represents uridine). The antisense strand alone, the sense strand alone or both of the antisense strand and the sense strand may have an overhang. In the present invention, a double-stranded nucleic acid having an overhang in the antisense strand is preferred. An oligonucleotide strand consisting of 17 to 30 nucleotides including a duplex region and a subsequent overhang in the antisense strand is sufficiently complementary to a target β2GPI mRNA sequence selected from the group described in Tables 4-1 to 4-16. A nucleic acid molecule that produces a double-stranded nucleic acid by an action of a ribonuclease such as Dicer (WO 2005/089287), a double-stranded nucleic acid forming a blunt end with no overhang at the 3' end and the 5' end, or a double-stranded nucleic acid having an overhang in only a sense strand (US 2012/0040459) may also be used as the double-stranded nucleic acid of the present invention.

A nucleic acid having a base sequence identical to the base sequence of a target gene or identical to the base sequence of its complement strand may be used as the double-stranded nucleic acid constituting the nucleic acid conjugate used in the present invention. It is also possible to use a double-stranded nucleic acid having a nucleic acid obtained by deleting 1 to 4 bases at the 5' end or the 3' end of at least one strand of said nucleic acid and having a nucleic acid containing a base sequence complementary to the base sequence of said nucleic acid.

The double-stranded nucleic acid constituting the nucleic acid conjugate used in the present invention may be a double-stranded RNA (dsRNA) formed from two strands of RNA, a double-stranded DNA (dsDNA) formed from two strands of DNA, or a hybrid nucleic acid formed from a strand of RNA and a strand of DNA. Alternatively, it may be a chimera nucleic acid in which one or both of the strands is formed from both DNA and RNA. Preferably it is double-stranded RNA (dsRNA).

The second nucleotide from the 5' end of the antisense strand of the nucleic acid conjugate of the present invention is preferably complementary to the second deoxyribonucleotide from the 3' end of the target β2GPI mRNA sequence, more preferably the 2nd to 7th nucleotides from the 5' end of the antisense strand are completely complimentary to the 2nd to 7th deoxyribonucleotides from the 3' end of the target β2GPI mRNA sequence, and still more preferably the 2nd to 11th nucleotides from the 5' end of the antisense strand are completely complimentary to the 2nd to 11th deoxyribonucleotides from the 3' end of the target β2GPI mRNA sequence. Moreover, the 11th nucleotide from the 5' end of the antisense strand in the nucleic acid of the present invention is preferably complementary to the 11th deoxyribonucleotide from the 3' end of the target β2GPI mRNA sequence, more preferably the 9th to 13th nucleotides from the 5' end of the antisense strand are completely complementary to the 9th to 13th deoxyribonucleotides from the 3' end of the target β2GPI mRNA sequence, and still more preferably the 7th to 15th nucleotides from the 5' end of the antisense strand are completely complementary to the 7th to 15th deoxyribonucleotides from the 3' end of the target β2GPI mRNA sequence.

The antisense strand and the sense strand of the nucleic acid conjugate of the present invention may be designed based on the base sequence (SEQ ID NO: 3541) of the cDNA (sense strand) of the full-length human β2GPI mRNA recorded as Genbank Accession No. NM_000042.

Example of a nucleic acid having β2GPI expression-suppressing activity include a double-stranded nucleic acid having an antisense strand nucleic acid of the present invention containing a base sequence complementary to β2GPI mRNA and having a sense strand nucleic acid of the present invention containing a base sequence complementary to the base sequence of said nucleic acid, and having β2GPI expression-suppressing activity. The single-stranded nucleic acids constituting this double-stranded nucleic acid consist of normally 11 to 30 bases, preferably 15 to 29 bases, more preferably 15 to 27 bases, still more preferably 15 to 25 bases, yet more preferably 17 to 23 bases, and especially preferably 19 to 21 bases. The double-stranded nucleic acid has a duplex region consisting of normally 15 to 27 base pairs, preferably 15 to 25 base pairs, more preferably 15 to 23 base pairs, and still more preferably 15 to 21 base pairs.

The double-stranded nucleic acid may be designed so as to interact with a target sequence in the β2GPI gene sequence.

The sequence of one strand of the double-stranded nucleic acid is complementary to the above target sequence. The double-stranded nucleic acid can be chemically synthesized using methods described herein.

RNA may be prepared by enzymatic or partial/total organic synthesis. Modified ribonucleotides may be introduced by enzymatic or organic synthesis in vitro. In one embodiment, each strand is prepared chemically. Methods for chemically synthesizing RNA molecules are well known in the technical field (see Nucleic Acids Research, 1998, Vol. 32, pp. 936-948). In general, double-stranded nucleic acids can be synthesized using solid-phase oligonucleotide synthesis methods (see for example Usman et al., U.S. Patent No. 5,804,683 (Specification); U.S. Patent No. 5,831,071 (Specification); U.S. Patent No. 5,998,203 (Specification); U.S. Patent No. 6,117,657 (Specification); U.S. Patent No. 6,353,098 (Specification); U.S. Patent No. 6,362,323 (Specification); U.S. Patent No. 6,437,117 (Specification); U.S. Patent No. 6,469,158 (Specification); Scaringe et al., U.S. Patent No. 6,111,086 (Specification); U.S. Patent No. 6,008,400 (Specification); and U.S. Patent No. 6,111,086 (Specification)).

The single-stranded nucleic acids are synthesized by the solid-phase phosphoramidite method (Nucleic Acids Research, 1993, Vol. 30, pp. 2435-2443), deprotected, and desalted on a NAP-5 column (Amersham Pharmacia Biotech, Piscataway, NJ). The oligomers are purified by ion-exchange high-performance liquid chromatography (IE-HPLC) in an Amersham Source 15Q column-1.0 cm, height .25 cm (Amersham Pharmacia Biotech, Piscataway, NJ) using a 15 minute process linear gradient. For the gradient, the buffer is changed from a 90:10 buffer A:B to a 52:48 buffer A:B, in which buffer A is 100 mmol/L Tris pH 8.5, and buffer B is 100 mmol/L Tris pH 8.5 (1 mol/L NaCl). Monitoring is performed at 260 nm, and peaks corresponding to full-length oligonucleotide species are collected, pooled, desalted on a NAP-5 column and freeze-dried.

The purity of each single-stranded nucleic acid is determined by capillary electrophoresis (CE) in a Beckman PACE 5000 (Beckman Coulter, Inc., Fullerton, CA). The CE capillarity has an inner diameter of 100 µm, and contains ssDNA 100R Gel (Beckman-Coulter). Typically, about 0.6 nmol of the oligonucleotide is injected into the capillary, and detected by UV absorption at 260 nm in a 444 V/cm electrical field. A modified tris-boric acid-7 mol/L urea running buffer is purchased from Beckman-Coulter. A single-stranded nucleic acid with a purity of at least 90% in the CE evaluation is obtained for use in the tests described below. Compound identity is verified by matrix-assisted laser desorption/ionization-time of flight (MALD-TOF) mass spectrometry using a Voyager-DE® Biospectrometry workstation (Applied Biosystems, Inc., Foster City, CA) in accordance with the manufacturer's recommended protocols. A relative molecular mass of the single-stranded nucleic acid can be obtained within 0.2% of the predicted molecular mass.

The single-stranded nucleic acids are re-suspended at a concentration of 100 µmol/L in a buffer with the pH of 7.5 consisting of 100 mmol/L potassium acetate and 30 mmol/L HEPES. The complementary sense strand and the antisense strand are mixed in the same molar amounts to obtain a final 50 µmol/L solution of the double-stranded nucleic acid. The sample is heated for 5 minutes at 95°C, and cooled to room temperature before use. The double-stranded nucleic acid is stored at -20°C. The single-stranded nucleic acids are either freeze-dried or stored at -80°C in nuclease-free water.

Examples of the double-stranded nucleic acid of the present invention consisting of a sense strand and an antisense strand and comprising a duplex region of at least 11 base pairs in which an oligonucleotide strand with a strand length of 17 to 30 nucleotides in the antisense strand is complementary to the target β2GPI mRNA sequence selected from the group described in Tables 4-1 to 4-16, include a double-stranded nucleic acid containing a sequence selected from the group consisting of the antisense strands described in Tables 2-1 to 2-18 and 3-1 to 3-18 and 4-1 to 4-16 below, a double-stranded nucleic acid containing a sequence selected from the group consisting of the sense strands described in Tables 2-1 to 2-18 and 3-1 to 3-18 and 4-1 to 4-16 below, or a double-stranded nucleic acid containing the sequence of a pair of sense/antisense strands selected from the group consisting of the sense/antisense strands described in Tables 2-1 to 2-18 and 3-1 to 3-18 and 4-1 to 4-16 below. That is, specific examples of the double-stranded nucleic acid constituting the nucleic acid conjugate of the present invention are double-stranded nucleic acids consisting of the sense strand and the antisense strand from Tables 2-1 to 2-18 and 3-1 to 3-18 and 4-1 to 4-16 below. In Tables 3-1 to 3-18, N(M) represents 2'-O-methyl modified RNA, N(F) represents 2'-fluorine modified RNA, and ^ represents phosphorothioate.

### [Table 3]

**Table 2-1**

| Double stranded nucleic acid No. | SEQ ID NO | Sense strand sequence (5'→3') | SEQ ID NO. | Antisense strand sequence (5'→3') |
|---|---|---|---|---|
| BH0033 | No 4001 | UGGUAGUGCCAGUGUGACUCA | No. 4703 | UGAGUCACACUGGCACUACCAAA |
| BH0034 | No 4002 | GGUAGUGCCAGUGUGACUCAU | No. 4704 | AUGAGUCAGACUGGCACUACCAA |
| BH0035 | No. 4003 | GUAGUGCCAGUGUGACUCAUC | No. 4705 | GAUGAGUCACACUGGCACUACCA |
| BH0036 | No. 4004 | UAGUGCCAGUGUGACUCAUCC | No. 4706 | GGAUGAGUCACACUGGCACUACC |
| BH0037 | No 4005 | AGUGCCAGUGUGACUCAUCCA | No. 4707 | UGGAUGAGUCACACUGGCACUAC |
| BH0038 | No. 4006 | GUGCCAGUGUGACUCAUCCAC | No. 4708 | GUGGAUGAGUCACACUGGCACUA |
| BH0039 | No. 4007 | UGCCAGUGUGACUCAUCCACA | No. 4709 | UGUGGAUGAGUCACACUGGCACU |
| BH0040 | No. 4008 | GCCAGUGUGACUCAUCCACAA | No. 4710 | UUGUGGAUGAGUCACACUGGCAC |
| BH0041 | No. 4009 | CCAGUGUGACUCAUCCACAAU | No. 4711 | AUUGUGGAUGAGUCACACUGGCA |
| BH0042 | No. 4010 | CAGUGUGACUCAUCCACAAUG | No. 4712 | CAUUGUGGAUGAGUCACACUGGC |
| BH0043 | No. 4011 | AGUGUGACUCAUCCACAAUGA | No. 4713 | UCAUUGUGGAUGAGUCACACUGG |
| BH0044 | No. 4012 | GUGUGACUCAUCCACAAUGAU | No. 4714 | AUCAUUGUGGAUGAGUCACACUG |
| BH0045 | No. 4013 | UGUGACUCAUCCACAAUGAUU | No. 4715 | AAUCAUUGUGGAUGAGUCACACU |
| BH0046 | No. 4014 | GUGACUCAUCCACAAUGAUUU | No. 4716 | AAAUCAUUGUGGAUGAGUCACAC |
| BH0047 | No. 4015 | UGACUCAUCCACAAUGAUUUC | No. 4717 | GAAAUCAUUGUGGAUGAGUCACA |
| BH0048 | No. 4016 | GACUCAUCCACAAUGAUUUCU | No. 4718 | AGAAAUCAUUGUGGAUGAGUCAC |
| BH0050 | No. 4017 | CUCAUCCACAAUGAUUUCUCC | No. 4719 | GGAGAAAUCAUUGUGGAUGAGUC |
| BH0051 | No. 4018 | UCAUCCACAAUGAUUUCUCCA | No. 4720 | UGGAGAAAUCAUUGUGGAUGAGU |
| BH0052 | No. 4019 | CAUCCACAAUGAUUUCUCCAG | No 4721 | CUGGAGAAAUCAUUGUGGAUGAG |
| BH0053 | No. 4020 | AUCCACAAUGAUUUCUCCAGU | No. 4722 | ACUGGAGAAAUCAUUGUGGAUGA |
| BH0054 | No. 4021 | UCCACAAUGAUUUCUCCAGUG | No. 4723 | CACUGGAGAAAUCAUUGUGGAUG |
| BH0055 | No. 4022 | CCACAAUGAUUUCUCCAGUGC | No. 4724 | GCACUGGAGAAAUCAUUGUGGAU |
| BH0056 | No. 4023 | CACAAUGAUUUCUCCAGUGCU | No. 4725 | AGCACUGGAGAAAUCAUUGUGGA |
| BH0057 | No 4024 | ACAAUGAUUUCUCCAGUGCUC | No. 4726 | GAGCACUGGAGAAAUCAUUGUGG |
| BH0058 | No. 4025 | CAAUGAUUUCUCCAGUGCUCA | No 4727 | UGAGCACUGGAGAAAUCAUUGUG |
| BH0059 | No. 4026 | KAUGAUUUCUCCAGUGCUCAU | No. 4728 | AUGAGCACUGGAGAAAUCAUUGU |
| BH0060 | No. 4027 | AUGAUUUCUCCAGUGCUCAUC | No. 4729 | GAUGAGCACUGGAGAAAUCAUUG |
| BH0061 | No. 4028 | UGAUUUCUCCAGUGCUCAUCU | No. 4730 | AGAUGAGCACUGGAGAAAUCAUU |
| BH0062 | No. 4029 | GAUUUCUCCAGUGCUCAUCUU | No. 4731 | AAGAUGAGCACUGGAGAAAUCAU |
| BH0063 | No. 4030 | AUUUCUCCAGUGCUCAUCUUG | No. 4732 | CAAGAUGAGCACUGGAGAAAUCA |
| BH0064 | No. 4031 | UUUCUCCAGUGCUCAUCUUGU | No 4733 | ACAAGAUGAGCACUGGAGAAAUC |
| BH0065 | No. 4032 | UUCUCCAGUGCUCAUCUUGUU | No. 4734 | AACAAGAUGAGCACUGGAGAAAU |
| BH0066 | No. 4033 | UCUCCAGUGCUCAUCUUGUUC | No. 4735 | GAACAAGAUGAGCACUGGAGAAA |
| BH0067 | No. 4034 | CUCCAGUGCUCAUCUUGUUCU | No. 4736 | AGAACAAGAUGAGCACUGGAGAA |
| BH0068 | No. 4035 | UCCAGUGCUCAUCUUGUUCUC | No. 4737 | GAGAACAAGAUGAGCACUGGAGA |
| BH0069 | No. 4036 | CCAGUGCUCAUCUUGUUCUCG | No. 4738 | CGAGAACAAGAUGAGCACUGGAG |
| BH0070 | No. 4037 | CAGUGCUCAUCUUGUUCUCGA | No. 4739 | UCGAGAACAAGAUGAGCACUGGA |
| BH0071 | No. 4038 | AGUGCUCAUCUUGUUCUCGAG | No. 4740 | CUCGAGAACAAGAUGAGCACUGG |
| BH0072 | No. 4039 | GUGCUCAUCUUGUUCUCGAGU | No. 4741 | ACUCGAGAACAAGAUGAGCACUG |
| BH0073 | No. 4040 | UGCUCAUCUUGUUCUCGAGUU | No. 4742 | AACUCGAGAACAAGAUGAGCACU |

### [Table 4]

**Table 2-2**

| Double stranded nucleic acid No. | SEQ ID NO. | Sense strand sequence (5'→3') | SEQ ID NO. | Antisense strand sequence (5'→3') |
|---|---|---|---|---|
| BH0074 | No. 4041 | GCUCAUCUUGUUCUCGAGUUU | No. 4743 | AAACUCGAGAACAAGAUGAGCAC |
| BH0075 | No. 4042 | CUCAUCUUGUUCUCGAGUUUU | No. 4744 | AAAACUCGAGAACAAGAUGAGCA |
| BH0076 | No. 4043 | UCAUCUUGUUCUCGAGUUUUC | No. 4745 | GAAAACUCGAGAACAAGAUGAGC |
| BH0077 | No. 4044 | CAUCUUGUUCUCGAGUUUUCU | No. 4746 | AGAAAACUCGAGAACAAGAUGAG |
| BH0078 | No. 4045 | AUCUUGUUCUCGAGUUUUCUC | No. 4747 | GAGAAAACUCGAGAACAAGAUGA |
| BH0079 | No. 4046 | UCUUGUUCUCGAGUUUUCUCU | No 4748 | AGAGAAAACUCGAGAACAAGAUG |
| BH0080 | No. 4047 | CUUGUUCUCGAGUUUUCUCUG | No. 4749 | CAGAGAAAACUCGAGAACAAGAU |
| BH0081 | No. 4048 | UUGUUCUCGAGUUUUCUCUGC | No. 4750 | GCAGAGAAAACUCGAGAACAAGA |
| BH0083 | No. 4049 | GUUCUCGAGUUUUCUCUGCCA | No. 4751 | UGGCAGAGAAAACUCGAGAACAA |
| BH0084 | No. 4050 | UUCUCGAGUUUUCUCUGCCAU | No. 4752 | AUGGCAGAGAAAACUCGAGAACA |
| BH0085 | No. 4051 | UCUCGAGUUUUCUCUGCCAUG | No. 4753 | CAUGGCAGAGAAAACUCGAGAAC |
| BH0086 | No. 4052 | CUCGAGUUUUCUCUGCCAUGU | No. 4754 | ACAUGGCAGAGAAAACUCGAGAA |
| BH0087 | No. 4053 | UCGAGUUUUCUCUGCCAUGUU | No. 4755 | AACAUGGCAGAGAAAACUCGAGA |
| BH0088 | No. 4054 | CGAGUUUUCUCUGCCAUGUUG | No. 4766 | CAACAUGGCAGAGAAAACUCGAG |
| BH0089 | No. 4055 | GAGUUUUCUCUGCCAUGUUGC | No. 4757 | GCAACAUGGCAGAGAAAACUCGA |
| BH0090 | No. 4056 | AGUUUUCUCUGCCAUGUUGCU | No. 4758 | AGCAACAUGGCAGAGAAAACUCG |
| BH0091 | No. 4057 | GUUUUCUCUGCCAUGUUGCUA | No. 4759 | UAGCAACAUGGCAGAGAAAACUC |
| BH0092 | No. 4058 | UUUUCUCUGCCAUGUUGCUAU | No. 4760 | AUAGCAACAUGGCAGAGAAAACU |
| BH0093 | No. 4059 | UUUCUCUGCCAUGUUGCUAUU | No 4761 | AAUAGCAACAUGGCAGAGAAAAC |
| BH0096 | No. 4060 | CUCUGCCAUGUUGCUAUUGCA | No. 4762 | UGCAAUAGCAACAUGGCAGAGAA |
| BH0097 | No. 4061 | UCUGCCAUGUUGCUAUUGCAG | No 4763 | CUGCAAUAGCAACAUGGCAGAGA |
| BH0098 | No. 4062 | CUGCCAUGUUGCUAUUGCAGG | No. 4764 | CCUGCAAUAGCAACAUGGCAGAG |
| BH0099 | No. 4063 | UGCCAUGUUGCUAUUGCAGGA | No. 4765 | UCCUGCAAUAGCAACAUGGCAGA |
| BH0103 | No. 4064 | AUGUUGCUAUUGCAGGACGGA | No. 4766 | UCCGUCCUGCAAUAGCAACAUGG |
| BH0106 | No. 4065 | UUGCUAUUGCAGGACGGACCU | No. 4767 | AGGUCCGUCCUGCAAUAGCAACA |
| BH0107 | No. 4066 | UGCUAUUGCAGGACGGACCUG | No. 4768 | CAGGUCCGUCCUGCAAUAGCAAC |
| BH0108 | No. 4067 | GCUAUUGCAGGACGGACCUGU | No. 4769 | ACAGGUCCGUCCUGCAAUAGCAA |
| BH0109 | No. 4068 | CUAUUGCAGGACGGACCUGUC | No. 4770 | GACAGGUCCGUCCUGCAAUAGCA |
| BH0112 | No. 4069 | UUGCAGGACGGACCUGUCCCA | No. 4771 | UGGGACAGGUCCGUCCUGCAAUA |
| BH0114 | No. 4070 | GCAGGACGGACCUGUCCCAAG | No. 4772 | CUUGGGACAGGUCCGUCCUGCAA |
| BH0115 | No. 4071 | CAGGACGGACCUGUCCCAAGC | No. 4773 | GCUUGGGACAGGUCCGUCCUGCA |
| BH0117 | No. 4072 | GGACGGACCUGUCCCAAGCCA | No. 4774 | UGGCUUGGGACAGGUCCGUCCUG |
| BH0118 | No. 4073 | GACGGACCUGUCCCAAGCCAG | No. 4775 | CUGGCUUGGGACAGGUCCGUCCU |
| BH0119 | No. 4074 | ACGGACCUGUCCCAAGCCAGA | No. 4776 | UCUGGCUUGGGACAGGUCCGUCC |
| BH0120 | No. 4075 | CGGACCUGUCCCAAGCCAGAU | No. 4777 | AUCUGGCUUGGGACAGGUCCGUC |
| BH0121 | No. 4076 | GGACCUGUCCCAAGCCAGAUG | No. 4778 | CAUCUGGCUUGGGACAGGUCCGU |
| BH0122 | No. 4077 | GACCUGUCCCAAGCCAGAUGA | No. 4779 | UCAUCUGGCUUGGGACAGGUCCG |
| BH0123 | No. 4078 | ACCUGUCCCAAGCCAGAUGAU | No. 4780 | AUCAUCUGGCUUGGGACAGGUCC |
| BH0124 | No. 4079 | CCUGUCCCAAGCCAGAUGAUU | No. 4781 | AAUCAUCUGGCUUGGGACAGGUC |
| BH0125 | No. 4080 | CUGUCCCAAGCCAGAUGAUUU | No. 4782 | AAAUCAUCUGGCUUGGGACAGGU |

### [Table 5]

**Table 2-3**

| Double stranded nucleic acid No. | SEQ ID NO. | Sense strand sequence (5'→3') | SEQ ID NO. | Antisense strand sequence (5'→3') |
|---|---|---|---|---|
| BH0126 | No. 4081 | UGUCCCAAGCCAGAUGAUUUA | No. 4783 | UAAAUCAUCUGGCUUGGGACAGG |
| BH0127 | No. 4082 | GUCCCAAGCCAGAUGAUUUAC | No. 4784 | GUAAAUCAUCUGGCUUGGGACAG |
| BH0128 | No. 4083 | UCCCAAGCCAGAUGAUUUACC | No 4785 | GGUAAAUCAUCUGGCUUGGGACA |
| SH0129 | No. 4084 | CCCAAGCCAGAUGAUUUACCA | No.4786 | UGGUAAAUCAUCUGGCUUGGGAC |
| BH0130 | No. 4085 | CCAAGCCAGAUGAUUUACCAU | No. 4787 | AUGGUAAAUCAUCUGGCUUGGGA |
| BH0131 | No 4086 | CAAGCCAGAUGAUUUACCAUU | No. 4788 | AAUGGUAAAUCAUCUGGCUUGGG |
| BH0132 | No. 4087 | AAGCCAGAUGAUUUACCAUUU | No. 4789 | AAAUGGUAAAUCAUCUGGCUUGG |
| BH0133 | No. 4088 | AGCCAGAUGAUUUACCAUUUU | No 4790 | AAAAUGGUAAAUCAUCUGGCUUG |
| BH0134 | No. 4089 | GCCAGAUGAUUUACCAUUUUC | No. 4791 | GAAAAUGGUAAAUCAUCUGGCUU |
| BH0135 | No. 4090 | CCAGAUGAUUUACCAUUUUCC | No. 4792 | GGAAAAUGGUAAAUCAUCUGGCU |
| BH0136 | No. 4091 | CAGAUGAUUUACCAUUUUCCA | No. 4793 | UGGAAAAUGGUAAAUCAUCUGGC |
| BH0137 | No. 4092 | AGAUGAUUUACCAUUUUCCAC | No. 4794 | GUGGAAAAUGGUAAAUCAUCUGG |
| BH0138 | No. 4093 | GAUGAUUUACCAUUUUCCACA | No. 4795 | UGUGGAAAAUGGUAAAUCAUCUG |
| BH0139 | No. 4094 | AUGAUUUACCAUUUUCCACAG | No. 4796 | CUGUGGAAAAUGGUAAAUCAUCU |
| BH0143 | No. 4095 | UUUACCAUUUUCCACAGUGGU | No. 4797 | ACCACUGUGGAAAAUGGUAAAUC |
| BH0144 | No. 4096 | UUACCAUUUUCCACAGUGGUC | No. 4798 | GACCACUGUGGAAAAUGGUAAAU |
| BH0145 | No. 4097 | UACCAUUUUCCACAGUGGUCC | No. 4799 | GGACCACUGUGGAAAAUGGUAAA |
| BH0149 | No. 4098 | AUUUUCCACAGUGGUCCCGUU | No. 4800 | AACGGGACCACUGUGGAAAAUGG |
| BH0150 | No. 4099 | UUUUCCACAGUGGUCCCGUUA | No. 4801 | UAACGGGACCACUGUGGAAAAUG |
| BH0151 | No. 4100 | UUUCCACAGUGGUCCCGUUAA | No. 4802 | UUAACGGGACCACUGUGGAAAAU |
| BH0152 | No. 4101 | UUCCACAGUGGUCCCGUUAAA | No. 4803 | UUUAACGGGACCACUGUGGAAAA |
| BH0153 | No. 4102 | UCCACAGUGGUCCCGUUAAAA | No. 4804 | UUUUAACGGGACCACUGUGGAAA |
| BH0154 | No. 4103 | CCACAGUGGUCCCGUUAAAAA | No. 4805 | UUUUUAACGGGACCACUGUGGAA |
| BH0155 | No. 4104 | CACAGUGGUCCCGUUAAAAAC | No. 4806 | GUUUUUAACGGGACCACUGUGGA |
| BH0156 | No. 4105 | ACAGUGGUCCCGUUAAAAACA | No. 4807 | UGUUUUUAACGGGACCACUGUGG |
| BH0157 | No. 4106 | CAGUGGUCCCGUUAAAAACAU | No. 4808 | AUGUUUUUAACGGGACCACUGUG |
| BH0158 | No 4107 | AGUGGUCCCGUUAAAAACAUU | No. 4809 | AAUGUUUUUAACGGGACCACUGU |
| BH0159 | No. 4108 | GUGGUCCCGUUAAAAACAUUC | No. 4810 | GAAUGUUUUUAACGGGACCACUG |
| SH0160 | No. 4109 | UGGUCCCGUUAAAAACAUUCU | No. 4811 | AGAAUGUUUUUAACGGGACCACU |
| SH0161 | No. 4110 | GGUCCCGUUAAAAACAUUCUA | No. 4812 | UAGAAUGUUUUUAACGGGACCAC |
| BH0162 | No. 4111 | GUCCCGUUAAAAACAUUCUAU | No. 4813 | AUAGAAUGUUUUUAACGGGACCA |
| BH0163 | No. 4112 | UCCCGUUAAAAACAUUCUAUG | No. 4814 | CAUAGAAUGUUUUUAACGGGACC |
| BH0164 | No. 4113 | CCCGUUAAAAACAUUCUAUGA | No. 4815 | UCAUAGAAUGUUUUUAACGGGAC |
| BH0173 | No. 4114 | AACAUUCUAUGAGCCAGGAGA | No. 4816 | UCUCCUGGCUCAUAGAAUGUUUU |
| BN0174 | No. 4115 | ACAUUCUAUGAGCCAGGAGAA | No. 4817 | UUCUCCUGGCUCAUAGAAUGUUU |
| SH0177 | No. 4116 | UUCUAUGAGCCAGGAGAAGAG | No. 4818 | CUCUUCUCCUGGCUCAUAGAAUG |
| BH0178 | No. 4117 | UCUAUGAGCCAGGAGAAGAGA | No. 4819 | UCUCUUCUCCUGGCUCAUAGAAU |
| BH0179 | No. 4118 | CUAUGAGCCAGGAGAAGAGAU | No. 4820 | AUCUCUUCUCCUGGCUCAUAGAA |
| BH0180 | No. 4119 | UAUGAGCCAGGAGAAGAGAUU | No. 4821 | AAUCUCUUCUCCUGGCUCAUAGA |
| BH0181 | No. 4120 | AUGAGCCAGGAGAAGAGAUUA | No. 4822 | UAAUCUCUUCUCCUGGCUCAUAG |

### [Table 6]

**Table 2-4**

| Double stranded nucleic acid No. | SEQ ID NO. | Sense strand sequence (5'→3') | SEQ ID NO. | Antisense strand sequence (5'→3') |
|---|---|---|---|---|
| BH0183 | No. 4121 | GAGCCAGGAGAAGAGAUUACG | No. 4823 | CGUAAUCUCUUCUCCUGGCUCAU |
| BH0184 | No. 4122 | AGCCAGGAGAAGAGAUUACGU | No. 4824 | ACGUAAUCUCUUCUCCUGGCUCA |
| BH0185 | No. 4123 | GCCAGGAGAAGAGAUUACGUA | No. 4825 | UACGUAAUCUCUUCUCCUGGCUC |
| BH0186 | No. 4124 | CCAGGAGAAGAGAUUACGUAU | No. 4826 | AUACGUAAUCUCUUCUCCUGGCU |
| BH0187 | No. 4125 | CAGGAGAAGAGAUUACGUAUU | No. 4827 | AAUACGUAAUCUCUUCUCCUGGC |
| BH0188 | No 4126 | AGGAGAAGAGAUUACGUAU UC | No. 4828 | GAAUACGUAAUCUCUUCUCCUGG |
| BH0189 | No. 4127 | GGAGAAGAGAUUACGUAUUCC | No. 4829 | GGAAUACGUAAUCUCUUCUCCUG |
| BH0190 | No. 4128 | GAGAAGAGAUUACGUAUUCCU | No. 4830 | AGGAAUACGUAAUCUCUUCUCCU |
| BH0193 | NO. 4129 | AAGAGAUUACGUAUUCCUGCA | No. 4831 | UGCAGGAAUACGUAAUCUCUUCU |
| BH0194 | No. 4130 | AGAGAUUACGUAUUCCUGCAA | No. 4832 | UUGCAGGAAUACGUAAUCUCUUC |
| BH0195 | No. 4131 | GAGAUUACGUAUUCCUGCAAG | No. 4833 | CUUGCAGGAAUACGUAAUCUCUU |
| BH0199 | No. 4132 | UUACGUAUUCCUGCAAGCCGG | No. 4834 | CCGGCUUGCAGGAAUACGUAAUC |
| BH0203 | No. 4133 | GUAUUCCUGCAAGCCGGGCUA | No. 4835 | UAGCCCGGCUUGCAGGAAUACGU |
| BH0201 | No. 4134 | UCCUGCAAGCCGGGCUAUGUG | No. 4836 | CACAUAGCCCGGCUUGCAGGAAU |
| BH0225 | No. 4135 | GUGUCCCGAGGAGGGAUGAGA | No. 4837 | UCUCAUCCCUCCUCGGGACACAU |
| BH0226 | No. 4136 | UGUCCCGAGGAGGGAUGAGAA | No. 4838 | UUCUCAUCCCUCCUCGGGACACA |
| BH0229 | No. 4137 | CCCGAGGAGGGAUGAGAAAGU | No. 4839 | ACUUUCUCAUCCCUCCUCGGGAC |
| BH0230 | No. 4138 | CCGAGGAGGGAUGAGAAAGUU | No. 4840 | AACUUUCUCAUCCCUCCUCGGGA |
| BH0231 | No. 4139 | CGAGGAGGGAUGAGAAAGUUU | No. 4841 | AAACUUUCUCAUCCCUCCUCGGG |
| BH0232 | No. 4140 | GAGGAGGGAUGAGAAAGUUUA | No. 4842 | UAAACUUUCUCAUCCCUCCUCGG |
| BH0233 | No. 4141 | AGGAGGGAUGAGAAAGUUUAU | No. 4843 | AUAAACUUUCUCAUCCCUCCUCG |
| BH0234 | No. 4142 | GGAGGGAUGAGAAAGUUUAUC | No. 4844 | GAUAAACUUUCUCAUCCCUCCUC |
| BH0235 | No. 4143 | GAGGGAUGAGAAAGUUUAUCU | No. 4845 | AGAUAAACUUUCUCAUCCCUCCU |
| BH0236 | No. 4144 | AGGGAUGAGAAAGUUUAUCUG | No. 4846 | CAGAUAAACUUUCUCAUCCCUCC |
| BH0242 | No. 4145 | GAGAAAGUUUAUCUGCCCUCU | No. 4847 | AGAGGGCAGAUAAACUUUCUCAU |
| BH0244 | No. 4146 | GAAAGUUUAUCUGCCCUCUCA | No. 4848 | UGAGAGGGCAGAUAAACUUUCUC |
| BH0255 | No. 4147 | UGCCCUCUCACAGGACUGUGG | No. 4849 | CCACAGUCCUGUGAGAGGGCAGA |
| BH0260 | No. 4148 | UCUCACAGGACUGUGGCCCAU | No. 4850 | AUGGGCCACAGUCCUGUGAGAGG |
| BH0262 | No. 4149 | UCACAGGACUGUGGCCCAUCA | No. 4851 | UGAUGGGCCACAGUCCUGUGAGA |
| BH0265 | No. 4150 | CAGGACUGUGGCCCAUCAACA | No. 4852 | UGUUGAUGGGCCACAGUCCUGUG |
| BH0266 | No. 4151 | AGGACUGUGGCCCAUCAACAC | No. 4853 | GUGUUGAUGGGCCACAGUCCUGU |
| BH0272 | No. 4152 | GUGGCCCAUCAACACUCUGAA | No. 4854 | UUCAGAGUGUUGAUGGGCCACAG |
| BH0274 | No. 4153 | GGCCCAUCAACACUCUGAAAU | No. 4855 | AUUUCAGAGUGUUGAUGGGCCAC |
| BH0215 | No. 4154 | GCCCAUCAACACUCUGAAAUG | No. 4856 | CAUUUCAGAGUGUUGAUGGGCCA |
| BH0276 | No. 4155 | CCCAUCAACACUCUGAAAUGU | No. 4857 | ACAUUUCAGAGUGUUGAUGGGCC |
| BH0277 | No. 4156 | CCAUCAACACUCUGAAAUGUA | No. 4858 | UACAUUUCAGAGUGUUGAUGGGC |
| BH0279 | No. 4157 | AUCAACACUCUGAAAUGUACA | No. 4859 | UGUACAUUUCAGAGUGUUGAUGG |
| BH0283 | No. 4158 | ACACUCUGAAAUGUACACCCA | No. 4860 | UGGGUGUACAUUUCAGAGUGUUG |
| BH0285 | No. 4159 | ACUCUGAAAUGUACACCCAGA | No. 4861 | UCUGGGUGUACAUUUCAGAGUGU |
| BH0288 | No. 4160 | CUGAAAUGUACACCCAGAGUA | No. 4862 | UACUCUGGGUGUACAUUUCAGAG |

### [Table 7]

**Table 2-5**

| Double stranded nucleic acid No. | SEQ ID NO. | Sense strand sequence (5'→3') | SEQ ID NO. | Antisense strand sequence (5'→3') |
|---|---|---|---|---|
| BH0289 | No. 4161 | U GAAAUGUACACCCAGAGUAU | No. 4863 | AUACUCUGGGUGUACAUUUCAGA |
| BH0291 | No. 4162 | AAAUGUACACCCAGAGUAUGU | No. 4864 | ACAUACUCUGGGUGUACAUUUCA |
| BH0294 | No. 4163 | UGUACACCCAGAGUAUGUCCU | No. 4865 | AGGACAUACUCUGGGUGUACAUU |
| BH0295 | No. 4164 | GUACACCCAGAGUAUGUCCUU | No. 4866 | AAGGACAUACUCUGGGUGUACAU |
| BH0296 | No. 4165 | UACACCCAGAGUAUGUCCUUU | No. 4867 | AAAGGACAUACUCUGGGUGUACA |
| BH0297 | No 4166 | ACACCCAGAGUAUGUCCUUUU | No. 4868 | AAAAGGACAUACUCUGGGUGUAC |
| BH0298 | No. 4167 | CACCCAGAGUAUGUCCUUUUG | No. 4869 | CAAAAGGACAUACUCUGGGUGUA |
| BH0300 | No. 4168 | CCCAGAGUAUGUCCUUUUGCU | No. 4870 | AGCAAAAGGACAUACUCUGGGUG |
| BH0301 | No. 4169 | CCAGAGUAUGUCCUUUUGCUG | No. 4871 | CAGCAAAAGGACAUACUCUGGGU |
| BH0302 | No. 4170 | CAGAGUAUGUCCUUUUGCUGG | No. 4872 | CCAGCAAAAGGACAUACUCUGGG |
| BH0303 | No. 4171 | AGAGUAUGUCCUUUUGCUGGA | No. 4873 | UCCAGCAAAAGGACAUACUCUGG |
| BH0304 | No. 4172 | GAGUAUGUCCUUUUGCUGGAA | No. 4874 | UUCCAGCAAAAGGACAUACUCUG |
| BH0306 | No. 4173 | GUAUGUCCUUUUGCUGGAAUC | No. 4875 | GAUUCCAGCAAAAGGACAUACUC |
| BH0307 | No. 4174 | UAUGUCCUUUUGCUGGAAUCU | No. 4876 | 4GAUUCCAGCAAAAGGACAUACU |
| BH0308 | No. 4175 | AUGUCCUUUUGCUGGAAUCUU | No 4877 | AAGAUUCCAGCAAAAGGACAUAC |
| BH0309 | No. 4176 | UGUCCUUUUGCUGGAAUCUUA | No. 4878 | UAAGAUUCCAGCAAAAGGACAUA |
| BH0310 | No. 4177 | GUCCUUUUGCUGGAAUCUUAG | No. 4879 | CUAAGAUUCCAGCAAAAGGACAU |
| BH0311 | No. 4178 | UCCUUUUGCUGGAAUCUUAGA | No. 4880 | UCUAAGAUUCCAGCAAAAGGACA |
| BH0314 | No. 4179 | UUUUGCUGGAAUCUUAGAAAA | No. 4881 | UUUUCUAAGAUUCCAGCAAAAGG |
| BH0315 | No. 4180 | UUUGCUGGAAUCUUAGAAAAU | No. 4882 | AUUUUCUAAGAUUCCAGCAAAAG |
| BH0316 | No. 4181 | UUGCUGGAAUCUUAGAAAAUG | No. 4883 | CAUUUUCUAAGAUUCCAGCAAAA |
| BH0317 | No. 4182 | UGCUGGAAUCUUAGAAAAUGG | No. 4884 | CCAUUUUCUAAGAUUCCAGCAAA |
| BH0318 | No. 4183 | GCUGGAAUCUUAGAAAAUGGA | No. 4885 | UCCAUUUUCUAAGAUUCCAGCAA |
| BH0319 | No. 4184 | CUGGAAUCUUAGAAAAUGGAG | No. 4886 | CUCCAUUUUCUAAGAUUCCAGCA |
| BH0324 | No. 4185 | AUCUUAGAAAAUGGAGCCGUA | No. 4887 | UACGGCUCCAUUUUCUAAGAUUC |
| BH0327 | No. 4186 | UUAGAAAAUGGAGCCGUACGC | No. 4888 | GCGUACGGCUCCAUUUUCUAAGA |
| BH0328 | No. 4187 | UAGAAAAUGGAGCCGUACGCU | No. 4889 | AGCGUACGGCUCCAUUUUCUAAG |
| BH0329 | No. 4188 | AGAAAAUGGAGCCGUACGCUA | No. 4890 | UAGCGUACGGCUCCAUUUUCUAA |
| BH0330 | No. 4189 | GAAAAUGGAGCCGUACGCUAU | No. 4891 | AUAGCGUACGGCUCCAUUUUCUA |
| SH0331 | No. 4190 | AAAAUGGAGCCGUACGCUAUA | No. 4892 | UAUAGCGUACGGCUCCAUUUUCU |
| BH0334 | No. 4191 | AUGGAGCCGUACGCUAUACGA | No. 4893 | UCGUAUAGCGUACGGCUCCAUUU |
| BH0335 | No. 4192 | UGGAGCCGUACGCUAUACGAC | No. 4894 | GUCGUAUAGCGUACGGCUCCAUU |
| BH0336 | No 4193 | GGAGCCGUACGCUAUACGACU | No. 4895 | AGUCGUAUAGCGUACGGCUCCAU |
| BH0337 | No. 4194 | GAGCCGUACGCUAUACGACUU | No. 4896 | AAGUCGUAUAGCGUACGGCUCCA |
| BH0338 | No. 4195 | AGCCGUACGCUAUACGACUUU | No. 4897 | AAAGUCGUAUAGCGUACGGCUCC |
| BH0339 | No. 4196 | GCCGUACGCUAUACGACUUUU | No. 4898 | AAAAGUCGUAUAGCGUACGGCUC |
| BH0340 | No. 4197 | CCGUACGCUAUACGACUUUUG | No. 4899 | CAAAAGUCGUAUAGCGUACGGCU |
| BH0341 | No. 4198 | CGUACGCUAUACGACUUUUGA | No. 4900 | UCAAAAGUCGUAUAGCGUACGGC |
| BH0342 | No. 4199 | GUACGCUAUACGACUUUUGAA | No. 4901 | UUCAAAAGUCGUAUAGCGUACGG |
| BH0343 | No. 4200 | UACGCUAUACGACUUUUGAAU | No. 4902 | AUUCAAAAGUCGUAUAGCGUACG |

### [Table 8]

**Table 2-6**

| Double stranded nucleic acid No. | SEQ ID NO. | Sense strand sequence (5'→3') | SEQ ID NO. | Antisense strand sequence (5'→3') |
|---|---|---|---|---|
| BH0345 | No. 4201 | CGCUAUACGACUUUUGAAUAU | No. 4903 | AUAUUCAAAAGUCGUAUAGCGUA |
| BH0346 | No. 4202 | GCUAUACGACUUUUGAAUAUC | No. 4904 | GAUAUUCAAAAGUCGUAUAGCGU |
| BH0349 | No. 4203 | AUACGACUUUUGAAUAUCCCA | No. 4905 | UGGGAUAUUCAAAAGUCGUAUAG |
| BH0350 | No. 4204 | UACGACUUUUGAAUAUCCCAA | No. 4906 | UUGGGAUAUUCAAAAGUCGUAUA |
| BH0351 | No. 4205 | ACGACUUUUGAAUAUCCCAAC | No. 4907 | GUUGGGAUAUUCAAAAGUCGUAU |
| BH0352 | No. 4206 | CGACUUUUGAAUAUCCCAACA | No. 4908 | UGUUGGGAUAUUCAAAAGUCGUA |
| BH0355 | No 4207 | CUUUUGAAUAUCCCAACACGA | No 4909 | UCGUGUUGGGAUAUUCAAAAGUC |
| BH0356 | No. 4208 | UUUUGAAUAUCCCAACACGAU | No. 4910 | AUCGUGUUGGGAUAUUCAAAAGU |
| BH0357 | No. 4209 | UUUGAAUAUCCCAACACGAUC | No. 4911 | GAUCGUGUUGGGAUAUUCAAAAG |
| BH0358 | No 4210 | UUGAAUAUCCCAACACGAUCA | No. 4912 | UGAUCGUGUUGGGAUAUUCAAAA |
| BH0361 | No. 4211 | AAUAUCCCAACACGAUCAGUU | No. 4913 | AACUGAUCGUGUUGGGAUAUUCA |
| BH0362 | No. 4212 | AUAUCCCAACACGAUCAGUUU | No. 4914 | AAACUGAUCGUGUUGGGAUAUUC |
| BH0363 | No. 4213 | UAUCCCAACACGAUCAGUUUU | No. 4915 | AAAACUGAUCGUGUUGGGAUAUU |
| BH0364 | No. 4214 | AUCCCAACACGAUCAGUUUUU | No. 4916 | AAAAACUGAUCGUGUUGGGAUAU |
| BH0365 | No. 4215 | UCCCAACACGAUCAGUUUUUC | No. 491 7 | GAAAAACUGAUCGUGUUGGGAUA |
| BH0366 | No 4216 | CCCAACACGAUCAGUUUUUCU | No. 4918 | AGAAAAACUGAUCGUGUUGGGAU |
| BH0367 | No 4217 | CCAACACGAUCAGUUUUUCUU | No. 491 9 | AAGAAAAACUGAUCGUGUUGGGA |
| BH0369 | No. 4218 | AACACGAUCAGUUUUUCUUGU | No. 4920 | ACAAGAAAAACUGAUCGUGUUGG |
| BH0370 | No 4219 | ACACGAUCAGUUUUUCUUGUA | No. 4921 | UACAAGAAAAACUGAUCGUGUUG |
| BH0371 | No. 4220 | CACGAUCAGUUUUUCUUGUAA | No. 4922 | UUACAAGAAAAACUGAUCGUGUU |
| BH0372 | No 4221 | ACGAUCAGUUUUUCUUGUAAC | No. 4923 | GUUACAAGAAAAACUGAUCGUGU |
| BH0373 | No. 4222 | CGAUCAGUUUUUCUUGUAACA | No. 4924 | UGUUACAAGAAAAACUGAUCGUG |
| BH0375 | No. 4223 | AUCAGUUUUUCUUGUAACACU | No 4925 | AGUGUUACAAGAAAAACUGAUCG |
| BH0376 | No. 4224 | UCAGUUUUUCUUGUAACACUG | No. 4926 | CAGUGUUACMGMAAACUGAUC |
| BH0382 | No. 4225 | UUUCUUGUAACACUGGGUUUU | No. 4927 | AAAACCCAGUGUUACAAGAAAAA |
| BH0383 | No. 4226 | UUCUUGUAACACUGGGUUUUA | No. 4928 | UAAAACCCAGUGUUACAAGAAAA |
| BH0384 | No. 4227 | UCUUGUAACACUGGGUUUUAU | No. 4929 | AUAAAACCCAGUGUUACAAGAAA |
| BH0385 | No. 4228 | CUUGUAACACUGGGUUUUAUC | No. 4930 | GAUAAAACCGAGUGUUACAAGAA |
| BH0386 | No. 4229 | UUGUAACACUGGGUUUUAUCU | No. 4931 | AGAUAAAACCCAGUGUUACAAGA |
| BH0388 | No. 4230 | GUAACACUGGGUUUUAUCUGA | No. 4932 | UCAGAUAAAACCCAGUGUUACAA |
| BH0389 | No. 4231 | UAACACUGGGUUUUAUCUGAA | No. 4933 | UUCAGAUAAAACCCAGUGUUACA |
| BH0390 | No. 4232 | AACACUGGGUUUUAUCUGAAU | No. 4934 | AUUCAGAUAAAACCCAGUGUUAC |
| BH0392 | No. 4233 | CACUGGGUUUUAUCUGAAUGG | No. 4935 | CCAUUCAGAUAAAACCCAGUGUU |
| BH0394 | No. 4234 | CUGGGUUUUAUCUGAAUGGCG | No. 4936 | CGCCAUUCAGAUAAAACCCAGUG |
| BH0395 | No. 4235 | UGGGUUUUAUCUGAAUGGCGC | No. 4937 | GCGCCAUUCAGAUAAAACCCAGU |
| BH0396 | No. 4236 | GGGUUUUAUCUGAAUGGCGCU | No. 4938 | AGCGCCAUUCAGAUAAAACCCAG |
| BH0399 | No. 4237 | UUUUAUCUGAAUGGCGCUGAU | No. 4939 | AUCAGCGCCAUUCAGAUAAAACC |
| BH0402 | No. 4238 | UAUCUGAAUGGCGCUGAUUCU | No. 4940 | AGAAUCAGCGCCAU UCAGAUAAA |
| BH0406 | No. 4239 | UGAAUGGCGCUGAUUCUGCCA | No. 4941 | UGGCAGAAUCAGCGCCAUUCAGA |
| BH0409 | No. 4240 | AUGGCGCUGAUUCUGCCAAGU | No. 4942 | ACUUGGCAGAAUCAGCGCCAUUC |

### [Table 9]

**Table 2-7**

| Double stranded nucleic acid No. | SEQ ID NO. | Sense strand sequence (5'→3') | SEQ ID NO. | Antisense strand sequence (5'→3') |
|---|---|---|---|---|
| BH0410 | No. 4241 | UGGCGCUGAUUCUGCCAAGUG | No. 4943 | CACUUGGCAGAAUCAGCGCCAUU |
| BH0411 | No. 4242 | GGCGCUGAUUCUGCCAAGUGC | No. 4944 | GCACUUGGCAGAAUCAGCGCCAU |
| BH0412 | No. 4243 | GCGCUGAUUCUGCCAAGUGCA | No. 4945 | UGCACUUGGCAGAAUCAGCGCCA |
| BH0413 | No. 4244 | CGCUGAUUCUGCCAAGUGCAC | No. 4946 | GUGCACUUGGCAGAAUCAGCGCC |
| BH0414 | No. 4245 | GCUGAUUCUGCCAAGUGCACU | No. 4947 | AGUGCACUUGGCAGAAUCAGCGC |
| BH0415 | No. 4246 | CUGAUUCUGCCAAGUGCACUG | No. 4948 | CAGUGCACUUGGCAGAAUCAGCG |
| BH0419 | No. 4247 | UUCUGCCAAGUGCACUGAGGA | No 4949 | UCCUCAGUGCACUUGGCAGAAUC |
| BH0420 | No. 4248 | UCUGCCAAGUGCACUGAGGAA | No. 4950 | UUCCUCAGUGCACUUGGCAGAAU |
| BH0422 | No. 4249 | UGCCAAGUGCACUGAGGAAGG | No. 4951 | CCUUCCUCAGUGCACUUGGCAGA |
| BH0423 | No. 4250 | GCCAAGUGCACUGAGGAAGGA | No. 4952 | UCCUUCCUCAGUGCACUUGGCAG |
| BH0424 | No. 4251 | CCAAGUGCACUGAGGAAGGAA | No. 4953 | UUCCUUCCUCAGUGCACUUGGCA |
| BH0425 | No. 4252 | CAAGUGCACUGAGGAAGGAAA | No. 4954 | UUUCCUUCCUCAGUGCACUUGGC |
| BH0426 | No. 4253 | AAGUGCACUGAGGAAGGAAAA | No. 4955 | UUUUCCUUCCUCAGUGCACUUGG |
| BH0427 | No. 4254 | AGUGCACUGAGGAAGGAAAAU | No. 4956 | AUUUUCCUUCCUCAGUGCACUUG |
| BH0436 | No. 4255 | AGGAAGGAAAAUGGAGCCCGG | No. 4957 | CCGGGCUCCAUUUUCCUUCCUCA |
| BH0449 | No. 4266 | GAGCCCGGAGCUUCCUGUCUG | No. 4958 | CAGACAGGAAGCUCCGGGCUCCA |
| BH0450 | No. 4257 | AGCCCGGAGCUUCCUGUCUGU | No. 4959 | ACAGACAGGAAGCUCCGGGCUCC |
| BH0453 | No. 4258 | CCGGAGCUUCCUGUCUGUGCU | No. 4960 | AGCACAGACAGGAAGCUCCGGGC |
| BH0454 | No. 4259 | CGGAGCUUCCUGUCUGUGCUC | No. 4961 | GAGCACAGACAGGAAGCUCCGGG |
| BH0457 | No. 4260 | AGCUUCCUGUCUGUGCUCCCA | No. 4962 | UGGGAGCACAGACAGGAAGCUCC |
| BH0461 | No. 4261 | UCCUGUCUGUGCUCCCAUCAU | No. 4963 | AUGAUGGGAGCACAGACAGGAAG |
| BH0463 | No. 4262 | CUGUCUGUGCUCCCAUCAUCU | No. 4964 | AGAUGAUGGGAGCACAGACAGGA |
| BH0472 | No. 4263 | CUCCCAUCAUCUGCCCUCCAC | No. 4965 | GUGGAGGGCAGAUGAUGGGAGCA |
| BH0475 | No. 4264 | CCAUCAUCUGCCCUCCACCAU | No. 4966 | AUGGUGGAGGGCAGAUGAUGGGA |
| BH0476 | No. 4265 | CAUCAUCUGCCCUCCACCAUC | No. 4967 | GAUGGUGGAGGGCAGAUGAUGGG |
| BH0480 | No. 4266 | AUCUGCCCUCCACCAUCCAUA | No. 4968 | UAUGGAUGGUGGAGGGCAGAUGA |
| BH0484 | No. 4267 | GCCCUCCACCAUCCAUACCUA | No. 4969 | UAGGUAUGGAUGGUGGAGGGCAG |
| BH0487 | No. 4268 | CUCCACCAUCCAUACCUACGU | No. 4970 | ACGUAGGUAUGGAUGGUGGAGGG |
| BH0488 | No. 4269 | UCCACCAUCCAUACCUACGUU | No. 4971 | AACGUAGGUAUGGAUGGUGGAGG |
| BH0489 | No. 4270 | CCACCAUCCAUACCUACGUUU | No. 4972 | AAACGUAGGUAUGGAUGGUGGAG |
| BH0490 | No 4271 | CACCAUCCAUACCUACGUUUG | No. 4973 | CAAACGUAGGUAUGGAUGGUGGA |
| BH0491 | No. 4272 | ACCAUCCAUACCUACGUUUGC | No. 4974 | GCAAACGUAGGUAUGGAUGGUGG |
| BH0492 | No. 4273 | CCAUCCAUACCUACGUUUGCA | No. 4976 | UGCAAACGUAGGUAUGGAUGGUG |
| BH0493 | No. 4274 | CAUCCAUACCUACGUUUGCAA | No. 4976 | UUGCAAACGUAGGUAUGGAUGGU |
| BH0495 | No. 4275 | UCCAUACCUACGUUUGCAACA | No. 4977 | UGUUGCAAACGUAGGUAUGGAUG |
| BH0498 | No. 4276 | AUACCUACGUUUGCAACACUU | No. 4978 | AAGUGUUGCAAACGUAGGUAUGG |
| BH0499 | No. 4277 | UACCUACGUUUGCAACACUUC | No. 4979 | GAAGUGUUGCAAACGUAGGUAUG |
| BH0500 | No. 4278 | ACCUACGUUUGCAACACUUCG | No. 4980 | CGAAGUGUUGCAAACGUAGGUAU |
| BH0501 | No. 4279 | CCUACGUUUGCAACACUUCGU | No. 4981 | ACGAAGUGUUGCAAACGUAGGUA |
| BH0502 | No 4280 | CUACGUUUGCAACACUUCGUG | No. 4982 | CACGAAGUGUUGCAAACGUAGGU |

### [Table 10]

**Table 2-8**

| Double stranded nucleic acid No. | SEQ ID NO. | Sense strand sequence (5'→3') | SEQ ID NO. | Antisense strand sequence (5'→3') |
|---|---|---|---|---|
| BH0503 | No. 4281 | UACGUUUGCAACACUUCGUGU | No. 4983 | ACACGAAGUGUUGCAAACGUAGG |
| BH0504 | No. 4282 | ACGUUUGCAACACUUCGUGUU | No. 4984 | AACAGGAAGUGUUGCAAACGUAG |
| BH0505 | No. 4283 | CGUUUGCAACACUUCGUGUUU | No. 4985 | AAACACGAAGUGUUGCAAACGUA |
| BH0506 | No. 4284 | GUUUGCAACACUUCGUGUUUA | No. 4986 | UAAACACGAAGUGUUGCAAACGU |
| BH0507 | No. 4285 | UUUGCAACACUUCGUGUUUAU | No. 4987 | AUAAACACGAAGUGUUGCAAACG |
| BH0508 | No. 4286 | UUGCAACACUUCGUGUUUAUA | No. 4988 | UAUAAACACGAAGUGUUGCAAAC |
| BH0509 | No. 4287 | UGCAACACUUCGUGUUUAUAA | No. 4989 | UUAUAAACACGAAGUGUUGCAAA |
| BH0510 | No. 4288 | GCAACACUUCGUGUUUAUAAG | No. 4990 | CUUAUAAACACGAAGUGUUGCAA |
| BH0513 | No 4289 | ACACUUCGUGUUUAUAAGCCA | No. 4991 | UGGCUUAUAAACACGAAGUGUUG |
| BH0514 | No. 4290 | CACUUCGUGUUUAUAAGCCAU | No. 4992 | AUGGCUUAUAAACACGAAGUGUU |
| RH0518 | No. 4291 | UCGUGUUUAUAAGCCAUCAGC | No. 4993 | GCUGAUGGCUUAUAAACACGAAG |
| BH0519 | No. 4292 | CGUGUUUAUAAGCCAUCAGCU | No. 4994 | AGCUGAUGGCUUAUAAACACGAA |
| BH0520 | No. 4293 | GUGUUUAUAAGCCAUCAGCU G | No. 4995 | CAGCUGAUGGCUUAUAAACACGA |
| BH0526 | No. 4294 | AUAAGCCAUCAGCUGGAAACA | No. 4996 | UGUUUCCAGCUGAUGGCUUAUAA |
| BH0527 | No. 4295 | UAAGCCAUCAGCUGGAAACAA | No. 4997 | UUGUUUCCAGCUGAUGGCUUAUA |
| BH0528 | No. 4296 | AAGCCAUCAGGCUGGAAACAAU | No 4998 | AUUGUUUCCAGCUGAUGGCUUAU |
| BH0529 | No. 4297 | AGCCAUCAGCUGGAAACAAUU | No. 4999 | AAUUGUUUCCAGCUGAUGGCUUA |
| BH0530 | No. 4298 | GCCAUCAGCUGGAAACAAUUC | No. 5000 | GAAUUGUUUCCAGCUGAUGGCUU |
| BH0534 | No. 4299 | UCAGCUGGAAACAAUUCCCUC | No. 5001 | GAGGGAAUUGUUUCCAGCUGAUG |
| BH0535 | No. 4300 | CAGCUGGAAACAAUUCCCUCU | No. 5002 | AGAGGGAAUUGUUUCCAGCUGAU |
| BH0538 | No. 4301 | CUGGAAACAAUUCCCUCUAUC | No. 5003 | GAUAGAGGGAAUUGUUUCCAGCU |
| BH0542 | No. 4302 | AAACAAUUCCCUCUAUCGGGA | No. 5004 | UCCCGAUAGAGGGAAUUGUUUCC |
| BH0549 | No. 4303 | UCCCUCUAUCGGGACACAGCA | No. 5005 | UGCUGUGUCCCGAUAGAGGGAAU |
| BH0552 | No. 4304 | CUCUAUCGGGACACAGCAGUU | No. 5006 | AACUGCUGUGUCCCGAUAGAGGG |
| BH0553 | No. 4305 | UCUAUCGGGACACAGCAGUUU | No. 5007 | AAACUGCUGUGUCCCGAUAGAGG |
| BH0554 | No. 4306 | CUAUCGGGACACAGCAGUUUU | No. 5008 | AAAACUGCUGUGUCCCGAUAGAG |
| BH0555 | No. 4307 | UAUCGGGACACAGCAGUUUUU | No. 5009 | AAAAACUGCUGUGUCCCGAUAGA |
| BH0557 | No. 4308 | UCGGGACACAGCAGUUUUUGA | No. 5010 | UCAAAAACUGCUGUGUCCCGAUA |
| BH0558 | No. 4309 | CGGGACACAGCAGUUUUUGAA | No. 5011 | UUCAAAAACUGCUGUGUCCCGAU |
| BH0559 | No. 4310 | GGGACACAGCAGUUUUUGAAU | No. 5012 | AUUCAAAAACUGCUGUGUCCCGA |
| BH0561 | No. 4311 | GACACAGCAGUUUUUGAAUGU | No. 5013 | ACAUUCAAAAACUGCUGUGUCCC |
| BH0562 | No. 4312 | ACACAGCAGUUUUUGAAUGUU | No 5014 | AACAUUCAAAAACUGCUGUGUCC |
| BH0563 | No. 4313 | CACAGCAGUUUUUGAAUGUUU | No. 5015 | AAACAUUCAAAAACUGCUGUGUC |
| BH0564 | No. 4314 | ACAGCAGUUUUUGAAUGUUUG | No. 5016 | CAAACAUUCAAAAACUGCUGUGU |
| BH0565 | No. 4315 | CAGCAGUUUUUGAAUGUUUGC | No. 5017 | GCAAACAUUCAAAAACUGCUGUG |
| BH0567 | No. 4316 | GCAGUUUUUGAAUGUUUGCCA | No. 501 8 | UGGCAAACAUUCAAAAACUGCUG |
| BH0568 | No. 4317 | CAGUUUUUGAAUGUUUGCCAC | No. 5019 | GUGGCAAACAUUCAAAAACUGCU |
| BH0570 | No. 4318 | GUUUUUGAAUGUUUGCCACAA | No. 5020 | UUGUGGCAAACAUUCAAAAACUG |
| BH0572 | No. 4319 | UUUUGAAUGUUUGCCACAACA | No. 5021 | UGUUGUGGCAAACAUUCAAAAAC |
| BH0573 | No. 4320 | UUUGAAUGUUUGCCACAACAU | No. 5022 | AUGUUGUGGCAAACAUUCAAAAA |

### [Table 11]

**Table 2-9**

| Double stranded nucleic acid No. | SEQ ID NO. | Sense strand sequence (5'→3') | SEQ ID NO. | Antisense strand sequence (5'→3') |
|---|---|---|---|---|
| BH0574 | No. 4321 | UUGAAUGUUUGCCACAACAUG | No. 5023 | CAUGUUGUGGCAAACAUUCAAAA |
| BH0578 | No. 4322 | AUGUUUGCCACAACAUGCGAU | No. 5024 | AUCGCAUGUUGUGGCAAACAUUC |
| BH0582 | No. 4323 | UUGCCACAACAUGCGAUGUUU | No. 5025 | AAACAUCGCAUGUUGUGGCAAAC |
| BH0583 | No 4324 | UGCCACAACAUGCGAUGUUUG | No. 5026 | CAAACAUCGCAUGUUGUGGCAAA |
| BH0585 | No. 4325 | CCACAACAUGCGAUGUUUGGA | No. 5027 | UCCAAACAUCGCAUGUUGUGGCA |
| BH0586 | No. 4326 | CACAACAUGCGAUGUUUGGAA | No. 5028 | UUCCAAACAUCGCAUGUUGUGGC |
| BH0588 | No. 4327 | CAACAUGCGAUGUUUGGAAAU | No. 5029 | AUUUCCAAACAUCGCAUGUUGUG |
| BH0590 | No. 4328 | ACAUGCGAUGUUUGGAAAUGA | No. 5030 | UCAUUUCCAAACAUCGCAUGUUG |
| BH0591 | No. 4329 | CAUGCGAUGUUUGGAAAUGAU | No. 5031 | AUCAUUUCCAAACAUCGCAUGUU |
| BH0592 | No. 4330 | AUGCGAUGUUUGGAAAUGAUA | No. 5032 | UAUCAUUUCCAAACAUCGCAUGU |
| BH0593 | No. 4331 | UGCGAUGUUUGGAAAUGAUAC | No. 5033 | GUAUCAUUUCCAAACAUCGCAUG |
| BH0594 | No. 4332 | GCGAUGUUUGGAAAUGAUACA | No. 5034 | UGUAUCAUUUCCAAACAUCGCAU |
| BH0595 | No. 4333 | CGAUGUUUGGAAAUGAUACAA | No. 5035 | UUGUAUCAUUUCCAAACAUCGCA |
| BH0597 | No. 4334 | AUGUUUGGAAAUGAUACAAUU | No. 5036 | AAUUGUAUCAUUUCCAAACAUCG |
| BH0598 | No. 4335 | UGUUUGGAAAUGAUACAAUUA | No. 5037 | UAAUUGUAUCAUUUCCAAACAUC |
| BH0601 | No. 4336 | UUGGAAAUGAUACAAUUACCU | No. 5038 | AGGUAAUUGUAUCAUUUCCAAAC |
| BH0607 | No. 4337 | AUGAUACAAUUACCUGCACGA | No. 5039 | UCGUGCAGGUAAUUGUAUCAUUU |
| BH0609 | No. 4338 | GAUACAAUUACCUGCACGACA | No. 5040 | UGUCGUGCAGGUAAUUGUAUCAU |
| BH0610 | No. 4339 | AUACAAUUACCUGCACGACAC | No. 5041 | GUGUCGUGCAGGUAAUUGUAUCA |
| BH0611 | No. 4340 | UACAAUUACCUGCACGACACA | No. 5042 | UGUGUCGUGCAGGUAAUUGUAUC |
| BH0612 | No. 4341 | ACAAUUACCUGCACGACACAU | No. 5043 | AUGUGUCGUGCAGGUAAUUGUAU |
| BH0616 | No. 4342 | UUACCUGCACGACACAUGGAA | No. 5044 | UUCCAUGUGUCGUGCAGGUAAUU |
| BH0617 | No. 4343 | UACCUGCACGACACAUGGAAA | No. 5045 | UUUCCAUGUGUCGUGCAGGUAAU |
| BH0618 | No. 4344 | ACCUGCACGACACAUGGAAAU | No. 5046 | AUUUCCAUGUGUCGUGCAGGUAA |
| BH0622 | No. 4345 | GCACGACACAUGGAAAUUGGA | No. 5047 | UCCAAUUUCCAUGUGUCGUGCAG |
| BH0624 | No. 4346 | ACGACACAUGGAAAUUGGACU | No. 5048 | AGUCCAAUUUCCAUGUGUCGUGC |
| BH0625 | No. 4347 | CGACACAUGGAAAUUGGACUA | No. 5049 | UAGUCCAAUUUCCAUGUGUCGUG |
| BH0627 | No. 4348 | ACACAUGGAAAUUGGACUAAA | No. 5050 | UUUAGUCCAAUUUCCAUGUGUCG |
| BH0628 | No. 4349 | CACAUGGAAAUUGGACUAAAU | No. 5061 | AUUUAGUCCAAUUUCCAUGUGUC |
| BH0629 | No. 4350 | ACAUGGAAAUUGGACUAAAUU | No. 5052 | AAUUUAGUCCAAUUUCCAUGUGU |
| BH0630 | No. 4351 | CAUGGAAAUUGGACUAAAUUA | No. 5053 | UAAUUUAGUCCAAUUUCCAUGUG |
| BH0631 | No. 4352 | AUGGAAAUUGGACUAAAUUAC | No. 5064 | GUAAUUUAGUCCAAUUUCCAUGU |
| BH0633 | No. 4353 | GGAAAUUGGACUAAAUUACCA | No. 5055 | UGGUAAUUUAGUCCAAUUUCCAU |
| BH0638 | No. 4354 | UUGGACUAAAUUACCAGAAUG | No. 5056 | CAUUCUGGUAAUUUAGUCCAAUU |
| BH0649 | No. 4355 | UACCAGAAUGCAGGGAAGUAA | No. 5057 | UUACUUCCCUGCAUUCUGGUAAU |
| BH0650 | No. 4356 | ACCAGAAUGCAGGGAAGUAAA | No. 5058 | UUUACUUCCCUGCAUUCUGGUAA |
| BH0651 | No. 4357 | CCAGAAUGCAGGGAAGUAAAA | No. 5059 | UUUUACUUCCCUGCAUUCUGGUA |
| BH0652 | No. 4358 | CAGAAUGCAGGGAAGUAAAAU | No. 5060 | AUUUUACUUCCCUGCAUUCUGGU |
| BH0653 | No. 4359 | AGAAUGCAGGGAAGUAAAAUG | No. 5061 | CAUUUUACUUCCCUGCAUUCUGG |
| BH0657 | No. 4360 | UGCAGGGAAGUAAAAUGCCCA | No. 5062 | UGGGCAUUUUACUUCCCUGCAUU |

### [Table 12]

**Table 2-10**

| Double stranded nucleic acid No. | SEQ ID NO. | Sense strand sequence (5'→3') | SEQ ID NO. | Antisense strand sequence (5'→3') |
|---|---|---|---|---|
| BH0661 | No. 4361 | GGGAAGUAAAAUGCCCAUUCC | No. 5063 | GGAAUGGGCAUUUUACUUCCCUG |
| BH0664 | No. 4362 | AAGUAAAAUGCCCAUUCCCAU | No. 5064 | AUGGGAAUGGGCAUUUUACUUCC |
| BH0669 | No. 4363 | AAAUGCCCAUUCCCAUCAAGA | No. 5065 | UCUUGAUGGGAAUGGGCAUUUUA |
| BH0670 | No. 4364 | AAUGCCCAUUCCCAUCAAGAC | No. 5066 | GUCUUGAUGGGAAUGGGCAUUUU |
| BH0672 | No. 4365 | UGCCCAUUCCCAUCAAGACCA | No. 5067 | UGGUCUUGAUGGGAAUGGGCAUU |
| BH0673 | No. 4366 | GCCCAUUCCCAUCAAGACCAG | No. 5068 | CUGGUCUUGAUGGGAAUGGGCAU |
| BH0674 | No. 4367 | CCCAUUCCCAUCAAGACCAGA | No. 5069 | UCUGGUCUUGAUGGGAAUGGGCA |
| BH0678 | No. 4368 | UUCCCAUCAAGACCAGACAAU | No. 5070 | AUUGUCUGGUCUUGAUGGGAAUG |
| BH0679 | No. 4369 | UCCCAUCAAGACCAGACAAUG | No. 5071 | CAUUGUCUGGUCUUGAUGGGAAU |
| BH0680 | No 4370 | CCCAUCAAGACCAGACAAUGG | No. 5072 | CCAUUGUCUGGUCUUGAUGGGAA |
| BH0681 | No. 4371 | CCAUCAAGACCAGACAAUGGA | No. 5073 | UCCAUUGUCUGGUCUUGAUGGGA |
| BH0682 | No. 4372 | CAUCAAGACCAGACAAUGGAU | No. 5074 | AUCCAUUGUCUGGUCUUGAUGGG |
| BH0683 | No. 4373 | AUCAAGACCAGACAAUGGAUU | No. 5075 | AAUCCAUUGUCUGGUCUUGAUGG |
| BH0684 | No. 4374 | UCAAGACCAGACAAUGGAUUU | No. 5076 | AAAUCCAUUGUCUGGUCUUGAUG |
| BH0685 | No. 4375 | CAAGACCAGACAAUGGAUUUG | No. 5077 | CAAAUCCAUUGUCUGGUCUUGAU |
| BH0687 | No. 4376 | AGACCAGACAAUGGAUUUGUG | No. 5078 | CACAAAUCCAUUGUCUGGUCUUG |
| BH0688 | No. 4377 | GACCAGACAAUGGAUUUGUGA | No. 5079 | UCACAAAUCCAUUGUCUGGUCUU |
| BH0689 | No. 4378 | ACCAGACAAUGGAUUUGUGAA | No. 5080 | UUCACAAAUCCAUUGUCUGGUCU |
| BH0690 | No. 4379 | CCAGACAAUGGAUUUGUGAAC | No. 5081 | GUUCACAAAUCCAUUGUCUGGUC |
| BH0692 | No. 4380 | AGACAAUGGAUUUGUGAACUA | No. 5082 | UAGUUCACAAAUCCAUUGUCUGG |
| BH0693 | No. 4381 | GACAAUGGAUUUGUGAACUAU | No. 5083 | AUAGUUCACAAAUCCAUUGUCUG |
| BH0694 | No. 4382 | ACAAUGGAUUUGUGAACUAUC | No. 5084 | GAUAGUUCACAAAUCCAUUGUCU |
| BH0697 | No. 4383 | AUGGAUUUGUGAACUAUCCUG | No. 5085 | CAGGAUAGUUCACAAAUCCAUUG |
| BH0699 | No. 4384 | GGAUUUGUGAACUAUCCUGCA | No. 5086 | UGCAGGAUAGUUCACAAAUCCAU |
| BH0700 | No. 4385 | GAUUUGUGAACUAUCCUGCAA | No. 5087 | UUGCAGGAUAGUUCACAAAUCCA |
| BH0701 | No. 4386 | AUUUGUGAACUAUCCUGCAAA | No. 5088 | UUUGCAGGAUAGUUCACAAAUCC |
| BH0702 | No. 4387 | UUUGUGAACUAUCCUGCAAAA | No. 5089 | UUUUGCAGGAUAGUUCACAAAUC |
| BH0705 | No. 4388 | GUGAACUAUCCUGCAAAACCA | No. 5090 | UGGUUUUGCAGGAUAGUUCACAA |
| BH0706 | No. 4389 | UGAACUAUCCUGCAAAACCAA | No. 5091 | UUGGUUUUGCAGGAUAGUUCACA |
| BH0708 | No. 4390 | AACUAUCCUGCAAAACCAACA | No. 5092 | UGUUGGUUUUGCAGGAUAGUUCA |
| BH0709 | No. 4391 | ACUAUCCUGCAAAACCAACAC | No. 5093 | GUGUUGGUUUUGCAGGAUAGUUC |
| BH0711 | No. 4392 | UAUCCUGCAAAACCAACACUU | No. 5094 | AAGUGUUGGUUUUGCAGGAUAGU |
| BH0712 | No. 4393 | AUCCUGCAAAACCAACACUUU | No. 5095 | AAAGUGUUGGUUUUGCAGGAUAG |
| BH0713 | No. 4394 | UCCUGCAAAACCAACACUUUA | No. 5096 | UAAAGUGUUGGUUUUGCAGGAUA |
| BH0714 | No. 4395 | CCUGCAAAACCAACACUUUAU | No. 5097 | AUAAAGUGUUGGUUUUGCAGGAU |
| BH0715 | No. 4396 | CUGCAAAACCAACACUUUAUU | No. 5098 | AAUAAAGUGUUGGUUUUGCAGGA |
| BH0716 | No. 4397 | UGCAAAACCAACACUUUAUUA | No. 6099 | UAAUAAAGUGUUGGUUUUGCAGG |
| BH0718 | No. 4398 | CAAAACCAACACUUUAUUACA | No. 5100 | UGUAAUAAAGUGUUGGUUUUGCA |
| BH0719 | No. 4399 | AAAACCAACACUUUAUUACAA | No. 5101 | UUGUAAUAAAGUGUUGGUUUUGC |
| BH0721 | No. 4400 | AACCAACACUUUAUUACAAGG | No. 5102 | CCUUGUAAUAAAGUGUUGGUUUU |

### [Table 13]

**Table 2-11**

| Double stranded nucleic acid No. | SEQ ID NO. | Sense strand sequence (5'→3') | SEQ ID NO. | Antisense strand sequence (5'→3') |
|---|---|---|---|---|
| SH0723 | No. 4401 | CCAACACUUUAUUACAAGGAU | No. 5103 | AUCCUUGUAAUAAAGUGUUGGUU |
| BH0724 | No. 4402 | CAACACUUUAUUACAAGGAUA | No. 5104 | UAUCCUUGUAAUAAAGUGUUGGU |
| BH0725 | No. 4403 | AACACUUUAUUACAAGGAUAA | No. 5105 | UUAUCCUUGUAAUAAAGUGUUGG |
| BH0735 | No. 4404 | UACAAGGAUAAAGCCACAUUU | No. 5106 | AAAUGUGGCUUUAUCCUUGUAAU |
| 8H0737 | No. 4405 | CAAGGAUAAAGCCACAUUUGG | No. 5107 | CCAAAUGUGGCUUUAUCCUUGUA |
| BH0739 | No. 4406 | AGGAUAAAGCCACAUUUGGCU | No. 5108 | AGCCAAAUGUGGCUUUAUCCUUG |
| BH0743 | No. 4407 | UAAAGCCACAUUUGGCUGCCA | No. 5109 | UGGCAGCCAAAUGUGGCUUUAUC |
| BH0744 | No. 4408 | AAAGCCACAUUUGGCUGCCAU | No. 5110 | AUGGCAGCCAAAUGUGGCUUUAU |
| BH0745 | No. 4409 | AAGCCACAUUUGGCUGCCAUG | No. 6111 | CAUGGCAGCCAAAUGUGGCUUUA |
| 8H0746 | No. 4410 | AGCCACAUUUGGCUGCCAUGA | No. 5112 | UCAUGGCAGCCAAAUGUGGCUUU |
| BH0747 | No 4411 | GCCACAUUUGGCUGCCAUGAU | No. 5113 | AUCAUGGCAGCCAAAUGUGGCUU |
| BH0748 | No. 4412 | CCACAUUUGGCUGCCAUGAUG | No. 5114 | CAUCAUGGCAGCCAAAUGUGGCU |
| 8H0749 | No. 4413 | CACAUUUGGCUGCCAUGAUGG | No. 5115 | CCAUCAUGGCAGCCAAAUGUGGC |
| BH0750 | No. 4414 | ACAUUUGGCUGCCAUGAUGGA | No. 5116 | UCCAUCAUGGCAGCCAAAUGUGG |
| BH0753 | No. 4415 | UUUGGCUGCCAUGAUGGAUAU | No. 5117 | AUAUCCAUCAUGGCAGCCAAAUG |
| BH0754 | No. 4416 | UUGGCUGCCAUGAUGGAUAUU | No. 5118 | AAUAUCCAUCAUGGCAGCCAAAU |
| BH0756 | No. 4417 | GGCUGCCAUGAUGGAUAUUCU | No. 5119 | AGAAUAUCCAUCAUGGCAGCCAA |
| BH0757 | No. 4418 | GCUGCCAUGAUGGAUAUUCUC | No. 5120 | GAGAAUAUCCAUCAUGGCAGCCA |
| BH0759 | No. 4419 | UGCCAUGAUGGAUAUUCUCUG | No. 5121 | CAGAGAAUAUCCAUCAUGGCAGC |
| BH0760 | No. 4420 | GCCAUGAUGGAUAUUCUCUGG | No. 5122 | CCAGAGAAUAUCCAUCAUGGCAG |
| BH0762 | No. 4421 | CAUGAUGGAUAUUCUCUGGAU | No. 5123 | AUCCAGAGAAUAUCCAUCAUGGC |
| BH0763 | No. 4422 | AUGAUGGAUAUUCUCUGGAUG | No. 5124 | CAUCCAGAGAAUAUCCAUCAUGG |
| BH0766 | No. 4423 | AUGGAUAUUCUCUGGAUGGCC | No. 5125 | GGCCAUCCAGAGAAUAUCCAUCA |
| BH0771 | No. 4424 | UAUUCUCUGGAUGGCCCGGAA | No. 5126 | UUCCGGGCCAUCCAGAGAAUAUC |
| BH0773 | No. 4425 | UUCUCUGGAUGGCCCGGAAGA | No. 5127 | UCUUCCGGGCCAUCCAGAGAAUA |
| BH0774 | No. 4426 | UCUCUGGAUGGCCCGGAAGAA | No. 5128 | UUCUUCCGGGCCAUCCAGAGAAU |
| BH0775 | No. 4427 | CUCUGGAUGGCCCGGAAGAAA | No. 5129 | UUUCUUCCGGGCCAUCCAGAGAA |
| BH0776 | No. 4428 | UCUGGAUGGCCCGGAAGAAAU | No. 5130 | AUUUCUUCCGGGCCAUCCAGAGA |
| BH0777 | No. 4429 | CUGGAUGGCCCGGAAGAAAUA | No. 5131 | UAUUUCUUCCGGGCCAUCCAGAG |
| BH0779 | No. 4430 | GGAUGGCCCGGAAGAAAUAGA | No. 5132 | UCUAUUUCUUCCGGGCCAUCCAG |
| BH0780 | No. 4431 | GAUGGCCCGGAAGAAAUAGAA | No. 5133 | UUCUAUUUCUUCCGGGCCAUCCA |
| BH0781 | No. 4432 | AUGGCCCGGAAGAAAUAGAAU | No. 5134 | AUUCUAUUUCUUCCGGGCCAUCC |
| BH0782 | No. 4433 | UGGCCCGGAAGAAAUAGAAUG | No. 5135 | CAUUCUAUUUCUUCCGGGCCAUC |
| BH0783 | No. 4434 | GGCCCGGAAGAAAUAGAAUGU | No. 6136 | ACAUUCUAUUUCUUCCGGGCCAU |
| BH0785 | No. 4435 | CCCGGAAGAAAUAGAAUGUAC | No. 5137 | GUACAUUCUAUUUCUUCCGGGCC |
| BH0786 | No. 4436 | CCGGAAGAAAUAGAAUGUACC | No. 5138 | GGUACAUUCUAUUUCUUCCGGGC |
| BH01S7 | No. 4437 | CGGAAGAAAUAGAAUGUACCA | No. 5139 | UGGUACAUUCUAUUUCUUCCGGG |
| BH0790 | No. 4438 | AAGAAAUAGAAUGUACCAAAC | No. 5140 | GUUUGGUACAUUCUAUUUCUUCC |
| BH0795 | No. 4439 | AUAGAAUGUACCAAACUGGGA | No. 5141 | UCCCAGUUUGGUACAUUCUAUUU |
| BH0796 | No. 4440 | UAGAAUGUACCAAACUGGGAA | No. 5142 | UUCCCAGUUUGGUACAUUCUAUU |

### [Table 14]

**Table 2-12**

| Double stranded nucleic acid No. | SEQ ID NO. | Sense strand sequence (5'→3') | SEQ ID NO. | Antisense strand sequence (5'→3') |
|---|---|---|---|---|
| BH0797 | No. 4441 | AGAAUGUACCAAACUGGGAAA | No. 5143 | UUUCCCAGUUUGGUACAUUCUAU |
| BH0800 | No. 4442 | AUGUACCAAACUGGGAAACUG | No. 5144 | CAGUUUCCCAGUUUGGUACAUUC |
| BH0804 | No. 4443 | ACCAAACUGGGAAACUGGUCU | No. 5145 | AGACCAGUUUCCCAGUUUGGUAC |
| BH0805 | No. 4444 | CCAAACUGGGAAACUGGUCUG | No. 5146 | CAGACCAGUUUCCCAGUUUGGUA |
| BH0808 | No. 4445 | AACUGGGAAACUGGUCUGCCA | No. 5147 | UGGCAGACCAGUUUCCCAGUUUG |
| BH0809 | No. 4446 | ACUGGGAAACUGGUCUGCCAU | No. 5148 | AUGGCAGACCAGUUUCCCAGUUU |
| BH0810 | No. 4447 | CUGGGAAACUGGUCUGCCAUG | No. 5149 | CAUGGCAGACCAGUUUCCCAGUU |
| BH0811 | No 4448 | UGGGAAACUGGUCUGCCAUGC | No. 6160 | GCAUGGCAGACCAGUUUCCCAGU |
| BH0813 | No. 4449 | GGAAACUGGUCUGCCAUGCCA | No. 5151 | UGGCAUGGCAGACCAGUUUCCCA |
| BH0814 | No. 4450 | GAAACUGGUCUGCCAUGCCAA | No. 6152 | UUGGCAUGGCAGACCAGUUUCCC |
| BH0815 | No. 4451 | AAACUGGUCUGCCAUGCCAAG | No. 5153 | CUUGGCAUGGCAGACCAGUUUCC |
| BH0816 | No. 4452 | AACUGGUCUGCCAUGCCAAGU | No. 5154 | ACUUGGCAUGGCAGACCAGUUUC |
| BH0817 | No. 4453 | ACUGGUCUGCCAUGCCAAGUU | No. 5155 | AACUUGGCAUGGCAGACCAGUUU |
| BH0818 | No. 4454 | CUGGUCUGCCAUGCCAAGUUG | No. 5156 | CAACUUGGCAUGGCAGACCAGUU |
| BH0819 | No. 4455 | UGGUCUGCCAUGCCAAGUUGU | No. 5157 | ACAACUUGGCAUGGCAGACCAGU |
| BH0820 | No. 4456 | GGUCUGCCAUGCCAAGUUGUA | No. 6158 | UACAACUUGGCAUGGCAGACCAG |
| BH0821 | No. 4457 | GUCUGCCAUGCCAAGUUGUAA | No. 5159 | UUACAACUUGGCAUGGCAGACCA |
| BH0822 | No. 4458 | CUGCCAUGCCAAGUUGUAAA | No. 5160 | UUUACAACUUGGCAUGGCAGACC |
| BH0823 | No. 4459 | OUGCCAUGCCAAGUUGUAAAG | No. 5161 | CUUUACAACUUGGCAUGGCAGAC |
| BH0825 | No. 4460 | GGGAUGCCAAGUUGUAAAGCA | No. 5162 | UGCUUUACAACUUGGCAUGGCAG |
| BH0826 | No. 4461 | CCAUGCCAAGUUGUAAAGCAU | No. 5163 | AUGCUUUACAACUUGGCAUGGCA |
| BH0827 | No. 4462 | CAUGCCAAGUUGUAAAGCAUC | No. 5164 | GAUGCUUUACAACUUGGCAUGGC |
| BH0828 | No. 4463 | AUGCCAAGUUGUAAAGCAUCU | No. 5165 | AGAUGCUUUACAACUUGGCAUGG |
| BH0829 | No. 4464 | UGCCAAGUUGUAAAGCAUCUU | No. 5166 | AAGAUGCUUUACAACUUGGCAUG |
| BM0830 | No. 4465 | GCCAAGUUGUAAAGCAUCUUG | No. 5167 | CAAGAUGCUUUACAACUUGGCAU |
| BH0831 | No. 4466 | CCAAGUUGUAAAGCAUCUUGU | No. 5168 | ACAAGAUGCUUUACAACUUGGCA |
| BH0832 | No. 4467 | CAAGUUGUAAAGCAUCUUGUA | No. 5169 | UACAAGAUGCUUUACAACUUGGC |
| BH0833 | No. 4468 | AAGUUGUAAAGGAUCUUGUAA | No. 5170 | UUACAAGAUGCUUUACAACUUGG |
| BH0834 | No. 4469 | AGUUGUAAAGCAUCUUGUAAA | No. 5171 | UUUACAAGAUGCUUUACAACUUG |
| BH0835 | No. 4470 | GUUGUAAAGCAUCUUGUAAAG | No. 5172 | CUUUACAAGAUGCUUUACAACUU |
| BH0837 | No. 4471 | UGUAAAGCAUCUUGUAAAGUA | No. 5173 | UACUUUACAAGAUGCUUUACAAC |
| BH0838 | No. 4472 | GUAAAGCAUCUUGUAAAGUAC | No. 5174 | GUACUUUACAAGAUGCUUUACAA |
| BH0840 | No. 4473 | AAAGCAUCUUGUAAAGUACCU | No. 5175 | AGGUACUUUACAAGAUGCUUUAC |
| BH0842 | No. 4474 | AGCAUCUUGUAAAGUACCUGU | No. 6176 | ACAGGUACUUUACAAGAUGCUUU |
| BH0845 | No. 4475 | AUCUUGUAAAGUACCUGUGAA | No. 5117 | UUCACAGGUACUUUACAAGAUGC |
| BH0846 | No. 4476 | UCUUGUAAAGUACCUGUGAAA | No. 5178 | UUUCACAGGUACUUUACAAGAUG |
| BH0847 | No. 4477 | CUUGUAAAGUACCUGUGAAAA | No. 5179 | UUUUCACAGGUACUUUACAAGAU |
| BH0852 | No. 4478 | AAAGUACCUGUGAAAAAAGCC | No. 5180 | GGCUUUUUUCACAGGUACUUUAC |
| BH0853 | No. 4479 | AAGUACCUGUGAAAAAAGCCA | No. 5181 | UGGCUUUUUUCACAGGUACUUUA |
| BH0854 | No. 4480 | AGUACCUGUGAAAAAAGCCAC | No. 5182 | GUGGCUUUUUUCACAGGUACUUU |

### [Table 15]

**Table 2-13**

| Double stranded nucleic acid No. | SEQ ID NO. | Sense strand sequence (5'→3') | SEQ ID NO. | Antisense strand sequence (5'→3') |
|---|---|---|---|---|
| BH0855 | No. 4481 | GUACCUGUGAAAAAAGCCACU | No. 5183 | AGUGGCUUUUUUCACAGGUACUU |
| BH0857 | No. 4482 | ACCUGUGAAAAAAGCCACUGU | No. 6184 | ACAGUGGCUUUUUUCACAGGUAC |
| BH0858 | No. 4483 | CCUGUGAAAAAAGCCACUGUG | No. 5185 | CACAGUGGCUUUUUUCACAGGUA |
| BH0859 | No. 4484 | CUGUGAAAAAAGCCACUGUGG | No. 5186 | CCACAGUGGCUUUUUUCACAGGU |
| BH0860 | No. 4485 | UGUGAAAAAAGCCACUGUGGU | No. 5187 | ACCACAGUGGCUUUUUUCACAGG |
| BH0861 | No. 4486 | GUGAAAAAAGCCACUGUGGUG | No. 5188 | CACCACAGUGGCUUUUUUCACAG |
| BH0863 | No. 4487 | GAAAAAAGCCACUGUGGUGUA | No. 5189 | UACACCACAGUGGCUUUUUUCAC |
| BH0864 | No. 4488 | AAAAAAGCCACUGUGGUGUAC | No. 6190 | GUACACCACAGUGGCUUUUUUCA |
| BH0866 | No. 4489 | AAAAGCCACUGUGGUGUACCA | No. 5191 | UGGUACACCACAGUGGCUUUUUU |
| BH0861 | No. 4490 | AAAGCCACUGUGGUGUACCAA | No. 5192 | UUGGUACACCACAGUGGCUUUUU |
| BH0868 | No. 4491 | AAGCCACUGUGGUGUACCAAG | No. 5193 | CUUGGUACACCACAGUGGCUUUU |
| BH0810 | No 4492 | GCCACUGU GGU GUACCAAGGA | No. 5194 | UCCUUGGUACACCACAGUGGCUU |
| BH0872 | No. 4493 | CACUGUGGUGUACCAAGGAGA | No. 5195 | UCUCCUUGGUACACCACAGUGGC |
| BH0873 | No. 4494 | ACUGUGGUGUACCAAGGAGAG | No. 5196 | CUCUCCUUGGUACACCACAGUGG |
| BH0874 | No. 4495 | CUGUGGUGUACCAAGGAGAGA | No. 5197 | UCUCUCCUUGGUACACCACAGUG |
| BH0875 | No. 4496 | UGUGGUGUACCAAGGAGAGAG | No. 5198 | CUCUCUCCUUGGUACACCACAGU |
| BH0876 | No. 4497 | GUGGUGUACCAAGGAGAGAGA | No. 5199 | UCUCUCUCCUUGGUACACCACAG |
| BH0877 | No. 4498 | UGGUGUACCAAGGAGAGAGAG | No. 5200 | CUCUCUCUCCUUGGUACACCACA |
| BH0879 | No. 4499 | GUGUACCAAGGAGAGAGAGUA | No. 5201 | UACUCUCUCUCCUUGGUACACCA |
| BH0880 | No. 4500 | UGUACCAAGGAGAGAGAGUAA | No. 5202 | UUACUCUCUCUCCUUGGUACACC |
| BH0881 | No. 4501 | GUACCAAGGAGAGAGAGUAAA | No. 5203 | UUUACUCUCUCUCCUUGGUACAC |
| BH0882 | No. 4502 | UACCAAGGAGAGAGAGUAAAG | No. 5204 | CUUUACUCUCUCUCCUUGGUACA |
| BH0883 | No. 4503 | ACCAAGGAGAGAGAGUAAAGA | No. 5205 | UCUUUACUCUCUCUCCUUGGUAC |
| BH0884 | No. 4504 | CCAAGGAGAGAGAGUAAAGAU | No. 5206 | AUCUUUACUCUCUCUCCUUGGUA |
| BH0885 | No. 4505 | CAAGGAGAGAGAGUAAAGAUU | No. 5207 | AAUCUUUACUCUCUCUCCUUGGU |
| BH0886 | No. 4506 | AAGGAGAGAGAGUAAAGAUUC | No. 5208 | GAAUCUUUACUCUCUCUCCUUGG |
| BH0887 | No. 4507 | AGGAGAGAGAGUAAAGAUUCA | No. 5209 | UGAAUCUUUACUCUCUCUCCUUG |
| BH0888 | No. 4508 | GGAGAGAGAGUAAAGAUUCAG | No. 5210 | CUGAAUCUUUACUCUCUCUCCUU |
| BH0890 | No. 4509 | AGAGAGAGUAAAGAUUCAGGA | No. 5211 | UCCUGAAUCUUUACUCUCUCUCC |
| BH0891 | No. 4510 | GAGAGAGUAAAGAUUCAGGAA | No. 5212 | UUCCUGAAUCUUUACUCUCUCUC |
| BH0892 | No. 4511 | AGAGAGUAAAGAUUCAGGAAA | No. 5213 | UUUCCUGAAUCUUUACUCUCUCU |
| BH0893 | No. 4512 | GAGAGUAAAGAUUCAGGAAAA | No. 5214 | UUUUCCUGAAUCUUUACUCUCUC |
| BH0894 | No. 4513 | AGAGUAAAGAUUCAGGAAAAA | No. 5215 | UUUUUCCUGAAUCUUUACUCUCU |
| BH0895 | No. 4514 | GAGUAAAGAUUCAGGAAAAAU | No. 5216 | AUUUUUCCUGAAUCUUUACUCUC |
| BH0896 | No. 4515 | AGUAAAGAUUCAGGAAAAAUU | No. 5217 | AAUUUUUCCUGAAUCUUUACUCU |
| BH0897 | No. 4516 | GUAAAGAUUCAGGAAAAAUUU | No. 5218 | AAAUUUUUCCUGAAUCUUUACUC |
| BH0898 | No. 4517 | UAAAGAUUCAGGAAAAAUUUA | No. 5219 | UAAAUUUUUCCUGAAUCUUUACU |
| BH0899 | No. 4518 | AAAGAUUCAGGAAAAAUUUAA | No. 5220 | UUAAAUUUUUCCUGAAUCUUUAC |
| BH0905 | No. 4519 | UCAGGAAAAAUUUAAGAAUGG | No. 5221 | CCAUUCUUAAAUUUUUCCUGAAU |
| BH0906 | No. 4520 | CAGGAAAAAUUUAAGAAUGGA | No. 5222 | UCCAUUCUUAAAUUUUUCCUGAA |

### [Table 16]

**Table 2-14**

| Double stranded nucleic acid No. | SEQ ID NO. | Sense strand sequence (5'→3') | SEQ ID NO. | Antisense strand sequence (5'→3') |
|---|---|---|---|---|
| BH0907 | No. 4521 | AGGAAAAAUUUAAGAAUGGAA | No. 5223 | UUCCAUUCUUAAAUUUUUCCUGA |
| BH0912 | No. 4522 | AAAUUUAAGAAUGGMUGCUA | No. 5224 | UAGCAUUCCAUUCUUAAAUUUUU |
| BH0914 | No. 4523 | AUUUAAGAAUGGAAUGCUACA | No. 5225 | UGUAGCAUUCCAUUCUUAAAUUU |
| BH0917 | No. 4524 | UAAGAAUGGAAUGCUACAUGG | No. 5226 | CCAUGUAGCAUUCCAUUCUUAAA |
| BH0918 | No. 4525 | AAGAAUGGAAUGCUACAUGGU | No. 5227 | ACCAUGUAGCAUUCCAUUCUUAA |
| BH0919 | No. 4526 | AGAAUGGAAUGCUACAUGGUG | No. 5228 | CACCAUGUAGCAUUCCAUUCUUA |
| BH0920 | No 4527 | GAAUGGAAUGCUACAUGGUGA | No. 5229 | UCACCAUGUAGCAUUCCAUUCUU |
| BH0921 | No. 4528 | AAUGGAAUGCUACAUGGUGAU | No. 5230 | AUCACCAUGUAGCAUUCCAUUCU |
| BH0922 | No. 4529 | AUGGAAUGCUACAUGGUGAUA | No. 5231 | UAUCACCAUGUAGCAUUCCAUUC |
| BH0923 | No. 4530 | UGGAAUGCUACAUGGUGAUAA | No. 5232 | UUAUCACCAUGUAGCAUUCCAUU |
| BH0924 | No. 4531 | GGAAUGCUACAUGGUGAUAAA | No. 5233 | UUUAUCACCAUGUAGCAUUCCAU |
| BH0926 | No. 4532 | AAUGCUACAUGGUGAUAAAGU | No. 5234 | ACUUUAUCACCAUGUAGCAUUCC |
| BH0927 | No. 4533 | AUGCUACAUGGUGAUAAAGUU | No. 5235 | AACUUUAUCACCAUGUAGCAUUC |
| BH0928 | No. 4534 | UGCUACAUGGUGAUAAAGUUU | No. 5236 | AAACUUUAUCACCAUGUAGCAUU |
| BH0930 | No. 4535 | CUACAUGGUGAUAAAGUUUCU | No. 5237 | AGAAACUUUAUCACCAUGUAGCA |
| BH0932 | No. 4536 | ACAUGGUGAUAAAGUUUCUUU | No. 5238 | AAAGAAACUUUAUCACCAUGUAG |
| BH0934 | No. 4537 | AUGGUGAUAAAGUUUCUUUCU | No. 6239 | AGAAAGAAACUUUAUCACCAUGU |
| BH0935 | No. 4538 | UGGUGAUAAAGUUUCUUUCUU | No. 5240 | AAGAAAGAAACUUUAUCACCAUG |
| BH0936 | No. 4539 | GGUGAUAAAGUUUCUUUCUUC | No. 5241 | GAAGAAAGAAACUUUAUCACCAU |
| BH0937 | No. 4540 | GUGAUAAAGUUUCUUUCUUCU | No. 5242 | AGAAGAAAGAAACUUUAUCACCA |
| BH0940 | No. 4541 | AUAAAGUUUCUUUCUUCUGCA | No. 5243 | UGCAGAAGAAAGAAACUUUAUCA |
| BH0941 | No. 4542 | UAAAGUUUCUUUCUUCUGCAA | No. 5244 | UUGCAGAAGAAAGAAACUUUAUC |
| BH0942 | No. 4543 | AAAGUUUCUUUCUUCUGCAAA | No. 5245 | U UUGCAGAAGAAAGAAACUUUAU |
| BH0943 | No. 4544 | AAGUUUCUUUCUUCUGCAAAA | No. 5246 | UUUUGCAGAAGAAAGAAACUUUA |
| BH0944 | No. 4545 | AGUUUCUUUCUUCUGCAAAAA | No. 5247 | UUUUUGCAGAAGAAAGAAACUUU |
| BH0945 | No. 4546 | GUUUCUUUCUUCUGCAAAAAU | No. 5248 | AUUUUUGCAGAAGAAAGAAACUU |
| BH0946 | No. 4547 | UUUCUUUCUUCUGCAAAAAUA | No 5249 | UAUUUUUGCAGAAGAAAGAAACU |
| BH0947 | No. 4548 | UUCUUUCUUCUGCAAAAAUAA | No. 5260 | UUAUUUUUGCAGAAGAAAGAAAC |
| BH0948 | No. 4549 | UCUUUCUUCUGCAAAAAUAAG | No. 5251 | CUUAUUUUUGCAGAAGAAAGAAA |
| BH0953 | No. 4550 | CUUCUGCAAAAAUAAGGAAAA | No. 5252 | UUUUCCUUAUUUUUGCAGAAGAA |
| BH0955 | No. 4551 | UCUGCAAAAAUAAGGAAAAGA | No. 5253 | UCUUUUCCUUAUUUUUGCAGAAG |
| BH0956 | No. 4552 | CUGCAAAAAUAAGGAAAAGAA | No. 5254 | UUCUUUUCCUUAUUUUUGCAGAA |
| BH0957 | No. 4553 | UGCAAAAAUAAGGAAAAGAAG | No. 5255 | CUUCUUUUCCUUAUUUUUGCAGA |
| BH0964 | No. 4554 | AUAAGGAAAAGAAGUGUAGCU | No. 5256 | AGCUACACUUCUUUUCCUUAUUU |
| BH0965 | No. 4555 | UAAGGAAAAGAAGUGUAGCUA | No. 5257 | UAGCUACACUUCUUUUCCUUAUU |
| BH0966 | No. 4556 | AAGGAAAAGAAGUGUAGCUAU | No. 5258 | AUAGCUACACUUCUUUUCCUUAU |
| BH0967 | No. 4657 | AGGAAAAGAAGUGUAGCUAUA | No. 6259 | UAUAGCUACACUUCUUUUCCUUA |
| BH0969 | No. 4558 | GAAAAGAAGUGUAGCUAUACA | No. 5260 | UGUAUAGCUACACUUCUUUUCCU |
| BH0972 | No. 4559 | AAGAAGUGUAGCUAUACAGAG | No. 5261 | CUCUGUAUAGCUACACUUCUUUU |
| BH0974 | No. 4560 | GAAGUGUAGCUAUACAGAGGA | No. 5262 | UCCUCUGUAUAGCUACACUUCUU |

### [Table 17]

**Table 2-15**

| Double stranded nucleic acid No. | SEQ ID NO. | Sense strand sequence (5'→3') | SEQ ID NO. | Antisense strand sequence (5'→3') |
|---|---|---|---|---|
| BH0975 | No. 4561 | AAGUGUAGCUAUACAGAGGAU | No. 5263 | AUCCUCUGUAUAGCUACACUUCU |
| BH0977 | No. 4562 | GUGUAGCUAUACAGAGGAUGC | No. 5264 | GCAUCCUCUGUAUAGCUACACUU |
| BH097S | No. 4563 | UGUAGCUAUACAGAGGAUGCU | No. 5265 | AGCAUCCUCUGUAUAGCUACACU |
| BH0980 | No. 4564 | UAGCUAUACAGAGGAUGCUCA | No. 5266 | UGAGCAUCCUCUGUAUAGCUACA |
| BH0981 | No. 4565 | AGCUAUACAGAGGAUGCUCAG | No. 5267 | CUGAGCAUCCUCUGUAUAGCUAC |
| BH0985 | No. 4566 | AUACAGAGGAUGCUCAGUGUA | No. 5268 | UACACUGAGCAUCCUCUGUAUAG |
| BH0987 | No. 4567 | ACAGAGGAUGCUCAGUGUAUA | No. 5269 | UAUACACUGAGCAUCCUCUGUAU |
| BH0988 | No. 4668 | CAGAGGAUGCUCAGUGUAUAG | No. 5270 | CUAUACACUGAGCAUCCUCUGUA |
| BH0989 | No. 4569 | AGAGGAUGCUCAGUGUAUAGA | No. 5271 | UCUAUACACUGAGCAUCCUCUGU |
| BH0990 | No. 4570 | GAGGAUGCUCAGUGUAUAGAU | No. 5272 | AUCUAUACACUGAGCAUCCUCUG |
| BH0991 | No. 4571 | AGGAUGCUCAGUGUAUAGAUG | No. 5273 | CAUCUAUACACUGAGCAUCCUCU |
| BH0996 | No. 4572 | GCUCAGUGUAUAGAUGGCACU | No. 5274 | AGUGCCAUCUAUACACUGAGCAU |
| BH0997 | No. 4573 | CUCAGUGUAUAGAUGGCACUA | No. 5275 | UAGUGCCAUCUAUACACUGAGCA |
| BH0998 | No. 4574 | UCAGUGUAUAGAUGGCACUAU | No. 5276 | AUAGUGCCAUCUAUACACUGAGC |
| BH0999 | No. 4575 | CAGUGUAUAGAUGGCACUAUC | No. 5277 | GAUAGUGCCAUCUAUACACUGAG |
| BH1000 | No. 4576 | AGUGUAUAGAUGGCACUAUCG | No. 5278 | CGAUAGUGCCAUCUAUACACUGA |
| BH1001 | No. 4577 | GUGUAUAGAUGGCACUAUCGA | No. 5279 | UCGAUAGUGCCAUCUAUACACUG |
| BH1002 | No. 4678 | UGUAUAGAUGGCACUAUCGAA | No. 5280 | UUCGAUAGUGCCAUCUAUACACU |
| BH1403 | No. 4579 | GUAUAGAUGGCACUAUCGAAG | No. 5281 | CUUCGAUAGUGCCAUCUAUACAC |
| BH1005 | No. 4580 | AUAGAUGGCACUAUCGAAGUC | No. 5282 | GACUUCGAUAGUGCCAUCUAUAC |
| BH1007 | No. 4581 | AGAUGGCACUAUCGAAGUCCC | No 5283 | GGGACUUCGAUAGUGCCAUCUAU |
| BH1009 | No. 4582 | AUGGCACUAUCGAAGUCCCCA | No. 6284 | UGGGGACUUCGAUAGUGCCAUCU |
| BH1010 | No. 4683 | UGGCACUAUCGAAGUCCCCAA | No. 5285 | UUGGGGACUUCGAUAGUGCCAUC |
| BH1011 | No. 4584 | GGCACUAUCGAAGUCCCCAAA | No. 5286 | UUUGGGGACUUCGAUAGUGCCAU |
| BH1013 | No. 4585 | CACUAUCGAAGUCCCCAAAUG | No. 5287 | CAUUUGGGGACUUCGAUAGUGCC |
| BH1015 | No. 4586 | CUAUCGAAGUCCCCAAAUGCU | No. 5288 | AGCAUUUGGGGACUUCGAUAGUG |
| BH1016 | No. 4587 | UAUCGAAGUCCCCAAAUGCUU | No. 5289 | AAGCAUUUGGGGACUUCGAUAGU |
| BH1017 | No. 4588 | AUCGAAGUCCCCAAAUGCUUC | No 5290 | GAAGCAUUUGGGGACUUCGAUAG |
| BH1018 | No. 4589 | UCGAAGUCCCCAAAUGCUUCA | No. 5291 | UGAAGCAUUUGGGGACUUCGAUA |
| BH1021 | No. 4590 | AAGUCCCCAAAUGCUUCAAGG | No. 5292 | CCUUGAAGCAUUUGGGGACUUCG |
| BH1022 | No. 4591 | AGUCCCCAAAUGCUUCAAGGA | No. 5293 | UCCUUGAAGCAUUUGGGGACUUC |
| BH1023 | No. 4592 | GUCCCCAAAUGCUUCAAGGAA | No. 5294 | UUCCUUGAAGCAUUUGGGGACUU |
| BH1024 | No. 4593 | UCCCCAAAUGCUUCAAGGAAC | No. 5295 | GUUCCUUGAAGCAUUUGGGGACU |
| BH1025 | No. 4594 | CCCCAAAUGCUUCAAGGAACA | No. 5296 | UGUUCCUUGAAGCAUUUGGGGAC |
| BH1026 | No. 4595 | CCCAAAUGCUUCAAGGAACAC | No 5297 | GUGUUCCUUGAAGCAUUUGGGGA |
| BH1027 | No. 4596 | CCAAAUGCUUCAAGGAACACA | No. 5298 | UGUGUUCCUUGAAGCAUUUGGGG |
| BH1028 | No. 4597 | CAAAUGCUUCAAGGAACACAG | No. 5299 | CUGUGUUCCUUGAAGCAUUUGGG |
| BH1029 | No. 4598 | AAAUGCUUCAAGGAACACAGU | No. 5300 | ACUGUGUUCCUUGAAGCAUUUGG |
| BH1030 | No. 4599 | AAUGCUUGAAGGAACACAGUU | No. 5301 | AACUGUGUUCCUUGAAGCAUUUG |
| BH1031 | No. 4600 | AUGCUUCAAGGAACACAGUUC | No. 5302 | GAACUGUGUUCCUUGAAGCAUUU |

### [Table 18]

**Table 2-16**

| Double stranded nucleic acid No. | SEQ ID NO. | Sense strand sequence (5'→3') | SEQ ID NO. | Antisense strand sequence (5'→3') |
|---|---|---|---|---|
| BH1032 | No. 4601 | UGCUUCAAGGAACACAGUUCU | No. 5303 | AGAACUGUGUUCCUUGAAGCAUU |
| BH1034 | No. 4602 | CUUCAAGGAACACAGUUCUCU | No. 5304 | AGAGAACUGUGUUCCUUGAAGCA |
| BH1035 | No. 4603 | UUCAAGGAACACAGUUCUCUG | No. 5305 | CAGAGAACUGUGUUCCUUGAAGC |
| BH1038 | No. 4604 | AAGGAACACAGUUCUCUGGCU | No. 5306 | AGCCAGAGAACUGUGUUCCUUGA |
| BH1039 | No. 4605 | AGGAACACAGUUCUCUGGCUU | No. 5307 | AAGCCAGAGAACUGUGUUCCUUG |
| BH1040 | No. 4606 | GGAACACAGUUCUCUGGCUUU | No. 5308 | AAAGCCAGAGAACUGUGUUCCUU |
| BH1041 | No. 4607 | GAACACAGUUCUCUGGCUUUU | No. 5309 | AAAAGCCAGAGAACUGUGUUCCU |
| BH1042 | No. 4608 | AACACAGUUCUCUGGCUUUUU | No. 5310 | AAAAAGCCAGAGAACUGUGUUCC |
| BH1043 | No. 4609 | ACACAGUUCUCUGGCUUUUUG | No. 5311 | CAAAAACCCAGAGAACUGUGUUC |
| BH1044 | No. 4610 | CACAGUUCUCUGGCUUUUUGG | No 5312 | CCAAAAAGCCAGAGAACUGUGUU |
| BH1045 | No. 4611 | ACAGUUCUCUGGCUUUUUGGA | No. 5313 | UCCAAAAAGCCAGAGAACUGUGU |
| BH1046 | No. 4612 | CAGUUCUCUGGCUUUUUGGAA | No. 5314 | UUCCAAAAAGCCAGAGAACUGUG |
| BH1047 | No. 4613 | AGUUCUCUGGCUUUUUGGAAA | No. 5315 | UUUCCAAAAAGCCAGAGAACUGU |
| BH1048 | No. 4614 | GUUCUCUGGCUUUUUGGAAAA | No. 5316 | UUUUCCAAAAAGCCAGAGAACUG |
| BH1051 | No. 4615 | CUCUGGCUUUUUGGAAAACUG | No. 5317 | CAGUUUUCCAAAAAGCCAGAGAA |
| BH1052 | No. 4616 | UCUGGCUUUUUGGAAAACUGA | No. 5318 | UCAGUUUUCCAAAAAGCCAGAGA |
| BH1053 | No. 4617 | CUGGCU UUUUGGAAAACUGAU | No. 5319 | AUCAGUUUUCCAAAAAGCCAGAG |
| BH1054 | No. 4618 | UGGCUUUUUGGAAAACUGAUG | No. 5320 | CAUCAGUUUUCCAAAAAGCCAGA |
| BH1056 | No. 4619 | GCUUUUUGGAAAACUGAUGCA | No. 5321 | UGCAUCAGUUUUCCAAAAAGCCA |
| BH1057 | No. 4620 | CUUUUUGGAAAACUGAUGCAU | No. 5322 | AUGCAUCAGUUUUCCAAAAAGCC |
| BH1061 | No. 4621 | UUGGAAAACUGAUGCAUCCGA | No. 5323 | UCGGAUGCAUCAGUUUUCCAAAA |
| BH1062 | No. 4622 | UGGAAAACUGAUGCAUCCGAU | No. 5324 | AUCGGAUGCAUCAGUUUUCCAAA |
| BH1063 | No. 4623 | GGAAAACUGAUGCAUCCGAUG | No 5325 | CAUCGGAUGCAUCAGUUUUCCAA |
| BH1064 | No. 4624 | GAAAACUGAUGCAUCCGAUGU | No. 5326 | ACAUCGGAUGCAUCAGUUUUCCA |
| BH1065 | No. 4625 | AAAACUGAUGCAUCCGAUGUA | No. 5327 | UACAUCGGAUGCAUCAGUUUUCC |
| BH1067 | No. 4626 | AACUGAUGCAUCCGAUGUAAA | No. 5328 | UUUACAUCGGAUGCAUCAGUUUU |
| BH1068 | No. 4627 | ACUGAUGCAUCCGAUGUAAAG | No. 5329 | CUUUACAUCGGAUGCAUCAGUUU |
| BH1072 | No 4628 | AUGCAUCCGAUGUAAAGCCAU | No. 5330 | AUGGCUUUACAUCGGAUGCAUCA |
| BH1073 | No. 4629 | UGCAUCCGAUGUAAAGCCAUG | No. 5331 | CAUGGCUUUACAUCGGAUGCAUC |
| BH1076 | No. 4630 | AUCCGAUGUAAAGCCAUGCUA | No. 5332 | UAGCAUGGCUUUACAUCGGAUGC |
| BH1077 | No. 4631 | UCCGAUGUAAAGCCAUGCUAA | No. 5333 | UUAGCAUGGCUUUACAUCGGAUG |
| BH1078 | No. 4632 | CCGAUGUAAAGCCAUGCUAAG | No. 5334 | CUUAGCAUGGCUUUACAUCGGAU |
| BH1083 | No. 4633 | GUAAAGCCAUGCUAAGGUGGU | No. 5335 | ACCACCUUAGCAUGGCUUUACAU |
| BH1084 | No. 4634 | UAAAGCCAUGCUAAGGUGGUU | No 5336 | AACCACCUUAGCAUGGCUUUACA |
| BH1085 | No. 4635 | AAAGCCAUGCUAAGGUGGUUU | No. 5337 | AAACCACCUUAGCAUGGCUUUAC |
| BH1086 | No. 4636 | AAGCCAUGCUAAGGUGGUUUU | No. 5338 | AAAACCACCUUAGCAUGGCUUUA |
| BH1087 | No. 4637 | AGCCAUGCUAAGGUGGUUUUC | No. 5339 | GAAAACCACCUUAGCAUGGCUUU |
| BH1088 | No. 4638 | GCCAUGCUAAGGUGGUUUUCA | No. 5340 | UGAAAACCACCUUAGCAUGGCUU |
| BH1089 | No. 4639 | CCAUGCUAAGGUGGUUUUCAG | No. 5341 | CUGAAAACCACCUUAGCAUGGCU |
| BH1090 | No. 4640 | CAUGCUAAGGUGGUUUUCAGA | No. 5342 | UCUGAAAACCACCUUAGCAUGGC |

### [Table 19]

**Table 2-17**

| Double stranded nucleic acid No. | SEQ ID NO. | Sense strand sequence (5'→3') | SEQ ID NO. | Antisense strand sequence (5'→3') |
|---|---|---|---|---|
| BH1092 | No. 4641 | UGCUAAGGUGGUUUUCAGAUU | No. 5343 | AAUCUGAAAACCACCU UAGCAUG |
| BH1093 | No. 4642 | GCUAAGGUGGUUUUCAGAUUC | No. 5344 | GAAUCUGAAAACCACCUUAGCAU |
| BH1095 | No. 4643 | UAAGGUGGUUUUCAGAUUCCA | No. 5345 | UGGAAUCUGAAAACCACCUUAGC |
| BH1096 | No. 4644 | AAGGUGGUUUUCAGAUUCCAC | No. 5346 | GUGGAAUCUGAAAACCACCUUAG |
| BH1097 | No. 4645 | AGGUGGUUUUCAGAUUCCACA | No. 5347 | UGUGGAAUCUGAAAACCACCUUA |
| BH1099 | No. 4646 | GUGGUUUUCAGAUUCCACACA | No. 5348 | UGUGUGGAAUCUGAAAACCACCU |
| BH1100 | No. 4647 | UGGUUUUCAGAUUCCACACAA | No. 5349 | UUGUGUGGAAUCUGAAAACCACC |
| BH1101 | No. 4648 | GGUUUUCAGAUUCCACACAAA | No. 5350 | UUUGUGUGGAAUCUGAAAACCAC |
| BH1102 | No. 4649 | GUUUUCAGAUUCCACACAAAA | No. 5351 | UUUUGUGUGGAAUCUGAAAACCA |
| BH1103 | No. 4650 | UUUUCAGAUUCCACACAAAAU | No. 5352 | AUUUUGUGUGGAAUCUGAAAACC |
| BH1104 | No. 4651 | UUUCAGAUUCCACACAAAAUG | No. 5353 | CAUUUUGUGUGGAAUCUGAAAAC |
| BH1105 | No. 4652 | UUCAGAUUCCACACAAAAUGU | No. 5354 | ACAUUUUGUGUGGAAUCUGAAAA |
| BH1106 | No. 4653 | UCAGAUUCCACACAAAAUGUC | No. 5355 | GACAUUUUGUGUGGAAUCUGAAA |
| BH1107 | No. 4654 | CAGAUUCCACACAAAAUGUCA | No. 5356 | UGACAUUUUGUGUGGAAUCUGAA |
| BH1109 | No. 4655 | GAUUCCACACAAAAUGUCACA | No. 5357 | UGUGACAUUUUGUGUGGAAUCUG |
| BH1111 | No. 4656 | UUCCACACAAAAUGUCACACU | No. 5368 | AGUGUGACAUUUUGUGUGGAAUC |
| BH1112 | No. 4657 | UCCACACAAAAUGUCACACUU | No. 5369 | AAGUGUGACAUUUUGUGUGGAAU |
| BH1113 | No. 4658 | CCACACAAAAUGUCACACUUG | No. 5360 | CAAGUGUGACAUUUUGUGUGGAA |
| BH1114 | No. 4659 | CACACAAAAUGUCACACUUGU | No. 5361 | ACAAGUGUGACAUUUUGUGUGGA |
| BH1115 | No. 4660 | ACACAAAAUGUCACACUUGUU | No. 5362 | AACAAGUGUGACAUUUUGUGUGG |
| BH1116 | No. 4661 | CACAAAAUGUCACACUUGUUU | No. 5363 | AAACAAGUGUGACAUUUUGUGUG |
| BH1118 | No. 4662 | CAAAAUGUCACACUUGUUUCU | No. 5364 | AGAAACAAGUGUGACAUUUUGUG |
| BH1120 | No. 4663 | AAAUGUCACACUUGUUUCUUG | No. 5365 | CAAGAAACAAGUGUGACAUUUUG |
| BH1121 | No. 4664 | AAUGUCACACUUGUUUCUUGU | No. 5366 | ACAAGAAACAAGUGUGACAUUUU |
| BH1122 | No. 4665 | AUGUCACACUUGUUUCUUGUU | No. 6367 | AACAAGAAACAAGUGUGACAUUU |
| BH1124 | No. 4666 | GUCACACUUGUUUCUUGUUCA | No. 5368 | UGAACAAGAAACAAGUGUGACAU |
| BH1125 | No. 4667 | UCACACUUGUUUCUUGUUCAU | No. 5369 | AUGAACAAGAAACAAGUGUGACA |
| BH1127 | No. 4668 | ACACUUGUUUCUUGUUCAUCC | No. 5370 | GGAUGAACAAGAAACAAGUGUGA |
| BH1128 | No. 4669 | CACUUGUUUCUUGUUCAUCCA | No. 5371 | UGGAUGAACAAGAAACAAGUGUG |
| BH1129 | No. 4670 | ACUUGUUUCUUGUUCAUCCAA | No. 5372 | UUGGAUGAACAAGAAACAAGUGU |
| BH1130 | No. 4671 | CUUGUUUCUUGUUCAUCCAAG | No. 5373 | CUUGGAUGAACAAGAAACAAGUG |
| BH1131 | No. 4672 | UUGUUUCUUGUUCAUCCAAGG | No. 5374 | CCUUGGAUGAACAAGAAACAAGU |
| BH1133 | No. 4673 | GUUUCUUGUUCAUCCAAGGAA | No. 5375 | UUCCUUGGAUGAACAAGAAACAA |
| BH1134 | No. 4674 | UUUCUUGUUCAUCCAAGGAAC | No. 5376 | GUUCCUUGGAUGAACAAGAAACA |
| BH1135 | No. 4675 | UUCUUGUUCAUCCAAGGAACC | No. 5377 | GGUUCCUUGGAUGAACAAGAAAC |
| BH1136 | No. 4676 | UCUUGUUCAUCCAAGGAACCU | No. 5378 | AGGUUCCUUGGAUGAACAAGAAA |
| BH1137 | No. 4677 | CUUGUUCAUCCAAGGAACCUA | No. 5379 | UAGGUUCCUUGGAUGAACAAGAA |
| BH1138 | No. 4678 | UUGUUCAUCCAAGGAACCUAA | No. 5380 | UUAGGUUCCUUGGAUGAACAAGA |
| BH1139 | No. 4679 | UGUUCAUCCAAGGAACCUAAU | No. 5381 | AUUAGGUUCCUUGGAUGAACAAG |
| BH1140 | No. 4680 | GUUCAUCCAAGGAACCUAAUU | No. 5382 | AAUUAGGUUCCUUGGAUGAACAA |

### [Table 20]

**Table 2-18**

| Double stranded nucleic acid No. | SEQ ID NO. | Sense strand sequence (5'→3') | SEQ ID NO. | Antisense strand sequence (5'→3') |
|---|---|---|---|---|
| BH1141 | No. 4681 | UUCAUCCAAGGAACCUAAUUG | No. 5383 | CAAUUAGGUUCCUUGGAUGAACA |
| BH1142 | No. 4682 | UCAUCCAAGGAACCUAAUUGA | No. 5384 | UCAAUUAGGUUCCUUGGAUGAAC |
| BH1143 | No. 4683 | CAUCCAAGGAACCUAAUUGAA | No. 5385 | UUCAAUUAGGUUCCUUGGAUGAA |
| BH1144 | No. 4684 | AUCCAAGGAACCUAAUUGAAA | No. 5386 | UUUCAAUUAGGUUCCUUGGAUGA |
| BH1145 | No. 4685 | UCCAAGGAACCUAAUUGAAAU | No. 5387 | AUUUCAAUUAGGUUCCUUGGAUG |
| BH1146 | No. 4686 | CCAAGGAACCUAAUUGAAAUU | No. 5388 | AAUUUCAAUUAGGUUCCUUGGAU |
| BH1147 | No. 4687 | CAAGGAACCUAAUUGAAAUUU | No. 5389 | AAAUUUCAAUUAGGUUCCUUGGA |
| BH1148 | No. 4688 | AAGGAACCUAAUUGAAAUUUA | No. 5390 | UAAAUUUCAAUUAGGUUCCUUGG |
| BH1149 | No. 4689 | AGGAACCUAAUUGAAAUUUAA | No. 5391 | UUAAAUUUCAAUUAGGUUCCUUG |
| BH1150 | No. 4690 | GGAACCUAAUUGAAAUUUAAA | No. 5392 | UUUAAAUUUCAAUUAGGUUCCUU |
| BH1151 | No. 4691 | GAACCUAAUUGAAAUUUAAAA | No. 5393 | UUUUAAAUUUCAAUUAGGUUCCU |
| BH1155 | No. 4692 | CUAAUUGAAAUUUAAAAAUAA | No. 5394 | UUAUUUUUAAAUUUCAAUUAGGU |
| BH1167 | No. 4693 | UAAAAAUAAAGCUACUGAAUU | No. 5395 | AAUUCAGUAGCUUUAUUUUUAAA |
| BH1168 | No. 4694 | AAAAAUAAAGCUACUGAAUUU | No. 5396 | AAAUUCAGUAGCUUUAUUUUUAA |
| BH1169 | No. 4695 | AAAAUAAAGCUACUGAAUUUA | No. 5397 | UAAAUUCAGUAGCUUUAUUUUUA |
| BH1171 | No. 4696 | AAUAAAGCUACUGAAUUUAUU | No. 5398 | AAUAAAUUCAGUAGCUUUAUUUU |
| BH1175 | No. 4697 | AAGCUACUGAAUUUAUUGCCG | No. 5399 | CGGCAAUAAAUUCAGUAGCUUUA |
| BH1176 | No. 4698 | AGCUACUGAAUUUAUUGCCGC | No. 5400 | GCGGCAAUAAAUUCAGUAGCUUU |
| BH1177 | No. 4699 | GCUACUGAAUUUAUUGCCGCA | No. 5401 | UGCGGCAAUAAAUUCAGUAGCUU |
| BH1178 | No. 4700 | CUACUGAAUUUAUUGCCGCAC | No. 5402 | GUGCGGCAAUAAAUUCAGUAGCU |
| BH1179 | No. 4701 | CUCUGCCAUGUUGCUAUUGCA | No. 5403 | UGCAAUAGCAACAUGGCAGAGGA |
| BH1180 | No. 4702 | GCUGGAAUCUUAGAAAAUGGA | No. 5404 | UCCAUUUUCUAAGAUUCCAGCGA |

### [Table 21]

**Table 3-1**

| Double stranded nucleid acid No. | SEQ ID NO. | Sense strand sequence (5'→3') | SEQ ID NO. | Antisense strand sequence (5'→3') |
|---|---|---|---|---|
| CH0033 | No. 5405 | U(F)^G(M)^G(F)U(M)A(F)G(M)U(F)G(M)C(F)C(M)A(F) G(F)U(M)G(F)U(M)G(F)A(M)C(F)U(M)C(F)A(M) | No. 6107 | U(F)^G(M)^A(F)G(M)U(F)C(M)A(F)C(M)A(F)C(M)C(M)U(M)G(F)G (M)C(F)A(M)C(F)U(M)A(F)C(M)C(F)A(M)^A(F)^A(M) |
| CH0034 | No. 5406 | G(F)^G(M)^U(F)A(M)G(F)U(M)G(F)C(M)C(F)A(M)G(F) U(F)G(M)U(F)G(M)A(F)C(M)U(F)C(M)A(F)U(M) | No. 6108 | A(F)^U(M)^G(F)A(M)G(F)U(M)C(F)A(M)C(F)A(M)C(M)U(F)G (M)G(F)C(M)A(F)C(M)U(F)A(M)C(F)C(M)^A(F)^A(M) |
| CH0035 | No. 5407 | G(F)^U(M)^A(F)G(M)U(F)G(M)C(F)C(M)A(F)G(M)U(F) G(F)U(M)G(F)A(M)C(F)U(M)C(F)A(M)U(F)C(M) | No. 6109 | G(F)^A(M)^U(F)G(M)A(F)G(M)U(F)C(M)A(F)C(M)A(M)C(F)U (M)G(F)G(M)C(F)A(M)C(F)U(M)A(F)C(M)^C(F)^A(M) |
| CH0036 | No. 5408 | U(F)^A(M)^G(F)U(M)G(F)C(M)C(F)A(M)G(F)U(M)G(F) U(F)G(M)A(F)C(M)U(F)C(M)A(F)U(M)C(F)C(M) | No. 6110 | G(F)^G(M)^A(F)U(M)G(F)A(M)G(F)U(M)C(F)A(M)C(M)A(F)C (M)U(F)G(M)G(F)C(M)A(F)C(M)U(F)A(M)^C(F)^C(M) |
| CH0037 | No. 5409 | A(F)G(M)U(F)G(M)C(F)C(M)A(F)G(M)U(F)G(M)U(F) G(F)A(M)C(F)U(M)C(F)A(M)U(F)C(M)C(F)A(M) | No. 6111 | U(F)^G(M)^G(F)A(M)U(F)G(M)A(F)G(M)U(F)C(M)A(M)C(F)A (M)C(F)U(M)G(F)G(M)C(F)A(M)C(F)U(M)^A(F)^C(M) |
| CH0038 | No. 5410 | G(F)^U(M)^G(F)C(M)C(F)A(M)G(F)U(M)G(F)U(M)G(F) A(F)C(M)U(F)C(M)A(F)U(M)C(F)C(M)A(F)C(M) | No. 6112 | G(F)^U(M)^G(F)G(M)A(F)U(M)G(F)A(M)G(F)U(M)C(M)A(F)C (M)A(F)C(M)U(F)G(M)G(F)C(M)A(F)C(M)^U(F)^A(M) |
| CH0039 | No. 5411 | U(F)^G(M)^C(F)C(M)A(F)G(M)U(F)G(M)U(F)G(M)A(F) C(F)U(M)C(F)A(M)U(F)G(M)C(F)A(M)C(F)A(M) | No. 6113 | U(F)^G(M)^U(F)G(M)G(F)A(M)U(F)G(M)A(F)G(M)U(M)C(F)A (M)C(F)A(M)C(F)U(M)G(F)G(M)C(F)A(M)^(F)^U(M) |
| CH0040 | No. 5412 | G(F)^C(M)^C(F)A(M)G(F)U(M)G(F)U(M)G(F)A(M) U(F)C(M)A(F)U(M)C(F)C(M)A(F)C(M)A(F)A(M) | No. 6114 | U(F)^U(M)^G(F)U(M)G(F)G(M)A(F)U(M)G(F)A(M)G(M)U(F)C (M)A(F)C(M)A(F)C(M)U(F)G(M)G(F)C(M)^A(F)^C(M) |
| CH0041 | No. 5413 | C(F)^C(M)^A(F)G(M)U(F)G(M)U(F)G(M)A(F)C(M)U(F) C(F)A(M)U(F)C(M)C(F)A(M)C(F)A(M)A(F)U(M) | No. 6115 | A(F)^U(M)^U(F)G(M)U(F)G(M)G(F)A(M)U(F)G(M)A(M)G(F)U (M)C(F)A(M)C(F)A(M)C(F)U(M)G(F)G(M)^C(F)^A(M) |
| CH0042 | No. 5414 | C(F)^A(M)^G(F)U(M)G(F)U(M)G(F)A(M)C(F)U(M)C(F) A(F)U(M)C(F)C(M)A(F)C(M)A(F)A(M)U(F)G(M) | No. 6116 | C(F)^A(M)^U(F)U(M)G(F)U(M)G(F)G(M)A(F)U(M)G(M)A(F)G (M)U(F)C(M)A(F)C(M)A(F)C(M)U(F)G(M)^G(F)^C(M) |
| CH0043 | No. 5415 | A(F)^G(M)^U(F)G(M)U(F)G(M)A(F)C(M)U(F)C(M)A(F) U(F)C(M)C(F)A(M)C(F)A(M)A(F)U(M)G(F)A(M) | No. 6117 | U(F)^C(M)^A(F)U(M)U(F)G(M)U(F)G(M)G(F)A(M)U(M)G(F)A (M)G(F)U(M)C(F)A(M)C(F)A(M)C(F)U(M)^C(F)^G(M) |
| CH0044 | No. 5416 | G(F)^U(M)^G(F)U(M)G(F)A(M)C(F)U(M)C(F)A(M)U(F) C(F)C(M)A(F)C(M)A(F)A(M)U(F)G(M)A(F)U(M) | No. 6118 | A(F)^U(M)^C(F)A(M)U(F)U(M)G(F)U(M)G(M)A(M)U(F)G (M)A(F)G(M)U(F)C(M)A(F)G(M)A(F)C(M)^U(F)^G(M) |
| CH0045 | No. 5417 | U(F)^G(M)^U(F)G(M)A(F)C(M)U(F)C(M)A(F)U(M)C(F) C(F)A(M)C(F)A(M)A(F)U(M)G(F)A(M)U(F)U(M) | No. 6119 | A(F)^A(M)^U(F)G(M)A(F)U(M)U(F)G(M)U(F)G(M)G(M)A(F)U( M)G(F)A(M)G(F)U(M)C(F)A(M)^C(F)^U(M) |
| CH0046 | No. 5418 | G(F)^U(M)^G(F)A(M)C(F)U(M)C(F)A(M)U(F)C(M)C(F) A(F)C(M)A(F)A(M)U(F)G(M)A(F)U(M)U(F)U(M) | No. 6120 | A(F)^A(M)^A(F)U(M)C(F)A(M)U(F)U(M)G(F)U(M)G(M)G(F)A( M)U(F)G(M)A(F)G(M)U(F)C(M)A(F)C(M)^A(F)^C(M) |
| CH0047 | No. 5419 | U(F)^G(M)^A(F)C(M)U(F)C(M)A(F)U(M)C(F)C(M)A(F) C(F)A(M)A(F)U(M)G(F)A(M)U(F)U(M)U(F)C(M) | No. 6121 | G(F)^A(M)^A(F)A(M)U(F)C(M)A(F)U(M)U(F)G(M)U(M)G(F)G (M)A(F)G(M)G(F)A(M)G(F)U(M)C(F)A(M)^C(F)^A(M) |
| CH0048 | No. 5420 | G(F)^A(M)^C(F)U(M)C(F)A(M)U(F)C(M)C(F)A(M)C(F) A(F)A(M)U(F)G(M)A(F)U(M)U(F)U(M)C(F)U(M) | No. 6122 | A(F)^G(M)^A(F)A(M)A(F)U(M)U(M)C(F)A(M)U(F)U(M)G(M)U(F)G( M)G(F)A(M)U(F)G(M)A(F)G(M)U(F)C(M)^A(F)^C(M) |
| CH0050 | No. 5421 | C(F)^U(M)^C(F)A(M)U(F)C(M)C(F)A(M)C(F)A(M)A(F) U(F)G(M)A(F)U(M)U(F)U(M)C(F)U(M)C(F)C(M) | No. 6123 | G(F)^G(M)^A(F)G(M)A(F)A(M)A(F)U(M)C(F)A(M)U(M)U(F)G (M)U(F)G(M)G(F)A(M)U(F)G(M)A(F)G(M)^U(F)^C(M) |
| CH0051 | No. 5422 | U(F)^C(M)^A(F)U(M)C(F)C(M)A(F)C(M)A(F)A(M)U(F) G(F)A(M)U(F)U(M)U(F)C(M)U(F)C(M)C(F)A(M) | No. 6124 | M)G(F)U(M)G(F)G(F)G(M)A(F)U(M)G(F)A(M)^G(F)^U(M) |
| CH0052 | No. 5423 | C(F)^A(M)^U(F)C(M)C(F)A(M)C(F)A(M)A(F)U(M)G(F) A(F)U(M)U(F)U(M)C(F)U(M)C(F)C(M)A(F)C(M) | No. 6125 | C(F)^U(M)^G(F)G(M)A(F)A(M)A(F)U(M)G(M)A(F)C(M)A(F)U (M)U(F)G(M)U(F)G(M)G(F)A(M)U(F)G(M)^A(F)^C(M) |
| CH0053 | No. 5424 | A(F)^U(M)^C(F)C(M)A(F)C(M)A(F)A(M)U(F)G(M)A(F) U(F)U(M)U(F)C(M)U(F)C(M)C(F)A(M)G(F)U(M) | No. 6126 | A(F)^C(M)^U(F)G(M)G(F)A(M)G(F)A(M)A(F)A(M)U(M)C(F)A (M)U(F)U(M)G(F)U(M)G(F)G(M)A(F)U(M)^G(F)^A(M) |
| CH0054 | No. 5425 | U(F)^C(M)^C(F)A(M)C(F)A(M)A(F)U(M)G(F)A(M)U(F) U(F)U(M)C(F)U(M)C(F)C(M)A(F)G(M)U(F)G(M) | No. 6127 | C(F)^A(M)^C(F)U(M)G(F)G(M)A(F)G(M)A(F)A(M)A(M)U(F)C (M)A(F)U(M)U(F)G(M)U(F)G(M)G(F)A(M)^U(F)^G(M) |
| CH0055 | No. 5426 | C(F)^C(M)^A(F)C(M)A(F)A(M)U(F)G(M)A(F)U(M)U(F) U(F)C(M)U(F)C(M)C(F)A(M)G(F)U(M)C(F)C(M) | No. 6128 | GF^C(M)^A(F)C(M)U(F)G(M)G(F)A(M)G(F)A(M)A(M)A(F)U (M)C(F)A(M)U(F)U(M)G(F)U(M)G(F)G(M)^A(F)^U(M) |
| CH0056 | No. 5427 | C(F)^A(M)^C(F)A(M)A(F)U(M)G(F)A(M)U(F)U(M)U(F) C(F)U(M)C(F)C(M)A(F)G(M)U(F)G(M)C(F)U(M) | No. 6129 | A(F)^G(M)^C(F)A(M)C(F)U(M)G(F)G(M)A(F)G(M)A(M)A(F)A (M)U(F)C(M)A(F)U(M)U(F)G(M)U(F)G(M)^G(F)^A(M) |
| CH0057 | No. 5428 | A(F)^C(M)^A(F)A(M)U(F)G(M)A(F)U(M)U(F)U(M)C(F) U(F)C(M)C(F)A(M)G(F)U(M)G(F)C(M)U(F)C(M) | No. 6130 | G(F)^A(M)^G(F)C(M)A(F)C(M)U(F)G(M)G(F)A(M)G(M)A(F)A (M)A (F)U(M)G(F)A(M)U(F)U(M)G(F)U(M)^G(F)^G(M) |
| CH0058 | No. 5429 | C(F)^A(M)^A(F)U(M)G(F)A(M)U(F)U(M)U(F)C(M)U(F) C(F)C(M)A(F)G(M)U(F)G(M)C(F)U(M)C(F)A(M) | No. 6131 | U(F)^G(M)^A(F)G(M)C(F)A(M)C(F)U(M)G(F)G(M)A(M)G(F)A (M)A(F)A(M)U(F)C(M)A(F)U(M)U(F)G(M)^U(F)^G(M) |
| CH0059 | No. 5430 | A(F)^A(M)^U(F)G(M)A(F)U(M)U(F)U(M)C(F)U(M)C(F) C(F)A(M)G(F)U(M)G(F)C(M)U(F)C(M)A(F)U(M) | No. 6132 | A(F)^U(M)^G(F)A(M)G(F)C(M)A(F)C(M)U(F)G(M)G(M)G(M)A(F)G (M)A(F)A(M)A(F)U(M)C(F)A(M)U(F)U(M)^G(F)^U(M) |
| CH0060 | No. 5431 | A(F)^U(M)^G(F)A(M)U(F)U(M)U(F)C(M)U(F)C(M)C(F) A(F)G(M)U(F)G(M)C(F)U(M)C(F)A(M)U(F)C(M) | No. 6133 | G(F)^A(M)^U(F)G(M)A(F)G(M)C(F)A(M)C(F)U(M)G(M)G(F)A (M)G(F)A(M)A(F)A(M)U(F)C(M)A(F)U(M)^U(F)^G(M) |
| CH0061 | No. 5432 | U(F)^G(M)^A(F)U(M)U(F)U(M)C(F)U(M)C(F)C(M)A(F) G(F)U(M)G(F)C(M)U(F)C(M)A(F)U(M)C(F)U(M) | No. 6134 | A(F)^G(M)^A(F)U(M)G(F)A(M)G(F)C(M)A(F)C(M)U(M)G(F)G (M)A(F)G(M)A(F)A(M)A(F)U(M)C(F)A(M)^U(F)^U(M) |
| CH0062 | No. 5433 | G(F)^A(M)^U(F)U(M)U(F)C(M)U(F)C(M)C(F)A(M)G(F) U(F)G(M)C(F)U(M)C(F)A(M)U(F)C(M)U(F)U(M) | No. 6135 | A(F)^A(M)^G(F)A(M)U(F)G(M)A(F)G(M)C(F)A(M)C(M)U(F)G (M)G(F)A(M)G(F)A(M)A(F)A(M)U(F)C(M)^A(F)^U(M) |
| CH0063 | No. 5434 | A(F)^U(M)^U(F)U(M)C(F)U(M)C(F)C(M)A(F)G(M)U(F) G(F)C(M)U(F)C(M)A(F)U(M)C(F)U(M)U(F)G(M) | No. 6136 | C(F)^A(M)^A(F)G(M)A(F)U(M)G(F)A(M)G(F)C(M)A(M)C(F)U (M)G(F)G(M)A(F)G(M)A(F)A(M)A(F)U(M)^C(F)^A(M) |
| CH0064 | No. 5435 | U(F)^U(M)^U(F)C(M)U(F)C(M)C(F)A(M)G(F)U(M)G(F) G(F)U(M)C(F)A(M)U(F)G(M)U(F)U(M)G(F)U(M) | No. 6137 | A(F)^C(M^A(F)A(M)G(F)A(M)U(F)G(M)A(F)G(M)C(M)A(F)C (M)U(F)G(M)G(F)A(M)G(F)A(M)A(F)A(M)^U(F)^G(M) |
| CH0065 | No. 5436 | U(F)^U(M)^C(F)^U(M)C(F)C(M)A(F)G(M)U(F)G(M)C(F) U(F)C(M)A(F)U(M)C(F)U(M)U(F)G(M)U(F)U(M) | No. 6138 | A(F)^A(M)^C(F)A(M)A(F)G(M)A(F)U(M)G(F)A(M)G(M)C(F)A (M)C(F)U(M)G(F)G(M)A(F)G(M)A(F)A(M)^A(F)^U(M) |
| CH0066 | No. 5437 | U(F)^C(M)^U(F)C(M)C(F)A(M)G(F)U(M)G(F)C(M)U(F) C(F)A(M)U(F)C(M)U(F)U(M)G(F)U(M)U(F)C(M) | No. 6139 | G(F)^A(M)^A(F)C(M)A(F)A(M)G(F)A(M)U(F)G(M)A(M)G(F)C (M)A(F)C(M)U(F)G(M)G(F)A(M)G(F)A(M)^A(F)^A(M) |
| CH0067 | No. 5438 | C(F)^U(M)^C(F)C(M)A(F)G(M)U(F)G(M)C(F)U(M)C(F) A(F)U(M)C(F)U(M)U(F)G(M)U(F)U(M)C(F)U(M) | No. 6140 | A(F)^G(M)^A(F)A(M)C(F)A(M)A(F)G(M)A(F)U(M)G(M)A(F)G (M)C(F)A(M)C(F)U(M)G(F)G(M)A(F)G(M)A(F)A(M) |
| CH0068 | No. 5439 | U(F)^C(M)^C(F)A(M)G(F)U(M)G(F)C(M)U(F)C(M)A(F) U(F)C(M)U(F)U(M)G(F)U(M)U(F)C(M)U(F)C(M) | No. 6141 | G(F)^A(M)^G(F)A(M)A(F)C(M)A(F)A(M)G(F)A(M)U(M)G(F)A (M)G(F)C(M)A(F)C(M)U(F)G(M)G(F)A(M)^G(F)^AM |
| CH0069 | No. 5440 | C(F)^C(M)^A(F)G(M)U(F)G(M)C(F)U(M)C(F)A(M)U(F) C(F)U(M)U(F)G(M)U(F)U(M)C(F)U(M)C(F)G(M) | No. 6142 | C(F)^G(M)^A(F)G(M)A(F)A(M)C(F)A(M)A(F)G(M)A(M)U(F)G (M)A(F)G(M)C(F)A(M)C(F)U(M)G(F)G(M)^A(F)^G(M) |
| CH0070 | No. 5441 | C(F)^A(M)^G(F)U(M)G(F)C(M)U(F)C(M)A(F)U(M)C(F) U(F)U(M)G(F)U(M)U(F)C(M)U(F)C(M)G(F)A(M) | No. 6143 | U(F)^C(M)^G(F)A(M)G(F)A(M)A(F)C(M)A(F)A(M)G(M)A(F)U (M)G(F)A(M)G(F)C(M)A(F)C(M)U(F)G(M)^G(F)^A(M) |
| CH0071 | No. 5442 | A(F)^G(M)^U(F)G(M)C(F)U(M)C(F)A(M)U(F)C(M)U(F) U(F)G(M)U(F)U(M)C(F)U(M)C(F)G(M)A(F)G(M) | No. 6144 | C(F)^U(M)^C(F)G(M)A(F)G(M)A(F)A(M)C(F)A(M)A(M)G(F)A (M)U(F)G(M)A(F)G(M)C(F)A(M)C(F)U(M)^G(F)^G(M) |
| CH0072 | NO. 5443 | G(F)^(M)^G(F)C(M)U(F)C(M)A(F)U(M)C(F)U(M)U(F) G(F)U(M)U(F)C(M)U(F)C(M)G(F)A(M)G(F)U(M) | No. 6145 | A(F)^C(M)^U(F)C(M)G(F)A(M)G(F)A(M)A(F)C(M)A(M)A(F)G (M)A(F)U(M)G(F)A(M)G(F)C(M)A(F)C(M)^U(F)^G(M) |
| CH0073 | No. 5444 | U(F)^G(M)^C(F)U(M)C(F)A(M)U(F)C(M)U(F)U(M)G(F) U(F)U(M)C(F)U(M)C(F)G(M)A(F)G(M)U(F)U(M) | No. 6146 | A(F)^A(M)^C(F)U(M)C(F)G(M)A(F)G(M)A(F)A(M)C(M)A(F)A (M)G(F)A(M)U(F)G(M)A(F)G(M)C(F)A(M)^C(F)^U(M) |

### [Table 22]

**Table 3-2**

| Double stranted nucleid acid No. | SEQ ID NO | Sense stand sequence (5'→3') | SEQ ID NO | Antisense strand sequence (5'→3') |
|---|---|---|---|---|
| CH0074 | No. 5445 | G(F)^C(M)^U(F)C(M)A(F)U(M)C(F)U(M)U(F)G(M)U(F) U(F)C(M)U(F)C(M)G(F)A(M)G(F)U(M)U(F)U(M) | No. 6147 | A(F)^A(M)^A(F)C(M)U(F)C(M)G(F)A(M)G(F)A(M)A(M)C(F)A (M)A(F)G(M)A(F)U(M)G(F)A(M)G(F)C(M)^A(F)^C(M) |
| CH0075 | No. 5446 | G(F)^U(M)^C(F)A(M)U(F)C(M)U(F)U(M)G(F)U(M)U(F) C(F)U(M)C(F)G(M)A(F)G(M)U(F)U(M)U(F)U(M) | No. 6148 | A(F)^A(M)A(F)A(M)G(F)U(M)C(F)G(M)A(F)G(M)A(M)A(F)C (M)A(F)A(M)G(F)A(M)U(F)G(M)A(F)G(M)^C(F)^A(M) |
| CH0076 | No. 5447 | U(F)^C(M)^A(F)U(M)C(F)U(M)U(F)G(M)U(F)U(M)C(F) U(F)C(M)G(F)A(M)G(F)U(M)U(F)U(M)U(F)C(M) | No. 6149 | G(F)^A(M)^A(F)A(M)A(F)C(M)U(F)C(M)G(F)A(M)G(M)A(F)A (M)C(F)A(M)A(F)G(M)A(F)U(M)G(F)A(M)^G(F)^C(M) |
| CH0077 | No. 5448 | C(F)^A(M)^U(F)C(M)U(F)U(M)G(F)U(M)U(F)C(M)U(F) C(F)G(M)A(F)G(M)U(F)U(M)U(F)U(M)C(F)U(M) | No. 6150 | A(F)^G(M)^A(F)A(M)A(F)A(M)C(F)U(M)C(F)G(M)A(M)G(F)A (M)A(F)C(M)A(F)A(M)G(F)A(M)U(F)G(M)^A(F)^G(M) |
| CH0078 | No. 5449 | A(F)^U(M)^C(F)U(M)U(F)G(M)U(F)U(M)C(F)U(M)C(F) G(F)A(M)G(F)U(M)U(F)U(M)U(F)C(M)U(F)C(M) | No. 6151 | G(F)^A(M)^G(F)A(M)A(F)A(M)A(F)C(M)U(F)C(M)G(M)A(F)G (M)A(F)A(M)C(F)A(M)A(F)G(M)A(F)U(M)^G(F)^A(M) |
| CH0078 | No. 5450 | U(F)^C(M)^U(F)U(M)G(F)U(M)U(F)C(M)U(F)C(M)G(F) A(F)G(M)U(F)U(M)U(F)U(M)C(F)U(M)C(F)U(M) | No. 6152 | A(F)^G(M)^A(F)G(M)A(F)A(M)A(F)A(M)C(F)U(M)C(M)G(F)A (M)G(F)A(M)A(F)C(M)A(F)A(M)G(F)A(M)^U(F)^G(M) |
| CH0080 | No. 5451 | C(F)^U(M)^U(F)G(M)U(F)U(M)C(F)G(M)A(F) G(F)U(M)U(F)U(M)U(F)C(M)U(F)C(M)U(F)G(M) | No. 6153 | C(F)^A(M)^G(F)A(M)G(F)A(M)A(F)A(M)A(F)C(M)U(M)C(F)G (M)A(F)G(M)A(F)A(M)C(F)A(M)A(F)G(M)^A(F)^U(M) |
| CH0081 | No. 5452 | U(F)^U(M)^G(F)U(M)U(F)C(M)U(F)C(M)G(F)A(M)G(F) U(F)U(M)U(F)U(M)C(F)U(M)C(F)U(M)G(F)C(M) | No. 6154 | G(F)^C(M)^A(F)G(M)A(F)G(M)A(F)A(M)A(F)A(M)C(M)U(F)C (M)G(F)A(M)G(F)A(M)A(F)C(M)A(F)A(M)^G(F)^A(M) |
| CH0083 | No. 5453 | G(F)^U(M)^U(F)C(M)U(F)C(M)G(F)A(M)G(F)U(M)U(F) U(F)U(M)C(F)U(M)C(F)U(M)G(F)C(M)C(F)A(M) | No. 6155 | U(F)^G(M)^G(F)C(M)A(F)G(M)A(F)G(M)A(F)A(M)A(M)A(F)C (M)U(F)C(M)G(F)A(M)G(F)A(M)A(F)C(M)^A(F)^A(M) |
| CH0084 | No. 5454 | U(F)^U(M)^C(F)U(M)C(F)G(M)A(F)G(M)U(F)U(M)U(F) U(F)C(M)U(F)C(M)U(F)G(M)C(F)C(M)A(F)U(M) | No. 6156 | A(F)^U(M)^G(F)G(M)C(F)A(M)G(F)A(M)G(F)A(M)A(M)A(F)A (M)C(F)U(M)C(F)G(M)A(F)G(M)A(F)A(M)^C(F)^A(M) |
| CH0085 | No. 5455 | U(F)^C(M)^U(F)C(M)G(F)A(M)G(F)U(M)U(F)U(M)U(F) C(F)U(M)C(F)U(M)G(F)C(M)C(F)A(M)U(F)G(M) | No. 6157 | C(F)^A(M)^U(F)G(M)G(F)C(M)A(F)G(M)A(M)A(F)A (M)A(F)C(M)U(F)C(M)G(F)A(M)G(F)A(M)^A(F)^C(M) |
| CH0086 | No. 5456 | C(F)^U(M)^C(F)G(M)A(F)G(M)U(F)U(M)U(F)U(M)C(F) U(F)C(M)U(F)G(M)C(F)C(M)A(F)U(M)G(F)U(M) | No. 6158 | A(F)^C(M)^A(F)U(M)G(F)G(M)C(F)A(M)G(F)A(M)G(M)A(F)A (M)A(F)A(M)C(F)U(M)C(F)G(M)A(F)G(M)^A(F)^A(M) |
| CH0087 | No. 5457 | U(F)^C(M)^G(F)A(M)G(F)U(M)U(F)U(M)U(F)C(M)U(F) C(F)U(M)G(F)C(M)C(F)A(M)U(F)G(M)U(F)U(M) | No. 6159 | A(F)^A(M)^C(F)A(M)U(F)G(M)G(F)C(M)A(F)G(M)A(M)G(F)A (M)A(F)A(M)A(F)C(M)U(F)C(M)G(F)A(M)^G(F)^A(M) |
| CH0088 | No. 5458 | C(F)^G(M)^A(F)G(M)U(F)U(M)U(F)U(M)C(F)U(M)G(F) U(F)G(M)C(F)C(M)A(F)U(M)G(F)U(M)U(F)G(M) | No. 6160 | C(F)^A(M)^A(F)C(M)A(F)U(M)G(F)G(M)C(F)A(M)G(M)A(F)G (M)A(F)A(M)A(F)A(M)C(F)U(M)C(F)G(M)^A(F)^G(M) |
| CH0089 | No. 5459 | G(F)^A(M)^G(F)U(M)U(F)U(M)U(F)C(M)U(F)C(M)U(F) G(F)C(M)C(F)A(M)U(F)G(M)U(F)U(M)G(F)C(M) | No. 6161 | G(F)^C(M)^A(F)A(M)C(F)A(M)U(F)G(M)G(M)A(M)G(F)A (M)G(F)A(M)A(F)A(M)A(F)C(M)U(F)C(M)^G(F)^A(M) |
| CH0090 | No. 5460 | A(F)^G(M)^U(F)U(M)U(F)U(M)C(F)U(M)C(F)U(M)G(F) C(F)C(M)A(F)U(M)G(F)U(M)U(F)G(M)C(F)U(M) | No. 6162 | A(F)^G(M)^C(F)A(M)A(F)C(M)A(F)U(M)G(F)G(M)C(M)A(F)G (M)A(F)G(M)A(F)A(M)A(F)A(M)C(F)U(M)^C(F)^G(M) |
| CH0091 | No. 5461 | G(F)^U(M)^U(F)U(M)U(F)C(M)U(F)C(M)U(F)G(M)G(F) C(F)A(M)U(F)G(M)U(F)U(M)G(F)C(M)U(F)A(M) | No. 6163 | U(F)^A(M)^G(F)C(M)A(F)A(M)C(F)A(M)U(F)G(M)G(M)C(F)A (M)G(F)A(M)G(F)A(M)A(F)A(M)A(F)C(M)^U(F)^C(M) |
| CH0092 | No. 5462 | U(F)^U(M)^U(F)U(M)C(F)U(M)C(F)U(M)G(F)C(M)C(F) A(F)U(M)G(F)U(M)U(F)G(M)C(F)U(M)A(F)U(M) | No. 6164 | A(F)^U(M)^ A(F)G(M)C(F)A(M)A(F)U(M)G(M)G(F)C (M)A(F)G(M)A(F)G(M)A(F)A(M)A(F)A(M)^C(F)^U(M) |
| CH0093 | No. 5463 | U(F)^U(M)^U(F)C(M)U(F)C(M)U(F)G(M)C(F)C(M)A(F) U(F)G(M)U(F)U(M)G(F)C(M)U(F)A(M)U(F)U(M) | No. 6165 | A(F)^A(M)^U(F)A(M)G(F)C(M)A(F)A(F)A(M)C(F)A(M)U(M)G(F)G (M)C(F)A(M)G(F)A(M)G(F)A(M)A(F)A(M)^A(F)^C(M) |
| CH0096 | No. 5464 | C(F)^U(M)^C(F)U(M)G(F)C(M)G(F)A(M)U(F)G(M)U(F) U(F)G(M)C(F)U(M)A(F)U(M)U(F)G(M)C(F)A(M) | No. 6166 | U(F)^G(M)^C(F)A(M)A(F)U(M)A(F)G(M)C(F)A(M)A(M)C(F)A (M)U(F)G(M)G(F)C(M)A(F)G(M)A(F)G(M)^A(F)^A(M) |
| CH0097 | No. 5465 | U(F)^C(M)^U(F)G(M)C(F)C(M)A(F)U(M)G(F)U(M)U(F) G(F)C(M)U(F)A(M)U(F)U(M)G(F)C(M)A(F)G(M) | No. 6167 | C(F)^U(M)^G(F)C(M)A(F)A(M)U(F)A(M)G(F)C(M)A(M)A(F)C (M)A(F)U(M)G(F)G(M)C(F)A(M)G(F)A(M)^G(F)^A(M) |
| CH0098 | No. 5466 | C(F)^U(M)^G(F)C(M)C(F)A(M)U(F)G(M)U(F)U(M)G(F) C(F)U(M)A(F)U(M)U(F)G(M)C(F)A(M)G(F)G(M) | No. 6168 | C(F)^C(M)^U(F)G(M)C(F)A(M)A(F)U(M)A(F)G(M)C(M)A(F)A (M)C(F)A(M)U(F)G(M)G(F)C(M)A(F)G(M)^A(F)^G(M) |
| CH0099 | No. 5467 | U(F)^G(M)^C(F)C(M)A(F)U(M)G(F)U(M)U(F)G(M)C(F) U(F)A(M)U(F)U(M)G(F)C(M)A(F)G(M)G(F)A(M) | No. 6169 | U(F)^C(M)^C(F)U(M)G(F)C(M)A(F)A(M)U(F)A(M)G(M)C(F)A (M)A(F)C(M)A(F)U(M)G(F)G(M)C(F)A(M)^G(F)^A(M) |
| CH0103 | No. 5468 | A(F)^U(M)^G(F)U(M)U(F)G(M)C(F)U(M)A(F)U(M)U(F) G(F)C(M)A(F)G(M)G(F)A(M)C(F)G(M)G(F)A(M) | No. 6170 | U(F)^C(M)^C(F)G(M)U(F)C(M)C(F)U(M)G(F)C(M)A(M)A(F)U (M)A(F)G(M)C(F)A(M)A(F)C(M)A(F)U(M)^G(F)^G(M) |
| CH0106 | No. 5469 | U(F)^U(M)^G(F)C(M)U(F)A(M)U(F)U(M)G(F)C(M)A(F) G(F)G(M)A(F)C(M)G(F)G(M)A(F)C(M)C(F)U(M) | No. 6171 | A(F)^G(M)^G(F)U(M)C(F)C(M)G(F)U(M)C(F)C(M)U(M)G(F) C(M)A(F)A(M)U(F)A(M)G(F)C(M)A(F)A(M)^C(F)^A(M) |
| CH0107 | No. 5470 | U(F)^G(M)^C(F)U(M)A(F)U(M)U(F)G(M)C(F)A(M)G(F) G(F)A(M)C(F)G(M)G(F)A(M)C(F)C(M)U(F)G(M) | No. 6172 | C(F)^A(M)^G(F)G(M)U(F)C(M)C(F)G(M)U(F)C(M)C(M)U(F) G(M)C(F)A(M)A(F)U(M)A(F)G(M)C(F)A(M)^A(F)^C(M) |
| CH0108 | No. 5471 | G(F)^C(M)^U(F)A(M)U(F)U(M)G(F)C(M)A(F)G(M)G(F) A(F)C(M)G(F)G(M)A(F)C(M)C(F)U(M)G(F)U(M) | No. 6173 | A(F)^C(M)^A(F)G(M)G(F)U(M)C(F)C(M)G(F)U(M)C(M)C(F)U (M)G(F)G(M)A(F)A(M)U(F)A(M)G(F)G(M)^A(F)^A(M) |
| CH0109 | No. 5472 | C(F)^U(M)^A(F)U(M)U(F)G(M)C(F)A(M)G(F)G(M)A(F) C(F)G(M)G(F)A(M)C(F)C(M)U(F)G(M)U(F)C(M) | No. 6174 | G(F)^A(M)^C(F)A(M)G(F)G(M)U(F)C(M)C(F)G(M)U(M)C(F) C(M)U(F)G(M)C(F)A(M)A(F)U(M)A(F)G(M)^G(F)^A(M) |
| CH0112 | No. 5473 | U(F)^U(M)^G(F)G(M)A(F)G(M)G(F)A(M)C(F)G(M)G(F) A(F)C(M)C(F)U(M)G(F)U(M)C(F)C(M)C(F)A(M) | No. 6175 | U(F)^G(M)^G(F)G(M)A(G)C(M)A(F)G(M)G(F)U(M)C(M)C(F) G(M)U(F)C(M)C(F)U(M)G(F)C(M)A(F)A(M)^U(F)^A(M) |
| CH0114 | No. 5474 | G(F)^C(M)^A(F)G(M)G(F)A(M)C(F)G(M)G(F)A(M)C(F) C(F)U(M)G(F)U(M)C(F)C(M)C(F)A(M)A(F)G(M) | No. 6176 | C(F)^U(M)^U(F)G(M)G(F)G(M)A(F)C(M)A(F)G(M)G(M)U(F)C (M)C(F)G(M)U(F)C(M)C(F)U(M)G(F)C(M)^A(F)^A(M) |
| CH0115 | No. 5475 | C(F)^A(M)^G(F)G(M)A(F)C(M)G(F)G(M)A(F)C(M)C(F) U(F)G(M)U(G)C(M)C(F)C(M)A(F)A(M)G(F)C(M) | No. 6177 | G(F)^C(M)^U(F)U(M)G(F)G(M)G(F)A(M)C(F)A(M)G(M)G(F)U (M)C(F)C(M)G(F)U(M)C(F)C(M)U(F)G(M)^C(F)^A(M) |
| CH0117 | No. 5476 | G(F)^G(M)^A(F)C(M)G(F)G(M)A(F)C(M)C(F)U(M)G(F) U(F)C(M)C(F)C(M)A(F)A(M)G(F)C(M)C(F)A(M) | No. 6178 | U(F)^G(M)^G(F)C(M)U(F)U(M)G(F)G(M)G(F)A(M)C(M)A(F)G (M)G(F)U(M)C(F)C(M)G(F)U(M)C(F)C(M)^U(F)^G(M) |
| CH0118 | No. 5477 | G(F)^A(M)^C(F)G(M)G(F)A(M)C(F)C(M)U(F)G(M)U(F) C(F)C(M)G(F)A(M)A(F)G(M)C(F)C(M)A(F)G(M) | No. 6179 | C(F)^U(M)^G(F)G(M)C(F)U(M)U(F)G(M)G(F)G(M)A(M)C(F)A (M)G(F)G(M)U(F)C(M)C(F)G(M)U(F)G(M)^C(F)^U(M) U(F)^C(M)^U(F)G(M)G(F)C(M)U(F)U(M)G(F)G(M)G(M)A(F)C |
| CH0119 | No. 5478 | A(F)^C(M)^G(F)G(M)A(F)C(M)C(F)U(M)G(F)U(M)C(F) C(F)C(M)A(F)A(M)G(F)C(M)C(F)A(M)G(F)A(M) | No. 6180 | (M)A(F)G(M)G(F)U(M)C(F)C(M)C(F)U(M)^C(F)^C(M) |
| CH0120 | No. 5479 | C(F)^G(M)^G(F)A(M)C(F)C(M)U(F)G(M)U(F)C(M)C(F) C(F)A(M)A(F)G(M)C(F)C(M)A(F)G(M)A(F)U(M) | No. 6181 | A(F)^U(M)^C(F)U(M)G(F)G(M)C(F)U(M)U(F)G(M)G(M)G(F)A (M)C(F)A(M)G(F)G(M)U(F)C(M)C(F)G(M)^U(F)^C(M) |
| CH0121 | No. 5480 | G(F)^G(M)^A(F)C(M)C(F)U(M)G(F)U(M)C(F)C(M)C(F) A(F)A(M)G(F)C(M)C(F)A(M)G(F)A(M)U(F)G(M) | No. 6182 | C(F)^A(M)^U(F)C(M)U(F)G(M)G(F)C(M)U(F)U(M)G(M)G(F)G (M)A(F)C(M)A(F)G(M)G(F)U(M)C(F)C(M)^G(F)^U(M) |
| CH0122 | No. 5481 | G(F)^A(M)^C(F)C(M)U(F)G(M)U(F)C(M)C(F)C(M)A(F) | No. 6183 | U(F)^C(M)^A(F)U(M)C(F)U(M)G(F)G(M)C(F)U(M)U(M)G(F)G (M)G(F)A(M)C(F)A(M)G(F)G(M)U(F)C(M)^C(F)^G(M) |
| CH0123 | No. 5482 | A(F)^C(M)^C(F)U(M)G(F)U(M)C(F)C(M)C(F)A(M)A(F) G(F)C(M)C(F)A(M)G(F)A(M)U(F)G(M)A(F)U(M) | No. 6184 | A(F)^U(M)^C(F)A(M)U(F)C(M)U(F)G(M)G(F)C(M)U(M)U(F)G (M)G(F)G(M)A(F)C(M)A(F)G(M)G(F)U(M)^C(F)^C(M) |
| CH0124 | No. 54 83 | C(F)^C(M)^U(F)G(M)U(F)C(M)C(F)C(M)A(F)A(M)G(F) G(F)G(M)A(F)G(M)A(F)U(M)G(F)A(M)U(F)U(M) | No. 6185 | A(F)^A(M)^U(F)C(M)A(F)U(M)C(F)U(M)G(F)G(M)C(M)U(F)U (M)G(F)G(M)G(F)A(M)C(F)A(M)G(F)G(M)^U(F)^C(M) |
| CH0125 | No. 5484 | C(F)^U(M)^G(F)U(M)C(F)C(M)C(F)A(M)A(F)G(M)C(F) C(F)A(M)G(F)A(M)U(F)G(M)A(F)U(M)U(F)U(M) | No. 6186 | A(F)^A(M)^A(F)U(M)C(F)A(M)U(F)C(M)U(F)G(M)G(M)C(F)U (M)U(F)G(M)G(F)G(M)A(F)C(M)A(F)G(M)^G(F)^U(M) |

### [Table 23]

**Table 3-3**

| Double stranded nucleic acid No. | SEQ ID NO | Sense strand sequence (5'-3') | SEQ ID NO | Antisense strand sequence (5'-3') |
|---|---|---|---|---|
| CH0126 | No. 5485 | U(F)^G(M)^U(F)C(M)C(F)C(M)A(F)A(M)G(F)C(M)C(F) A(F)G(M)A(F)U(M)G(F)A(M)U(F)U(M)U(F)A(M) | No. 6187 | U(F)^A(M)^A(F)A(M)U(F)G(M)A(F)U(M)C(F)U(M)G(M)G(F)C (M)U(F)U(M)G(F)G(M)G(F)A(M)C(F)A(M)^G(F)^G(M) |
| CH0127 | No. 5486 | Q(F)^U(M)^C(F)C(M)C(F)A(M)A(F)G(M)C(F)C(M)A(F) G(F)A(M)U(F)G(M)A(F)U(M)U(F)U(M)A(F)C(M) | No. 6188 | G(F)^U(M)^A(F)A(M)A(F)U(M)C(F)A(M)U(F)C(M)U(M)G(F)G (M)C(F)U(M)U(F)G(M)G(F)G(M)A(F)C(M)^A(F)^G(M) |
| CH0128 | No. 5487 | U(F)^C(M)^C(F)C(M)A(F)A(M)G(F)C(M)C(F)A(M)G(F) A(F)U(M)G(F)A(M)U(F)U(M)U(F)A(M)C(F)C(M) | No. 6189 | G(F)^G(M)^U(F)A(M)A(F)A(M)U(F)C(M)A(F)U(M)C(M)U(F)G (M)G(F)C(M)U(F)U(M)G(F)G(M)G(F)A(M)^C(F)^A(M) |
| CM0129 | No. 5488 | C(F)^C(M)^C(F)A(M)A(F)G(M)C(F)C(M)A(F)G(M)A(F) U(F)G(M)A(F)U(M)U(F)U(M)A(F)C(M)C(F)A(M) | No. 6190 | U(F)^G(M)^G(F)U(M)A(F)A(M)A(F)U(M)C(F)A(M)U(M)C(F)U (M)G(F)G(M)C(F)U(M)U(F)G(M)G(F)G(M)^A(F)^C(M) |
| CH0130 | No. 5489 | C(F)^C(M)^A(F)A(M)G(F)C(M)C(F)A(M)G(F)A(M)U(F) G(F)A(M)U(F)U(M)U(F)A(M)C(F)C(M)A(F)U(M) | No. 6191 | A(F)^U(M)^G(F)G(M)U(F)A(M)A(F)A(M)U(F)C(M)A(M)U(F)C (M)U(F)G(M)G(F)C(M)U(F)U(M)G(F)G(M)^G(F)^A(M) |
| CH0131 | No. 5490 | C(F)^A(M)^A(F)G(M)C(F)C(M)A(F)G(M)A(F)U(M)G(F) A(F)U(M)U(F)U(M)A(F)C(M)C(F)A(M)U(F)U(M) | No. 6192 | A(F)^A(M)^U(F)G(M)G(F)U(M)A(F)A(M)A(F)U(M)C(M)A(F)U( M)C(F)U(M)G(F)G(M)C(F)U(M)U(F)G(M)^F)^G(M) |
| CH0132 | No. 5491 | A(F)^A(M)^G(F)C(M)C(F)A(M)G(F)A(M)U(F)G(M)A(F) U(F)U(M)U(F)A(M)C(F)C(M)A(F)U(M)U(F)U(M) | No. 6193 | A(F)^A(M)^A(F)U(M)G(F)G(M)U(F)A(M)A(F)A(M)U(M)C(F)A( M)U(F)C(M)U(F)G(M)G(F)C(M)U(F)U(M)^G(F)^G(M) |
| CM0133 | No. 5492 | A(F)^G(M)^C(F)C(M)A(F)G(M)A(F)U(M)G(F)A(M)U(F) U(F)U(M)A(F)C(M)C(F)A(M)U(F)U(M)U(F)U(M) | No. 6194 | A(F)^A(M)^A(F)A(M)U(F)G(M)G(F)U(M)A(F)A(M)A(M)U(F)C( M)A(F)U(M)C(F)U(M)G(F)G(M)C(F)U(M)^U(F)^GM) |
| CH0134 | No. 5493 | G(F)^C(M)^C(F)A(M)G(F)A(M)U(F)G(M)A(F)U(M)U(F) U(F)A(M)C(F)C(M)A(F)U(M)U(F)U(M)U(F)C(M) | | G(F)^A(M)^A(F)A(M)A(F)U(M)G(F)G(M)U(F)A(M)A(M)A(F)U( M)C(F)A(M)U(F)C(M)U(F)G(M)G(F)C(M)^U(F)^U(M) |
| CH0135 | No. 5494 | C(F)^C(M)^A(F)G(M)A(F)U(M)G(F)A(M)U(F)U(M)U(F) A(F)C(M)C(F)A(M)U(F)U(M)U(F)U(M)C(F)C(M) | No. 6196 | G(F)^G(M)^A(F)A(M)A(F)A(M)U(F)G(M)G(F)U(M)A(M)A(F)A( M)U(F)C(M)A(F)U(M)C(F)U(M)G(F)G(M)^C(F)^U(M) |
| CH0136 | No. 5495 | C(F)^A(M)^G(F)A(M)U(F)G(M)A(F)U(M)U(F)U(M)A(F) C(F)C(M)A(F)U(M)U(F)U(M)U(F)C(M)C(F)A(M) | No. 6197 | U(F)^G(M)^G(F)A(M)A(F)A(M)A(F)U(M)G(F)G(M)U(M)A(F)A( M)A(F)U(M)C(F)A(M)U(F)C(M)U(F)G(M)^G(F)^C(M) |
| CH0137 | No. 5496 | A(F)^G(M)^A(F)U(M)G(F)A(M)U(F)U(M)U(F)A(M)C(F) C(F)A(M)U(F)U(M)U(F)U(M)C(F)C(M)A(F)C(M) | No. 6198 | G(F)^U(M)^G(F)G(M)A(F)A(M)A(F)A(M)U(F)G(M)G(M)U(F)A (M)A(F)A(M)U(F)C(M)A(F)U(M)C(F)U(M)^G(F)^G(M) |
| CH0138 | No. 5497 | G(F)^AM)^U(F)G(M)A(F)U(M)U(F)U(M)A(F)C(M)C(F) A(F)U(M)U(F)U(M)U(F)C(M)C(F)A(M)C(F)A(M) | No. 6199 | U(F)^G(M)^U(F)G(M)G(F)A(M)A(F)A(M)A(F)U(M)G(M)G(F)U (M)A(F)A(M)A(F)U(M)C(F)A(M)U(F)C(M)^U(F)^G(M) |
| CH0139 | No. 5498 | A(F)^U(M)^G(F)A(M)U(F)U(M)U(F)A(M)C(F)C(M)A(F) U(F)U(M)U(F)U(M)C(F)C(M)A(F)C(M)A(F)G(M) | No. 6200 | C(F)^U(M)^G(F)U(M)G(F)G(M)A(F)A(M)A(F)A(M)U(M)G(F)G (M)U(F)A(M)A(F)A(M)U(F)C(M)A(F)U(M)^C(F)^U(M) |
| CH0143 | No. 5499 | U(F) C(F)C(M)A(F)C(M)A(F)G(M)U(F)G(M)G(F)U(M) | No. 6201 | A(F)^C(M)^C(F)A(M)C(F)U(M)G(F)U(M)G(F)G(M)A(M)A(F)A (M)A(F)U(M)G(F)G(M)U(F)A(M)A(F)A(M)^U(F)^C(M) |
| CH0144 | No. 5500 | U(F)^U(M)^A(F)C(M)C(F)A(M)U(F)U(M)U(F)U(M)C(F) C(F)A(M)C(F)A(M)G(F)U(M)G(F)G(M)U(F)C(M) | No. 6202 | G(F)^A(M)^C(F)C(M)A(F)C(M)U(F)G(M)U(F)G(M)G(M)A(F)A (M)A(F)A(M)U(F)G(M)G(F)U(M)A(F)A(M)^A(F)^U(M) |
| CH0145 | No. 5501 | U(F)^A(M)^C(F)C(M)A(F)U(M)U(F)U(M)U(F)C(M)C(F) A(F)C(M)A(F)G(M)U(F)G(M)G(F)U(M)C(F)C(M) | No. 6203 | G(F)^G(M)^A(F)C(M)C(F)A(M)C(F)A(M)C(F)U(M)G(F)U(M)G(M)G(F)A (M)A(F)A(M)A(F)U(M)G(F)G(M)U(F)A(M)^A(F)^A(M) |
| CH0148 | No. 5502 | A(F)^U(M)^U(F)U(M)U(F)C(M)C(F)A(M)C(F)A(M)G(F) U(F)G(M)G(F)U(M)C(F)C(M)C(F)G(M)U(F)U(M) | No. 6204 | A(F)^A(M)^C(F)G(M)G(F)G(M)A(F)C(M)C(F)A(M)C(M)U(F)G (M)U(F)G(M)G(F)A(M)A(F)A(M)A(F)U(M)^G(F)^G(M) |
| CH0150 | No. 5503 | U(F)^U(M)^U(F)U(M)C(F)C(M)A(F)C(M)A(F)G(M)U(F) G(F)G(M)U(F)C(M)C(F)C(M)G(F)U(M)U(F)A(M) | | U(F)^A(M)^A(F)C(M)G(F)G(M)G(F)A(M)C(F)C(M)A(M)C(F)U (M)G(F)U(M)G(F)G(M)A(F)A(M)A(F)A(M)^ U(F)^G(M) |
| CH0151 | No. 5604 | U(F)^U(M)^U(F)C(M)C(F)A(M)C(F)A(M)G(F)U(M)G(F) G(F)U(M)C(F)C(M)C(F)G(M)U(F)U(M)A(F)A(M) | No. 6206 | U(F)^U(M)^A(F)A(M)C(F)G(M)G(F)G(M)A(F)C(M)C(M)A(F)C (M)U(F)G(M)U(F)G(M)G(F)A(M)A(F)A(M)^A(F)^U(M) |
| CH0152 | No. 5505 | U(F)^U(M)^C(F)C(M)A(F)C(M)A(F)G(M)U(F)G(M)G(F) U(F)C(M)C(F)C(M)G(F)U(M)U(F)A(M)A(F)A(M) | No. 6207 | U(F)^U(M)^U(F)A(M)A(F)C(M)G(F)G(M)G(F)A(M)C(M)C(F)A (M)C(F)U(M)G(F)U(M)G(F)G(M)A(F)A(M)^A(F)^A(M) |
| CH0153 | No. 5506 | U(F)^C(M)^C(F)A(M)C(F)A(M)G(F)U(M)G(F)G(M)U(F) C(F)C(M)C(F)G(M)U(F)U(M)A(F)A(M)A(F)A(M) | No. 6208 | U(F)^U(M)^U(F)U(M)A(F)A(M)C(F)G(M)G(F)G(M)A(M)C(F)C (M)A(F)C(M)U(F)G(M)U(F)G(M)G(F)A(M)^A(F)^A(M) |
| CH0154 | No. 5507 | C(F)^C(M)^A(F)C(M)A(F)G(M)U(F)G(M)G(F)U(M)C(F) C(F)C(M)G(F)U(M)U(F)A(M)A(F)A(M)A(F)A(M) | No. 6209 | U(F)^U(M)^U(F)U(M)U(F)A(M)A(F)C(M)G(F)G(M)G(M)A(F)C (M)C(F)A(M)C(F)U(M)G(F)U(M(G)FG(M)^A(F)^A(M) |
| CH0155 | No. 5508 | C(F)^A(M)^C(F)A(M)G(F)U(M)G(F)G(M)U(F)C(M)C(F) C(F)G(M)U(F)U(M)A(F)A(M)A(F)A(M)A(F)C(M) | No. 6210 | G(F)^U(M)^U(F)U(M)U(F)U(M)A(F)A(M)C(F)G(M)G(M)G(F)A (M)C(F)C(M)A(F)C(M)U(F)G(M)U(F)G(M)^G(F)^A(M) |
| CH0156 | No. 5509 | A(F)^C(M)^A(F)G(M)U(F)G(M)G(F)U(M)C(F)C(M)C(F) G(F)U(M)U(F)A(M)A(F)A(M)A(F)A(M)C(F)A(M) | No. 6211 | U(F)^G(M)^U(F)U(M)U(F)U(M)U(F)A(M)A(F)C(M)G(M)G(F)G (M)A(F)C(M)C(F)A(M)C(F)U(M)G(F)U(M)^G(F)^G(M) |
| CH0157 | No. 5510 | C(F)^A(M)^G(F)U(M)G(F)G(M)U(F)C(M)C(F)C(M)G(F) U(F)U(M)A(F)A(M)A(F)A(M)A(F)C(M)A(F)U(M) | No. 6212 | A(F)^U(M)^G(F)U(M)U(F)U(M)U(M)A(F)A(M)G(M)G(F)G( M)G(F)A(M)C(F)C(M)A(F)C(M)U(F)G(M)^U(F)^G(M) |
| CH0158 | No. 5511 | A(F)^G(M)^U(F)G(M)G(F)U(M)C(F)C(M)C(F)G(M)U(F) U(F)A(M)A(F)A(M)A(F)A(M)C(F)A(M)U(F)U(M) | No. 6213 | A(F)^A(M)^U(F)G(M)U(F)U(M)U(F)U(M)U(F)A(M)A(M)C(F)G( M)G(F)G(M)A(F)C(M)C(F)A(M)C(F)U(M)^G(F)^U(M) |
| CH0159 | No. 5512 | G(F)^U(M)^G(F)G(M)U(F)C(M)C(F)C(M)G(F)U(M)U(F) A(F)A(M)A(F)A(M)A(F)C(M)A(F)U(M)U(F)C(M) | No. 6214 | G(F)^A(M)^A(F)U(M)G(F)U(M)U(F)U(M)U(F)U(M)A(M)A(F)C( M)G(F)G(M)G(F)A(M)C(F)C(M)A F)C(M)^U(F)^G(M) |
| CH0160 | No. 5513 | U(F)^G(M)^G(F)U(M)C(F)C(M)C(F)G(M)U(F)U(M)A(F) A(F)A(M)A(F)A(M)C(F)A(M)U(F)U(M)C(F)U(M) | No. 6215 | A(F)^G(M)^A(F)A(M)U(F)G(M)U(F)U(M)U(F)U(M)U(M)A(F)A( M)C(F)G(M)G(F)G(M)A(F)C(M)C(F)A(M)^C(F)^U(M) |
| CH0161 | No. 5514 | G(F)^G(M)^U(F)C(M)C(F)C(M)G(F)U(M)U(F)A(M)A(F) A(F)A(M)A(F)C(M)A(F)U(M)U(F)C(M)U(F)A(M) | No. 6216 | U(F)^A(M)^G(F)A(M)A(F)U(M)G(F)U(M)U(F)U(M)U(M)U(F)A( M)A(F)C(M)G(F)G(M)G(F)A(M)C(F)C(M)^A(F)^C(M) |
| CH0162 | No. 5515 | G(F)^U(M)^C(F)C(M)C(F)G(M)U(F)U(M)A(F)A(M)A(F) A(F)A(M)C(F)A(M)U(F)U(M)C(F)U(M)A(F)U(M) | No. 6217 | A(F)^U(M)^A(F)G(M)A(F)A(M)U(F)G(M)U(F)U(M)U(M)U(F)U( (M)A(F)A(M)C(F)G(M)G(F)G(M)A(F)C(M)^C(F)^A(M) |
| CH0163 | No. 5516 | U(F)^C(M)C(F)C(M)G(F)U(M)U(F)A(M)A(F)A(M)A(F) A(F)C(M)A(F)U(M)U(F)C(M)U(F)A(M)U(F)G(M) | No. 6218 | C(F)^A(M)^U(F)A(M)G(F)A(M)A(F)U(M)G(F)U(M)U(M)U(F)( M)U(F)A(M)A(F)C(M)G(F)G(M)G(F)A(M)^C(F)^C(M) |
| CH0164 | No. 5517 | C(F)^C(M)^C(F)G(M)U(F)U(M)A(F)A(M)A(F)A(M)A(F) C(F)A(M)U(F)U(M)C(F)U(M)A(F)U(M)G(F)A(M) | No. 6219 | U(F)^C(M)^A(F)U(M)A(F)G(M)A(F)A(M)U(F)G(M)U(M)U(F)U( M)U(F)U(M)A(F)A(M)C(F)G(M)G(F)G(M)^A(F)^C(M) |
| CH0173 | No. 5518 | A(F)^A(M)^C(F)A(M)U(F)U(M)C(F)U(M)A(F)U(M)G(F) A(F)G(M)C(F)C(M)A(F)G(M)G(F)A(M)G(F)A(M) | No. 6220 | U(F)^C(M)^U(F)C(M)C(F)U(M)G(F)G(M)C(F)U(M)C(M)A(F)U (M)A(F)G(M)A(F)A(M)U(F)G(M)U(F)U(M)^U(F)^U(M) |
| CH0174 | No. 5519 | A(F)^C(M)^A(F)U(M)U(F)C(M)U(F)A(M)U(F)G(M)A(F) G(F)C(M)C(F)A(M)G(F)G(M)A(F)G(M)A(F)A(M) | No. 6221 | U(F)^U(M)C(F)U(M)C(F)C(M)U(F)G(M)G(F)C(M)U(M)C(F)A (M)U(F)A(M)G(F)A(M)A(F)U(M) G(F)U(M)^U(F)^U(M) |
| CH0177 | No. 5520 | U(F)^U(M)^C(F)U(M)A(F)U(M)G(F)A(M)G(F)C(M)C(F) A(F)G(M)G(F)A(M)G(F)A(M)A(F)G(M)A(F)G(M) | No. 6222 | C(F)^U(M)^C(F)U(M)U(F)C(M)U(F)C(M)C(F)U(M)G(M)G(F)C (M)U(F)C(M)A(F)U(M)A(F)G(M)A(F)A(M)^U(F)^G(M) |
| CH0178 | No. 5521 | U(F)^C(M)^U(F)A(M)U(F)G(M)A(F)G(M)C(F)C(M)A(F) G(F)G(M)A(F)G(M)A(F)A(M)G(F)A(M)G(F)A(M) | No. 6223 | U(F)^C(M)^U(F)C(M)U(F)U(M)C(F)U(M)C(F)C(M)U(M)G(F)G (M)C(F)U(M)C(F)A(M)U(F)A(M)G(F)A(M)^A(F)^U(M) |
| CH0179 | No. 5522 | C(F)^U(M)^A(F)U(M)G(F)A(M)G(F)C(M)C(F)A(M)G(F) G(F)A(M)G(F)A(M)A(F)G(M)A(F)G(M)A(F)U(M) | No. 6224 | A(F)^U(M)^C(F)U(M)C(F)U(M)U(F)C(M)U(F)C(M)C(M)U(F)G (M)G(F)C(M)U(F)C(M)A(F)U(M)A(F)G(M)^A(F)^A(M) |
| CH0180 | No. 5523 | U(F)^A(M)^U(F)G(M)A(F)G(M)C(F)C(M)A(F)G(M)G(F) A(F)G(M)A(F)A(M)G(F)A(M)G(F)A(M)U(F)U(M) | No. 6225 | A(F)^A(M)^U(F)C(M)U(F)C(M)U(F)U(M)C(F)U(M)C(M)C(F)U (M)G(F)G(M)C(F)U(M)C(F)A(M)U(F)A(M)^G(F)^A(M) |
| CH0181 | No. 5524 | A(F)^U(M)^G(F)A(M)G(F)C(M)C(F)A(M)G(F)G(M)A(F) G(F)A(M)A(F)G(M)A(F)G(M)A(F)U(M)U(F)A(M) | No. 6226 | U(F)^A(M)^A(F)U(M)C(F)U(M)C(F)U(M)U(F)C(M)U(M)C(F)C (M)U(F)G(M)G(F)C(M)U(F)C(M)A(F)U(M)^A(F)^G(M) |

### [Table 24]

**Table 3-4**

| Double stranded nucleic acid No. | SEQ ID NO. | Sense strand sequence (5'-3') | SEQ ID NO | Antisence strand sequence (5'-3') |
|---|---|---|---|---|
| CH0183 | No.5525 | G(F)^A(M)^G(F)C(M)C(F)A(M)G(F)G(M)A(F)G(M)A(F) A(F)G(M)A(F)G(M)A(F)U(M)U(F)A(M)C(F)G(M) | No. 6227 | C(F)^G(M)^U(F)A(M)A(F)U(M)C(F)U(M)C(F)U(M)U(M)C(F)U (M)C(F)C(M)U(F)G(M)(G(F)C(M)U(F)C(M)^A(F)^U(M) |
| CH0184 | No. 5526 | A(F)^G(M)^C(F)C(M)A(F)G(M)G(F)A(M)G(F)A(M)A(F) G(F)A(M)G(F)A(M)U(F)U(M)A(F)C(M)G(F)U(M) | No. 6228 | A(F)^C(M)^G(F)U(M)A(F)A(M)U(F)C(M)U(F)C(M)U(M)U(F)C(M)U(F)C(M)U(F)C(M)(G(F)G(M)C(F)U(M)^C(F)^A(M) |
| CH0185 | No. 5527 | G(F)^C(M)^C(F)A(M)G(F)G(M)A(F)G(M)A(F)A(M)G(F) A(F)G(M)A(F)U(M)U(F)A(M)C(F)G(M)U(F)A(M) | No. 6220 | U(F)^A(M)C(F)G(M)U(F)A(M)A(F)U(M)C(F)U(M)C(M)U(F)U( M)C(F)U(M)C(F)C(M)U(F)G(M)G(F)C(M)^U(F)^C(M) |
| CH0186 | No. 5528 | C(F)^C(M)^A(F)G(M)G(F)A(M)G(F)A(M)A(F)G(M)A(F) G(F)A(M)U(F)U(M)A(F)C(M)G(F)U(M)A(F)U(M) | No. 6230 | A(F)^U(M)^A(F)C(M)G(F)U(M)A(F)A(M)U(F)C(M)U(M)C(F)U( M)U(F)C(M)U(F)C(M)C(F)U(M)G(F)G(M)^C(F)^U(M) |
| CH0187 | No.5529 | C(F)^A(M)^G(F)G(M)A(F)G(M)A(F)A(M)G(F)A(M)G(F) A(F)U(M)U(F)A(M)C(F)G(M)U(F)A(M)U(F)U(M) | No. 6231 | A(F)^A(M)^U(F)A(M)C(F)G(M)U(F)A(M)A(F)U(M)C(M)U(F)C( M)U(F)U(M)C(F)U(M)C(F)C(M)U(F)G(M)^G(F)^C(M) |
| CH0188 | No. 5530 | A(F)^G(M)^G(F)A(M)G(F)A(M)A(F)G(M)A(F)G(M)A(F) U(F)U(M)A(F)C(M)G(F)U(M)A(F)U(M)U(F)C(M) | No. 6232 | G(F)^A(M)^A(F)U(M)A(F)C(M)G(F)U(M)A(F)A(M)U(M)C(F)U( M)C(F)U(M)U(F)C(M)U(F)C(M)C(F)U(M)^G(F)^G(M) |
| CH0189 | No. 5531 | Q(F)^G(M)^A(F)G(M)A(F)A(M)G(F)A(M)G(F)A(M)U(F) U(F)A(M)C(F)G(M)U(F)A(M)U(F)U(M)C(F)C(M) | No. 6233 | G(F)^G(M)^A(F)A(M)U(G)A(M)C(F)C(M)U(F)A(M)A(M)U(F)C (M)U(F)C(M)U(F)U(M)C(F)U(M)C(F)C(M)^U(F)^G(M) |
| CH0190 | No.5532 | G(F)^A(M)^G(F)A(M)A(F)G(M)A(F)G(M)A(F)U(M)U(F) A(F)C(M)G(F)U(M)A(F)U(M)U(F)C(M)C(F)U(M) | No. 6234 | A(F)^G(M)^ G(F)A(M)A(F)U(M)A(F)C(M)G(F)U(M)A(M)A(F)U( M)C(F)U(M)C(F)U(M)U(F)C(M)U(F)C(M)C(F)U(M) |
| CH0193 | No. 5533 | A(F)^A(M)^G(F)A(M)G(F)A(M)U(F)U(M)A(F)C(M)G(F) U(F)A(M)U(F)U(M)C(F)C(M)U(F)G(M)C(F)A(M) | No.6235 | U(F)^G(M)^C(F)A(M)G(F)G(M)A(F)A(M)U(F)A(M)C(M)G(F)U (M)A(F)A(M)U(F)C(M)U(F)U(M)^CF^UM |
| CH0194 | No. 5534 | A(F)^G(M)^A(F)G(M)A(F)U(M)U(F)A(M)C(F)G(M)U(F) A(F)U(M)U(F)C(M)C(F)U(M)G(F)C(M)A(F)A(M) | No. 6236 | U(F)^U(M)^G(F)C(J)M(A)F(G)M(F)G(A)M(A)F(U)M(M)C(F)G (M)U(F)A(M)A(F)U(M)C(F)U(M)C(F)U(M)^U(F)^C(M) |
| CH0195 | No. 5535 | G(F)^A(M)^G(F)A(M)U(F)U(M)A(F)C(M)G(F)U(M)A(F) U(F)U(M)C(F)C(M)U(F)G(M)C(F)A(M)A(F)G(M) | No. 6237 | C(F)^U(M)^U(F)G(M)C(F)A(M)G(F)G(M)A(F)A(M)A(F)C (M)G(F)U(M)A(F)A(M)U(F)C(M)U(F)C(M)U(F)U(M) |
| CH0199 | No. 5536 | U(F)^U(M)^ A(F)C(M)G(F)U(M)A(F)U(M)U(F)C(M)C(F) U(F)G(M)C(F)A(M)A(F)G(M)C(F)C(M)G(F)G(M) | No. 6238 | C(F)^C(M)^G(F)G(M)C(F)U(M)U(F)G(M)C(F)A(M)G(M)G(F) A(M)A(F)U(M)A(F)C(M)G(F)U(M)A(F)A(M)^U(F)^C(M) |
| CH0203 | No. 5537 | G(F)^U(M)^A(F)U(M)U(F)C(M)C(F)U(M)G(F)C(M)A(F) A(F)G(M)C(F)C(M)G(F)G(M)G(M)G(F)C(M)U(F)A(M) | No. 6239 | U(F)^A(M)G(F)C(M)C(F)C(M)G(F)G(M)C(F)U(M)U(M)G(F) C(M)A(F)G(M)G(F)A(M)A(F)U(M)A(F)C(M)^G(F)^U(M) |
| CH0207 | No. 5538 | U(F)^C(M)^C(F)U(M)G(F)C(M)A(F)A(M)G(F)C(M)C(F) G(F)G(M)G(F)C(M)U(F)A(M)U(F)G(M)U(F)G(M) | No. 6240 | C(M)U(F)U(M)G(F)C(M)A(F)G(M)G(F)A(M)^A(F)^U(M) |
| CH0225 | No. 5539 | G(F)^U(M)^G(F)U(M)C(F)C(M)C(F)G(M)A(F)G(M)G(F) A(F)G(M)G(F)G(M)A(F)U(M)G(F)A(M)G(F)A(M) | No. 6241 | U(F)^C(M)^U(F)C(M)A(F)U(M)C(F)C(M)C(F)U(M)C(M)C(F)U (M)C(F)G(M)G(F)G(M)A(F)C(M)A(F)C(M)^A(F)^U(M) |
| CH0226 | No.5540 | G(F)G(M)G(F)A(M)U(F)G(M)A(F)G(M)A(F)A(M) | No. 6242 | U(F)^U(M)^C(F)U(M)C(F)A(M)U(F)C(M)C(F)C(L)U(M)C(F)C (M)U(F)C(M)G(F)G(M)G(F)G(F)G(F)A(M)C(F)A(M)^C(F)^A(M) |
| CH0229 | No.5541 | C(F)^C(M)^C(F)G(M)A(F)G(M)G(F)A(M)G(F)G(M)G(F) A(F)U(M)G(F)A(M)G(F)A(M)A(F)A(M)G(F)U(M) | No. 6243 | A(F)^C(M)^U(F)U(M)U(F)C(L)U(F)C(L)A(F)U(L)C(L)C(F)C (M)U(F)C(M)C(F)U(M)C(F)G(M)G(F)G(M)^A(F)^C(M) |
| CH0230 | No.5542 | C(F)^C(M)^G(F)A(M)G(F)G(M)A(F)G(M)G(F)G(M)A(F) U(F)G(M)A(F)G(M)A(F)A(M)A(F)G(M)U(F)U(M) | No. 6244 | A(F)^A(M)^C(F)U(M)U(F)U(M)C(F)U(M)C(F)A(M)U(M)C(F)C (M)C(F)U(M)C(F)C(M)U(F)C(M)G(F)G(M)^G(F)^A(M) |
| CH0231 | No. 5543 | C(F)^G(M)^A(F)G(M)G(F)A(M)G(F)G(M)G(F)A(M)U(F) G(F)A(M)G(F)A(M)A(F)A(M)G(F)U(M)U(F)U(M) | No. 6245 | A(F)^A(M)^A(F)C(M)U(F)U(M)U(F)U(M)U(F)C(M)U(F)C(M)U(F)C(M)A(M)U(F)C( M)C(F)C(M)U(F)M(C)F(U)M(C)F(G)^M(F)^G(M) |
| CH0232 | No. 5544 | G(F)^A(M)^G(F)G(M)A(F)G(M)G(F)G(M)A(F)U(M)G(F) A(F)G(M)A(F)A(M)A(F)G(M)U(F)U(M)U(F)A(M) | No. 6246 | U(F)^A(M)^A(F)A(M)C(F)U(M)U(F)U(M)C(F)U(M)C(M)A(F)U( M)C(F)C(M)C(F)U(M)C(F)C(M)U(F)C(M)^G(F)^G(M) |
| CH0233 | No.5545 | A(F)^G(M)^G(F)A(M)G(F)G(M)G(F)A(M)U(F)G(M)A(F) G(F)A(M)A(F)A(M)G(F)U(M)U(F)U(M)A(F)U(M) | No. 6247 | A(F)^U(M)^A(F)A(M)A(F)C(M)U(F)U(M)U(F)C(M)U(M)C(F)A( M)U(F)C(M)C(F)C(M)U(F)C(M)C(F)U(M)^C(F)^G(M) |
| CH0234 | No.5546 | G(F)^G(M)^A(F)G(M)G(F)G(M)A(F)U(M)G(F)A(M)G(F) A(F)A(M)A(F)G(M)U(F)U(M)U(F)A(M)U(F)C(M) | No. 6248 | G(F)^A(M)^U(F)A(M)A(F)A(M)C(F)U(M)U(F)U(M)C(M)U(F)C( M)A(F)U(M)C(F)C(M)C(F)U(M)C(F)C(M)^U(F)^C(M) |
| CH0235 | No.5547 | G(F)^A(M)^G(F)G(M)G(F)A(M)U(F)G(M)A(F)G(M)A(F) A(F)A(M)G(F)U(M)U(F)U(M)A(F)U(M)C(F)U(M) | No. 6249 | A(F)^G(M)^A(F)U(M)A(F)A(M)A(F)C(M)U(F)U(M)U(M)C(F)U( M)C(F)A(M)U(F)C(M)C(F)C(M)I(F)C(M)C(F)U(M) |
| CH0236 | No.5548 | A(F)G(M)U(F)U(M)U(F)A(M)U(F)C(M)U(F)G(M) | No. 6260 | C(F)^A(M)^G(F)A(M)U(F)A(M)A(F)A(M)C(F)U(M)U(M)U(F)C( M)U(F)C(M)A(F)U(M)C(F)C(M)C(F)U(M)^C(F)^C(M) |
| CH0242 | No. 5549 | G(F)^A(M)^G(F)A(M)A(F)A(M)G(F)U(M)U(F)U(M)A(F) U(F)C(M)U(F)G(M)C(F)C(M)C(F)U(M)C(F)U(M) | No. 6261 | A(F)^G(M)^A(F)G(M)G(F)G(M)C(F)A(M)G(F)A(M)U(M)A(F)A (M)A(F)C(M)U(F)U(M)U(F)C(M)U(F)C(M)^A(F)^U(M) |
| CH0244 | No.5550 | G(F)^A(M)^A(F)A(M)G(F)U(M)U(F)U(M)A(F)U(M)C(F) U(F)G(M)C(F)C(M)C(F)U(M)C(F)U(M)C(F)A(M) | No. 6352 | U(F)^G(M)^A(F)G(M)A(F)G(M)G(F)G(M)C(F)A(M)G(M)A(F)U (M)A(F)A(M)A(F)C(M)U(F)U(M)U(F)C(M)U(F)C(M) |
| CH0255 | No.5551 | U(F)^G(M)^C(F)C(M)C(F)U(M)C(F)U(M)C(F)A(M)C(F) A(F)G(M)G(F)A(M)C(F)U(M)G(F)U(M)G(F)G(M) | No. 6253 | C(F)^C(M)^A(F)C(M)A(F)G(M)U(F)C(M)C(F)U(M)G(M)U(F)G (M)A(F)G(M)A(F)G(M)G(F)G(M)C(F)A(M)^G(F)^A(M) |
| CH0260 | No.5552 | U(F)^C(M)^U(F)C(M)A(F)C(M)A(F)C(M)A(F)C(M)G(F)A(M)C(F) U(F)G(M)U(F)G(M)G(F)C(M)C(F)C(M)A(F)U(M) | No. 6254 | A(F)^U(M)^G(F)G(M)G(F)C(M)C(F)A(M)C(F)A(M)G(M)U(F)C (M)C(F)U(M)G(F)U(M)G(F)A(M)G(F)A(M)^G(F)^G(M) |
| CH0262 | No. 5553 | U(F)^C(M)^A(F)C(M)A(F)G(M)G(F)A(M)C(F)U(M)G(F) U(F)G(M)G(F)C(M)C(F)C(M)A(F)U(M)C(F)A(M) | No. 6255 | U(F)^G(M)^A(F)U(M)G(F)G(M)G(F)C(M)C(F)A(M)C(M)A(F)G (M)U(F)C(M)C(F)U(M)G(F)U(M)G(F)A(M)^G(F)^A(M) |
| CH0265 | No. 5554 | C(F)^A(M)^G(F)G(M)A(F)C(M)U(F)G(M)U(F)G(M)G(F) C(F)C(M)C(F)A(M)U(F)C(M)A(F)A(M)C(F)A(M) | No. 6356 | U(F)^G(M)^U(F)U(M)G(F)A(M)U(F)G(M)G(F)G(M)C(M)C(F)A (M)C(F)A(M)G(F)U(M)C(F)C(M)U(F)G(M)^U(F)^G(M) |
| CH0266 | No. 5555 | A(F)^G(M)^G(F)A(M)C(F)U(M)G(F)U(M)G(F)G(M)C(F) C(F)C(M)A(F)U(M)C(F)A(M)A(F)C(M)A(F)C(M) | No. 6257 | G(F)^U(M)^G(F)U(M)U(F)G(M)A(F)U(M)G(F)G(M)G(M)C(F)C (M)A(F)C(M)A(F)G(M)U(F)C(M)C(F)U(M)^G(F)^U(M) |
| CH0272 | No.5556 | G(F)^U(M)^G(F)G(M)C(F)C(M)C(F)A(M)U(F)C(M)A(F) A(F)C(M)A(F)C(M)U(F)C(M)U(F)G(M)A(F)A(M) | No. 6258 | U(F)^U(M)^C(F)A(M)G(F)A(M)G(F)U(M)G(F)U(M)U(M)G(F)A (M)U(F)G(M)G(F)G(M)C(F)C(M)A(F)C(M)^A(F)^G(M) |
| CH0274 | No.5557 | G(F)^G(M)^C(F)C(M)C(F)A(M)U(F)C(M)A(F)A(M)C(F) A(F)G(M)U(F)C(M)U(F)G(M)A(F)A(M)A(F)U(M) | No. 6269 | A(F)^U(M)^U(F)U(M)C(F)A(M)G(F)A(M)G(F)U(M)G(M)U(F)U( M)G(F)A(M)U(F)G(M)G(F)G(M)C(F)C(M)^A(F)^C(M) |
| CH0275 | No. 5558 | G(F)^C(M)^C(F)C(M)A(F)U(M)C(F)A(M)A(F)C(M)A(F) C(F)U(M)C(F)U(M)G(F)A(M)A(F)A(M)U(F)G(M) | No. 6260 | G(F)^A(M)^U(F)U(M)U(F)C(M)A(F)G(M)A(F)G(M)U(M)G(F)U (M)U(F)G(M)A(F)U(M)G(F)G(M)G(F)C(M)^C(F)^A(M) |
| CH0276 | No. 5559 | C(F)^C(M)^C(F)A(M)U(F)C(M)A(F)A(M)C(F)A(M)C(F) U(F)C(M)U(F)G(M)A(F)A(M)A(F)U(M)G(F)U(M) | No. 6261 | A(F)^C(M)^A(F)U(M)U(F)U(M)C(F)A(M)G(F)A(M)G(M)U(F)G (M)U(F)U(M)G(F)A(M)U(F)G(M)G(F)G(M)^C(F)^C(M) |
| CH0277 | No. 5560 | C(F)^C(M)^A(F)U(M)C(F)A(M)A(F)C(M)A(F)C(M)U(F) C(F)U(M)G(F)A(M)A(F)A(M)U(F)G(M)U(F)A(M) | No. 6262 | U(F)^A(M)^C(F)A(M)U(F)U(M)U(F)C(M)A(F)G(M)A(M)G(F)U( M)G(F)U(M)U(F)G(M)A(F)U(M)G(F)G(M)^G(F)^C(M) |
| CH0279 | No. 5561 | A(F)^U(M)^C(F)A(M)A(F)C(M)A(F)C(M)U(F)C(M)U(F) G(F)A(M)A(F)A(M)U(F)G(M)U(F)A(M)C(F)A(M) | No. 6263 | U(F)^G(M)^U(F)A(M)C(F)A(M)U(F)U(M)U(F)C(M)A(M)G(F)A( M)G(F)U(M)G(F)U(M)U(G)F(M)A(F)U(M)^G(F)^G(M) |
| CH0283 | No. 5562 | A(F)^C(M)^A(F)C(M)U(F)C(M)U(F)G(M)A(F)A(M)A(F)U(F)G(M)U(F)A(M)C(F)A(M)C(F)C(M)C(F)A(M) | No. 6264 | U(F)^G(M)^G(F)G(M)U(F)G(M)U(F)A(M)C(F)A(M)U(M)U(F)U (M)C(F)A(M)G(F)A(M)G(F)U(M)G(F)U(M)^U(F)^G(M) |
| CH0285 | No. 5563 | A(F)^C(M)^U(F)C(M)U(F)G(M)A(F)A(M)A(F)U(M)G(F) U(F)A(M)C(F)A(M)C(F)C(M)C(F)A(M)G(F)A(M) | No. 6265 | U(F)^C(M)^U(F)G(M)G(F)G(M)U(F)G(M)U(F)A(M)C(M)A(F)U (M)U(F)U(M)C(F)A(M)G(F)A(M)G(F)U(M)^G(F)^U(M) |
| CH0288 | No.5564 | C(F)^U(M)^G(F)A(M)A(F)A(M)U(F)G(M)U(F)A(M)C(F) A(F)C(M)C(F)C(M)A(F)G(M)A(F)G(M)U(F)A(M) | No. 6266 | U(F)^A(M)^C(F)U(M)C(F)U(M)G(F)U(M)G(M)U(F)A (M)C(F)A(M)U(F)U(M)U(F)C(M)A(G)M^A(F)^G(M) |

### [Table 25]

**Table 3-5**

| Double stranded nucleic acid No. | SEQ ID NO | Serse strand sequence (5'→3') | SEQ ID NO. | Antisense strand sequence (5'→3') |
|---|---|---|---|---|
| CH0289 | No. 5565 | U(F)^G(M)^A(F)A(M)A(F)U(M)G(F)U(M)A(F)C(M)A(F) C(F)C(M)C(F)A(M)G(F)A(M)G(F)U(M)A(F)U(M) | No. 6267 | A(F)^U(M)^A(F)C(M)U(F)C(M)U(F)G(M)G(F)G(M)U(M)G(F)U (M)A(F)C(M)A(F)U(M)U(F)U(M)C(F)A(M)G(F)A(M) |
| CH0291 | No. 5566 | A(F)^A(M)^A(F)U(M)G(F)U(M)A(F)C(M)A(F)C(M)C(F) C(F)A(M)G(F)A(M)G(F)U(M)A(F)U(M)G(F)U(M) | No. 6268 | A(F)^C(M)^A(F)U(M)A(F)C(M)U(F)C(M)U(F)G(M)G(M)G(F)U (M)G(F)Y(L)A(F)C(M)A(F)U(M)U(F)U(M)^C(F)^A(M) |
| CH0284 | No. 5567 | U(F)^G(M)^U(F)A(M)C(F)A(M)C(F)C(M)C(F)A(M)G(F) A(F)G(M)U(F)A(M)U(F)G(M)U(F)C(M)C(F)U(M) | No. 6269 | A(F)^G(M)^G(F)A(M)C(F)A(M)U(F)A(M)C(F)U(M)C(M)U(F)G (M)G(F)G(M)U(G)G(M)U(G)A(M)C(F)A(M)^U(F)^U(M) |
| CH0295 | No. 5568 | G(F)^U(M)^A(F)C(M)A(F)C(M)C(F)C(M)A(F)G(M)A(F) G(F)U(M)A(F)U(M)G(F)U(M)C(F)C(M)U(F)U(M) | No. 6270 | A(F)^A(M)^G(F)G(M)A(F)C(M)A(F)U(M)A(F)C(M)U(M)C(F)U (M)G(F)G(M)G(F)U(M)G(F)U(M)A(F)C(M)^A(F)^U(M) |
| CH0296 | No. 5569 | U(F)^A(M)^C(F)A(M)C(F)C(M)C(F)A(M)G(F)A(M)G(F) U(F)A(M)U(F)G(M)U(F)C(M)C(F)U(M)U(F)U(M) | No. 6271 | A(F)^A(M)^A(F)G(M)G(F)A(M)C(F)A(M)U(F)A(M)C(M)U(F)G (M)U(F)G(M)G(F)G(M)U(F)G(M)U(F)A(M)^C(F)^A(M) |
| CH0297 | No. 5570 | A(F)^C(M)^A(F)C(M)C(F)C(M)A(F)G(M)A(F)G(M)U(F) A(F)U(M)G(F)U(M)C(F)C(M)U(F)U(M)U(F)U(M) | No. 6272 | A(F)^A(M)^A(F)A(L)G(F)G(L)A(F)C(M)A(F)U(M)A(M)C(F)U( M)C(F)U(M)G(F)G(M)G(F)U(M)G(F)U(M)^A(F)^C(M) |
| CH0298 | No. 5571 | C(F)^A(M)^C(F)C(M)C(F)A(M)G(F)A(M)G(F)U(M)A(F) U(F)G(M)U(F)C(M)C(F)U(M)C(F)U(M)U(F)U(M)U(F)G(M) | No. 6273 | C(F)^A(M)^A(F)A(M)A(F)G(M)G(F)A(M)C(F)A(M)U(M)A(F)C (M)U(F)C(M)U(F)G(M)G(F)G(M)U(F)G(M)^U(F)^A(M) |
| CH0300 | No. 5572 | C(F)^C(M)^C(F)A(M)G(F)A(M)G(F)U(M)A(F)U(M)G(F) U(F)C(M)C(F)U(M)U(F)U(M)U(F)G(M)C(F)U(M) | No. 6274 | A(F)^G(M)^C(F)A(M)A(F)A(M)A(F)G(M)G(F)A(M)C(M)A(F)U (M)A(F)C(M)U(F)C(M)U(F)G(M)G(F)G(M)^U(F)^G(M) |
| CH0301 | No. 5573 | C(F)C(M)A(F)G(M)A(F)G(M)U(F)A(M)U(F)G(M)U(F) C(F)C(M)U(F)U(M)U(F)U(M)G(F)C(M)U(F)G(M) | No. 6275 | C(F)^A(M)^G(F)C(M)A(F)A(M)A(F)A(M)G(F)G(M)A(M)C(F)A (M)U(F)A(M)C(F)U(M)C(F)U(M)G(F)G(M)^G(F)^U(M) |
| CH0302 | No. 5574 | C(F)^A(M)^G(F)A(M)G(F)U(M)A(F)U(M)G(F)U(M)C(F) C(F)U(M)U(F)U(M)U(F)G(M)C(F)U(M)G(F)G(M) | No. 6276 | C(F)^C(M)^A(F)G(M)C(F)A(M)A(F)A(M)A(F)G(M)G(M)A(F)C (M)A(F)U(M)A(F)C(M)U(F)C(M)U(F)G(M)^G(F)^G(M) |
| CH0303 | No. 5575 | A(F)^G(M)^A(F)G(M)U(F)A(M)U(F)G(M)U(F)C(M)C(F) U(F)U(M)U(F)U(M)G(F)C(M)U(F)G(M)G(F)A(M) | No. 6277 | U(F)^C(M)^C(F)A(M)G(F)C(M)A(F)A(M)G(M)G(F)A (M)C(F)A(M)U(F)A(M)C(F)U(M)C(F)U(M)^G(F)^G(M) |
| CH0304 | No. 5576 | G(F)^A(M)^G(F)U(M)A(F)U(M)G(F)U(M)C(F)C(M)U(F) U(F)U(M)U(F)G(M)C(F)U(M)G(F)G(M)A(F)A(M) | No. 6278 | U(F)^U(M)^C(F)C(M)A(F)G(M)C(F)A(M)A(F)A(M)A(M)G(F)G (M)A(F)C(M)A(F)U(M)A(F)C(M)U(F)C(M)^U(F)^G(M) |
| CH0306 | No. 5577 | G(F)^U(M)^A(F)U(M)G(F)U(M)C(F)C(M)U(F)U(M)U(F) U(F)G(M)C(F)U(M)G(F)G(M)A(F)A(M)U(F)C(M) | No. 6279 | G(F)^A(M)^U(F)U(M)C(F)C(M)A(F)G(M)C(F)A(M)A(M)A(F)A (M)G(F)G(M)A(F)C(M)A(F)U(M)A(F)C(M)^U(F)^C(M) |
| CH0307 | No. 5518 | U(F)^A(M)^U(F)G(M)U(F)C(M)C(F)U(M)U(F)U(M)U(F) G(F)C(M)U(F)G(M)G(F)A(M)A(F)U(M)C(F)U(M) | No. 6280 | A(F)^G(M)^A(F)U(M)U(F)C(M)C(F)A(M)G(F)C(M)A(M)A(F)A (M)A(F)G(M)G(F)A(M)C(F)A(M)U(F)A(M)^C(F)^U(M) |
| CH0308 | No. 5579 | A(F)^U(M)^G(F)U(M)C(F)C(M)U(F)U(M)U(F)U(M)G(F) C(F)U(M)G(F)G(M)A(F)A(M)U(F)C(M)U(F)U(M) | No. 6281 | A(F)^A(M)^G(F)A(M)U(F)U(M)C(F)C(M)A(F)G(M)C(M)A(F)A (M)A(F)A(M)G(F)G(M)A(F)C(M)A(F)U(M)^A(F)^C(M) |
| CH0309 | No. 5580 | U(F)^G(M)^U(F)C(M)C(F)U(M)U(F)U(M)U(F)G(M)C(F) U(F)G(M)G(F)A(M)A(F)U(M)C(F)U(M)U(F)A(M) | No. 6282 | U(F)^A(M)^A(F)G(M)A(F)U(M)U(F)C(M)C(F)A(M)G(M)C(F)A (M)A(F)A(M)A(F)G(M)G(F)A(M)C(F)A(M)^U(F)^A(M) |
| CH0310 | No. 5581 | G(F)^U(M)^C(F)C(M)U(F)U(M)U(F)U(M)G(F)C(M)U(F) G(F)G(M)A(F)A(M)U(F)C(M)U(F)U(M)A(F)G(M) | No. 6283 | C(F)^U(M)^A(F)A(M)G(F)A(M)U(F)U(M)C(F)C(M)A(M)G(F)C (M)A(F)A(M)A(F)A(M)G(F)G(M)A(F)C(M)^A(F)^U(M) |
| CH0311 | No. 5582 | U(F)^C(M)^C(F)U(M)U(F)U(M)U(F)G(M)C(F)U(M)G(F) G(F)A(M)A(F)U(M)C(F)U(M)U(F)A(M)G(F)A(M) | No. 6284 | U(F)^C(M)^U(F)A(M)A(F)G(M)A(F)U(M)U(F)C(M)C(M)A(F)G (M)C(F)A(M)A(F)A(M)A(F)G(M)G(F)A(M)^C(F)^A(M) |
| CH0314 | No. 5583 | U(F)^U(M)^U(F)U(M)G(F)C(M)U(F)G(M)G(F)A(M)A(F) U(F)C(M)U(F)U(M)A(F)G(M)A(F)A(M)A(F)A(M) | No. 6285 | U(F)U(M)U(F)U(M)C(F)U(M)A(F)A(M)G(F)A(M)U(M)U(F)C( M)C(F)A(M)G(F)C(M)A(F)A(M)A(F)A(M)^G(F)^G(M) |
| CH0315 | No. 5584 | U(F)^U(M)^U(F)G(M)C(F)U(M)G(F)G(M)A(F)A(M)U(F) C(F)U(M)U(F)A(M)G(F)A(M)A(F)A(M)A(F)U(M) | No. 6286 | A(F)U(M)U(F)U(M)U(F)C(M)U(F)A(M)A(F)G(M)A(M)U(F)U( M)C(F)C(M)A(F)G(M)C(F)A(M)A(F)A(M)^A(F)^G(M) |
| CH0316 | No. 5585 | U(F)^U(M)^G(F)C(M)U(F)G(M)G(F)A(M)A(F)U(M)C(F) U(F)U(M)A(F)G(M)A(F)A(M)A(F)A(M)U(F)G(M) | No. 6287 | C(F)^A(M)^U(F)U(M)U(F)U(M)C(F)U(M)A(F)A(M)G(M)A(F)U( M)U(F)C(M)C(F)A(M)G(F)C(M)A(F)A(M)^A(F)^A(M) |
| CH0317 | No. 5586 | U(F)^G(M)^C(F)U(M)G(F)G(M)A(F)A(M)U(F)C(M)U(F) U(F)A(M)G(F)A(M)A(F)A(M)A(F)U(M)G(F)G(M) | No. 6288 | C(F)^C(M)^A(F)U(M)U(F)U(M)U(F)C(M)U(F)A(M)A(M)G(F)A( M)U(F)U(M)C(F)C(M)A(F)G(M)C(F)A(M)^A(F)^A(M) |
| CH0318 | No. 5587 | G(F)^C(M)^U(F)G(M)G(F)A(M)A(F)U(M)C(F)U(M)U(F) A(F)G(M)A(F)A(M)A(F)A(M)U(F)G(M)G(F)A(M) | No. 6289 | A(F)^A(M) |
| CH0319 | No. 5588 | C(F)U(M)G(F)G(M)A(F)A(M)U(F)C(M)U(F)U(M)A(F) G(F)A(M)A(F)A(M)A(F)U(M)G(F)G(M)A(F)G(M) | No. 6290 | C(F)U(M)C(F)C(M)A(F)U(M)U(F)U(M)U(F)U(M)U(F)C(M)U(M)A(F)A( M)G(F)A(M)U(F)U(M)C(F)C(M)A(F)G(M)^C(F)^A(M) |
| CH0324 | No. 5589 | A(F)^U(M)^C(F)U(M)U(F)A(M)G(F)A(M)A(F)A(M)A(F)U (F)G(M)G(F)A(M)G(F)C(M)C(F)G(M)U(F)A(M) | No. 6291 | U(F)^A(M)^C(F)G(M)G(F)C(M)C(F)A(M)U(M)U(F)U (M)U(F)C(M)U(F)A(M)A(F)G(M)A(F)U(M)^U(F)^C(M) |
| CH0327 | No. 5590 | U(F)^U(M)^A(F)G(M)A(F)A(M)A(F)A(M)U(F)G(M)G(F) A(F)G(M)C(F)C(M)G(F)U(M)A(F)C(M)G(F)C(M) | No. 6292 | G(F)^C(M)^G(F)U(M)A(F)C(M)G(F)G(M)C(F)U(M)C(M)C(F) A(M)U(F)U(M)U(F)U(M)C(F)U(M)A(F)A(M)^G(F)^A(M) |
| CH0328 | No. 5591 | U(F)^A(M)^G(F)A(M)A(F)A(M)A(F)U(M)G(F)G(M)A(F) G(F)C(M)C(F)G(M)U(F)A(M)C(F)G(M)C(F)U(M) | No. 6293 | A(F)^G(M)^C(F)G(M)U(F)A(M)C(F)G(M)G(F)C(M)U(M)C(F) C(M)A(F)U(M)U(F)U(M)U(F)U(M)U(F)C(M)U(F)A(M)A(F)G(M) |
| CH0329 | No. 5592 | A(F)^G(M)^A(F)A(M)A(F)A(M)U(F)G(M)G(F)A(M)G(F) C(F)C(M)G(F)U(M)A(F)C(M)G(F)C(M)U(F)A(M) | No. 6294 | U(F)^A(M)^G(F)C(M)G(F)U(M)A(F)C(M)G(F)G(M)C(M)U(F)C (M)C(F)A(M)U(F)U(F)U(M)C(F)U(M)^A(F)^A(M) |
| CH0330 | No. 5593 | G(F)^A(M)^A(F)A(M)A(F)U(M)G(F)G(M)A(F)G(M)C(F) C(F)G(M)U(F)A(M)C(F)G(M)C(F)U(M)A(F)U(M) | No. 6295 | A(F)^U(M)^A(F)G(M)C(F)G(M)U(F)A(M)C(F)G(M)G(M)C(F)U (M)C(F)C(M)A(F)U(M)U(F)U(M)U(F)C(M)^U(F)^A(M) |
| CH0331 | No. 5594 | A(F)^A(M)^A(F)A(M)U(F)G(M)G(F)A(M)G(F)C(M)C(F) G(F)U(M)A(F)C(M)G(F)C(M)U(F)A(M)U(F)A(M) | No. 6296 | UF^AM^U(F)A(M)G(F)C(M)G(F)U(M)A(F)C(M)G(M)G(F)C (M)U(F)C(M)C(F)A(M)U(F)U(M)U(F)U(M)^C(F)^U(M) |
| CH0334 | No. 5595 | A(F)^U(M)^G(F)G(M)A(F)G(M)C(F)C(M)G(F)U(M)A(F) C(F)G(M)C(F)U(M)A(F)U(M)A(F)C(M)G(F)A(M) | No. 6297 | U(F)^C(M)^G(F)U(M)A(F)U(M)A(F)G(M)C(F)G(M)U(M)A(F)C (M)G(F)G(M)C(F)U(M)C(F)C(M)A(F)U(M)^U(F)^U(M) |
| CH0335 | No. 5596 | U(F)^G(M)^G(F)A(M)G(F)C(M)C(F)G(M)U(F)A(M)G(F) G(F)C(M)U(F)A(M)U(F)A(M)C(F)G(M)A(F)C(M) | No. 6298 | G(F)U^(M)^C(F)G(M)U(F)A(M)U(F)A(M)G(F)C(M)G(M)U(F)A (M)C(F)G(M)G(F)C(M)U(F)C(M)C(F)A(M)^U(F)^U(M) |
| CH0336 | No. 5597 | G(F)^G(M)^A(F)G(M)C(F)C(M)G(F)U(M)A(F)C(M)G(F) C(F)U(M)A(F)U(M)A(F)C(M)G(F)A(L)C(F)U(M) | No. 6299 | A(F)^G(M)^U(F)C(M)G(F)U(M)A(F)U(M)A(F)G(M)C(M)G(F)U (M)A(F)C(M)G(F)G(M)C(F)U(M)C(F)C(M)^A(F)^U(M) |
| CH0337 | No. 5598 | G(F)^A(M)^G(F)C(M)C(F)G(M)U(F)A(M)C(F)G(M)C(F) U(F)A(M)U(F)A(M)C(F)G(M)A(F)C(M)U(F)U(M) | No. 6300 | A(F)^A(M)^G(F)U(M)C(F)G(M)U(F)A(M)U(F)A(M)G(M)C(F)G (M)U(F)A(M)C(F)G(M)G(F)C(M)U(F)C(M)^C(F)^A(M) |
| CH0338 | No. 5599 | A(F)^G(M)^C(F)C(M)G(F)U(M)A(F)C(M)G(F)C(M)U(F) A(F)U(M)A(F)C(M)G(F)A(M)C(F)U(M)U(F)U(M) | No. 6301 | A(F)^A(M)^A(F)G(M)U(F)C(M)G(F)U(M)A(F)U(M)A(M)G(F)C (M)G(F)U(M)A(F)C(M)G(F)G(M)C(F)U(M)^C(F)^C(M) |
| CH0339 | No. 5600 | G(F)^C(M)^C(F)G(M)U(F)A(M)C(F)G(M)C(F)U(M)A(F) U(F)A(M)C(F)G(M)A(F)C(M)U(F)U(M)U(F)U(M) | No. 6302 | A(F)^A(M)^A(F)A(M)G(F)U(M)C(F)G(M)U(F)A(M)U(M)A(F)G( M)C(F)G(M)U(F)A(M)C(F)G(M)G(F)C(M)^U(F)^C(M) |
| CH0340 | No. 5601 | C(F)^C(M)^ G(F)U(M)A(F)C(M)G(F)C(M)U(F)A(M)U(F) A(F)C(M)G(F)A(M)C(F)U(M)U(F)U(M)U(F)G(M) | No. 6303 | C(F)^A(M)^A(F)A(M)A(F)G(M)U(F)C(M)G(F)U(M)A(M)U(F)A( M)G(F)C(M)G(F)U(M)A(F)C(M)G(F)G(M)^C(F)^U(M) |
| CH0341 | Nο. 5602 | C(F)^G(M)^U(F)A(M)C(F)G(M)C(F)U(M)A(F)U(M)A(F) C(F)G(M)A(F)C(M)U(F)U(M)U(F)U(M)G(F)A(M) | No. 6304 | U(F)^C(M)^A(F)A(M)A(F)A(M)G(F)U(M)C(F)G(M)U(M)A(F)U( M)A(F)G(M)C(F)G(M)U(F)A(M)C(F)G(M)^G(F)^C(M) |
| CH0342 | No. 5603 | G(F)^U(M)^A(F)C(M)G(F)C(M)U(F)A(M)U(F)A(M)C(F) G(F)A(M)C(F)U(M)U(F)U(M)U(F)G(M)A(F)A(M) | No. 6305 | U(F)^U(M)^C(F)A(M)A(F)A(M)A(F)G(M)U(F)C(M)G(M)U(F)A( M)U(F)A(M)G(F)C(M)G(F)U(M)A(F)C(M)G(F)G(M) |
| CH0343 | No. 5604 | U(F)^A(M)^C(F)G(M)C(F)U(M)A(F)U(M)A(F)C(M)G(F) A(F)C(M)U(F)U(M)U(F)U(M)G(F)A(M)A(F)U(M) | No. 6306 | A(F)U(M)U(F)C(M)A(F)A(M)A(F)A(M)G(F)U(M)C(M)G(F)U( M)A(F)U(M)A(F)G(M)C(F)G(L)U(F)A(M)^C(F)^G(M) |

### [Table 26]

**Table 3-6**

| Double stranded nucleic acid No. | SEQ ID NO. | Sense strand sequence (5'→3') | SEQ ID NO. | Antisense strand sequence (5'→3') |
|---|---|---|---|---|
| CH0345 | No. 5605 | C(F)^G(M)^C(F)U(M)A(F)U(M)A(F)C(M)G(F)A(M)C(F) U(F)U(M)U(F)U(M)G(F)A(M)A(F)U(M)A(F)U(M) | No. 6307 | A(F)^U(M)^A(F)U(M)U(F)C(M)A(F)A(M)A(F)G(M)U(F)C( M)G(F)U(M)A(F)U(M)A(F)G(M)C(F)G(M)^U(F)^A(M) |
| CH0346 | No. 5606 | G(F)^C(M)^U(F)A(M)U(F)A(M)C(F)G(M)A(F)C(M)U(F) U(F)U(M)U(F)G(M)A(F)A(M)U(F)A(M)U(F)C(M) | No. 6308 | G(F)^A(M)^U(F)A(M)U(F)U(M)C(F)A(M)A(F)A(M)A(M)G(F)U( M)C(F)G(M)U(F)A(M)U(F)A(M)G(F)C(M)^G(F)^U(M) |
| CH0349 | No. 5607 | A(F)^U(M)^A(F)C(M)G(F)A(M)C(F)U(M)U(F)U(M)U(F) G(F)A(M)A(F)U(M)A(F)U(M)C(F)C(M)C(F)A(M) | No. 6309 | U(F)^G(M)^G(F)G(M)A(F)U(M)A(F)U(M)U(F)C(M)A(M)A( M)A(F)G(M)U(F)C(M)G(F)U(M)A(F)U(M)^A(F)^G(M) |
| CH0350 | No. 5608 | U(F)^A(M)^C(F)G(M)A(F)C(M)U(F)U(M)U(F)U(M)C(F) A(F)A(M)U(F)A(M)U(F)C(M)C(F)C(M)A(F)A(M) | No. 6310 | U(F)^U(M)^G(F)G(M)G(F)A(M)U(F)U(M)C(M)A(F)A( M)A(F)A(M)G(F)U(M)C(F)G(M)U(F)A(M)^U(F)^A(M) |
| CH0351 | No. 5609 | A(F)^C(M)^G(F)A(M)C(F)U(M)U(F)U(M)U(F)G(M)A(F) A(F)U(M)A(F)U(M)C(F)C(M)C(F)A(M)A(F)C(M) | No. 6311 | G(F)^U(M)^U(F)G(M)G(F)G(M)A(F)U(M)A(F)U(M)U(M)C(F)A (M)A(F)A(M)A(F)G(M)U(F)C(M)G(F)U(M)^A(F)^U(M) |
| CH0352 | No. 5610 | C(F)^G(M)^A(F)C(M)U(F)U(M)U(F)U(M)G(F)A(M)A(F) U(F)A(M)U(F)C(M)C(F)C(M)A(F)A(M)C(F)A(M) | No. 6312 | U(F)^G(M)^U(F)U(M)G(F)G(M)G(F)A(M)U(F)A(M)U(M)U(F)C |
| CH0355 | No. 5611 | C(F)^U(M)U(F)U(M)U(F)G(M)A(F)A(M)U(F)A(M)U(F) C(F)C(M)C(F)A(M)A(F)C(M)A(F)C(M)G(F)A(M) | No. 6313 | (M)U(F)U(M)C(F)A(M)A(F)A(M)A(F)G(M)^U(F)^C(M) |
| CH0356 | No. 5612 | U(F)^U(M)^U(F)U(M)G(F)A(M)A(F)U(M)A(F)U(M)C(F) C(F)C(M)A(F)A(M)C(F)A(M)C(F)G(M)A(F)U(M) | No. 6314 | A(F)^U(M)^C(F)G(M)U(F)G(M)U(F)U(M)G(F)G(M)G(M)A(F)U (M)A(F)U(M)U(F)C(M)A(F)A(M)A(F)A(M)^G(F)U(M) |
| CH0357 | No. 5613 | U(F)^U(M)^U(F)G(M)A(F)A(M)U(F)A(M)U(F)C(M)C(F) C(F)A(M)A(F)C(M)A(F)C(M)G(F)A(M)U(F)C(M) | No. 6315 | G(F)^A(M)^U(F)C(M)G(F)U(M)G(F)U(M)U(F)G(M)G(M)A(F)A (M)U(F)A(M)U(F)U(M)C(F)A(M)A(F)A(M)^A(F)^G(M) |
| CH0358 | No. 5614 | U(F)^U(M)^G(F)A(M)A(F)U(M)A(F)U(M)C(F)C(M)C(F) A(F)A(M)C(F)A(M)C(F)G(M)A(F)U(M)C(F)A(M) | No. 6316 | U(F)^G(M)^A(F)U(M)C(F)G(M)U(F)G(M)U(F)U(M)G(M)G(F)G (M)A(F)U(M)A(F)U(M)U(F)C(M)A(F)A(M)^A(F)^A(F) |
| CH0361 | No. 5616 | A(F)^A(M)^U(F)A(M)U(F)C(M)C(F)C(M)A(F)A(M)C(F) A(F)C(M)G(F)A(M)U(F)C(M)A(F)G(M)U(F)U(M) | No. 6317 | A(F)^A(M)^C(F)U(M)G(F)A(M)U(F)C(M)G(F)U(M)G(M)U(F)C (M)G(F)G(M)G(F)A(M)U(F)A(M)U(F)U(M)^C(F)^A(M) |
| CH0362 | No. 5616 | A(F)^U(M)^A(F)U(M)C(F)C(M)C(F)A(M)A(F)C(M)A(F) C(F)G(M)A(F)U(M)C(F)A(M)G(F)U(M)U(F)U(M) | No. 6318 | A(F)^A(M)^A(F)C(M)U(F)G(M)A(F)U(M)C(F)G(M)U(M)G(F)U (M)U(F)G(M)G(F)G(M)A(F)U(M)A(F)U(M)^U(F)^C(M) |
| CH0363 | No. 5617 | U(F)^A(M)^U(F)C(M)C(F)C(M)A(F)A(M)C(F)A(M)C(F) G(F)A(M)U(F)C(M)A(F)G(M)U(F)U(M)U(F)U(M) | No. 6319 | A(F)^A(M)^A(F)A(M)C(F)U(M)G(F)A(M)U(F)C(M)G(M)U(F)G (M)U(F)U(M)G(F)G(M)G(F)A(M)U(F)A(M)^U(F)^U(M) |
| CH0364 | No. 5618 | A(F)^U(M)^C(F)C(M)C(F)A(M)A(F)C(M)A(F)C(M)G(F) A(F)U(M)C(F)A(M)G(F)U(M)U(F)U(M)U(F)U(M) | No. 6320 | A(F)^A(M)^A(F)A(M)A(F)C(M)U(F)G(M)A(F)U(M)C(M)G(F)U (M)G(F)U(M)U(F)G(M)G(F)G(M)A(F)U(M)^A(F)^U(M) |
| CH0365 | No. 5619 | U(F)^C(M)^C(F)C(M)A(F)A(M)C(F)A(M)C(F)G(M)A(F) U(F)C(M)A(F)G(M)U(F)U(M)U(F)U(M)U(F)C(M) | No. 6321 | G(F)^A(M)^A(F)A(M)A(F)A(M)C(F)U(M)G(F)A(M)U(M)C(F)G (M)U(F)G(M)U(F)U(M)G(F)G(M)G(F)A(M)^U(F)^A(M) |
| CH0366 | No. 5620 | C(F)^C(M)^C(F)A(M)A(F)C(M)A(F)C(M)G(F)A(M)U(F) C(F)A(M)G(F)U(M)U(F)U(M)U(F)U(M)C(F)U(M) | No. 6322 | A(F)^G(M)^A(F)A(M)A(F)A(M)A(F)C(M)U(F)G(M)A(M)U(F)C( M)G(F)U(M)G(F)U(M)U(F)G(M)G(F)G(M)^A(F)^U(M) |
| CH0367 | No. 5621 | C(F)^C(M)^A(F)A(M)C(F)A(M)C(F)G(M)A(F)U(M)C(F) A(F)G(M)U(F)U(M)U(F)U(M)U(F)C(M)U(F)U(M) | No. 6323 | A(F)^A(M)^G(F)A(M)A(F)A(M)A(F)A(M)C(F)U(M)G(M)A(F)U( M)C(F)G(M)U(F)G(M)U(F)U(M)G(F)G(M)^G(F)^A(M) |
| CH0358 | No. 5622 | A(F)^A(M)^C(F)A(M)C(F)G(M)A(F)U(M)C(F)A(M)G(F) U(F)U(M)U(F)U(M)U(F)C(M)U(F)U(M)G(F)U(M) | No. 6324 | A(F)^C(M)^A(F)A(M)G(F)A(M)A(F)A(M)A(F)A(M)C(M)U(F)G( M)A(F)U(M)C(F)G(M)U(F)G(M)U(F)U(M)^G(F)^G(M) |
| CH0370 | No. 5623 | A(F)^C(M)^A(F)C(M)G(F)A(M)U(F)C(M)A(F)G(M)U(F) U(F)U(M)U(F)U(M)C(F)U(M)U(F)G(M)U(F)A(M) | No. 6325 | U(F)^A(M)^C(F)A(M)A(F)G(M)A(F)A(M)A(F)A(M)A(M)C(F)U( M)G(F)A(M)U(F)C(M)G(F)U(M)G(F)U(M)^U(F)^G(M) |
| CH0371 | No. 5624 | C(F)^A(M)^C(F)G(M)A(F)U(M)C(F)A(M)G(F)U(M)U(F) U(F)U(M)U(F)C(M)U(F)U(M)G(F)U(M)A(F)A(M) | No. 6326 | U(F)^U(M)^A(F)C(M)A(F)A(M)G(F)A(M)A(F)A(M)A(M)A(F)C( M)U(F)G(M)A(F)U(M)C(F)G(M)U(F)G(M)^U(F)^U(M) |
| CH0372 | No. 5625 | A(F)^C(M)^G(F)A(M)U(F)C(M)A(F)G(M)U(F)U(M)U(F) U(F)U(M)C(F)U(M)U(F)G(M)U(F)A(M)A(F)C(M) | No. 6327 | G(F)^U(M)^U(F)A(M)C(F)A(M)A(F)G(M)A(F)A(M)A(M)A(FA( M)C(F)U(M)G(F)A(M)U(F)C(M)G(F)U(M)^G(F)^U(M) |
| CH0373 | No. 5626 | C(F)^G(M)^A(F)U(M)C(F)A(M)G(F)U(M)U(F)U(M)U(F) U(F)C(M)U(F)U(M)G(F)U(M)A(F)A(M)C(F)A(M) | No. 6328 | U(F)^G(M)^U(F)U(M)A(F)C(M)A(F)A(M)G(F)A(M)A(M)A(F)A( M)A(F)C(M)U(F)G(M)A(F)U(M)C(F)G(M)^U(F)^G(M) |
| CH0375 | No. 5627 | A(F)^U(M)^C(F)A(M)G(F)U(M)U(F)U(M)U(F)U(M)C(F) U(F)U(M)G(F)U(M)A(F)A(M)C(F)A(M)C(F)U(M) | No. 6329 | A(F)^G(M)^U(F)G(M)U(F)U(M)A(F)C(M)A(F)A(M)C(M)A(F)A( M)A(F)A(M)A(F)C(M)U(F)G(M)A(F)U(M)^C(F)^G(M) |
| CH0376 | No. 5628 | U(F)^C(M)^A(F)G(M)U(F)U(M)U(F)U(M)U(F)C(M)U(F) U(F)G(M)U(F)A(M)A(F)C(M)A(F)C(M)U(F)G(M) | No. 6330 | C(F)^A(M)^G(F)U(M)G(F)U(M)U(F)A(M)C(F)A(M)A(M)G(F)A (M)A(F)A(M)A(F)A(M)C(F)U(M)G(F)A(M)^U(F)^C(M) |
| CH0382 | No. 5629 | U(F)^U(M)^U(F)C(M)U(F)U(M)G(F)U(M)A(F)A(M)C(F) A(F)C(M)U(F)G(M)G(F)G(M)U(F)U(M)U(F)U(M) | No. 6331 | A(F)^A(M)^A(F)A(M)C(F)C(M)C(F)A(M)G(F)U(M)G(M)U(F)U (M)A(F)C(M)A(F)A(M)G(F)A(M)A(F)A(M)^A(F)^A(M) |
| CH0383 | No. 5630 | U(F)^U(M)^C(F)U(M)U(F)G(M)U(F)A(M)A(F)C(M)A(F) C(F)U(M)G(F)G(M)G(F)U(M)U(F)U(M)U(F)A(M) | No. 6332 | U(F)^A(M)^A(F)A(M)A(F)C(M)C(F)C(M)A(F)G(M)U(M)G(F)U (M)U(F)A(M)C(F)A(M)A(F)G(M)A(F)A(M)^A(F)^A(M) |
| CH0384 | No. 5631 | U(F)^C(M)^U(F)U(M)G(F)U(M)A(F)A(M)C(F)A(M)C(F) U(F)G(M)G(F)G(M)U(F)U(M)U(F)U(M)A(F)U(M) | No. 6333 | A(F)^U(M)^A(F)A(M)A(F)A(M)C(F)C(M)C(F)A(M)G(M)U(F)G (M)U(F)U(M)A(F)C(M)A(F)A(M)G(F)A(M)^A(F)^A(M) |
| CH0385 | No. 5632 | C(F)^U(M)^U(F)G(M)U(F)A(M)A(F)C(M)A(F)C(M)U(F) G(F)G(M)G(F)U(M)U(F)U(M)U(F)A(M)U(F)C(M) | No. 6334 | G(F)^A(M)^U(F)A(M)A(F)A(M)A(F)C(M)C(F)C(M)A(M)G(F)U (M)G(F)U(M)U(F)A(M)C(F)A(M)A(F)G(M)^A(F)^A(M) |
| CH0386 | No. 5633 | U(F)^U(M)^G(F)U(M)A(F)A(M)C(F)A(M)C(F)U(M)G(F) G(F)G(M)U(F)U(M)U(F)U(M)A(F)U(M)C(F)U(M) | No. 6335 | A(F)^G(M)^A(F)U(M)A(F)A(M)A(F)A(M)C(F)C(M)C(M)A(F)G (M)U(F)G(M)U(F)U(M)A(F)C(M)A(F)A(M)^G(F)^A(M) |
| CH0388 | No. 5634 | G(F)^U(M)^A(F)A(M)C(F)A(M)C(F)U(M)G(F)G(M)G(F) U(F)U(M)U(F)U(M)A(F)U(M)C(F)U(M)G(F)A(M) | No. 6336 | U(F)^C(M)^A(F)G(M)A(F)U(M)A(F)A(M)A(F)A(M)C(M)C(F)C (M)A(F)G(M)U(F)G(M)U(F)U(M)A(F)C(M)^A(F)^A(M) |
| CH0389 | No. 5635 | U(F)^A(M)^A(F)C(M)A(F)C(M)U(F)G(M)G(F)G(M)U(F) U(F)U(M)U(F)A(M)U(F)C(M)U(F)G(M)A(F)A(M) | No. 6337 | U(F)^U(M)^C(M)A(M)G(F)A(M)U(F)A(M)A(F)A(M)A(M)C(F)C (M)C(F)A(M)G(F)U(M)G(F)U(M)U(F)A(M)^C(F)^A(M) |
| CH0390 | No. 5636 | A(F)^A(M)^C(F)A(M)C(F)U(M)G(F)G(M)G(F)U(M)U(F) U(F)U(M)A(F)U(M)C(F)U(M)G(F)A(M)A(F)U(M) | No. 6338 | A(F)^U(M)^U(F)C(M)A(F)G(M)A(F)U(M)A(F)A(M)A(M)A(F)G( M)C(F)C(M)A(F)G(M)U(F)G(M)U(F)U(M)^A(F)^C(M) |
| CH0392 | No. 5637 | C(F)^A(M)^C(F)U(M)G(F)G(M)G(F)U(M)U(F)U(M)U(F) A(F)U(M)C(F)U(M)G(F)A(M)A(F)U(M)G(F)G(M) | No. 6339 | C(F)^C(M)^A(F)U(M)U(F)C(M)A(F)G(M)A(F)U(M)A(M)A(F)A( M)A(F)C(M)C(F)C(M)A(F)F(M)U(F)G(M)^U(F)^U(M) |
| CH0394 | No. 5638 | C(F)^U(M)^G(F)G(M)G(F)U(M)U(F)U(M)U(F)A(M)U(F) C(F)U(M)G(F)A(M)A(F)U(M)G(F)G(M)C(F)G(M) | No. 6340 | C(F)^G(M)^C(F)C(M)A(F)U(M)U(F)C(M)A(F)G(M)A(M)U(F)A (M)A(F)A(M)A(F)C(M)C(F)C(M)A(F)G(M)^U(F)^G(M) |
| CH0395 | No. 5639 | U(F)^G(M)^G(F)G(M)U(F)U(M)U(F)U(M)A(F)U(M)C(F) U(F)G(M)A(F)A(M)U(F)G(M)G(F)C(M)G(F)C(M) | No. 6341 | G(F)^C(M)^G(F)C(M)C(F)A(M)U(F)U(M)C(F)A(M)G(M)A(F)U (M)A(F)A(M)A(F)A(M)C(F)C(M)C(F)A(M)^G(F)^U(M) |
| CH0396 | No. 5640 | G(F)^G(M)^G(F)U(M)U(F)U(M)U(F)A(M)U(F)C(M)U(F) G(F)A(M)A(F)U(M)G(F)G(M)C(F)G(M)C(F)U(M) | No. 6342 | A(F)^G(M)^C(F)G(M)C(F)C(M)A(F)U(M)U(F)C(M)A(M)G(F)A (M)U(F)A(M)A(F)A(M)A(F)C(M)C(F)C(M)^A(F)^G(M) |
| CH0399 | No. 5641 | U(F)^U(M)^U(F)U(M)A(F)U(M)C(F)U(M)G(F)A(M)U(F)U (F)G(M)G(F)C(M)G(F)C(M)U(F)G(M)A(F)U(M) | No. 6343 | A(F)^U(M)^C(F)A(M)G(F)C(M)G(F)C(M)C(F)A(M)U(M)U(F)C (M)A(F)G(M)A(F)U(M)A(F)A(M)A(F)A(M)^C(F)^C(M) |
| CH0402 | No. 5642 | U(F)^A(M)^U(F)C(M)U(F)G(M)A(F)A(M)U(F)G(M)G(F) C(F)G(M)C(F)U(M)G(F)A(M)U(F)U(M)C(F)U(M) | No. 6344 | A(F)^G(M)^A(F)A(M)U(F)C(M)A(F)G(M)C(F)G(M)C(M)C(F)A (M)U(F)U(M)C(F)A(M)G(F)A(M)U(F)A(M)^A(F)^A(M) |
| CH0406 | No. 5643 | U(F)^G(M)^A(F)A(M)U(F)G(M)G(F)C(M)G(F)C(M)U(F) G(F)A(M)U(F)U(M)C(F)U(M)G(F)C(M)C(F)A(M) | No. 6345 | U(F)^G(M)^G(F)C(M)A(F)G(M)A(F)A(M)U(F)C(M)A(M)G(F)C (M)G(F)C(M)C(F)A(M)U(F)U(M)C(F)A(M)^G(F)^A(M) |
| CH0409 | No. 5644 | A(F)^U(M)^G(F)G(M)C(F)G(M)C(F)U(M)G(F)A(M)U(F) U(F)C(M)U(F)G(M)C(F)C(M)A(F)A(M)G(F)U(M) | No. 6346 | A(F)^C(M)^U(F)U(M)G(F)G(M)C(F)A(M)G(F)A(M)A(M)U(F)C (M)A(F)G(M)C(F)G(M)V(F)C(M)A(F)U(M)^U(F)^C(M) |

### [Table 27]

**Table 3-7**

| Double stranded nucleic acid No. | SEQ ID NO. | Sense strand sequence (5'→3') | SEQ ID NO. | Antisense strand sequence (5'→3') |
|---|---|---|---|---|
| CH0410 | No. 5645 | U(F)^G(M)^G(F)C(M)U(F)G(M)A(F)U(M)U(F) C(F)U(M)G(F)C(M)C(F)A(M)A(F)G(M)U(F)G(M) | No. 6347 | C(F)^A(M)^C(F)U(M)U(F)G(M)G(F)C(M)A(F)G(M)A(M)A(F)U (M)C(F)A(M)G(F)C(M)G(F)C(M)C(F)A(M)^U(F)^U(M) |
| CH0411 | No. | G(F)^G(M)^C(F)G(M)C(F)U(M)G(F)A(M)U(F)U(M)G(F) U(F)G(M)C(F)C(M)A(F)A(M)G(F)U(M)G(F)G(M) | No. 6348 | G(F)^C(M)^A(F)C(M)U(F)U(M)G(F)G(M)C(F)A(M)G(M)A(F)A (M)U(F)C(M)A(F)G(M)C(F)G(M)C(F)C(M)^A(F)^U(M) |
| CH0412 | No. 5647 | G(F)^C(M)^G(F)C(M)U(F)G(M)A(F)U(M)U(F)C(M)U(F) G(F)C(M)C(F)A(M)A(F)G(M)U(F)G(M)C(F)A(M) | No. 6349 | U(F)^G(M)^C(F)A(M)C(F)U(M)U(F)G(M)G(F)C(M)A(M)G(F)A (M)A(F)U(M)C(F)A(M)G(F)C(M)G(F)C(M)^C(F)^A(M) |
| CH0413 | No. 5648 | C(F)^G(M)^C(F)U(M)G(F)A(M)U(F)U(M)C(F)U(M)G(F) C(F)C(M)A(F)A(M)G(F)U(M)G(F)C(M)A(F)C(M) | No. 6350 | G(F)^U(M)^G(F)C(M)A(F)C(M)U(F)U(M)G(F)G(M)C(M)A(F)G (M)A(F)A(M)U(F)C(M)A(F)G(M)C(F)G(M)^C(F)^C(M) |
| CH0414 | No. 5649 | G(F)^C(M)^U(F)G(M)A(F)U(M)U(F)C(M)U(F)G(M)C(F) C(F)A(M)A(F)G(M)U(F)G(M)C(F)A(M)C(F)U(M) | No. 6351 | A(F)^G(M)^U(F)G(M)C(F)A(M)C(F)U(M)U(F)G(M)G(M)C(F)A (M)G(F)A(M)A(F)U(M)C(F)A(M)G(F)C(M)^G(F)^C(M) |
| CH0415 | No. 5650 | G(F)^U(M)^G(F)A(M)U(F)U(M)C(F)U(M)G(F)C(M)C(F) A(F)A(M)G(F)U(M)G(F)C(M)A(F)C(M)U(F)G(M) | No. 6352 | C(F)^A(M)^G(F)U(M)G(F)C(M)A(F)C(M)U(F)U(M)G(M)G(F)C (M)A(F)G(M)A(F)A(M)U(F)C(M)A(F)G(M)^C(F)^G(M) |
| CH0419 | No. 5651 | U(F)^U(M)^C(F)U(M)G(F)C(M)C(F)A(M)A(F)G(M)U(F) G(F)C(M)A(F)C(M)U(F)G(M)A(F)G(M)G(F)A(M) | No. 6353 | U(F)^C(M)^C(F)U(M)C(F)A(M)G(F)U(M)G(F)C(M)A(M)C(F)U (M)U(F)G(M)G(F)C(M)A(F)G(M)A(F)A(M)^U(F)^C(M) |
| CH0420 | No. 5652 | U(F)^C(M)^U(F)G(M)C(F)C(M)A(F)A(M)G(F)U(M)G(F) C(F)A(M)C(F)U(M)G(F)A(M)G(F)G(M)A(F)A(M) | No. 6354 | U(F)^U(M)^C(F)C(M)U(F)C(M)A(F)G(M)U(F)G(M)C(M)A(F)C (M)U(F)U(M)G(F)G(M)C(F)A(M)G(F)A(M)^A(F)^U(M) |
| CH0422 | No. 5653 | U(F)^G(M)^C(F)C(M)A(F)A(M)G(F)U(M)G(F)C(M)A(F) C(F)U(M)G(F)A(M)G(F)G(M)A(F)A(M)G(F)G(M) | No. 6366 | C(F)^C(M)^U(F)U(M)C(F)C(M)U(F)C(M)A(F)G(M)U(M)G(F)C (M)A(F)C(M)U(F)U(M)G(F)G(M)C(F)A(M)^G(F)^A(M) |
| CH0423 | No. 5654 | G(F)^C(M)^C(F)A(M)A(F)G(M)U(F)G(M)C(F)A(M)C(F) U(F)G(M)A(F)G(M)G(F)A(M)A(F)G(M)G(F)A(M) | No. 6356 | U(F)^C(M)^C(F)U(M)U(F)C(M)C(F)U(M)C(F)A(M)G(M)U(F)G (M)C(F)A(M)C(F)U(M)U(F)G(M)G(F)C(M)^A(F)^G(M) |
| CH0424 | No. 5655 | C(F)^C(M)^A(F)A(M)G(F)U(M)G(F)C(M)A(F)C(M)U(F) G(F)A(M)G(F)G(M)A(F)A(M)G(F)G(M)A(F)A(M) | No. 6357 | U(F)^U(M)^C(F)C(M)U(F)U(M)C(F)C(M)U(F)C(M)A(M)G(F)U (M)G(F)C(M)A(F)C(M)U(F)U(M)G(F)G(M)^C(F)^A(M) |
| CH0425 | No. 5656 | C(F)^A(M)^A(F)G(M)U(F)G(M)C(F)A(M)C(F)U(M)G(F) A(F)G(M)G(F)A(M)A(F)G(M)G(F)A(M)A(F)A(M) | No. 6358 | U(F)^U(M)^U(F)C(M)C(F)U(M)U(F)C(M)C(F)U(M)C(M)A(F)G (M)U(F)G(M)C(F)A(M)C(F)U(M)U(F)G(M)^G(F)^G(M) |
| CH0426 | No. 5657 | A(F)^A(M)^G(F)U(M)G(F)C(M)A(F)C(M)U(F)G(M)A(F) G(F)G(M)A(F)A(M)G(F)G(M)A(F)A(M)A(F)A(M)C(F) | No. 6359 | U(F)^U(M)^U(F)U(M)C(F)C(M)U(F)U(M)C(F)C(M)U(M)C(F)A (M)G(F)U(M)G(F)C(M)A(F)C(M)U(F)U(M)^G(F)^G(M) |
| CH0427 | No. 5658 | A(F)^G(M)^U(F)G(M)C(F)A(M)C(F)U(M)G(F)A(M)G(F) G(F)A(M)A(F)G(M)G(F)A(M)A(F)A(M)A(F)U(M) | No. 6360 | A(F)^U(M)^U(F)U(M)U(F)C(M)U(F)U(M)U(F)C(M)C(M)U(F)C (M)A(F)G(M)U(F)G(M)C(F)A(M)C(F)U(M)^U(F)^G(M) |
| CH0436 | No. 5659 | A(F)^G(M)^G(F)A(M)A(F)G(M)G(F)A(M)A(F)A(M)A(F) U(F)G(M)G(F)A(M)G(F)C(M)C(F)C(M)G(F)G(M) | No. 6361 | C(F)^C(M)^G(F)G(M)G(F)C(M)U(F)C(M)C(F)A(M)U(M)U(F)U (M)U(F)C(M)C(F)U(M)U(F)C(M)C(F)U(M)^C(F)^A(M) |
| CH0449 | No. 5660 | G(F)^A(M)^G(F)C(M)C(F)C(M)G(F)G(M)A(F)G(M)C(F) U(F)U(M)C(F)C(M)U(F)G(M)U(F)C(M)U(F)G(M) | No. 6362 | C(F)^A(M)^G(F)A(M)C(F)A(M)G(F)G(M)A(F)A(M)G(M)C(F)U (M)C(F)C(M)G(F)G(M)G(F)C(M)U(F)C(M)^C(F)^A(M) |
| CH0450 | No. 5661 | A(F)^G(M)^C(F)C(M)C(F)G(M)G(F)A(M)G(F)C(M)U(F) U(F)C(M)C(F)U(M)G(F)U(M)C(F)U(M)G(F)U(M) | No. 6363 | A(F)^C(M)^A(F)G(M)A(F)C(M)A(F)G(M)G(F)A(M)A(M)G(F)C (M)U(F)C(M)C(F)G(M)G(F)G(M)C(F)U(M)^C(F)^C(M) |
| CH0453 | No. 5662 | C(F)^C(M)^G(F)G(M)A(F)G(M)C(F)U(M)U(F)C(M)C(F) U(F)G(M)U(F)C(M)U(F)G(M)U(F)G(M)C(F)U(M) | No. 6364 | A(F)^G(M)^C(F)A(M)C(F)A(M)G(F)A(M)C(F)A(M)G(M)G(F)A (M)A(F)G(M)C(F)U(M)C(F)C(M)G(F)G(M)^G(F)^C(M) |
| CH0454 | No. 5663 | C(F)^G(M)^G(F)A(M)G(F)C(M)U(F)U(M)C(F)C(M)U(F) G(F)U(M)C(F)U(M)G(F)U(M)G(F)C(M)U(F)C(M) | No. 6365 | G(F)^A(M)^G(F)C(M)A(F)C(M)A(F)G(M)A(F)C(M)A(M)G(F)G (M)A(F)A(M)G(F)C(M)U(F)C(M)C(F)G(M)^G(F)^G(M) |
| CH0457 | No. 5664 | A(F)^G(M)^C(F)U(M)U(F)C(M)C(F)U(M)G(F)U(M)C(F) U(F)G(M)U(F)G(M)C(F)U(M)C(F)C(M)C(F)A(M) | No. 6366 | U(F)^G(M)^G(F)G(M)A(F)G(M)C(F)A(M)C(F)A(M)G(M)A(F)C (M)A(F)G(M)G(F)A(M)A(F)G(M)C(F)U(M)^C(F)^C(M) |
| CH0461 | No. 5665 | U(F)^C(M)^C(F)U(M)G(F)U(M)C(F)U(M)G(F)U(M)G(F) C(F)U(M)C(F)C(M)C(F)A(M)U(F)C(M)A(F)U(M) | No. 6367 | A(F)^U(M)^G(F)A(M)U(F)G(M)G(F)G(M)A(F)G(M)C(M)A(F)C (M)A(F)G(M)A(F)C(M)A(F)G(M)G(F)A(M)^A(F)^G(M) |
| CH0483 | No. 5666 | C(F)^U(M)^G(F)U(M)C(F)U(M)G(F)U(M)G(F)C(M)U(F) C(F)C(M)C(F)A(M)U(F)C(M)A(F)U(M)C(F)U(M) | No. 6368 | A(F)^G(M)^A(F)U(M)G(F)A(M)U(F)G(M)C(F)G(M)A(M)C(F)C (M)A(F)C(M)A(F)G(M)A(F)C(M)A(F)G(M)^G(F)^A(M) |
| CH0472 | No. 5667 | C(F)^U(M)^C(F)C(M)C(F)A(M)U(F)C(M)A(F)U(M)C(F) U(F)G(M)C(F)C(M)C(F)U(M)C(F)C(M)A(F)C(M) | No. 6369 | G(F)^U(M)^G(F)G(M)A(F)G(M)G(F)G(M)C(F)A(M)G(M)A(F)U (M)G(F)A(M)U(F)G(M)G(F)G(M)A(F)G(M)^C(F)^A(M) |
| CH0475 | No. 5668 | C(F)^C(M)^A(F)U(M)C(F)A(M)U(F)C(M)U(F)G(M)C(F) C(F)C(M)U(F)C(M)C(F)A(M)C(F)C(M)A(F)U(M) | No. 6370 | A(F)^U(M)^G(F)G(M)U(F)G(M)G(F)A(M)G(F)G(M)G(M)C(F)A (M)G(F)A(M)U(F)G(M)A(F)U(M)G(F)G(M)^G(F)^A(M) |
| CH0476 | No. 5669 | C(F)^A(M)^U(F)C(M)A(F)U(M)C(F)U(M)G(F)C(M)C(F) C(F)U(M)C(F)C(M)A(F)C(M)C(F)A(M)U(F)C(M) | No. 6371 | G(F)^A(M)^U(F)G(M)G(F)U(M)G(F)G(M)A(F)G(M)G(M)G(F) C(M)A(F)G(M)A(F)U(M)G(F)A(M)U(F)G(M)^G(F)^G(M) |
| CH0480 | No. 5670 | A(F)^U(M)^C(F)U(M)G(F)C(M)C(F)C(M)U(F)C(M)C(F) A(F)C(M)C(F)A(M)U(F)C(M)C(F)A(M)U(F)A(M) | No. 6372 | U(F)^A(M)^U(F)G(M)G(F)A(M)U(F)G(M)G(F)U(M)G(M)G(F)A (M)G(F)G(M)G(F)C(M)A(F)G(M)A(F)U(M)^G(F)^A(M) |
| CH0484 | No. 5671 | G(F)^C(M)^C(F)C(M)U(F)C(M)C(F)A(M)C(F)C(M)A(F) U(F)C(M)C(F)A(M)U(F)A(M)C(F)C(M)U(F)A(M) | No. 6373 | U(F)^A(M)^G(F)G(M)U(F)A(M)U(F)G(M)G(F)A(M)U(M)G(F)C (M)U(F)G(M)G(F)A(M)G(F)G(M)G(F)C(M)^A(F)^G(M) |
| CH0487 | No. 5672 | C(F)^U(M)^C(F)C(M)A(F)C(M)C(F)A(M)U(F)C(M)C(F) A(F)U(M)A(F)C(M)C(F)U(M)A(F)C(M)G(F)U(M) | No. 6374 | A(F)^C(M)^G(F)U(M)A(F)G(M)G(F)U(M)A(F)U(M)G(M)G(F)A (M)U(F)G(M)G(F)U(M)G(F)G(M)A(F)G(M)^G(F)^G(M) |
| CH0488 | No. 5673 | U(F)^C(M)^C(F)A(M)C(F)G(M)A(F)U(M)C(F)C(M)A(F) U(F)A(M)C(F)C(M)U(F)A(M)C(F)G(M)U(F)U(M) | No. 6375 | A(F)^A(M)^C(F)G(M)U(F)A(M)G(F)G(M)U(F)A(M)U(M)G(F)G (M)A(F)U(M)C(F)G(M)U(F)G(M)G(F)A(M)^G(F)^G(M) |
| CH0489 | No. 5674 | C(F)^C(M)^A(F)C(M)C(F)A(M)U(F)C(M)C(F)A(M)U(F) A(F)C(M)C(F)U(M)A(F)C(M)G(F)U(M)U(F)U(M) | No. 6376 | A(F)^A(M)^A(F)C(M)G(F)U(M)A(F)G(M)G(F)U(M)A(M)U(F)G (M)G(F)A(M)U(F)G(M)G(F)U(M)G(F)G(M)^A(F)^G(M) |
| CH0490 | No. 5675 | C(F)^A(M)^C(F)C(M)A(F)U(M)C(F)C(M)A(F)U(M)A(F) C(F)C(M)U(F)A(M)C(F)G(M)U(F)U(M)U(F)G(M) | No. 6377 | C(F)^A(M)^A(F)A(M)C(F)G(M)U(F)A(M)G(F)G(M)U(M)A(F)U (M)G(F)G(M)A(F)U(M)G(F)G(M)U(F)G(M)^G(F)^A(M) |
| CH0491 | No. 5676 | A(F)^C(M)^C(F)A(M)U(F)C(M)C(F)A(M)U(F)A(M)C(F) C(F)U(M)A(F)C(M)G(F)U(M)U(F)U(M)G(F)C(M) | No. 6378 | G(F)^C(M)^A(F)A(M)A(F)C(M)G(F)U(M)A(F)G(M)G(M)U(F)A (M)U(F)G(M)G(F)A(M)U(F)G(M)G(F)U(M)^G(F)^G(M) |
| CH0492 | No. 5677 | C(F)^C(M)^A(F)U(M)C(F)C(M)A(F)U(M)A(F)C(M)C(F) U(F)A(M)C(F)G(M)U(F)U(M)U(F)G(M)G(F)A(M) | No. 6379 | U(F)^G(M)^C(F)A(M)A(F)A(M)C(F)G(M)U(F)A(M)G(M)G(F)U (M)A(F)U(M)G(F)G(M)A(F)U(M)G(F)G(M)^U(F)^G(M) |
| CH0493 | No. 5678 | C(F)^A(M)^U(F)C(M)C(F)A(M)U(F)A(M)C(F)C(M)U(F) A(F)C(M)G(F)U(M)U(F)U(M)G(F)C(M)A(F)A(M) | No. 6380 | U(F)^U(M)^G(F)C(M)A(F)A(M)A(F)C(M)G(F)U(M)A(M)G(F)G (M)U(F)A(M)U(F)G(M)G(F)A(M)U(F)G(M)^G(F)^U(M) |
| CH0495 | No. 5679 | U(F)^C(M)^C(F)A(M)U(F)A(M)C(F)C(M)U(F)A(M)C(F) G(F)U(M)U(F)U(M)G(F)C(M)A(F)A(M)C(F)A(M) | No. 6381 | U(F)^G(M)^U(F)U(M)G(F)C(M)A(F)A(M)A(F)C(M)G(M)U(F)A (M)G(F)G(M)U(F)A(M)U(F)G(M)G(F)A(M)^U(M)^G(M) |
| CH0498 | No. 5680 | A(F)^U(M)^A(F)C(M)C(F)U(M)A(F)C(M)G(F)U(M)U(F) U(F)G(M)C(F)A(M)A(F)C(M)A(F)C(M)U(F)U(M) | No. 6382 | A(F)^A(M)^G(F)U(M)G(F)U(M)U(F)G(M)C(F)A(M)A(M)A(F)C (M)G(F)U(M)A(F)G(M)G(F)U(M)A(F)U(M)^G(F)^G(M) |
| CH0499 | No. 5681 | U(F)^A(M)^C(F)C(M)U(F)A(M)C(F)G(M)U(F)U(M)U(F) G(F)C(M)A(F)A(M)C(F)A(M)C(F)U(M)U(F)C(M) | No. 6383 | G(F)^A(M)^A(F)G(M)U(F)G(M)U(F)U(M)G(F)C(M)A(M)A(F)A (M)C(F)G(M)U(F)A(M)G(F)G(M)U(F)A(M)^U(F)^G(M) |
| CH0500 | No. 5682 | A(F)^C(M)^C(F)U(M)A(F)C(M)G(F)U(M)U(F)U(M)G(F) C(F)A(M)A(F)C(M)A(F)C(M)U(F)U(M)C(F)G(M) | No. 6384 | C(F)^G(M)^A(F)A(M)G(F)U(M)U(F)G(M)U(F)G(M)C(M)A(F)A (M)A(F)C(M)G(F)U(M)A(F)G(M)G(F)U(M)^A(F)^U(M) |
| CH0501 | No. 5683 | C(F)^C(M)^U(F)A(M)C(F)G(M)U(F)U(M)U(F)G(M)C(F) A(F)A(M)C(F)A(M)C(F)U(M)U(F)C(M)G(F)U(M) | No. 6385 | A(F)^C(M)^G(F)A(M)A(F)G(M)U(F)G(M)U(F)U(M)G(M)C(F)A (M)A(F)A(M)C(F)G(M)U(F)A(M)G(F)G(M)^U(F)^A(M) |
| CH0502 | No. 5684 | C(F)^U(M)^A(F)C(M)G(F)U(M)U(F)U(M)G(F)C(M)A(F) A(F)C(M)A(F)C(M)U(F)U(M)C(F)G(M)U(F)G(M) | No. 6396 | C(F)^A(M)^C(F)GM)A(F)A(M)G(F)U(M)G(F)U(M)U(M)G(F)C (M)A(F)A(M)A(F)C(M)G(F)U(M)A(F)G(M)^G(F)^U(M) |

### [Table 28]

**Table 3-8**

| Double stranded nucleic acid No. | SEQ ID NO | Sense strand sequence (5'→3') | SEQ ID NO. | Antisense strand sequence (5'→3') |
|---|---|---|---|---|
| CH0503 | No. 5685 | U(F)^A(M)^C(F)G(M)U(F)U(M)U(F)G(M)C(F)A(M)A(F) A(F)A(M)C(F)U(M)U(F)C(M)G(F)U(M)G(F)U(M) | No. 6387 | A(F)^C(M)^A(F)C(M)G(F)A(M)A(F)G(M)U(F)G(M)U(M)U(F)G (M)C(F)A(M)A(F)A(M)C(F)G(M)U(F)A(M)^G(F)^G(M) |
| CH0504 | No. 5686 | A(F)^C(M)^G(F)U(M)U(F)U(M)G(F)C(M)A(F)A(M)C(F) A(F)C(M)U(F)U(M)C(F)G(M)U(F)G(M)U(F)U(M) | No. 6388 | A(F)^A(M)^C(F)A(M)C(F)G(M)A(F)A(M)G(F)U(M)G(M)U(F)U (M)G(F)C(M)A(F)A(M)A(F)C(M)G(F)U(M)^A(F)^G(M) |
| CH0505 | No. 5687 | C(F)^G(M)^U(F)U(M)U(F)G(M)C(F)A(M)A(F)C(M)A(F) C(F)U(M)U(F)C(M)G(F)U(M)G(F)U(M)U(F)U(M) | | A(F)^A(M)^A(F)C(M)A(F)C(M)G(F)A(M)A(F)G(M)U(M)G(F)U (M)U(F)G(M)C(F)A(M)A(F)A(M)C(F)G(M)^U(F)^A(M) |
| CH0506 | No. 5688 | G(F)^U(M)^U(F)U(M)G(F)C(M)A(F)A(M)C(F)A(M)C(F) U(F)U(M)C(F)G(M)U(F)G(M)U(F)U(M)U(F)A(M) | No. 6390 | U(F)^A(M)^A(F)A(M)C(F)A(M)C(F)G(M)A(F)A(M)G(M)U(F)G (M)U(F)U(M)G(F)C(M)A(F)A(M)A(F)C(M)^G(F)^U(M) |
| CH0507 | No. 5689 | U(F)^U(M)^U(F)G(M)C(F)A(M)A(F)C(M)A(F)C(M)U(F) U(F)C(M)G(F)U(M)G(F)U(M)U(F)U(M)A(F)U(M) | No. 6391 | A(F)^U(M)^A(F)A(M)A(F)C(M)A(F)C(M)G(F)A(M)A(M)G(F)U (M)G(F)U(M)U(F)G(M)C(F)A(M)A(F)A(M)^C(F)^G(M) |
| CH0508 | No. 5690 | U(F)^U(M)^G(F)C(M)A(F)A(M)C(F)A(M)C(F)U(M)U(F) C(F)G(M)U(F)G(M)U(F)U(M)U(F)A(M)U(F)A(M) | No. 6392 | U(F)^A(M)^U(F)A(M)A(F)A(M)C(F)A(M)C(F)G(M)A(M)A(F)G( M)U(F)G(M)U(F)U(M)G(F)C(M)A(F)A(M)^A(F)^C(M) |
| CH0509 | No. 5691 | U(F)^G(M)^C(F)A(M)A(F)C(M)A(F)C(M)U(F)U(M)C(F) G(F)U(M)G(F)U(M)U(F)U(M)A(F)U(M)A(F)A(M) | No. 6393 | U(F)^U(M)^A(F)U(M)A(F)A(M)A(F)C(M)A(F)C(M)G(M)A(F)A( M)G(F)U(M)G(F)U(M)U(F)G(M)C(F)A(M)^A(F)^A(M) |
| CH0510 | No. 5692 | G(F)^C(M)^A(F)A(M)C(F)A(M)C(F)U(M)U(F)C(M)G(F) U(F)G(M)U(F)U(M)U(F)A(M)U(F)A(M)A(F)G(M) | No; 6394 | C(F)^U(M)^U(F)A(M)U(F)A(M)A(F)A(M)C(F)A(M)C(M)G(F)A( M)A(F)G(M)U(F)G(M)U(F)U(M)G(F)C(M)^A(F)^A(M) |
| CH0513 | No. 5693 | A(F)^C(M)^A(F)C(M)U(F)U(M)C(F)G(M)U(F)G(M)U(F) U(F)U(M)A(F)U(M)A(F)A(M)G(F)C(M)C(F)A(M) | No. 6395 | U(F)^G(M)^G(F)C(M)U(F)U(M)A(F)U(M)A(F)A(M)A(M)C(F)A( M)C(F)G(M)A(F)A(M)G(F)U(M)G(F)U(M)^U(F)^G(M) |
| CH0514 | No. 5694 | C(F)^A(M)^C(F)U(M)U(F)C(M)G(F)U(M)G(F)U(M)U(F) U(F)A(M)U(F)A(M)A(F)G(M)C(F)C(M)A(F)U(M) | No. 6396 | A(F)^U(M)^G(F)G(M)C(F)U(M)U(F)A(M)U(F)A(M)A(M)A(F)C( M)A(F)C(M)G(F)A(M)A(F)G(M)U(F)G(M)^U(F)^U(M) |
| CH0518 | No. 5695 | U(F)^C(M)^G(F)U(M)G(F)U(M)U(F)U(M)A(F)U(M)A(F) A(F)G(M)C(F)C(M)A(F)U(M)C(F)A(M)G(F)C(M) | No. 6397 | G(F)^C(M)^U(F)G(M)A(F)U(M)G(F)G(M)C(F)U(M)U(M)A(F)U (M)A(F)A(M)A(F)C(M)A(F)C(M)G(F)A(M)^A(F)^G(M) |
| CH0519 | No. 5696 | C(F)^G(M)^U(F)G(M)U(F)U(M)U(F)A(M)U(F)A(M)A(F) G(F)C(M)C(F)A(M)U(F)C(M)A(F)G(M)C(F)U(M) | No. 6398 | A(F)^G(M)^C(F)U(M)G(F)A(M)U(F)G(M)G(F)C(M)U(M)U(F)A (M)U(F)A(M)A(F)A(M)C(F)A(M)C(F)G(M)^A(F)^A(M) |
| CH0520 | No. 5697 | G(F)^U(M)^G(F)U(M)U(F)U(M)A(F)U(M)A(F)A(M)G(F) C(F)C(M)A(F)U(M)C(F)A(M)G(F)C(M)U(F)G(M) | No. 6399 | C(F)^A(M)^G(F)C(M)U(F)G(M)A(F)U(M)G(F)G(M)C(M)U(F)U (M)A(F)U(M)A(F)A(M)A(F)C(M)A(F)C(M)^G(F)^A(M) |
| CH0526 | No. 5698 | U(F)^U(M)^A(F)A(M)G(F)C(M)C(F)A(M)U(F)C(M)A(F) G(F)C(M)U(F)G(M)G(F)A(M)A(F)A(M)C(F)A(M) | No. 6400 | U(F)^G(M)^U(F)U(M)U(F)C(M)C(F)A(M)G(F)C(M)U(M)G(F)A(M)U(F)G(M)G(F)C(M)U(F)U(M)A(F)U(M)^A(F)^A(M) |
| CH0527 | No. 5699 | U(F)^A(M)^A(F)G(M)C(F)C(M)A(F)U(M)C(F)A(M)G(F) C(F)U(M)G(F)G(M)A(F)A(M)A(F)C(M)A(F)A(M) | No. 6401 | U(F)^U(M)^G(F)U(M)U(F)U(M)C(F)C(M)A(F)G(M)C(M)U(F)G(M)A(F)U(M)G(F)G(M)C(F)U(M)U(F)A(M)^U(F)^A(M) |
| CH0528 | No. 5700 | A(F)^A(M)^G(F)C(M)C(F)A(M)U(F)C(M)A(F)G(M)G(F) U(F)G(M)G(F)A(M)A(F)A(M)C(F)A(M)A(F)U(M) | Nο. 6402 | A(F)^U(M)^U(F)G(M)U(F)U(M)U(F)C(M)C(F)A(M)G(M)C(F)U (M)G(F)A(M)U(F)G(M)G(F)C(M)U(F)U(M)^A(F)^U(M) |
| CH0529 | No. 5701 | A(F)^G(M)^C(F)C(M)A(F)U(M)C(F)A(M)G(F)C(M)U(F) G(F)G(M)A(F)A(M)A(F)C(M)A(F)A(M)U(F)U(M) | No. 6403 | A(F)^A(M)^U(F)U(M)U(F)U(M)U(F)U(M)U(F)C(M)C(M)U(F)U (M)U(F)G(M)A(F)U(M)G(F)G(M)C(F)U(M)^U(F)^A(M) |
| CH0530 | No. 5702 | G(F)^C(M)^C(F)A(M)U(F)C(M)A(F)G(M)C(F)U(M)G(F) G(F)A(M)A(F)A(M)C(F)A(M)A(F)U(M)U(F)C(M) | No. 6404 | G(F)^A(M)^A(F)U(M)U(F)G(M)U(F)U(M)U(F)C(M)C(M)A(F)G (M)C(F)U(M)G(F)A(M)U(F)G(M)G(F)C(M)^U(F)^U(M) |
| CH0534 | No. 5703 | U(F)^C(M)^A(F)G(M)C(F)U(M)G(F)G(M)A(F)A(M)A(F) C(F)A(M)A(F)U(M)U(F)C(M)C(F)C(M)U(F)C(M) | No. 6405 | G(F)^A(M)^G(F)G(M)G(F)A(M)A(F)U(M)U(F)G(M)U(M)U(F)U (M)C(F)C(M)A(F)G(M)C(F)U(M)G(F)A(M)^U(F)^G(M) |
| CH0535 | No. 5704 | C(F)^A(M)^G(F)C(M)U(F)G(M)G(F)A(M)A(F)A(M)C(F) A(F)A(M)U(F)U(M)C(F)C(M)C(F)U(M)C(F)U(M) | No. 6406 | A(F)^G(M)^A(F)G(M)G(F)G(M)A(F)A(M)U(F)U(M)G(M)U(F)U (M)U(F)C(M)C(F)A(M)G(F)C(M)U(F)G(M)^A(F)^U(M) |
| CH0538 | No. 5705 | C(F)^U(M)^G(F)G(M)A(F)A(M)A(F)C(M)A(F)A(M)U(F) U(F)C(M)C(F)C(M)U(F)C(M)U(F)A(M)U(F)C(M) | No. 6407 | G(F)^A(M)^U(F)A(M)C(F)A(M)G(F)G(M)G(F)A(M)A(M)U(F)U (M)G(F)U(M)U(F)U(M)C(F)C(M)A(F)G(M)^C(F)^U(M) |
| CH0542 | No. 5706 | A(F)^A(M)^A(F)C(M)A(F)A(M)U(F)U(M)C(F)C(M)C(F) U(F)C(M)U(F)A(M)U(F)C(M)G(F)G(M)G(F)A(M) | No. 6408 | U(F)^C(M)^C(F)C(M)C(F)A(M)U(F)A(M)G(F)A(M)G(M)G(F)G (M)A(F)A(M)U(F)U(M)G(F)U(M)U(F)U(M)^C(F)^C(M) |
| CH0549 | No. 5707 | U(F)^C(M)^C(F)C(M)U(F)C(M)U(F)A(M)U(F)C(M)G(F) G(F)G(M)A(F)C(M)A(F)C(M)A(F)G(M)C(F)A(M) | No. 6409 | U(F)^G(M)^C(F)U(M)G(F)U(M)G(F)U(M)C(F)C(M)C(M)G(F)A (M)U(F)A(M)G(F)A(M)G(F)G(M)G(F)A(M)^A(F)^U(M) |
| CH0552 | No. 5708 | C(F)^U(M)^C(F)U(M)A(F)U(M)C(F)G(M)G(F)G(M)A(F) C(F)A(M)C(F)A(M)G(F)C(M)A(F)G(M)U(F)U(M) | No. 6410 | A(F)^A(M)^C(F)U(M)G(F)C(M)U(F)G(M)U(F)G(M)U(M)C(F)C (M)C(F)G(M)A(F)U(M)A(F)G(M)A(F)G(M)^G(F)^G(M) |
| CH0553 | No. 5709 | U(F)^C(M)^U(F)A(M)U(F)C(M)G(F)G(M)G(F)A(M)C(F) A(F)C(M)A(F)G(M)C(F)A(M)G(F)U(M)U(F)U(M) | No. 6411 | A(F)^A(M)^A(F)C(M)U(F)G(M)C(F)U(M)G(F)U(M)G(M)U(F)C (M)C(F)C(M)G(F)A(M)U(F)A(M)G(F)A(M)^G(F)^G(M) |
| CH0554 | No. 5710 | C(F)^U(M)^A(F)U(M)C(F)G(M)G(F)G(M)A(F)C(M)A(F) C(F)A(M)G(F)G(M)A(F)G(M)U(F)U(M)U(F)U(M) | No. 6412 | A(F)^A(M)^A(F)A(M)C(F)U(M)G(F)C(M)U(F)G(M)U(M)G(F)U (M)C(F)C(M)C(F)G(M)A(F)U(M)A(F)G(M)^A(F)^G(M) |
| CH0555 | No. 5711 | U(F)^A(M)^U(F)C(M)G(F)G(M)G(F)A(M)C(F)A(M)C(F) A(F)G(M)C(F)A(M)G(F)U(M)U(F)U(M)U(F)U(M) | No. 6413 | A(F)^A(M)^A(F)A(M)A(F)C(M)U(F)G(M)C(F)U(M)G(M)U(F)G (M)U(F)C(M)C(F)C(M)G(F)A(M)U(F)A(M)^G(F)^A(M) |
| CH0557 | No. 5712 | U(F)^C(M)^G(F)G(M)G(F)A(M)C(F)A(M)C(F)A(M)G(F) C(F)A(M)G(F)U(M)U(F)U(M)U(F)U(M)G(F)A(M) | No. 6414 | U(F)^C(M)^A(F)A(M)A(F)A(M)A(F)C(M)U(F)G(M)C(M)U(F)G (M)U(F)G(M)U(F)C(M)C(F)C(M)G(F)A(M)^U(F)^A(M) |
| CH0558 | No. 5713 | C(F)^G(M)^G(F)G(M)A(F)C(M)A(F)C(M)A(F)G(M)C(F) A(F)G(M)U(F)U(M)U(F)U(M)U(F)G(M)A(F)A(M) | No. 6415 | U(F)^U(M)^C(F)A(M)A(F)A(M)A(F)A(M)C(F)U(M)G(M)V(F)U( M)G(F)U(M)G(F)U(M)C(F)C(M)C(F)G(M)^A(F)^U(M) |
| CH0559 | No. 5714 | G(F)^G(M)^G(F)A(M)C(F)A(M)C(F)A(M)G(F)C(M)A(F) G(F)U(M)U(F)U(M)U(F)U(M)G(F)A(M)A(F)U(M) | No. 6416 | A(F)^U(M)^U(F)C(M)A(F)A(M)A(F)A(M)A(F)C(M)U(M)G(F)C( M)U(F)G(M)U(F)G(M)U(F)C(M)C(F)C(M)^G(F)^A(M) |
| CH0561 | No. 5715 | G(F)^A(M)^C(F)A(M)C(F)A(M)G(F)C(M)A(F)G(M)U(F) U(F)U(M)U(F)U(M)G(F)A(M)A(F)U(M)G(F)U(M) | No. 6417 | A(F)^C(M)^A(F)U(M)U(F)C(M)A(F)A(M)A(F)A(M)A(M)C(F)U( M)G(F)C(M)U(F)G(M)U(F)G(M)U(F)C(M)^C(F)^C(M) |
| CH0562 | No. 5716 | A(F)^C(M)^A(F)C(M)A(F)G(M)C(F)A(M)G(F)U(M)U(F) U(F)U(M)U(F)G(M)A(F)A(M)U(F)G(M)U(F)U(M) | No. 6418 | A(F)^A(M)^C(F)A(M)U(F)U(M)C(F)A(M)A(F)A(M)A(M)A(F)C( M)U(F)G(M)C(F)U(M)G(F)U(M)G(F)U(M)^C(F)^C(M) |
| CH0563 | No. 5717 | C(F)^A(M)^C(F)A(M)G(F)C(M)A(F)G(M)U(F)U(M)U(F) U(F)U(M)G(F)A(M)A(F)U(M)G(F)U(M)U(F)U(M) | No. 6419 | A(F)^A(M)^A(F)C(M)A(F)U(M)U(F)C(M)A(F)A(M)A(M)A(F)C( M)C(F)U(M)G(F)C(M)U(F)G(M)U(F)G(M)^U(F)^C(M) |
| CH0564 | No. 5718 | A(F)^C(M)^A(F)G(M)C(F)A(M)G(F)U(M)U(F)U(M)U(F) U(F)G(M)A(F)A(M)U(F)G(M)U(F)U(M)U(F)G(M) | No. 6420 | C(F)^A(M)^A(F)A(M)C(F)A(M)U(F)U(M)C(F)A(M)A(M)A(F)A( M)A(F)C(M)U(F)G(M)C(F)U(M)G(F)U(M)^G(F)^U(M) |
| CH0585 | No. 5719 | C(F)^A(M)^G(F)C(M)A(F)G(M)U(F)U(M)U(F)U(M)U(F) G(F)A(M)A(F)U(M)G(F)U(M)U(F)U(M)G(F)C(M) | No. 6421 | G(F)^C(M)^A(F)A(M)A(F)C(M)A(F)U(M)U(F)C(M)A(M)A(F)A( M)A(F)A(M)C(F)U(M)G(F)C(M)U(F)G(M)^U(F)^G(M) |
| CH0567 | No. 5720 | G(F)^C(M)^A(F)G(M)U(F)U(M)U(F)U(M)U(F)G(M)A(F) A(F)U(M)G(F)U(M)U(F)U(M)G(F)C(M)C(F)A(M) | No. 6422 | U(F)^G(M)^G(F)C(M)A(F)A(M)A(F)C(M)A(F)U(M)U(M)C(F)A (M)A(F)A(M)A(F)A(M)C(F)U(M)G(F)C(M)^U(F)^G(M) |
| CH0568 | No. 5721 | C(F)^A(M)^G(F)U(M)U(F)U(M)U(F)U(M)G(F)A(M)A(F) U(F)G(M)U(F)U(M)U(F)G(M)C(F)C(M)A(F)C(M) | No. 6423 | G(F)^U(M)^G(F)G(M)C(F)A(M)A(F)A(M)C(F)A(M)U(M)U(F)C (M)A(F)A(M)A(F)A(M)A(F)C(M)U(F)G(M)^C(F)^U(M) |
| CH0570 | No. 5722 | G(F)^U(M)^U(F)U(M)U(F)U(M)G(F)A(M)A(F)U(M)G(F) U(F)U(M)U(F)G(M)C(F)C(M)A(F)C(M)A(F)A(M) | No. 6424 | U(F)^U(M)^G(F)U(M)G(F)G(M)C(F)A(M)A(F)A(M)C(M)A(F)U (M)U(F)C(M)A(F)A(M)A(F)A(M)A(F)C(M)^U(F)^C(M) |
| CH0572 | No. 5723 | U(F)^U(M)^U(F)U(M)G(F)A(M)A(F)U(M)G(F)U(M)U(F)U (F)G(M)C(F)C(M)A(F)C(M)A(F)A(M)C(F)A(M) | No. 6425 | U(F)^G(M)^U(F)U(M)G(F)U(M)G(F)G(M)C(F)A(M)A(M)A(F)C(M)A(F)U(M)U(F)C(M)A(F)A(M)A(F)A(M)^A(F)^C(M) |
| CH0573 | No. 5724 | U(F)^U(M)^U(F)G(M)A(F)A(M)U(F)G(M)U(F)U(M)U(F) G(F)C(M)C(F)A(M)C(F)A(M)A(F)C(M)A(F)U(M) | No. 6426 | A(F)^U(M)^G(F)U(M)U(F)G(M)U(F)G(M)G(F)C(M)A(M)A(F)A(M)C(F)A(M)U(F)U(M)C(F)A(M)A(F)A(M)^A(F)^A(M) |

### [Table 29]

**Table 3-9**

| Double stranded nucleic acide No. | SEQ ID NO. | Sense strand sequence (5'→3') | SEQ ID NO. | Antisense strand sequence (5'→3') |
|---|---|---|---|---|
| CH0574 | No. 5725 | U(F)^U(M)^G(F)A(M)A(F)U(M)G(F)U(M)U(F)U(M)G(F) C(F)C(M)A(F)C(M)A(F)A(M)C(F)A(M)U(F)G(M) | No. 6427 | C(F)^A(M)^U(F)G(M)U(F)U(M)G(F)U(M)G(F)G(M)C(M)A(F)A (M)A(F)C(M)A(F)U(M)U(F)C(M)A(F)A(M)^A(F)^A(M) |
| CH0578 | No. 5726 | A(F)^U(M)^G(F)U(M)U(F)U(M)G(F)C(M)C(F)A(M)C(F) A(F)A(M)C(F)A(M)U(F)G(M)C(F)G(M)A(F)U(M) | No. 6428 | A(F)^U(M)^C(F)G(M)C(F)A(M)U(F)G(M)U(F)U(M)G(M)U(F)G (M)G(F)C(M)A(F)A(M)A(F)C(M)A(F)U(M)^U(F)^C(M) |
| CH0582 | No. 5727 | U(F)^U(M)^G(F)C(M)C(F)A(M)C(F)A(M)A(F)C(M)A(F) U(F)G(M)C(F)G(M)A(F)U(M)G(F)U(M)U(F)U(M) | No. 6429 | A(F)^A(M)^A(F)C(M)A(F)U(M)C(F)G(M)C(F)A(M)U(M)G(F)U (M)U(F)G(M)U(F)G(M)G(F)C(M)A(M)^A(F)C(M) |
| CH0583 | No. 5728 | U(F)^G(M)^C(F)C(M)A(F)C(M)A(F)A(M)C(F)A(M)U(F) G(F)C(M)G(F)A(M)U(F)G(M)U(F)(M)U(F)G(M) | No. 6430 | C(F)^A(M)^A(F)A(M)C(F)A(M)U(F)C(M)G(F)C(M)A(M)U(F)G (M)U(F)U(M)G(F)U(M)G(F)G(M)C(F)A(M)^A(F)^A(M) |
| CH0585 | No. 5729 | C(F)^C(M)^A(F)C(M)A(F)A(M)C(M)A(M)U(F)G(M)C(F) G(F)A(M)U(F)G(M)U(F)U(M)U(F)G(M)G(F)A(M) | No. 6431 | U(F)^C(M)^C(F)A(M)A(F)A(M)C(F)A(M)U(F)C(M)G(M)C(F)A (M)U(F)G(M)U(F)U(M)G(F)U(M)G(F)G(M)^C(F)^A(M) |
| CH0586 | No. 5730 | C(M)^A(M)^C(F)A(M)A(F)C(M)A(F)U(M)G(F)C(M)G(F) A(F)U(M)G(F)U(M)U(F)U(M)G(F)G(M)A(F)A(M) | No. 6432 | U(F)^U(M)^C(F)C(M)A(F)A(M)A(F)C(M)A(F)U(M)C(M)G(F)C (M)A(F)U(M)G(F)U(M)U(F)U(M)U(F)G(M)U(F)G(M)^G(F)^C(M) |
| CH0588 | No. 5731 | C(F)^A(M)^A(F)C(M)A(F)U(M)G(F)C(M)G(F)A(M)U(F) G(F)U(M)U(F)U(M)G(F)G(M)A(F)A(M)A(F)U(M) | No. 6433 | A(F)^U(M)^U(F)U(M)C(F)C(F)C(M)A(F)A(M)A(F)C(M)A(M)U(F)C( M)G(F)C(M)A(F)U(M)G(F)U(M)U(F)G(M)^U(F)^G(M) |
| CH0590 | No. 5732 | A(F)^G(M)^A(F)U(M)G(F)C(M)G(F)A(M)U(F)G(M)U(F) U(F)U(M)G(F)G(M)A(F)A(M)A(F)U(M)G(F)A(M) | No. 6434 | U(F)^C(M)^A(F)U(M)U(F)U(M)C(F)C(M)A(F)A(M)A(M)C(F)A( M)U(F)C(M)G(F)C(M)A(F)U(M)G(F)U(M)^U(F)^G(M) |
| CH0591 | No. 5733 | C(F)^A(M)^U(F)G(M)C(F)G(M)A(F)U(M)G(F)U(M)U(F) U(F)G(M)G(F)A(M)A(F)A(M)U(F)G(M)A(F)U(M) | No. 0436 | A(F)^U(M)^C(F)A(M)U(F)U(M)U(F)C(M)C(F)A(M)A(M)A(F)C( M)A(F)U(M)C(F)G(M)C(F)A(M)U(F)G(M)^U(F)^U(M) |
| CH0592 | No. 5734 | A(F)^U(M)^G(F)C(M)G(F)A(M)U(F)G(M)U(F)U(M)U(F) G(F)G(M)A(F)A(M)A(F)U(M)G(F)A(M)U(F)A(M) | No. 6436 | U(F)^A(M)^U(F)C(M)A(F)U(M)U(F)U(M)C(F)C(M)A(F)A(F)A( M)C(F)A(M)U(F)C(M)G(F)C(M)A(F)U(M)^G(F)^U(M) |
| CH0593 | No. 5735 | U(F)^G(M)^C(F)G(M)A(F)U(M)G(F)U(M)U(F)U(M)G(F) G(F)A(M)A(F)A(M)U(F)G(M)A(F)U(M)A(F)C(M) | No. 6437 | G(F)^U(M)^A(F)U(M)C(F)A(M)U(F)U(M)U(F)C(M)A(F)A( M)A(F)C(M)A(F)U(M)C(F)G(M)C(F)G(M)C(F)A(M)^U(F)^G(M) |
| CN0594 | No. 5736 | G(F)^C(M)^G(F)A(M)U(F)G(M)U(F)U(M)U(F)G(M)G(F) A(F)A(M)A(F)U(M)G(F)A(M)U(F)A(M)C(F)A(M) | No. 6438 | U(F)^G(M)^U(F)A(M)U(F)C(M)A(F)U(M)U(F)U(M)C(M)C(F)A( M)A(F)A(M)C(F)A(M)U(F)C(M)G(F)C(M)^A(F)^U(M) |
| CH0595 | No. 5737 | C(F)^G(M)^A(F)U(M)G(F)U(M)U(F)U(M)G(F)G(M)A(F) A(F)A(M)U(F)G(M)A(F)U(M)A(F)C(M)A(F)A(M) | No. 6439 | U(F)^U(M)^G(F)U(M)A(F)U(M)A(F)U(M)C(F)A(M)U(F)U(M)U(M)C(F)C( M)A(F)A(M)A(F)C(M)A(F)U(M)C(F)C(M)^C(F)^A(M) |
| CH0597 | No. 5738 | A(F)^U(M)^G(F)U(M)U(F)U(M)G(F)G(M)A(F)A(M)A(F) U(F)G(M)A(F)U(M)A(F)C(M)A(F)A(M)U(F)U(M) | No. 6440 | A(F)^A(M)^U(F)U(M)G(F)U(M)A(F)U(M)C(F)A(M)U(M)U(F)U( M)C(F)C(M)A(F)A(M)A(F)C(M)A(F)U(M)^C(F)^G(M) |
| CH0598 | Nο. 5739 | U(F)^G(M)^U(F)U(M)U(F)G(M)G(F)A(M)A(F)A(M)U(F) G(F)A(M)U(F)A(M)C(F)A(M)A(F)U(M)U(F)A(M) | No. 6441 | U(F)^A(M)^A(F)U(M)U(F)G(M)U(F)A(M)U(F)C(M)A(M)U(F)U( M)U(F)C(F)C(F)A(M)A(F)A(F)C(F)A(M)^U(F)C(M) |
| CH0601 | No. 5740 | U(F)^U(M)^G(F)G(M)A(F)A(M)A(F)U(M)G(F)A(M)U(F) A(F)C(M)A(F)A(M)U(F)U(M)A(F)C(M)C(F)U(M) | No. 6442 | A(F)^G(M)^G(F)U(M)A(F)A(M)U(F)U(M)G(F)U(M)A(M)U(F)C( M)A(F)U(M)U(F)U(M)C(F)C(M)A(F)A(M)^A(F)^C(M) |
| CH0607 | No. 5741 | A(F)^U(M)^G(F)A(M)U(F)A(M)C(F)A(M)A(F)U(M)U(F)A (F)C(M)C(F)U(M)G(F)C(M)A(F)C(M)G(F)A(M) | No. 6443 | U(F)^C(M)^G(F)U(M)G(F)C(M)A(F)G(M)G(F)U(M)A(M)A(F)U (M)U(F)G(M)U(F)A(M)U(F)C(M)A(F)U(M)^U(F)^U(M) |
| CH0609 | No. 5742 | G(F)^A(M)^U(F)A(M)C(F)A(M)A(F)U(M)U(F)A(M)C(F) C(F)U(M)G(F)C(M)A(F)C(M)G(F)A(M)C(F)A(M) | No. 6444 | U(F)^G(M)^U(F)C(M)G(F)U(M)G(F)C(M)A(F)G(M)G(M)U(F)A (M)A(F)U(M)U(F)G(M)U(F)A(M)^A(F)^U(M) |
| CH0610 | No. 5743 | A(F)^U(M)^A(F)C(M)A(F)A(M)U(F)U(M)A(F)C(M)C(F) U(F)G(M)C(F)A(M)C(F)G(M)A(F)C(M)A(F)C(M) | No. 6445 | G(F)^U(M)^G(F)U(M)C(F)G(M)U(F)G(M)C(F)A(M)G(F)U (M)A(F)A(M)U(F)U(M)G(F)U(M)A(F)U(M)^C(F)^A(M) |
| CH0611 | No. 5744 | U(F)^A(M)^C(F)A(M)A(F)U(M)U(F)A(M)C(F)C(M)U(F) G(F)C(M)A(F)C(M)G(F)A(M)C(F)A(M)C(F)A(M) | No. 6446 | U(F)^G(M)^U(F)G(M)U(F)C(M)G(F)U(F)G(F)C(M)A(M)G(F)G (M)U(F)A(M)A(F)U(M)U(F)G(M)U(F)A(M)^U(F)^C(M) |
| CH0612 | No. 5745 | A(F)^C(M)^A(F)A(M)U(F)U(M)A(F)C(M)C(F)U(M)G(F) C(F)A(M)C(F)G(M)A(F)C(M)A(F)C(M)A(F)U(M) | No. 6447 | A(F)^U(M)^G(F)U(M)G(F)U(M)C(F)G(M)U(F)G(M)C(M)A(F)G (M)G(F)U(M)A(F)A(M)U(F)U(M)G(F)U(M)^A(F)^U(M) |
| CH0616 | No. 5746 | U(F)^U(M)^A(F)C(M)C(F)U(M)G(F)C(M)A(F)C(M)G(F) A(F)C(M)A(F)C(M)A(F)U(M)G(F)G(M)A(F)A(M) | No. 6448 | U(F)^U(M)^C(F)C(M)A(F)U(M)G(F)U(M)G(F)U(M)C(M)G(F)U (M)G(F)C(M)A(F)G(M)G(F)U(M)^U(F)^U(M) |
| CH0611 | No. 5747 | U(F)^A(M)^C(F)C(M)U(F)G(M)C(F)A(M)C(F)G(M)A(F) C(F)A(M)C(F)A(M)U(F)G(M)G(F)A(M)A(F)A(M) | No. 6449 | U(F)^U(M)^U(F)C(M)C(F)A(M)U(F)G(M)U(F)G(M)U(M)C(F)G (M)U(F)G(M)C(F)A(M)G(F)G(M)U(F)A(M)^A(F)^U(M) |
| CH0618 | No. 6748 | A(F)^C(M)^C(F)U(M)G(F)C(M)A(F)C(M)G(F)A(M)G(F) A(F)C(M)A(F)U(M)G(F)G(M)A(F)A(M)A(F)U(M) | No. 6450 | A(F)^U(M)^U(F)U(M)C(F)C(F)C(M)G(F)U(M)G(M)U(F)G (M)G(F)U(M)G(F)C(M)A(F)G(M)G(F)U(M)^A(F)^A(M) |
| CH0622 | No. 5749 | G(F)^C(M)^A(F)C(M)G(F)A(M)C(F)A(M)C(F)A(M)U(F) G(F)G(M)A(F)A(M)A(F)U(M)U(F)G(M)G(F)A(M) | No. 6451 | U(F)^C(M)^C(F)A(M)A(F)U(M)U(F)U(F)C(F)C(M)A(M)U(F)G (M)U(F)G(M)U(F)UC(M)G(F)U(M)G(F)C(M)^A(F)^G(M) |
| CH0624 | No. 5750 | A(F)^C(M)^G(F)A(M)C(F)A(M)C(F)A(M)U(F)G(M)G(F) A(F)A(M)A(F)U(M)U(F)G(M)G(F)A(M)C(F)U(M) | No. 6452 | A(F)^G(M)^U(F)C(M)A(M)A(F)U(M)U(F)U(M)C(M)C(F)A (M)U(F)G(M)U(F)G(M)U(F)C(M)G(F)U(M)^G(F)^C(M) |
| CH0625 | No. 5751 | C(F)^G(M)^A(F)C(M)A(F)C(M)A(F)U(M)G(F)G(M)A(F) A(F)A(M)U(F)U(M)G(F)G(M)A(F)C(M)U(F)A(M) | No. 6453 | U(F)^A(M)^G(F)U(M)C(F)C(M)A(F)A(M)U(F)U(M)U(M)C(F)C (M)A(F)U(M)G(F)U(F)(F)U(M)C(F)G(M)^U(F)^G(M) |
| CH0627 | No. 5752 | A(F)^C(M)^A(F)C(M)A(F)U(F)G(F)G(M)A(F)A(M)A(F) U(F)U(M)G(F)G(M)A(F)C(M)U(F)A(M)A(F)A(M) | No. 6454 | U(F)^U(M)^U(F)A(M)G(F)U(M)C(F)C(M)A(F)A(M)U(M)U( M)C(F)C(M)A(F)U(M)G(F)U(M)G(F)U(M)^C(F)^G(M) |
| CH0628 | No. 5753 | C(F)^A(M)^C(F)A(M)U(F)G(M)G(F)A(M)A(F)A(M)U(F) U(F)G(M)G(F)A(M)C(F)U(M)A(F)A(M)A(F)U(M) | No. 6455 | A(F)^U(M)^U(F)U(M)A(F)G(M)U(F)C(M)C(F)A(M)A(M)U(F)U( M)U(F)C(M)C(F)A(M)U(F)G(M)U(F)G(M)U(F)G(M)^U(F)^C(M) |
| CH0629 | No. 5754 | A(F)^C(M)^A(F)U(M)G(F)G(M)A(F)A(M)A(F)U(M)U(F) G(F)G(M)A(F)C(M)U(F)A(M)A(F)A(M)U(F)U(M) | No. 6456 | A(F)^A(M)^U(F)U(M)U(F)A(M)G(F)U(M)C(F)C(M)A(M)A(F)U( M)U(F)U(M)C(F)C(M)A(F)U(F)G(F)U(M)^G(F)^U(M) |
| CH0630 | No. 5755 | C(F)^A(M)^U(F)G(M)G(F)A(M)A(F)A(M)U(F)U(M)G(F) G(F)A(M)C(F)U(M)A(F)A(M)A(F)A(M)U(F)A(M) | No. 6457 | U(F)^A(M)^A(F)U(M)U(F)U(M)A(F)G(M)U(F)C(M)C(M)(F)A( M)U(F)U(M)U(F)C(M)C(F)A(M)U(F)G(M)^U(F)^G(M) |
| CH0631 | No. 5756 | A(F)^U(M)^G(F)G(M)A(F)A(M)A(F)U(M)U(F)G(M)G(F) A(F)C(M)U(F)A(M)A(F)A(M)U(F)U(M)A(F)C(M) | No. 6458 | G(F)^U(M)^A(F)A(M)U(F)U(M)U(F)A(M)G(F)U(M)C(M)C(F)A( M)A(F)U(M)U(F)U(M)C(F)C(M)A(F)U(M)^G(F)^U(M) |
| CH0633 | No. 5757 | G(F)^G(M)^A(F)A(M)A(F)U(M)U(F)G(M)G(F)A(M)C(F) U(F)A(M)A(F)A(M)U(F)U(M)A(F)C(M)C(F)A(M) | No. 6459 | U(F)^G(M)^G(F)U(M)A(F)A(M)U(F)U(M)U(F)A(M)G(M)U(F)C( M)C(F)A(M)A(F)U(M)U(F)U(M)C(F)C(M)^A(F)^U(M) |
| CH0638 | No. 5758 | U(F)^U(M)^G(F)G(M)A(F)C(M)U(F)A(M)A(F)A(M)U(F) U(F)A(M)C(F)C(M)A(F)G(M)A(F)A(M)U(F)G(M) | No. 6460 | C(F)^A(M)^U(F)U(M)C(F)U(M)G(F)G(M)U(F)A(M)A(M)U(F)U( M)U(F)A(M)G(F)U(M)C(F)C(M)A(F)A(M)^U(F)^U(M) |
| CH0649 | No. 5759 | U(F)^A(M)^C(F)C(M)A(F)G(M)A(F)A(M)U(F)G(M)C(F) A(F)G(M)G(F)G(M)A(F)A(M)G(F)U(M)A(F)A(M) | No. 6461 | U(F)^U(M)^A(F)C(M)U(F)U(M)C(F)C(M)U(F)U(M)G(M)C(F)A (M)U(F)U(M)C(F)U(M)G(F)G(M)U(F)A(M)^A(F)^U(M) |
| CH0650 | No. 5760 | A(F)^C(M)^C(F)A(M)G(F)A(M)A(F)U(M)G(F)C(M)A(F) G(F)G(M)G(F)A(M)A(F)G(M)U(F)A(M)A(F)A(M) | No. 6462 | U(F)^A(M)^U(F)A(M)C(F)U(M)U(F)C(M)C(F)C(M)U(M)G(F)C (M)A(F)U(M)U(F)C(M)U(F)G(M)G(F)U(M)^A(F)^A(M) |
| CH0651 | No. 5761 | C(F)^C(M)^A(F)G(M)A(F)A(M)U(F)G(M)C(F)A(M)G(F) G(F)G(M)A(F)A(M)G(F)U(M)A(F)A(M)A(F)A(M) | No. 6463 | U(F)^U(M)^U(F)U(M)A(F)C(M)U(F)U(M)C(F)C(M)C(M)U(F)G (M)C(F)A(M)U(F)U(M)C(F)U(M)G(F)G(M)^U(F)^A(M) |
| CH0652 | No. 5762 | C(F)^A(M)^G(F)A(M)A(F)U(M)G(F)C(M)A(F)G(M)G(F) G(F)A(M)A(F)G(M)U(F)A(M)A(F)A(M)A(F)U(M) | No. 6464 | A(F)^U(M)^U(F)U(M)U(F)A(M)C(F)U(M)U(F)C(M)C(M)C(F)U( M)G(F)C(M)A(F)U(M)U(F)C(M)U(F)G(M)^G(F)^G(M) |
| CH0653 | No. 5763 | A(F)^G(M)^A(F)A(M)U(F)G(M)C(F)A(M)G(F)G(M)G(F) A(F)A(M)G(F)U(M)A(F)A(M)A(F)A(M)U(F)G(M) | No. 6465 | C(F)^A(M)^U(F)U(M)U(F)U(M)A(F)C(M)U(F)U(M)C(M)C(F)C (M)U(F)G(M)C(F)A(M)U(F)U(M)C(F)U(M)^G(F)^G(M) |
| CH0657 | No. 5764 | U(F)^G(M)^C(F)A(M)G(F)G(M)G(F)A(M)A(F)G(M)U(F) A(F)A(M)A(F)A(M)U(F)G(M)C(F)C(M)C(F)A(M) | No. 6466 | U(F)^G(M)^G(F)G(M)C(F)A(M)U(F)U(M)U(F)U(M)A(M)C(F)U (M)U(F)C(M)C(F)C(M)U(F)G(M)C(F)A(M)^U(F)^U(M) |

### [Table 30]

**Table 3-10**

| Double stranded nucleic acid No. | SEQ ID NO. | Sense strand sequence (5'→3') | SEQ ID NO. | Antisense strand sequence (5→3) |
|---|---|---|---|---|
| CH0661 | No. 5765 | G(F)^G(M)^G(F)A(M)A(F)G(M)U(F)A(M)A(F)A(M)A(F) U(F)G(M)C(F)C(M)C(F)A(M)U(F)U(M)C(F)C(M) | No. 6467 | G(F)^G(M)^A(F)A(M)U(F)G(M)G(F)G(M)C(F)A(M)U(M)U(F)U (M)U(F)A(M)C(F)U(M)U(F)C(M)C(F)C(M)^U(F)^G(M) |
| CH0664 | No. 5766 | A(F)^A(M)^G(F)U(M)A(F)A(M)A(F)A(M)U(F)G(M)C(F) C(F)C(M)A(F)U(M)U(F)C(M)C(F)C(M)A(F)U(M) | No. 6468 | A(F)^U(M)^G(F)G(M)G(F)A(M)A(F)U(M)G(F)C(M)G(M)C(F)A (M)U(F)U(M)U(F)U(M)A(F)C(M)U(F)U(M)^C(F)^C(M) |
| CH0669 | No. 5767 | A(F)^A(M)^A(F)U(M)G(F)C(M)C(F)C(M)A(F)U(M)U(F) C(F)C(M)C(F)A(M)U(F)C(M)A(F)A(M)G(F)A(M) | No. 6469 | U(F)^C(M)^U(F)U(M)G(F)A(M)U(F)G(M)G(F)G(M)A(M)A(F)U (M)G(F)G(M)G(F)C(M)A(F)U(M)U(F)U(M)^U(F)^A(M) |
| CH0670 | No. 5768 | A(F)^A(M)^U(F)G(M)C(F)C(M)C(F)A(M)U(F)U(M)C(F) C(F)C(M)A(F)U(M)C(F)A(M)A(F)G(M)A(F)C(M) | No. 6470 | G(F)^U(M)^C(F)U(M)U(F)G(M)A(F)U(M)G(F)G(M)G(M)A(F)A (M)U(F)G(M)G(F)G(M)C(F)A(M)U(F)U(M)^U(F)^U(M) |
| CH0672 | No. 5769 | U(F)^G(M)^C(F)C(M)C(F)A(M)U(F)U(M)C(F)C(M)C(F) A(F)U(M)C(F)A(M)A(F)G(M)A(F)C(M)C(F)A(M) | No. 6471 | U(F)^G(M)^G(F)U(M)C(F)U(M)U(F)G(M)A(F)U(M)G(M)G(F)G (M)A(F)A(M)U(F)G(M)G(F)G(M)C(F)A(M)^U(F)^U(M) |
| CH0673 | No. 5770 | G(F)^C(M)^C(F)C(M)A(F)U(M)U(F)C(M)C(F)C(M)A(F) U(F)C(M)A(F)A(M)G(F)A(M)C(F)C(M)A(F)G(M) | No. 6472 | C(F)^U(M)^G(F)G(M)U(F)C(M)U(F)U(M)G(F)A(M)U(M)G(F)G (M)G(F)A(M)A(F)U(M)G(F)G(M)G(F)C(M)^A(F)^U(M) |
| CH0674 | No. 5771 | C(F)^C(M)^C(F)A(M)U(F)U(M)C(F)C(M)C(F)A(M)U(F) C(F)A(M)A(F)G(M)A(F)C(M)C(F)A(M)G(F)A(M) | No. 6473 | U(F)^C(M)^U(F)G(M)G(F)U(M)C(F)U(M)U(F)G(M)A(M)U(F)G (M)G(F)G(M)A(F)A(M)U(F)G(M)G(F)G(M)^C(F)^A(M) |
| CH0678 | No. 5772 | U(F)^U(M)^G(F)C(M)C(F)A(M)U(F)C(M)A(F)A(M)G(F) A(F)C(M)C(F)A(M)G(F)A(M)G(F)A(M)A(F)U(M) | No. 6474 | A(F)^U(M)^U(F)G(M)U(F)C(M)U(F)G(M)G(F)U(M)C(M)U(F)U (M)G(F)A(M)U(F)G(M)G(F)G(M)A(F)A(M)^U(F)^G(M) |
| CH0679 | No. 5773 | U(F)^C(M)^C(F)C(M)A(F)U(M)C(F)A(M)A(F)G(M)A(F) C(F)C(M)A(F)G(M)A(F)C(M)A(F)A(M)U(F)G(M) | No. 6475 | C(F)^A(M)^U(F)U(M)G(F)U(M)C(F)U(M)G(F)G(M)U(M)C(F)U (M)U(F)G(M)A(F)U(M)G(F)G(M)G(F)A(M)^A(F)^U(M) |
| CH0680 | No. 5774 | C(F)^C(M)^C(F)A(M)U(F)C(M)A(F)A(M)G(F)A(M)C(F) C(F)A(M)G(F)A(M)C(F)A(M)A(F)U(M)G(F)G(M) | No. 6476 | C(F)^C(M)^A(F)U(M)U(F)G(M)U(F)C(M)U(F)G(M)G(M)U(F)C (M)U(F)U(M)G(F)A(M)U(F)G(M)G(F)G(M)^A(F)^A(M) |
| CH0681 | No. 5775 | C(F)^C(M)^A(F)U(M)C(F)A(M)A(F)G(M)A(F)C(M)C(F) A(F)G(M)A(F)C(M)A(F)A(M)U(F)G(M)G(F)A(M) | No. 6477 | U(F)^C(M)^C(F)A(M)U(F)U(M)G(F)U(M)C(F)U(M)G(M)G(F)U (M)C(F)U(M)U(F)G(M)A(F)U(M)G(F)G(M)^G(F)^A(M) |
| CH0682 | No. 5776 | C(F)^A(M)^U(F)C(M)A(F)A(M)G(F)A(M)C(F)C(M)A(F) G(F)A(M)C(F)A(M)A(F)U(M)G(F)G(M)A(F)U(M) | No. 6478 | A(F)^U(M)^C(F)C(M)A(F)U(M)U(F)G(M)U(F)C(M)U(M)G(F)G (M)U(F)C(M)U(F)U(M)G(F)A(M)U(F)G(M)^G(F)^G(M) |
| CH0683 | No. 5777 | A(F)^U(M)^G(F)A(M)A(F)G(M)A(F)C(M)C(F)A(M)G(F) A(F)C(M)A(F)A(M)U(F)G(M)G(F)A(M)U(F)U(M) | No. 6479 | A(F)^A(M)^U(F)C(M)C(F)A(M)U(F)U(M)G(F)U(M)C(M)U(F)G (M)G(F)U(M)C(F)U(M)U(F)G(M)A(F)U(M)^G(F)^G(M) |
| CH0684 | No. 5778 | U(F)^C(M)^A(F)A(M)G(F)A(M)C(F)C(M)A(F)G(M)A(F) C(F)A(M)A(F)U(M)G(F)G(M)A(F)U(M)U(F)U(M) | No. 6480 | A(F)^A(M)^A(F)U(M)C(F)C(M)A(F)U(M)U(F)G(M)U(M)C(F)U( M)G(F)G(M)U(F)C(M)U(F)U(M)G(F)A(M)^U(F)^G(M) |
| CH0685 | No. 5779 | C(F)^A(M)^A(F)G(M)A(F)C(M)C(F)A(M)G(F)A(M)C(F) A(F)A(M)U(F)G(M)G(F)A(M)U(F)U(M)U(F)G(M) | No. 6481 | C(F)^A(M)^A(F)A(M)U(F)C(M)C(F)A(M)U(F)U(M)G(M)U(F)G (M)U(F)G(M)G(F)U(M)C(F)U(M)U(F)G(M)^A(F)^U(M) |
| CH0687 | No. 5780 | A(F)^G(M)^A(F)C(M)C(F)A(M)G(F)A(M)C(F)A(M)A(F) U(F)G(M)G(F)A(M)U(F)U(M)U(F)G(M)U(F)G(M) | No. 6482 | C(F)^A(M)^C(F)A(M)A(F)A(M)U(F)C(M)C(F)A(M)U(M)U(F)G (M)U(F)C(M)U(F)G(M)G(F)U(M)C(F)U(M)^U(F)^G(M) |
| CH0688 | No. 5781 | G(F)^A(M)^C(F)C(M)A(F)G(M)A(F)C(M)A(F)A(M)U(F) G(F)G(M)A(F)U(M)U(F)U(M)G(F)U(M)G(F)A(M) | No. 6483 | U(F)^C(M)^A(F)C(M)A(F)A(M)A(F)U(M)C(F)C(M)A(M)U(F)U( M)G(F)U(M)C(F)U(M)G(F)G(M)U(F)C(M)^U(F)^U(M) |
| CH0689 | No. 5782 | A(F)^C(M)^C(F)A(M)G(F)A(M)C(F)A(M)A(F)U(M)G(F) G(F)A(M)U(F)U(M)U(F)G(M)U(F)G(M)A(F)A(M) | No. 6484 | U(F)^U(M)^C(F)A(M)C(F)A(M)A(F)A(M)U(F)C(M)C(M)A(F)U( M)U(F)G(M)U(F)C(M)U(F)G(M)G(F)U(M)^C(F)^U(M) |
| CH0890 | No. 5783 | G(F)^C(M)^A(F)G(M)A(F)C(M)A(F)A(M)U(F)G(M)G(F) A(F)U(M)U(F)U(M)G(F)U(M)G(F)A(M)A(F)C(M) | No. 6485 | G(F)^U(M)^U(F)C(M)A(F)C(M)A(F)A(M)A(F)U(M)C(M)C(F)A (M)U(F)U(M)G(F)U(M)C(F)U(M)G(F)G(M)^U(F)^C(M) |
| CH0692 | No. 5784 | A(F)^G(M)^A(F)C(M)A(F)A(M)U(F)G(M)G(F)A(M)U(F) U(F)U(M)G(F)U(M)G(F)A(M)A(F)C(M)U(F)A(M) | No. 6486 | U(F)^A(M)^G(F)U(M)U(F)C(M)A(F)C(M)A(F)A(M)A(M)U(F)C( M)C(F)A(M)U(F)U(M)G(F)U(M)C(F)U(M)^G(F)^G(M) |
| CH0693 | No. 5785 | G(F)^A(M)^C(F)A(M)A(F)U(M)G(F)G(M)A(F)U(M)U(F) U(F)G(M)U(F)G(M)A(F)A(M)C(F)U(M)U(F)U(M) | No. 6487 | A(F)^U(M)^A(F)G(M)U(F)U(M)C(F)A(M)C(F)A(M)A(M)A(F)U( M)C(F)C(M)A(F)U(M)U(F)G(M)U(F)C(M)^U(F)^G(M) |
| CH0694 | No. 5786 | A(F)^C(M)^A(F)A(M)U(F)G(M)G(F)A(M)U(F)U(M)U(F) G(F)U(M)G(F)A(M)A(F)C(M)U(F)A(M)U(F)C(M) | No. 6488 | G(F)^A(M)^U(F)A(M)G(F)U(M)U(F)C(M)A(F)C(M)A(M)A(F)A( M)U(F)C(M)C(F)A(M)U(F)U(M)G(F)U(M)^C(F)^U(M) |
| CH0697 | No. 5787 | A(F)^U(M)^G(F)G(M)A(F)U(M)U(F)U(M)G(F)U(M)G(F) A(F)A(M)C(F)U(M)A(F)U(M)C(F)C(M)U(F)G(M) | No. 6489 | C(F)^A(M)^G(F)G(M)A(F)U(M)A(F)G(M)U(F)U(M)C(M)A(F)C (M)A(F)A(M)A(F)U(M)C(F)C(M)A(F)U(M)^U(F)^G(M) |
| CH0699 | No. 5788 | G(F)^G(M)^A(F)U(M)U(F)U(M)G(F)U(M)G(F)U(M)A(F) C(F)U(M)A(F)U(M)G(F)C(M)U(F)G(M)C(F)A(M) | No. 6490 | U(F)^G(M)^C(F)A(M)G(F)G(M)A(F)U(M)A(F)G(M)U(M)U(F)C (M)A(F)C(M)A(F)A(M)A(F)U(M)C(F)C(M)^A(F)^U(M) |
| CH0700 | No. 5789 | G(F)^A(M)^U(F)U(M)U(F)G(M)U(F)G(M)A(F)A(M)C(F) U(F)A(M)U(F)C(M)C(F)U(M)G(F)C(M)A(F)A(M) | No. 6491 | U(F)^U(M)^G(F)C(M)A(F)G(M)G(F)A(M)U(F)A(M)G(M)U(F)U (M)C(F)A(M)C(F)A(M)A(F)A(M)U(F)C(M)^C(F)^A(M) |
| CH0701 | No. 5790 | A(F)^U(M)^U(F)U(M)G(F)U(M)G(F)A(M)U(F)C(M)U(F) A(F)U(M)C(F)C(M)U(F)G(M)C(F)A(M)A(F)A(M) | No. 6492 | U(F)^U(M)^U(F)G(M)C(F)A(M)G(F)G(M)A(F)U(M)A(M)G(F)U (M)U(F)C(M)A(F)C(M)A(F)A(M)A(F)U(M)^C(F)^C(M) |
| CH0702 | No. 5791 | U(F)^U(M)^U(F)G(M)U(F)G(M)A(F)A(M)C(F)U(M)A(F) U(F)C(M)C(F)U(M)G(F)C(M)A(F)A(M)A(F)A(M) | No. 6493 | U(F)^U(M)^U(F)U(M)G(F)C(M)A(F)G(M)G(F)A(M)U(M)A(F)G (M)U(F)U(M)C(F)A(M)C(F)A(M)A(F)A(M)^U(F)^C(M) |
| CH0705 | No. 5792 | G(F)^U(M)^G(F)A(M)A(F)C(M)U(F)A(M)U(F)C(M)C(F) U(F)G(M)C(F)A(M)A(F)A(M)A(F)C(M)C(F)A(M) | No. 6494 | U(F)^G(M)^G(F)U(M)U(F)U(M)U(F)G(M)C(F)A(M)G(M)G(F)A (M)U(F)A(M)G(F)U(M)U(F)C(M)A(F)C(M)^A(F)^A(M) |
| CH0706 | No. 5793 | U(F)^G(M)^A(F)A(M)C(F)U(M)A(F)U(M)C(F)C(M)U(F) G(F)C(M)A(F)A(M)A(F)A(M)C(F)C(M)A(F)A(M) | No. 6495 | U(F)^U(M)^G(F)G(M)U(F)U(M)U(F)U(M)G(F)C(M)A(M)G(F)G (M)A(F)U(M)A(F)G(M)U(F)U(M)C(F)A(M)^C(F)^A(M) |
| CH0708 | No. 5794 | A(F)^A(M)^C(F)U(M)A(F)U(M)C(F)C(M)U(F)G(M)C(F) A(F)A(M)A(F)A(M)C(F)C(M)A(F)A(M)C(F)A(M) | No. 6496 | U(F)^G(M)^U(F)U(M)G(F)G(M)U(F)U(M)U(F)U(M)G(M)C(F)A (M)G(F)G(M)A(F)U(M)A(F)G(M)U(F)U(M)^C(F)^A(M) |
| CH0709 | No. 5795 | A(F)^C(M)^U(F)A(M)U(F)C(M)C(F)U(M)G(F)C(M)A(F) A(F)A(M)A(F)C(M)C(F)A(M)A(F)C(M)A(F)C(M) | No. 6497 | G(F)^U(M)^G(F)U(M)G(M)G(F)U(M)U(F)U(M)U(M)G(F)C (M)A(F)G(M)G(F)A(M)U(F)A(M)G(F)U(M)^U(F)^C(M) |
| CH0711 | No. 5796 | U(F)^A(M)^U(F)C(M)C(F)U(M)G(F)C(M)A(F)A(M)A(F) A(F)C(M)C(F)A(M)A(F)C(M)A(F)C(M)U(F)U(M) | No. 6498 | A(F)^A(M)^G(F)U(M)G(F)U(M)U(F)G(M)G(F)U(M)U(M)U(F)U( M)G(F)C(M)A(F)G(M)G(F)A(M)U(F)A(M)^G(F)^U(M) |
| CH0712 | No. 5797 | A(F)^U(M)^C(F)C(M)U(F)G(M)C(F)A(M)A(F)A(M)A(F) C(F)C(M)A(F)A(M)C(F)A(M)C(F)U(M)U(F)U(M) | No. 6499 | A(F)^A(M)^A(F)G(M)U(F)G(M)U(F)U(M)G(F)G(M)U(M)U(F)U( M)U(F)G(M)C(F)A(M)G(F)G(M)A(F)U(M)^A(F)^G(M) |
| CH0713 | No. 5798 | U(F)^C(M)^C(F)U(M)G(F)C(M)A(F)A(M)A(F)A(M)C(F) C(F)A(M)A(F)C(M)A(F)C(M)U(F)U(M)U(F)A(M) | No. 6500 | U(F)^A(M)^A(F)A(M)G(F)U(M)G(F)U(M)U(F)G(M)G(M)U(F)U( M)U(F)U(M)G(F)C(M)A(F)G(M)G(F)A(M)^U(F)^A(M) |
| CH0714 | No. 5799 | C(F)^C(M)^U(F)G(M)C(F)A(M)A(F)A(M)A(F)C(M)C(F) A(F)A(M)C(F)A(M)C(F)U(M)U(F)U(M)A(F)U(M) | No. 6501 | A(F)^U(M)^A(F)A(M)A(F)G(M)U(F)G(M)U(F)U(M)G(M)G(F)U( M)U(F)U(M)U(F)G(M)C(F)A(M)G(F)G(M)^A(F)^U(M) |
| CH0715 | No. 5800 | C(F)^U(M)^G(F)C(M)A(F)A(M)A(F)A(M)C(F)C(M)A(F) A(F)C(M)A(F)C(M)U(F)U(M)U(F)A(M)U(F)U(M) | No. 6502 | A(F)^A(M)^U(F)A(M)A(F)A(M)G(F)U(M)G(F)U(M)U(M)G(F)G( M)U(F)U(M)U(F)U(M)G(F)C(M)A(F)G(M)^G(F)^A(M) |
| CH0716 | No. 5801 | U(F)^G(M)^C(F)A(M)A(F)A(M)A(F)C(M)C(F)A(M)A(F) C(F)A(M)C(F)U(M)U(F)U(M)A(F)U(M)U(F)A(M) | No. 6503 | U(F)^A(M)^A(F)U(M)A(F)A(M)A(F)G(M)U(F)G(M)U(M)U(F)G( M)G(F)U(M)U(F)U(M)U(F)G(M)C(F)A(M)^G(F)^G(M) |
| CH0718 | No. 5802 | C(F)^A(M)^A(F)A(M)A(F)C(M)C(F)A(M)A(F)C(M)A(F) C(F)U(M)U(F)U(M)U(F)U(M)U(F)A(M)C(F)A(M) | No. 6604 | U(F)^G(M)^U(F)A(M)A(F)U(M)A(F)A(M)A(F)G(M)G(M)G(F)U( M)U(F)G(M)G(F)U(M)U(F)U(M)U(F)G(M)^C(F)^A(M) |
| CH0719 | No. 5803 | A(F)^A(M)^A(F)A(M)C(F)C(M)A(F)A(M)C(F)A(M)C(F) U(F)U(M)U(F)A(M)U(F)U(M)A(F)C(M)A(F)A(M) | No. 6505 | U(F)^U(M)^G(F)U(M)A(F)A(M)U(F)A(M)A(F)A(M)G(M)U(F)G( M)U(F)U(M)G(F)G(M)U(F)U(M)U(F)U(M)^G(F)^C(M) |
| CH0721 | No. 5804 | A(F)^A(M)^C(F)C(M)A(F)A(M)C(F)A(M)C(F)U(M)U(F) U(F)A(M)U(F)U(M)A(F)C(M)A(F)A(M)G(F)G(M) | No. 6506 | C(F)^C(M)^U(F)U(M)G(F)U(M)A(F)A(M)U(F)A(M)A(M)A(F)G( M)U(F)G(M)U(F)U(M)G(F)G(M)U(F)U(M)^U(F)^U(M) |

### [Table 31]

**Table 3-11**

| Double stranded nucleic acid No. | SEQ ID NO. | Sense strand sequence (5'→3') | SEQ ID NO. | Antisense strand sequence (5'→3') |
|---|---|---|---|---|
| CH0723 | No. 5805 | C(F)^C(M)^A(F)A(M)C(F)A(M)C(F)U(M)U(F)U(M)A(F) U(F)U(M)A(F)C(M)A(F)A(M)G(F)G(M)A(F)U(M) | No. . 6507 | A(F)^U(M)^C(F)C(M)U(F)U(M)G(F)U(M)A(F)A(M)U(M)A(F)A( M)A(F)G(M)U(F)G(M)U(F)U(M)G(F)G(M)^U(F)^U(M) |
| CH0724 | No. 5806 | C(F)^A(M)^A(F)C(M)A(F)C(M)U(F)U(M)U(F)A(M)U(F) U(F)A(M)C(F)A(M)A(F)G(M)G(F)A(M)U(F)A(M) | No. 6508 | U(F)^A(M)^U(F)C(M)C(F)U(M)U(F)G(M)U(F)A(M)A(M)U(F)A( M)A(F)A(M)G(F)U(M)G(F)U(M)U(F)G(M)^G(F)^U(M) |
| CH0725 | No. 5807 | A(F)^A(M)^C(F)A(M)C(F)U(M)U(F)U(M)A(F)U(M)U(F)A (F)C(M)A(F)A(M)G(F)G(M)A(F)U(M)A(F)A(M) | No. 6509 | U(F)^U(M)^A(F)U(M)C(F)C(M)U(F)U(M)G(F)U(M)A(M)A(F)U( M)A(F)A(M)A(F)G(M)U(F)G(M)U(F)U(M)^G(F)^G(M) |
| CH0735 | No. 5808 | U(F)^A(M)^C(F)A(M)A(F)G(M)G(F)A(M)U(F)A(M)A(F) A(F)G(M)C(F)C(M)A(F)C(M)A(F)U(M)U(F)U(M) | No. 6510 | A(F)^A(M)^A(F)U(M)G(F)U(M)G(F)G(M)C(F)U(M)U(M)U(F)A( M)U(F)C(M)C(F)U(M)U(F)G(M)U(F)A(M)^A(F)^U(M) |
| CH0737 | No. 5809 | C(F)^A(M)^A(F)G(M)G(F)A(M)U(F)A(M)A(F)A(M)G(F) C(F)C(M)A(F)C(M)A(F)U(M)U(F)U(M)G(F)G(M) | No. 6511 | C(F)^C(M)^A(F)A(M)A(F)U(M)G(F)U(M)G(F)G(M)C(M)U(F)U (M)U(F)A(M)U(F)C(M)C(F)U(M)U(F)G(M)^U(F)^A(M) |
| CH0739 | No. 5810 | A(F)^G(M)^G(F)A(M)U(F)A(M)A(F)A(M)G(F)C(M)C(F) A(F)C(M)A(F)U(M)U(F)U(M)G(F)G(M)C(F)U(M) | No. 6512 | A(F)^G(M)^C(F)C(M)A(F)A(M)A(F)U(M)G(F)U(M)G(M)G(F)C (M)U(F)U(M)U(F)A(M)U(F)C(M)C(F)U(M)^U(F)^G(M) |
| CH0743 | No. 5811 | U(F)^A(M)^A(F)A(M)G(F)C(M)C(F)A(M)C(F)A(M)U(F) U(F)U(M)G(F)G(M)C(F)U(M)G(F)C(M)C(F)A(M) | No. 6513 | U(F)^G(M)^G(F)C(M)A(F)G(M)C(F)C(M)A(F)A(M)A(M)U(F)G (M)U(F)G(M)G(F)C(M)U(F)U(M)U(F)A(M)^U(F)^C(M) |
| CH0744 | No. 5812 | A(F)^A(M)^A(F)G(M)C(F)C(M)A(F)C(M)A(F)U(M)U(F) U(F)G(M)G(F)C(M)U(F)G(M)C(F)C(M)A(F)U(M) | No. 6514 | A(F)^U(M)^G(F)G(M)C(F)A(M)G(F)C(M)C(F)A(M)A(M)A(F)U (M)G(F)U(M)G(F)G(M)C(F)U(M)U(F)U(M)^A(F)^U(M) |
| CH0745 | No. 5813 | A(F)^A(M)^G(F)C(M)C(F)A(M)C(F)A(M)U(F)U(M)U(F) G(F)G(M)C(F)U(M)G(F)C(M)C(F)A(M)U(F)G(M) | No. 6515 | C(F)^A(M)^U(F)G(M)G(F)C(M)A(F)G(M)C(F)C(M)A(M)A(F)A (M)U(F)G(M)U(F)G(M)G(F)G(M)U(F)U(M)^U(F)^A(M) |
| CH0746 | No. 5814 | A(F)^G(M)^C(F)C(M)A(F)C(M)A(F)U(M)U(F)U(M)G(F) G(F)C(M)U(F)G(M)C(F)C(M)A(F)U(M)G(F)A(M) | No. 6516 | U(F)^C(M)^A(F)U(M)G(F)G(M)C(F)A(M)G(F)C(M)C(M)A(F)A (M)A(F)U(M)G(F)U(M)G(F)G(M)C(F)U(M)^U(F)U(M) |
| CH0747 | No. 5815 | G(F)^G(M)^C(F)A(M)C(F)A(M)U(F)G(M)G(F) C(F)U(M)C(F)C(M)C(F)A(M)U(F)G(M)A(F)U(M) | No. 6517 | A(F)^U(M)^C(F)A(M)U(F)G(M)G(F)C(M)A(F)G(M)C(M)C(F)A(M)A(F)A(M)U(F)G(M)U(F)G(M)G(F)C(M)^U(F)^U(M) |
| CH0748 | No. 5816 | C(F)^C(M)^A(F)C(M)A(F)U(M)U(F)U(M)G(F)G(M)C(F) U(F)G(M)C(F)C(M)A(F)U(M)G(F)A(M)U(F)G(M) | No. 6518 | C(F)^A(M)^U(F)C(M)A(F)U(M)G(F)G(M)C(F)A(M)G(M)C(F)C(M)A(F)A(M)A(F)U(M)G(F)U(M)G(F)C(M)^C(F)^U(M) |
| CH0749 | No. 5817 | C(F)^A(M)^C(F)A(M)U(F)U(M)U(F)G(M)G(F)C(M)U(F) G(F)C(M)C(F)A(M)U(F)G(M)A(F)U(M)G(F)G(M) | No. 6519 | C(F)^C(M)^A(F)U(M)C(F)A(M)U(F)G(M)G(F)C(M)A(M)G(F)C(M)C(F)A(M)A(F)A(M)U(F)G(M)U(F)G(M)^G(F)^C(M) |
| CH0750 | No. 5818 | A(F)^C(M)^A(F)U(M)U(F)U(M)G(F)G(M)C(F)U(M)G(F) C(F)C(M)A(F)U(M)G(F)A(M)U(F)G(M)G(F)A(M) | No. 6520 | U(F)^C(M)^C(F)A(M)U(F)C(M)A(F)U(M)G(F)G(M)C(M)A(F)G(M)C(F)C(M)A(F)A(M)A(F)U(M)G(F)U(M)^G(F)^G(M) |
| CH0753 | No. 5819 | U(F)^U(M)^U(F)G(M)G(F)C(M)U(F)G(M)C(F)C(M)A(F) U(F)G(M)A(F)U(M)G(F)G(M)A(F)U(M)A(F)U(M) | No. 6521 | A(F)^U(M)^A(F)U(M)C(F)C(M)A(F)U(M)C(F)A(M)U(M)G(F)G(M)C(F)A(M)G(F)C(M)C(F)A(M)A(F)A(M)^U(F)^G(M) |
| CH0754 | No. 5820 | U(F)^U(M)^G(F)G(M)C(F)U(M)G(F)C(M)C(F)A(M)U(F) G(F)A(M)U(F)G(M)G(F)A(M)U(F)A(M)U(F)U(M) | No. 6522 | A(F)^A(M)^U(F)A(M)U(F)C(M)C(F)A(M)U(F)C(M)A(M)U(F)G(M)G(F)C(M)A(F)G(M)C(F)C(M)A(F)A(M)^A(F)^U(M) |
| CH0756 | No. 5821 | G(F)^G(M)^C(F)U(M)G(F)C(M)C(F)A(M)U(F)G(M)A(F) U(F)G(M)G(F)A(M)U(F)A(M)U(F)U(M)C(F)U(M) | No. 6523 | A(F)^G(M)^A(F)A(M)U(F)A(M)U(F)C(M)C(F)A(M)U(M)C(F)A(M)U(F)G(M)G(F)C(M)A(F)G(M)C(F)C(M)^A(F)^A(M) |
| CH0757 | No. 5822 | G(F)^C(M)^U(F)G(M)C(F)C(M)A(F)U(M)G(F)A(M)U(F) G(F)G(M)A(F)U(M)A(F)U(M)U(F)C(M)U(F)C(M) | No. 6524 | G(F)^A(M)^G(F)A(M)A(F)U(M)A(F)U(M)C(F)C(M)A(M)U(F)C(M)A(F)U(M)G(F)G(M)C(F)A(M)G(F)C(M)^C(F)^A(M) |
| CH0759 | No. 5823 | U(F)^G(M)^C(F)C(M)A(F)U(M)G(F)A(M)U(F)G(M)G(F) A(F)U(M)A(F)U(M)U(F)C(M)U(F)C(M)U(F)G(M) | No. 6525 | C(F)^A(M)^G(F)A(M)G(F)A(M)A(F)U(M)A(F)U(M)C(M)C(F)A(M)U(F)C(M)A(F)U(M)G(F)G(M)C(F)A(M)^G(F)^C(M) |
| CH0760 | No. 5824 | G(F)^C(M)^C(F)A(M)U(F)G(M)A(F)U(M)G(M)A(F) U(F)A(M)U(F)U(M)C(F)U(M)C(F)U(M)G(F)G(M) | No. 6526 | C(F)^C(M)^A(F)G(M)A(F)G(M)A(F)A(M)U(F)A(M)U(M)C(F)C(M)A(F)U(M)C(F)A(M)U(F)G(M)G(F)C(M)^A(F)^G(M) |
| CH0762 | No. 5825 | C(F)^A(M)^U(F)G(M)A(F)U(M)G(F)G(M)A(F)U(M)A(F) U(F)U(M)C(F)U(M)C(F)U(M)G(F)G(M)A(F)U(M) | No. 6527 | A(F)^U(M)^C(F)C(M)A(F)G(M)A(F)G(M)A(F)A(M)U(M)A(F)U(M)C(F)C(M)A(F)U(M)C(F)A(M)U(F)G(M)^G(F)^C(M) |
| CH0763 | No. 5826 | A(F)^U(M)^G(F)A(M)U(F)G(M)G(F)A(M)U(F)A(M)U(F) U(F)C(M)U(F)C(M)U(F)G(M)G(F)A(M)U(F)G(M) | No. 6528 | C(F)^A(M)^U(F)C(M)C(F)A(M)G(F)A(M)G(F)A(M)A(M)U(F)A(M)U(F)C(M)C(F)A(M)U(F)C(M)A(F)U(M)^G(F)^G(M) |
| CH0766 | No. 5827 | A(F)^U(M)^G(F)G(M)A(F)U(M)A(F)U(M)U(F)C(M)U(F) C(F)U(M)G(F)G(M)A(F)U(M)G(F)G(M)C(F)C(M) | No. 6529 | G(F)^G(M)^C(F)C(M)A(F)U(M)C(F)C(M)A(F)G(M)A(M)G(F)A(M)A(F)U(M)A(F)U(M)C(F)C(M)A(F)U(M)^C(F)^A(M) |
| CH0771 | No. 5828 | U(F)^A(M)^U(F)U(M)C(F)U(M)C(F)U(M)G(F)G(M)A(F) U(F)G(M)G(F)C(M)C(F)C(M)G(F)G(M)A(F)A(M) | No. 6530 | U(F)^U(M)^C(F)C(M)G(F)G(M)G(F)C(M)C(F)A(M)U(M)C(F)C(M)A(F)G(M)A(F)G(M)A(F)A(M)U(F)A(M)^U(F)^A(M) |
| CH0773 | No. 5829 | U(F)^U(M)^C(F)U(M)C(F)U(M)G(F)G(M)A(F)U(M)G(F) G(F)C(M)C(F)C(M)G(F)G(M)A(F)A(M)G(F)A(M) | No. 6531 | U(F)^C(M)^U(F)U(M)C(F)C(M)G(F)G(M)G(F)C(M)C(M)A(F)U(M)C(F)C(M)A(F)G(M)A(F)G(M)A(F)A(M)^U(F)^A(M) |
| CH0774 | No. 5830 | U(F)^C(M)^U(F)C(M)U(F)G(M)G(F)A(M)U(F)G(M)G(F) C(F)C(M)C(F)G(M)G(F)A(M)A(F)G(M)A(F)A(M) | No. 6532 | U(F)^U(M)^C(F)U(M)U(F)C(M)C(F)G(M)G(F)G(M)C(M)C(F)A(M)U(F)C(M)C(F)A(M)G(F)A(M)G(F)A(M)^A(F)^U(M) |
| CH0775 | No. 5831 | C(F)^U(M)^C(F)U(M)G(F)G(M)A(F)U(M)G(F)G(M)C(F) C(F)C(M)G(F)G(M)A(F)A(M)G(F)A(M)A(F)A(M) | No. 6533 | U(F)^U(M)^U(F)C(M)U(F)U(M)C(F)C(M)G(F)G(M)G(M)C(F)C(M)A(F)U(M)C(F)C(M)A(F)G(M)A(F)G(M)^A(F)^A(M) |
| CH0776 | No. 5832 | U(F)^C(M)^U(F)G(M)G(F)A(M)U(F)G(M)G(F)C(M)C(F) C(F)G(M)G(F)A(M)A(F)G(M)A(F)A(M)A(F)U(M) | No. 6534 | A(F)^U(M)^U(F)U(M)C(F)U(M)U(F)C(M)C(F)G(M)G(M)G(F)C(M)C(F)A(M)U(F)C(M)C(F)A(M)G(F)A(M)^G(F)^A(M) |
| CH0777 | No. 5833 | C(F)^U(M)^G(F)G(M)A(F)U(M)G(F)G(M)C(F)C(M)C(F) G(F)G(M)A(F)A(M)G(F)A(M)A(F)A(M)U(F)A(M) | No. 6535 | U(F)^A(M)^U(F)U(M)U(F)C(M)U(F)C(M)C(F)C(M)G(M)G(F)G(M)C(F)C(M)A(F)U(M)C(F)C(M)A(F)G(M)^A(F)^G(M) |
| CH0779 | No. 5834 | G(F)^G(M)^A(F)U(M)G(F)G(M)C(F)C(M)C(F)G(M)G(F) A(F)A(M)G(F)A(M)A(F)A(M)U(F)A(M)G(F)A(M) | No. 6536 | U(F)^C(M)^U(F)A(M)U(F)U(M)U(F)C(M)U(F)U(M)C(M)C(F)G(M)G(F)G(M)C(F)C(M)A(F)U(M)C(F)C(M)^A(F)^G(M) |
| CH0180 | No. 5835 | G(F)^A(M)^U(F)G(M)G(F)C(M)C(F)C(M)G(F)G(M)A(F) A(F)G(M)A(F)A(M)A(F)U(M)A(F)G(M)A(F)A(M) | No. 6537 | U(F)^U(M)^C(F)U(M)A(F)U(M)U(F)U(M)C(F)U(M)U(M)C(F)G(M)G(F)G(M)C(F)C(M)A(F)U(M)C(F)C(M)^C(F)^A(M) |
| CH0781 | No. 5836 | A(F)^U(M)^G(F)G(M)C(F)C(M)C(F)G(M)G(F)A(M)A(F) G(F)A(M)A(F)A(M)U(F)A(M)G(F)A(M)A(F)U(M) | No. 6538 | A(F)^U(M)^U(F)C(M)U(F)A(M)U(F)U(M)U(F)C(M)U(M)U(F)C(M)C(F)G(M)G(F)G(M)C(F)C(M)A(F)U(M)^C(F)^C(M) |
| CH0782 | No. 5837 | U(F)^G(M)^G(F)C(M)C(F)C(M)G(F)G(M)A(F)A(M)G(F) A(F)A(M)A(F)U(M)A(F)G(M)A(F)A(M)U(F)G(M) | No. 6539 | C(F)^A(M)^U(F)U(M)C(F)U(M)A(F)U(M)U(F)U(M)C(M)U(F)U(M)C(F)C(M)G(F)G(M)G(F)C(M)C(F)A(M)^U(F)^C(M) |
| CH0783 | No. 5838 | G(F)^G(M)^C(F)C(M)C(F)G(M)G(F)A(M)A(F)G(M)A(F) A(F)A(M)U(F)A(M)G(F)A(M)A(F)U(M)G(F)U(M) | No. 6540 | A(F)^C(M)^A(F)U(M)U(F)C(M)U(F)A(M)U(F)U(M)U(M)C(F)U(M)U(F)C(M)C(F)G(M)G(F)G(M)C(F)C(M)^A(F)^U(M) |
| CH0785 | No. 5839 | C(F)^C(M)^C(F)G(M)G(F)A(M)A(F)G(M)A(F)A(M)A(F) U(F)A(M)G(F)A(M)A(F)U(M)G(F)U(M)A(F)C(M) | No. 6541 | G(F)^U(M)^A(F)C(M)A(F)U(M)U(F)C(M)U(F)A(M)U(M)U(F)U(M)C(F)U(M)U(F)C(M)C(F)G(M)G(F)G(M)^C(F)^C(M) |
| CH0786 | No. 5840 | C(F)^C(M)^G(F)G(M)A(F)A(M)G(F)A(M)A(F)A(M)U(F) A(F)G(M)A(F)A(M)U(F)G(M)U(F)A(M)C(F)C(M) | No. 6542 | G(F)^G(M)^U(F)A(M)C(F)A(M)U(F)U(M)C(F)U(M)A(M)U(F)U(M)U(F)C(M)U(F)U(M)C(F)C(M)G(F)G(M)^G(F)^C(M) |
| CH0787 | No. 5841 | C(F)^G(M)^G(F)A(M)A(F)G(M)A(F)A(M)A(F)U(M)A(F) G(F)A(M)A(F)U(M)G(F)U(M)A(F)C(M)C(F)A(M) | No. 6543 | U(F)^G(M)G(F)U(M)A(F)C(M)A(F)U(M)U(F)C(M)U(M)A(F)U(M)U(F)U(M)C(F)U(M)U(F)C(M)C(F)G(M)^G(F)^G(M) |
| CH0790 | No. 5842 | A(F)^A(M)^G(F)A(M)A(F)A(M)U(F)A(M)G(F)A(M)A(F)U (F)G(M)U(F)A(M)C(F)C(M)A(F)A(M)A(F)C(M) | No. 6544 | G(F)^U(M)^U(F)U(M)G(F)G(M)U(F)A(M)C(F)A(M)U(M)U(F)C(M)U(F)A(M)U(F)U(M)U(F)C(M)U(F)U(M)^C(F)^C(M) |
| CH0795 | No. 5843 | A(F)^U(M)^A(F)G(M)A(F)A(M)U(F)G(M)U(F)A(M)C(F) C(F)A(M)A(F)A(M)C(F)U(M)G(F)G(M)G(F)A(M) | No. 6545 | U(F)^C(M)^C(F)C(M)A(F)G(M)U(F)U(M)U(F)G(M)G(M)U(F)A(M)C(F)A(M)U(F)U(M)C(F)U(M)A(F)U(M)^U(F)^U(M) |
| CH0796 | No. 5844 | U(F)^A(M)^G(F)A(M)A(F)U(M)G(F)U(M)A(F)C(M)C(F) A(F)A(M)A(F)C(M)U(F)G(M)G(F)G(M)A(F)A(M) | No. 6546 | U(F)^U(M)^C(F)C(M)C(F)A(M)G(F)U(M)U(F)U(M)G(M)G(F)U(M)A(F)C(M)A(F)U(M)U(F)C(M)U(F)A(M)^U(F)^U(M) |

### [Table 32]

**Table 3-12**

| Double stranded nucleic acid No. | SEQ ID NO | Sense strand sequence (5'→3') | SEQ ID NO | Antisense strand sequence (5'→3') |
|---|---|---|---|---|
| CH0797 | No. 5845 | A(F)^G(M)^A(F)A(M)U(F)G(M)U(F)A(M)C(F)C(M)A(F) A(F)A(M)C(F)U(M)G(F)G(M)G(F)A(M)A(F)A(M) | No. 6547 | U(F)^U(M)^U(F)C(M)C(F)C(M)A(F)G(MU(M)U(M)G(F)G (M)U(F)A(M)C(F)A(M)U(F)U(M)C(F)U(M))^A(F)^U(M) |
| CH0800 | No. 5846 | A(F)^U(M)^G(F)U(M)A(F)C(M)C(F)A(M)A(F)A(M)C(F) U(F)G(M)G(F)G(M)A(F)A(M)A(F)C(M)U(F)G(M) | No. 6548 | C(F)^A(M)^G(F)U(M)U(F)U(M)C(F)C(M)C(F)A(M)G(M)U(F)U (M)U(F)G(M)G(F)U(M)A(F)C(M)A(F)U(M)^U(F)^C(M) |
| CH0804 | No. 5847 | A(F)^C(M)^C(F)A(M)A(F)A(M)C(F)U(M)G(F)G(M)G(F) A(F)A(M)A(F)C(M)U(F)G(M)G(F)U(M)C(F)U(M) | No. 6549 | A(F)^G(M)^A(F)C(M)C(F)A(M)G(F)U(M)U(F)U(M)C(M)C(F)C (M)A(F)G(M)U(F)U(M)U(F)G(M)G(F)U(M)^A(F)^C(M) |
| CH0805 | No. 5848 | C(F)^C(M)^A(F)A(M)A(F)C(M)U(F)G(M)G(F)G(M)A(F) A(F)A(M)C(F)U(M)G(F)G(M)U(F)C(M)U(F)G(M) | No. 6550 | C(F)^A(M)^G(F)A(M)C(F)C(M)A(F)G(M)U(F)U(M)U(M)C(F)C (M)C(F)A(M)G(F)U(M)U(F)U(M)G(F)G(M)^U(F)^A(M) |
| CH0808 | No. 5849 | A(F)^A(M)^C(F)U(M)G(F)G(M)G(F)A(M)A(F)A(M)C(F) U(F)G(M)G(F)U(M)C(F)U(M)G(F)C(M)C(F)A(M) | No. 6551 | U(F)^G(M)^G(F)C(M)A(F)G(M)A(F)C(M)C(F)A(M)G(M)U(F)U (M)U(F)C(M)C(F)C(M)A(F)G(M)U(F)U(M)^U(F)^G(M) |
| CH0809 | No. 5850 | A(F)^C(M)^U(F)G(M)G(F)G(M)A(F)A(M)A(F)C(M)U(F) G(F)G(M)U(F)C(M)U(F)G(M)C(F)C(M)A(F)U(M) | No. 6552 | A(F)^U(M)^G(F)G(M)C(F)A(M)G(F)A(M)C(F)C(M)A(M)G(F)U (M)U(F)U(M)C(F)C(M)C(F)A(M)G(F)U(M)^U(F)^U(M) |
| CH0810 | No. 5851 | C(F)^U(M)^G(F)G(M)G(F)A(M)A(F)A(M)C(F)U(M)G(F) G(F)U(M)C(F)U(M)G(F)C(M)C(F)A(M)U(F)G(M) | No. 6553 | C(F)^A(M)^U(F)G(M)G(F)C(M)A(F)G(M)A(F)C(M)C(M)A(F)G (M)U(F)U(M)U(F)C(M)C(F)C(M)A(F)G(M)^U(F)^U(M) |
| CH0811 | No. 5852 | U(F)^G(M)^G(F)G(M)A(F)A(M)A(F)C(M)U(F)G(M)G(F) U(F)C(M)U(F)G(M)C(F)C(M)A(F)U(M)G(F)C(M) | No. 6554 | G(F)^C(M)^A(F)U(M)G(F)G(M)C(F)A(M)G(F)A(M)C(M)C(F)A (M)G(F)U(M)U(F)U(M)C(F)C(M)C(F)A(M)^G(F)^U(M) |
| CH0813 | No. 5853 | G(F)^G(M)^A(F)A(M)A(F)C(M)U(F)G(M)G(F)U(M)C(F) U(F)G(M)C(F)C(M)A(F)U(M)G(F)C(M)C(F)A(M) | No. 6555 | U(F)^G(M)^G(F)C(M)A(F)U(M)G(F)G(M)C(F)A(M)G(M)A(F)C (M)C(F)A(M)G(F)U(M)U(F)U(F)C(F)C(M)^C(F)^A(M) |
| CH0814 | No. 5854 | G(F)^A(M)^A(F)A(M)C(F)U(M)G(F)G(M)U(F)C(M)U(F) G(F)C(M)C(F)A(M)U(F)G(M)C(F)C(M)A(F)A(M) | No. 6556 | U(F)^U(M)^G(F)G(M)C(F)A(M)U(F)G(M)G(F)C(M)A(M)G(F)A (M)C(F)C(M)A(F)G(M)U(F)U(M)U(F)C(M)^C(F)^C(M) |
| CH0815 | No. 5855 | A(F)^A(M)^A(F)C(M)U(F)G(M)G(F)U(M)C(F)U(M)G(F) C(F)C(M)A(F)U(M)G(F)C(M)C(F)A(M)A(F)G(M) | No. 6557 | C(F)^U(M)^U(F)G(M)G(F)C(M)A(F)U(M)G(F)G(M)C(M)A(F)G (M)A(F)C(M)C(F)A(M)G(F)U(M)U(F)U(M)^C(F)^C(M) |
| CH0816 | No. 5856 | A(F)^A(M)^C(F)U(M)G(F)G(M)U(F)C(M)U(F)G(M)C(F) C(F)A(M)U(F)G(M)C(F)C(M)A(F)A(M)G(F)U(M) | No. 6558 | A(F)^C(M)^U(F)U(M)G(F)G(M)C(F)A(M)U(F)G(M)G(M)C(F)A (M)G(F)A(M)C(F)C(M)A(F)G(M)U(F)U(M)^U(F)^C(M) |
| CH0817 | No. 5857 | A(F) ^C(M)^U(F)G(M)G(F)U(M)C(F)U(M)G(F)C(M)C(F) A(F)U(M)G(F)C(M)C(F)A(M)A(F)G(M)U(F)U(M) | No. 6559 | A(F)^A(M)^C(F)U(M)U(F)G(M)G(F)C(M)A(F)U(M)G(M)G(F)C (M)A(F)G(M)A(F)C(M)C(F)A(M)G(F)U(M)^U(F)^U(M) |
| CH0818 | No. 5858 | C(F)^U(M)^G(F)G(M)U(F)C(M)U(F)G(M)C(F)C(M)A(F) U(F)G(M)C(F)C(M)A(F)A(M)G(F)U(M)U(F)G(M) | No. 6560 | C(F)^A(M)^A(F)C(M)U(F)U(M)G(F)G(M)C(F)A(M)U(M)G(F)G (M)C(F)A(M)G(F)A(M)C(F)C(M)A(F)G(M)^U(F)^U(M) |
| CH0819 | No. 5859 | U(F)^G(M)^G(F)U(M)C(F)U(M)G (F)C(M)C(F)A(M)U(F) G(F)C(M)C(F)A(M)A(F)G(M)U(F)U(M)G(F)U(M) | No. 6561 | A(F)^C(M)^A(F)A(M)C(F)U(M)U(F)G(M)G(F)C(M)A(M)U(F)G (M)G(F)C(M)A(F)G(M)A(F)C(M)C(F)A(M)^G(F)^U(M) |
| CH0820 | No. 5860 | G(F)^G(M)^U(F)C(M)U(F)G(M)C(F)C(M)A(F)U(M)G(F) C(F)C(M)A(F)A(M)G(F)U(M)U(F)G(M)U(F)A(M) | No. 6562 | U(F)^A(M)^C(F)A(M)A(F)C(M)U(F)U(M)G(F)G(M)C(M)A(F)U (M)G(F)G(M)C(F)A(M)G(F)A(M)C(F)C(M)^A(F)^G(M) |
| CH0821 | No. 5861 | G(F)^U(M)^C(F)U(M)G(F)C(M)C(F)A(M)U(F)G(M)C(F) C(F)A(M)A(F)G(M)U(F)U(M)G(F)U(M)A(F)A(M) | No. 6563 | U(F)^U(M)^A(F)C(M)A(F)A(M)C(F)U(M)U(F)G(M)G(M)C(F)A (M)U(F)G(M)G(F)C(M)A(F)G(MA(F)C(M)^C(F)^A(M) |
| CH0822 | No. 5862 | U(F)^C(M)^U(F)G(M)C(F)C(M)A(F)U(M)G(F)C(M)C(F) A(F)A(M)G(F)U(M)U(F)G(M)U(F)A(M)A(F)A(M) | No. 6564 | U(F)^U(M)^U(F)A(M)C(F)A(M)C(F)A(M)A(F)C(M)U(F)U(M)G(M)G(F)C (M)A(F)U(M)G(F)G(M)C(F)A(M)G(F)A(M)^C(F)^C(M) |
| CH0823 | No. 5863 | C(F)^U(M)^G(F)C(M)A(M)U(F)G(M)C(F)C(M)A(F) A(F)G(M)U(F)U(M)G(F)U(M)A(F)A(M)A(F)G(M) | No. 6565 | C(F)^U(M)^U(F)U(M)A(F)C(M)A(F)A(M)C(F)U(M)U(M)G(F)G (M)C(F)A(M)U(F)G(M)G(F)C(M)A(F)G(M)^A(F)^C(M) |
| CH0825 | No. 5864 | G(F)^C(M)^C(F)A(M)U(F)G(M)C(F)C(M)A(F)A(M)G(F) U(F)U(M)G(F)U(M)A(F)A(M)A(F)G(M)C(F)A(M) | No. 6566 | U(F)^G(M)^C(F)U(M)U(F)U(M)A(F)C(M)A(F)A(M)C(M)U(F) (M)G(F)G(M)C(F)A(M)U(F)G(M)G(F)C(M)^A(F)^G(M) |
| CH0826 | No. 5865 | C(F)^C(M)^A(F)U(M)G(F)C(M)C(F)A(M)A(F)G(M)U(F) UF)G(M)U(F)A(M)A(F)A(M)G(F)C(M)A(F)U(M) | No. 6567 | A(F)^U(M)^G(F)C(M)U(F)U(M)U(F)A(M)C(F)A(M)A(M)C(F)U M)U(F)G(M)G(F)C(M)A(F)U(M)G(F)G(M)^C(F)^A(M) |
| CH0827 | No. 5866 | C(F)^A(M)^U(F)G(M)C(F)C(M)A(F)A(M)G(F)U(M)U(F) G(F)U(M)A(F)A(M)A(F)G(M)C(F)A(M)U(F)C(M) | No. 6568 | G(F)^A(M)^U(F)G(M)C(F)U(M)U(F)U(M)A(F)C(M)A(M)A(F)C (M)U(F)U(M)G(F)G(M)C(F)A(M)U(F)G(M)^G(F)^C(M) |
| CH0828 | No. 5867 | A(F)^U(M)^G(F)C(M)C(F)A(M)A(F)G(M)U(F)U(M)G(F) U(F)A(M)A(F)A(M)G(F)C(M)A(F)U(M)C(F)U(M) | No. 6569 | A(F)^G(M)^A(F)U(M)G(F)C(M)U(F)U(M)U(F)A(M)C(M)A(F)A (M)C(F)U(M)U(F)G(M)G(F)C(M)A(F)U(M)^G(F)^G(M) |
| CH0829 | No. 5868 | U(F)^G(M)^C(F)C(M)A(F)A(M)G(F)U(M)U(F)G(M)U(F) A(F)A(M)A(F)G(M)C(F)A(M)U(F)C(M)U(F)U(M) | No. 6570 | A(F)^A(M)^G(F)A(M)U(F)G(M)C(F)U(M)U(F)U(M)A(M)C(F)A (M)A(F)C(M)U(F)U(M)G(F)G(M)C(F)A(M)^U(F)^G(M) |
| CH0830 | No. 5869 | G(F)^C(M)^C(F)A(M)A(F)G(M)U(F)U(M)G(F)U(M)A(F) A(F)A(M)G(F)C(M)A(F)U(M)C(F)U(M)U(F)G(M) | No. 6571 | C(F)^A(M)^A(F)G(M)A(F)U(M)G(F)C(M)U(F)U(M)U(M)A(F)C (M)A(F)A(M)C(F)U(M)U(F)G(M)G(F)C(M)^A(F)^U(M) |
| CH0831 | No. 5870 | C(F)^C(M)^A(F)A(M)G(F)U(M)U(F)G(M)U(F)A(M)A(F) A(F)G(M)C(F)A(M)U(F)C(M)U(F)U(M)G(F)U(M) | No. 6572 | A(F)^C(M)^A(F)A(M)G(F)A(M)U(F)G(M)C(F)U(M)U(M)U(F)A (M)C(F)A(M)A(F)C(M)U(F)U(M)G(F)G(M)^C(F)^A(M) |
| CH0832 | No. 5871 | C(F)^A(M)^A(F)G(M)U(F)U(M)G(F)U(M)A(F)A(M)A(F) G(F)C(M)A(F)U(M)C(F)U(M)U(F)G(M)U(F)A(M) | No. 6573 | U(F)^A(M)^C(F)A(M)A(F)G(M)A(F)U(M)G(F)C(M)U(M)U(F)U M)A(F)C(M)A(F)A(M)C(F)U(M)U(F)G(M)^G(F)^C(M) |
| CH0833 | No. 5872 | A(F)^A(M)^G(F)U(M)U(F)G(M)U(F)A(M)A(F)A(M)G(F) C(F)A(M)U(F)C(M)U(F)U(M)G(F)U(M)A(F)A(M) | No. 6574 | U(F)^U(M)^A(F)C(M)A(F)A(M)G(F)A(M)U(F)G(M)C(M)U(F)U (M)U(F)A(M)C(F)A(M)A(F)C(M)U(F)U(M)^G(F)^G(M) |
| CH0834 | No. 5873 | A(F)^G(M)^U(F)U(M)G(F)U(M)A(F)G(M)C(F) A(F)U(M)C(F)U(M)U(F)G(M)U(F)A(M)A(F)A(M) | No. 6575 | U(F)^U(M)^U(F)A(M)C(F)A(M)A(F)G(M)A(F)U(M)G(M)C(F)U (M)U(F)U(M)A(F)C(M)A(F)A(M)C(F)U(M)^U(F)^G(M) |
| CH0835 | No. 5874 | G(F)^U(M)^ U(F)G(M)U(F)A(M)A(F)A(M)G(F)C(M)A(F) U(F)C(M)U(F)U(M)G(F)U(M)A(F)A(M)A(F)G(M) | No. 6576 | C(F)^U(M)^U(F)U(M)A(F)C(M)A(F)A(M)G(F)A(M)U(M)G(F)C (M)U(F)U(M)U(F)A(M)C(F)A(M)A(F)C(M)^U(F)^U(M) |
| CH0837 | No. 5875 | U(F)^G(M)^U(F)A(M)A(F)A(M)G(F)C(M)A(F)U(M)C(F) U(F)U(M)G(F)U(M)A(F)A(M)A(F)G(M)U(F)A(M) | No. 6577 | U(F)^A(M)^C(F)U(M)U(F)U(M)A(F)C(M)A(F)A(M)G(M)A(F)U (M)G(F)C(M)U(F)U(M)U(F)A(M)C(F)A(M)^A(F)^C(M) |
| CH0838 | No. 5876 | G(F)^U(M)^A(F)A(M)A(F)G(M)C(F)A(M)U(F)C(M)U(F) U(F)G(M)U(F)A(M)A(F)A(M)G(F)U(M)A(F)C(M) | No. 6578 | G(F)^U(M)^ A(F)C(M)U(F)U(M)U(F)A(M)C(F)A(M)A(M)G(F)A (M)U(F)G(M)C(F)U(M)U(F)U(M)A(F)C(M)^ A(F)^A(M) |
| CH0840 | No. 5877 | A(F)^A(M)^A(F)G(M)C(F)A(M)U(F)C(M)U(F)U(M)G(F) U(F)A(M)A(F)A(M)G(F)U(M)A(F)C(M)C(F)U(M) | No. 6579 | A(F)^G(M)^G(F)U(M)A(F)C(M)U(F)U(M)U(F)A(M)C(M)A(F)A (M)G(F)A(M)U(F)G(M)C(F)U(M)U(F)U(M)^A(F)C(M) |
| CH0842 | No. 5878 | A(F)^G(M)^C(F)A(M)U(F)C(M)U(F)U(M)G(F)U(M)A(F) A(F)A(M)G(F)U(M)A(F)C(M)C(F)U(M)G(F)U(M) | No. 6580 | A(F)^C(M)^A(F)G(M)G(F)U(M)A(F)C(M)U(F)U(M)U(M)A(F)C (M)A(F)A(M)G(F)A(M)U(F)G(M)C(F)U(M)^U(F)^U(M) |
| CH0845 | No. 5879 | A(F)^U(M)^C(F)U(M)U(F)G(M)U(F)A(M)A(F)A(M)G(F) U(F)A(M)C(F)C(M)U(F)G(M)U(F)G(M)A(F)A(M) | No. 6581 | U(F)^U(M)^C(F)A(M)C(F)A(M)G(F)G(M)U(F)A(M)C(M)U(F)U (M)U(F)A(M)C(F)A(M)A(F)G(M)A(F)U(M)^G(F)^C(M) |
| CH0846 | No. 5880 | U(F)^C(M)^U(F)U(M)G(F)U(M)A(F)A(M)A(F)G(M)U(F) A(F)C(M)C(F)U(M)G(F)U(M)G(F)A(M)A(F)A(M) | No. 6582 | U(F)^U(M)^U(F)C(M)A(F)C(M)A(F)G(M)G(F)U(M)A(M)C(F)U (M)U(F)U(M)A(F)C(M)A(F)A(M)G(F)A(M) ^U(F)^G(M) |
| CH0847 | No. 5881 | C(F)^U(M)^U(F)G(M)U(F)A(M)A(F)A(M)G(F)U(M)A(F) C(F)C(M)U(F)G(M)U(F)G(M)A(F)A(M)A(F)A(M) | No. 6583 | U(F)^U(M)^U(F)U(M)C(F)A(M)C(F)A(M)G(F)G(M)U(M)A(F)C (M)U(F)U (M)U(F)A(M)C(F)A(M)A(F)G(M)^A(F)^U(M) |
| CH0852 | No. 5882 | A(F)^A(M)^A(F)G(M)U(F)A(M)C(F)C(M)U(F)G(M)U(F) G(F)A(M)A(F)A(M)A(F)A(M)A(F)G(M)C(F)C(M) | No. 6584 | G(F)^G(M)^C(F)U(M)U(F)U(M)U(F)U(M)U(F)C(M)A(M)C(F)A (M)G(F)G(M)U(F)A(M)C(F)U(M)U(F)U(M)^A(F)^C(M) |
| CH0853 | No. 5883 | A(F)^A(M)^G(F)U(M)A(F)C(M)C(F)U(M)G(F)U(M)G(F) A(F)A(M)A(F)A(M)A(F)A(M)G(F)C(M)C(F)A(M) | No. 6585 | U(F)^G(M)^G(F)C(M)U(F)U(M)U(F)U(M)U(F)U(M)C(M)A(F)C (M)A(F)G(M)G(F)U(M)A(F)C(M)U(F)U(M)^U(F)^A(M) |
| CH0854 | No. 5884 | A(F)^G(M)^U(F)A(M)C(F)C(M)U(F)G(M)U(F)G(M)A(F) A(F)A(M)A(F)A(M)A(F)G(M)C(F)C(M)A(F)C(M) | No. 6586 | G(F)^U(M)^G(F)G(M)C(F)U(M)U(F)U(M)U(F)U(M)U(M)C(F)A (M)C(F)A(M)G(F)G(M)U(F)A(M)C(F)U(M)^U(F)^U(M) |

### [Table 33]

**Table 3-13**

| Double stranded nucleic acid No. | SEQ ID NO | Sense strand sequence (5'→3') | SEQ ID NO | Antisense strand sequence (5'→3') |
|---|---|---|---|---|
| CH0855 | No. 5895 | G(F)^U(M)^A(F)C(M)C(F)U(M)G(F)U(M)G(F)A(M)A(F) A(F)A(M)A(F)A(M)G(F)C(M)C(F)A(M)C(F)U(M) | No. 6587 | A(F)*G(M)*U(F)G(M)G(F)C(M)U(F)U(M)U(F)U(M)U(M)U(F)C (M)A(F)C(M)A(F)G(M)G(F)U(M)A(F)C(M)*U(F)*U(M) |
| CH0857 | No. 5886 | A(F)^C(M)^C(F)U(M)G(F)U(M)G(F)A(M)A(F)A(M)A(F) A(F)A(M)G(F)C(M)C(F)A(M)C(F)U(M)G(F)U(M) | No. 6588 | A(F)^C(M)^A(F)G(M)U(F)G(M)G(F)C(M)U(F)U(M)U(F)U (M)U(F)C(M)A(F)C(M)A(F)G(M)G(F)U(M)^A(F)^C(M) |
| CH0858 | No. 5887 | C(F)^C(M)^U(F)G(M)U(F)G(M)A(F)A(M)A(F)A(M)A(F) A(F)G(M)C(F)C(M)A(F)C(M)U(F)G(M)U(F)G(M) | No. 6589 | C(F)^A(M)^C(F)A(M)G(F)U(M)G(F)G(M)C(F)U(M)U(M)U(F)U (M)U(F)U(M)C(F)A(M)C(F)A(M)G(F)G(M)^U(F)^A(M) |
| CH0859 | No. 5888 | C(F)^U(M)^G(F)U(M)G(F)A(M)A(F)A(M)A(F)A(M)A(F) G(F)C(M)C(F)A(M)C(F)U(M)G(F)U(M)G(F)G(M) | No. 6590 | C(F)^C(M)^A(F)C(M)A(F)G(M)U(F)G(M)G(F)C(M)U(M)U(F)U (M)U(F)U(M)U(F)C(M)A(F)C(M)A(F)G(M)^G(F)^U(M) |
| CH0860 | No. 5889 | U(F)^G(M)^U(F)G(M)A(F)A(M)A(F)A(M)A(F)A(M)G(F) C(F)C(M)A(F)C(M)U(F)G(M)U(F)G(M)G(F)U(M) | No. 6591 | A(F)^C(M)^C(F)A(M)C(F)A(M)G(F)U(M)G(F)G(M)C(M)U(F)U (M)U(F)U(M)U(F)U(M)C(F)A(M)C(F)A(M)^G(F)^G(M) |
| CH0861 | No. 5890 | G(F)^U(M)^G(F)A(M)A(F)A(M)A(F)A(M)A(F)G(M)C(F) C(F)A(M)C(F)U(M)G(F)U(M)G(F)G(M)U(F)G(M) | No. 6592 | C(F)^A(M)C(F)C(M)A(F)C(M)A(F)G(M)U(F)G(M)G(M)C(F)U (M)U(F)U(M)U(F)U(M)U(F)C(M)A(F)C(M)^A(F)^G(M) |
| CH0863 | No. 5891 | G(F)^A(M)^A(F)A(M)A(F)A(M)A(F)G(M)C(F)C(M)A(F) C(F)U(M)G(F)U(M)G(F)G(M)U(F)G(M)U(F)A(M) | No. 6593 | U(F)^A(M)^C(F)A(M)C(F)C(M)A(F)C(M)A(F)G(M)U(M)G(F)G (M)C(F)U(M)U(F)U(M)U(F)U(M)U(F)C(M)^A(F)^C(M) |
| CH0864 | No. 5892 | A(F)^A(M)^A(F)A(M)A(F)A(M)G(F)C(M)C(F)A(M)C(F) U(F)G(M)U(F)G(M)G(F)U(M)G(F)U(M)A(F)C(M) | No. 6594 | G(F)^U(M)^A(F)C(M)A(F)C(M)C(F)A(M)C(F)A(M)G(M)U(F)G (M)G(F)C(M)U(F)U(M)U(F)U(M)U(F)U(M)^C(F)^A(M) |
| CH0866 | No. 5893 | A(F)^A(M)^A(F)A(M)G(F)C(M)C(F)A(M)C(F)U(M)G(F) U(F)G(M)G(F)U(M)G(F)U(M)A(F)C(M)C(F)A(M) | No. 6595 | U(F)^G(M)^G(F)U(M)A(F)C(M)A(F)C(M)C(F)A(M)C(M)A(F)G (M)U(F)G(M)G(F)C(M)U(F)U(M)U(F)U(M)^U(F)^U(M) |
| CH0867 | No. 5894 | A(F)^A(M)^A(F)G(M)C(F)C(M)A(F)C(M)U(F)G(M)U(F) G(F)G(M)U(F)G(M)U(F)A(M)C(F)C(M)A(F)A(M) | No. 6596 | U(F)^U(M)^G(F)G (M)U(F)A(M)C(F)A(M)C(F)C(M)A(M)C(F)A (M)G(F)U(M)G(F)G(M)C(F)U(M)U(F)U(M)^U(F)^U(M) |
| CH0868 | No. 5895 | A(F)^A(M)^G(F)C(M)C(F)A(M)C(F)U(M)G(F)U(M)G(F) G(F)U(M)G(F)U(M)A(F)C(M)C(F)A(M)A(F)G(M) | No. 6597 | C(F)^U(M)^U(F)G(M)G(F)U(M)A(F)C(M)A(F)C(M)C(M)A(F)C (M)A(F)G(M)U(F)G(M)G(F)C(M)U(F)U(M)^U(F)^U(M) |
| CH0870 | No. 5896 | G(F)^C(M)^C(F)A(M)C(F)U(M)G(F)U(M)G(F)G(M)U(F) G(F)U(M)A(F)C(M)G(F)A(M)A(F)G(M)G(F)A(M) | No. 6598 | U(F)^C(M)^C(F)U(M)U(F)G(M)G(F)U(M)A(F)C(M)A(M)C(F)C (M)A(F)C(M)A(F)G(M)U(F)G(M)G(F)C(M)^U(F)^U(M) |
| CH0872 | No. 5897 | C(F)^A(M)^C(F)U(M)G(F)U(M)G(F)G(M)U(F)G(M)U(F) A(F)C(M)C(F)A(M)A(F)G(M)G(F)A(M)G(F)A(M) | No. 6599 | U(F)^C(M)^U(F)C(M)C(F)U(M)U(F)G(M)G(F)U(M)A(M)C(F)A (M)C(F)C(M)A(F)C(M)A(F)G(M)U(F)G(M)^G(F)^C(M) |
| CH0873 | No. 5898 | A(F)^C(M)^U(F)G(M)U(F)G(M)G(F)U(M)G(F)U(M)A(F) C(F)C(M)A(F)A(M)G(F)G(M)A(F)G(M)A(F)G(M) | No. 6600 | C(F)^U(M)^C(F)U(M)C(F)C(M)U(F)U(M)G(F)G(M)U(M)A(F)C (M)A(F)C(M)C(F)A(M)C(F)A(M)G(F)U(M)^G(F)^G(M) |
| CH0874 | No. 5899 | C(F)^U(M)^G(F)U(M)G(F)G(M)U(F)G(M)U(F)A(M)C(F) C(F)A(M)A(F)G(M)G(F)A(M)G(F)A(M)G(F)A(M) | No. 6601 | U(F)^C(M)^U(F)C(M)U(F)C(M)C(F)U(M)U(F)G(M)G(M)U(F)A (M)C(F)A(M)C(F)C(M)A(F)C(M)A(F)G(M)^U(F)^G(M) |
| CH0875 | No. 5900 | U(F)^G(M)^U(F)G(M)G(F)U(M)G(F)U(M)A(F)C(M)C(F) A(F)A(M)G(F)G(M)A(F)G(M) A(F)G(M)A(F)G(M) | No. 6602 | C(F)^U(M)^C(F)U(M)C(F)U(M)C(F)C(M)U(F)U(M)G(M)G(F)U (M)A(F)C(M)A(F)C(M)C(F)A(M)C(F)A(M)^G(F)^U(M) |
| CH0876 | No. 5901 | G(F)^U(M)^G(F)G(M)U(F)G(M)U(F)A(M)C(F)C(M)A(F) A(F)G(M)G(F)A(M)G(F)A(M)G(F)A(M)G(F)A(M) | No. 6603 | U(F)^C(M)^U(F)C(M)U(F)C(M)U(F)C(M)C(F)U(M)U(M)G(F)G (M)U(F)A(M)C(F)A(M)C(F)C(M)A(F)C(M) ^A(F)^G(M) |
| CH0877 | No. 5902 | U(F)^G(M)^G(F)U(M)G(F)U(M)A(F)C(M)C(F)A(M)A(F) G(F)G(M)A(F)G(M)A(F)G(M)A(F)G(M)A(F)G(M) | No. 6604 | C(F)^U(M)^C(F)U(M)C(F)U(M)C(F)U(M)C(F)C(M)U(m)U(F)G (M)G(F)U(M)A(F)C(M)A(F)C(M)C(F)A(M)^C(F)^A(M) |
| CH0879 | No. 5903 | G(F)^U(M)^G(F)U(M)A(F)C(M)C(F)A(M)A(F)G(M)G(F) A(F)G(M)A(F)G(M)A(F)G(M)A(F)G(M)U(F)A(M) | No. 6605 | U(F)^A(M)^C(F)U(M)C(F)U(M)C(F)U(M)C(F)U(M)C(M)C(F)U (M)U(F)G(M)G(F)U(M)A(F)C(M)A(F)C(M)^C(F)^A(M) |
| CH0880 | No. 5904 | U(F)^G(M)^U(F)A(M)C(F)C(M)A(F)A(M)G(F)G(M)A(F) G(F)A(M)G(F)A(M)G(F)A(M)G(F)U(M)A(F)A(M) | No. 6606 | U(F)^U(M)^A(F)C(M)U(F)C(M)U(F)C(M)U(F)C(M)U(M)C(F)C (M)U(F)U(M)G(F)G(M)U(F)A(M)C(F)A(M)^C(F)^C(M) |
| CH0881 | No. 5905 | G(F)^U(M)^A(F)C(M)C(F)A(M)A(F)G(M)G(F)A(M)G(F) A(F)G(M)A(F)G(M)A(F)G(M)U(F)A(M)A(F)A(M) | No. 6607 | U(F)^U(M)^U(F)A(M)C(F)U(M)C(F)U(M)C(M)U(F)C (M)C(F)U(M)U(F)G(M)G(F)U(M)A(F)C(M)^ A(F)^C(M) |
| CH0882 | No. 5906 | UF)^A(M)^C(F)C(M)A(F)A(M)G(F)G(M)A(F)G(M)A(F) G(F)A(M)G(F)A(M)G(F)U(M)A(F)A(M)A(F)G(M) | No. 6608 | C(F)^U(M)^U(F)U(M)A(F)C(M)U(F)C(M)U(F)C(M)U(M)C(F)U (M)C(F)C(M)U(F)U(M)G(F)G(M)U(F)A(M)^C(F)^A(M) |
| CH0883 | No. 5907 | A(F)^C(M)^C(F)A(M)A(F)G(M)G(F)A(M)G(F)A(M)G(F) A(F)G(M)A(F)G(M)U(F)A(M)A(F)A(M)G(F)A(M) | No. 6609 | U(F)^C(M)^U(F)U(M)U(F)A(M)C(F)U(M)C(F)U(M)C(M)U(F)C (M)U(F)C(M)C(F)U(M)U(F)G(M)G(F)U(M)^A(F)C(M) |
| CH0884 | No. 5908 | C(F)^C(M)^A(F)A(M)G(F)G(M)A(F)G(M)A(F)G(M)A(F) G(F)A(M)G(F)U(M)A(F)A(M)A(F)G(M)A(F)U(M) | No. 6610 | A(F)^U(M)^C(F)U(M)U(F)U(M)A(F)C(M)U(F)C(M)U(M)C(F)U (M)C(F)U(M)C(F)C(M)U(F)U(M)G(F)G(M)^U(F)^A(M) |
| CH0885 | No. 5909 | C(F)^A(M)^A(F)G(M)G(F)A(M)G(F)A(M)G(F)A(M)G(F) A(F)G(M)U(F)A(M)A(F)A(M)G(F)A(M)U(F)U(M) | No. 6611 | A(F)^A(M)^U(F)C(M)U(F)U(M)U(F)A(M)C(F)C(F)U(M)C(M)U(F)C (M)U(F)C(M)U(F)C(M)C(F)U(M)U(F)G(M)^G(F)^U(M) |
| CH0886 | No. 5910 | A(F)^A(M)^G(F)G(M)A(F)G(M)A(F)G(M)A(F)G(M)A(F) G(F)U(M)A(F)A(M)A(F)G(M)A(F)U(M)U(F)C(M) | No. 6612 | G(F)^A(M)^A(F)U(M)C(F)U(M)U(F)U(M)A(F)C(M)U(M)C(F)U (M)C(F)U(M)C(F)U(M)C(F)C(M)U(F)U(M)^G(F)^G(M) |
| CH0887 | No. 5911 | A(F)^G(M)^G(F)A(M)G(F)A(M)G(F)A(M)G(F)A(M)G(F) U(F)A(M)A(F)A(M)G(F)A(M)U(F)U(M)C(F)A(M) | No. 6613 | U(F)^G(M)^A(F)A(M)U(F)C(M)U(F)U(M)A(M)C(M)U(F)C (M)U(F)C(M)U(F)C(M)U(F)C(M)C(F)U(M)^U(F)^G(M) |
| CH0888 | No. 5912 | G(F)^G(M)^A(F)G(M)A(F)G(M)A(F)G(M)A(F)G(M)U(F) A(F)A(M)A(F)G(M)A(F)U(M)U(F)C(M)A(F)G(M) | No. 6614 | C(F)^U(M)^G(F)A(M)A(F)U(M)C(F)U(M)U(F)U(M)A(M)C(F)U (M)C(F)U(M)C(F)U(M)C(F)U(M)C(F)C(M)^U(F)U(M) |
| CH0890 | No. 5913 | A(F)^G(M)^A(F)G(M)A(F)G(M)A(F)G(M)U(F)A(M)A(F) A(F)G(M)A(F)U(M)U(F)C(M)A(F)G(M)G(F)A(M) | No. 6615 | U(F)^C(M)^G(F)U(M)G(F)A(M)A(F)U(M)C(F)U(M)U(M)U(F)A (M)C(F)U(M)C(F)U(M)C(F)U(M)C(F)U(M)^C(F)^C(M) |
| CH0891 | No. 5914 | G(F)^A(M)A(M)G(F)A(M)G(F)U(M)A(F)A(M)A(F) G(F)A(M)U(F)U(M)C(F)A(M)G(F)G(M)A(F)A(M) | No. 6616 | U(F)^U(M)^C(F)C(M)U(F)G(M)A(F)A(M)U(F)C(M)U(M)U(F)U (M)A(F)C(M)U(F)C(M)U(F)C(M)U(F)C(M)^U(F)^C(M) |
| CH0892 | No. 5915 | A(F)^G(M)^A(F)G(M)A(F)G(M)U(F)A(M)A(F)A(M)G(F) A(F)U(M)U(F)C(M)A(F)G(M)G(F)A(M)A(F)A(M) | No. 6617 | U(F)^U(M)^U(F)C(M)C(F)U(M)G(F)A(M)A(F)U(M)C(M)U(F)U (M)U(F)A(M)C(F)U(M)C(F)U(M)C(F)U(M)^C(F)^U(M) |
| CH0893 | No. 5916 | G(F)^A(M)^G(F)A(M)G(F)U(M)A(F)A(M)A(F)G(M)A(F) U(F)U(M)C(F)A(M)G(F)G(M)A(F)A(M)A(F)A(M) | No. 6618 | U(F)^U(M)^U(F)U(M)C(F)C(M)U(F)G(M)A(F)A(M)U(M)C(F)U (M)U(F)U(M)A(F)C(M)U(F)C(M)U(F)C(M)^U(F)^C(M) |
| **C**H0894 | Nο. 5917 | A(F)^G(M)^A(F)G(M)U(F)A(M)A(F)A(M)G(F)A(M)U(F) U(F)C(M)A(F)G(M)G(F)A(M)A(F)A(M)A(F)A(M) | No. 6619 | U(F)^U(M)^U(F)U(M)U(F)C(M)C(F)U(M)G(F)A(M)A(M)U(F)C (M)U(F)U(M)U(F)A(M)C(F)U(M)C(F)U(M)^C(F)^U(M) |
| CH0895 | No. 5918 | G(F)^A(M)^G(F)U(M)A(F)A(M)A(F)G(M)A(F)U(M)U(F) C(F)A(M)G(F)G(M)A(F)A(M)A(F)A(M)A(F)U(M) | No. 6620 | A(F)^U(M)^U(F)U(M)U(F)U(M)C(F)C(M)U(F)G(M)A(M)A(F)U (M)C(F)U(M)U(F)U(M)A(F)C(M)U(F)C(M)^U(F)^C(M) |
| CH0896 | No. 5919 | A(F)^G(M)^U(F)A(M)A(F)A(M)G(F)A(M)U(F)U(M)C(F) A(F)G(M)G(F)A(M)A(F)A(M)A(F)A(M)U(F)U(M) | No. 6621 | A(F)^A(M)^U(F)U(M)U(F)U(M)U(F)C(M)C(F)U(M)G(M)A(F)A (M)U(F)C(M)U(F)U(M)U(F)A(M)C(F)U(M)^C(F)^U(M) |
| CH0897 | No. 5920 | G(F)^U(M)^A(F)A(M)A(F)G(M)A(F)U(M)U(F)C(M)A(F) G(F)G(M)A(F)A(M)A(F)A(M)A(F)U(M)U(F)U(M) | No. 6622 | A(F)^A(M)^A(F)U(M)U(F)U(M)U(F)U(M)C(F)C(M)U(M)U(M)G(F)A (M)A(F)U(M)C(F)U(M)U(F)U(M)A(F)C(M)^U(F)^C(M) |
| CH0898 | No. 5921 | U(F)^A(M)^A(F)A(M)G(F)A(M)U(F)U(M)C(F)A(M)G(F) G(F)A(M)A(F)A(M)A(F)A(M)U(F)U(M)U(F)A(M) | No. 6623 | U(F)^A(M)^A(F)A(M)U(F)U(M)U(F)U(M)U(F)C(M)C(M)U(F)G M)A(F)A(M)U(F)C(M)U(F)U(M)U(F)A(M)^C(F)^U(M) |
| CH0899 | No. 5922 | A(F)^A(M)^A(F)G(M)A(F)U(M)U(F)C(M)A(F)G(M)G(F) A(F)A(M)A(F)A(M)A(F)U(M)U(F)U(M)A(F)A(M) | No. 6624 | U(F)^U(M)^A(F)A(M)A(F)U(M)U(F)U(M)U(F)U(M)C(M)C(F)U (M)G(F)A(M)A(F)U(M)C(F)U(M)U(F)U(M)^A(F)^C(M) |
| CH0905 | No. 5923 | U(F)^C(M)^A(F)G(M)G(F)A(M)A(F)A(M)A(F)A(M)U(F) U(F)U(M)A(F)A(M)G(F)A(M)A(F)U(M)G(F)G(M) | No. 6625 | C(F)^C(M)^A(F)U(M)U(F)C(M)U(F)U(M)A(F)A(M)A(M)U(F)U (M)U(F)U(M)U(F)C(M)C(F)U(M)G(F)A(M)^A(F)^U(M) |
| CH0906 | No. 5924 | C(F)^A(M)^G(F)G(M)A(F)A(M)A(F)A(M)A(F)U(M)U(F) U(F)A(M)A(F)G(M)A(F)A(M)U(F)G(M)G(F)A(M) | No. 6626 | U(F)^C(M)^C(F)A(M)U(F)U(M)C(F)U(M)U(F)A(M)A(M)A(F)U (M)U(F)U(M)U(F)U(M)C(F)C(M)U(F)G(M)^A(F)^A(M) |

### [Table 34]

**Table 3-14**

| Double stranded nucleic acid No. | SEQ ID NO. | Sense strand sequence (5'→3') | SEQ ID NO | Antisense strand sequence (5'→3') |
|---|---|---|---|---|
| CH0907 | No. 5925 | A(F)^G(M)^G(F)A(M)A(F)A(M)A(F)A(M)U(F)U(M)U(F)A (F)A(M)G(F)A(M)A(F)U(M)G(F)G(M)A(F)A(M) | No. 6627 | U(F)^U(M)^C(F)C(M)A(F)U(M)U(F)C(M)U(F)U(M)A(M)A(F)A( M)U(F)U(M)U(F)U(M)U(F)C(M)C(F)U(M)^G(F)^A(M) |
| CH0912 | No. 5926 | A(F)^A(M)^A(F)U(M)U(F)U(M)A(F)A(M)G(F)A(M)A(F)U (F)G(M)G(F)A(M)A(F)U(M)G(F)C(M)U(F)A(M) | No. 6628 | U(F)^A(M)^G(F)C(M)A(F)U(M)U(F)C(M)C(F)A(M)U(M)U(F)C (M)U(F)U(M)A(F)A(M)A(F)U(M)U(F)U(M)^U(F)^U(M) |
| CH0914 | No. 5927 | A(F)^U(M)^U(F)U(M)A(F)A(M)G(F)A(M)A(F)U(M)G(F) G(F)A(M)A(F)U(M)G(F)C(M)U(F)A(M)C(F)A(M) | No. 6629 | U(F)^G(M)^U(F)A(M)G(F)C(M)A(F)U(M)U(F)C(M)C(M)A(F)U (M)U(F)C(M)U(F)U(M)A(F)A(M)A(F)U(M)^U(F)^U(M) |
| CH0917 | No. 5928 | U(F)^A(M)^A(F)G(M)A(F)A(M)U(F)G(M)G(F)A(M)A(F) U(F)G(M)C(F)U(M)A(F)C(M)A(F)U(M)G(F)G(M) | No. 6630 | C(F)^C(M)^A(F)U(M)G(F)U(M)A(F)G(M)C(F)A(M)U(M)U(F)C (M)C(F)A(M)U(F)U(M)C(F)U(M)U(F)A(M)^A(F)^A(M) |
| CH0918 | No. 5929 | A(F)^A(M)^G(F)A(M)A(F)U(M)G(F)G(M)A(F)A(M)U(F) G(F)C(M)U(F)A(M)C(F)A(M)U(F)G(M)G(F)U(M) | No. 6631 | A(F)^C(M)^C(F)A(M)U(F)G(M)U(F)A(M)G(F)C(M)A(M)U(F)U (M)C(F)C(M)A(F)U(M)U(F)C(M)U(F)U(M)^A(F)^A(M) |
| CH0919 | No. 5930 | A(F)^G(M)^A(F)A(M)U(F)G(M)G(F)A(M)A(F)U(M)G(F) C(F)U(M)A(F)C(M)A(F)U(M)G(F)G(M)U(F)G(M) | No. 6632 | C(F)^A(M)^C(F)C(M)A(F)U(M)G(F)U(M)A(F)G(M)C(M)A(F)U (M)U(F)C(M)C(F)A(M)U(F)U(M)C(F)U(M)^U(F)^A(M) |
| CH0920 | No. 5931 | G(F)^A(M)^A(F)U(M)G(F)G(M)A(F)A(M)U(F)G(M)C(F) U(F)A(M)C(F)A(M)U(F)G(M)G(F)U(M)G(F)A(M) | No. 6633 | U(F)^C(M)^A(F)C(M)C(F)A(M)U(F)G(M)U(F)A(M)G(M)C(F)A (M)U(F)U(M)C(F)C(M)A(F)U(M)U(F)C(M)^U(F)^U(M) |
| CH0921 | No. 5932 | A(F)^A(M)^U(F)G(M)G(F)A(M)A(F)U(M)G(F)C(M)U(F) A(F)C(M)A(F)U(M)G(F)G(M)U(F)G(M)A(F)U(M) | No. 6634 | A(F)^U(M)^C(F)A(M)C(F)C(M)A(F)U(M)G(F)U(M)A(M)G(F)C (M)A(F)U(M)U(F)C(M)C(F)A(M)U(F)U(M)^C(F)^U(M) |
| CH0922 | No. 5933 | A(F)^U(M)^G(F)G(M)A(F)A(M)U(F)G(M)C(F)U(M)A(F) C(F)A(M)U(F)G(M)G(F)U(M)G(F)A(M)U(F)A(M) | No. 6635 | U(F)^A(M)^U(F)C(M)A(F)C(M)C(F)A(M)U(F)G(M)U(M)A(F)G (M)C(F)A(M)U(F)U(M)C(F)C(M)A(F)U(M)^U(F)^C(M) |
| CH0923 | No. 5934 | U(F)^G(M)^G(F)A(M)A(F)U(M)G(F)C(M)U(F)A(M)C(F) A(F)U(M)G(F)G(M)U(F)G(M)A(F)U(M)A(F)A(M) | No. 6636 | U(F)^U(M)^A(F)U(M)C(F)A(M)C(F)C(M)A(F)U(M)G(M)U(F)A (M)G(F)C(M)A(F)U(M)U(F)C(M)C(F)A(M)^U(F)^U(M) |
| CH0924 | No. 5935 | G(F)^G(M)^A(F)A(M)U(F)G(M)C(F)U(M)A(F)C(M)A(F) U(F)G(M)G(F)U(M)G(F)A(M)U(F)A(M)A(F)A(M) | No. 6637 | U(F)^U(M)^U(F)A(M)U(F)C(M)A(F)C(M)C(F)A(M)U(M)G(F)U (M)A(F)G(M)C(F)A(M)U(F)U(M)C(F)C(M)^A(F)^U(M) |
| CH0926 | No. 5936 | A(F)^A(M)^U(F)G(M)C(F)U(M)A(F)C(M)A(F)U(M)G(F) G(F)U(M)G(F)A(M)U(F)A(M)A(F)A(M)G(F)U(M) | No. 6638 | A(F)^C(M)^U(F)U(M)U(F)A(M)U(F)C(M)A(F)C(M)C(M)A(F)U( M)G(F)U(M)A(F)G(M)C(F)A(M)U(F)U(M)^C(F)^C(M) |
| CH0927 | No. 5937 | A(F)^U(M)^G(F)C(M)U(F)A(M)C(F)A(M)U(F)G(M)G(F) U(F)G(M)A(F)U(M)A(F)A(M)A(F)G(M)U(F)U(M) | No. 6639 | A(F)^A(M)^C(F)U(M)U(F)U(M)A(F)U(M)C(F)A(M)C(M)C(F) (M)U(F)G(M)U(F)A(M)G(F)C(M)A(F)U(M)^U(F)^C(M) |
| CH0928 | No. 5938 | U(F)^G(M)^C(F)U(M)A(F)C(M)A(F)U(M)G(F)G(M)U(F) G(F)A(M)U(F)A(M)A(F)A(M)G(F)U(M)U(F)U(M) | No. 6640 | A(F)^A(M)^A(F)C(M)U(F)U(M)U(F)A(M)U(F)C(M)A(M)C(F)C( M)A(F)U(M)G(F)U(M)A(F)G(M)C(F)A(M)^U(F)^U(M) |
| CH0930 | No. 5939 | C(F)^U(M)^A(F)C(M)A(F)U(M)G(F)G(M)U(F)G(M)A(F) U(F)A(M)A(F)A(M)G(F)U(M)U(F)U(M)C(F)U(M) | No. 6641 | A(F)^G(M)^A(F)A(M)A(F)C(M)U(F)U(M)U(F)A(M)U(M)C(F)A (M)C(F)C(M)A(F)U(M)G(F)U(M)A(F)G(M)^C(F)^A(M) |
| CH0932 | No. 5940 | A(F)^C(M)^A(F)U(M)G(F)G(M)U(F)G(M)A(F)U(M)A(F) A(F)A(M)G(F)U(M)U(F)U(M)C(F)U(M)U(F)U(M) | No. 6642 | A(F)^A(M)^A(F)G(M)A(F)A(M)A(F)C(M)U(F)U(M)U(M)A(F)U( M)C(F)A(M)C(F)C(M)A(F)U(M)G(F)U(M)^A(F)^G(M) |
| CH0934 | No. 5941 | A(F)^U(M)^G(F)G(M)U(F)G(M)A(F)U(M)A(F)A(M)A(F) G(F)U(M)U(F)U(M)C(F)U(M)U(F)U(M)C(F)U(M) | No. 6643 | A(F)^G(M)^ A(F)A(M)A(F)G(M)A(F)A(M)A(F)C(M)U(M)U(F)U( M)A(F)U(M)C(F)A(M)C(F)C(M)A(F)U(M)^G(F)^U(M) |
| CH0935 | No. 5942 | U(F)^ ^G(M)^G(F)U(M)G(F)A(M)U(F)A(M)A(F)A(M)G(F) U(F)U(M)U(F)C(M)U(F)U(M)U(F)C(M)U(F)U(M) | No. 6644 | A(F)^A(M)^G(F)A(M)A(F)A(M)G(F)A(M)A(F)A(M)C(M)U(F)U( M)U(F)A(M)U(F)C(M)A(F)C(M)C(F)A(M)^U(F)^G(M) |
| CH0936 | No. 5943 | G(F)^G(M)^U(F)G(M)A(F)U(M)A(F)A(M)A(F)G(M)U(F) U(F)U(M)C(F)U(M)U(F)U(M)C(F)U(M)U(F)C(M) | No.6645 | G(F)^A(M)^A(F)G(M)A(F)A(M)A(F)G(M)A(F)A(M)A(M)C(F)U( M)U(F)U(M)A(F)U(M)C(F)A(M)C(F)C(M)^A(F)^U(M) |
| CH0937 | No. 5944 | G(F)^U(M)^G(F)A(M)U(F)A(M)A(F)A(M)G(F)U(M)U(F) U(F)C(M)U(F)U(M)U(F)C(M)U(F)U(M)C(F)U(M) | No. 6646 | A(F)^G(M)^A(F)A(M)G(F)A(M)A(F)A(M)G(F)A(M)A(M)A(F)C( M)U(F)U(M)U(F)A(M)U(F)C(M)A(F)C(M)^C(F)^A(M) |
| CH0940 | No. 5945 | A(F)^U(M)^A(F)A(M)A(F)G(M)U(F)U(M)U(F)C(M)U(F)U (F)U(M)C(F)U(M)U(F)C(M)U(F)G(M)C(F)A(M) | No. 6647 | U(F)^G(M)^C(F)A(M)G(F)A(M)A(F)G(M)A(F)A(M)A(M)G(F)A (M)A(F)A(M)C(F)U(M)U(F)U(M)A(F)U(M)^C(F)^A(M) |
| CH0941 | No. 5946 | U(F)^A(M)^A(F)A(M)G(F)U(M)U(F)U(M)C(F)U(M)U(F)U (F)C(M)U(F)U(M)C(F)U(M)G(F)C(M)A(F)A(M) | No. 6648 | U(F)^U(M)^G(F)C(M)A(F)G(M)A(F)A(M)G(F)A(M)A(M)A(F)G (M)A(F)A(M)A(F)C(M)U(F)U(M)U(F)A(M)^U(F)^C(M) |
| CH0942 | No. 5947 | A(F)^A(M)^A(F)G(M)U(F)U(M)U(F)C(M)U(F)U(M)U(F) C(F)U(M)U(F)C(M)U(F)G(M)C(F)A(M)A(F)A(M) | No. 6649 | U(F)^U(M)^U(F)G(M)C(F)A(M)G(F)A(M)A(F)G(M)A(M)A(F)A (M)G(F)A(M)A(F)A(M)C(F)U(M)U(F)U(M)^A(F)^U(M) |
| CH0943 | No. 5948 | A(F)^A(M)^G(F)U(M)U(F)U(M)C(F)U(M)U(F)U(M)C(F) U(F)U(M)C(F)U(M)G(F)C(M)A(F)A(M)A(F)A(M) | No. 6650 | U(F)^U(M)^U(F)U(M)G(F)C(M)A(F)G(M)A(F)A(M)G(M)A(F)A (M)A(F)G(M)A(F)A(M)A(F)C(M)U(F)U(M)^U(F)^A(M) |
| CH0944 | No. 5949 | A(F)^ G(M)^U(F)U(M)U(F)C(M)U(F)U(M)U(F)C(M)U(F) U(F)C(M)U(F)G(M)C(F)A(M)A(F)A(M)A(F)A(M) | No. 6651 | U(F)^U(M)^U(F)U(M)U(F)G(M)C(F)A(M)G(F)A(M)A(M)G(F)A( M)A(F)A(M)G(F)A(M)A(F)A(M)C(F)U(M)^U(F)^U(M) |
| CH0945 | No. 5950 | G(F)^U(M)^U(F)U(M)C(F)U(M)U(F)U(M)C(F)U(M)U(F) C(F)U(M)G(F)C(M)A(F)A(M)A(F)A(M)A(F)U(M) | No. 6652 | A(F)^U(M)^U(F)U(M)U(F)U(M)G(F)C(M)A(F)G(M)A(M)A(F)G( M)A(F)A(M)A(F)G(M)A(F)A(M)A(F)C(M)^U(F)^U(M) |
| CH0946 | No. 5951 | U(F)^U(M)^U(F)C(M)U(F)U(M)U(F)C(M)U(F)U(M)C(F) U(F)G(M)C(F)A(M)A(F)A(M)A(F)A(M)U(F)A(M) | No. 6653 | U(F)^A(M)^U(F)U(M)U(F)U(M)U(F)G(M)C(F)A(M)G(M)A(F)A (M)G(F)A(M)A(F)A(M)G(F)A(M)A(F)A(M)^C(F)^U(M) |
| CH0947 | No. 5952 | U(F)^U(M)^C(F)U(M)U(F)U(M)C(F)U(M)U(F)C(M)U(F) G(F)C(M)A(F)A(M)A(F)A(M)A(F)U(M)A(F)A(M) | No. 6654 | U(F)^U(M)^A(F)U(M)U(F)U(M)U(F)U(M)G(F)C(M)A(M)G(F)A( M)A(F)G(M)A(F)A(M)A(F)G(M)A(F)A(M)^A(F)^C(M) |
| CH0948 | No. 5953 | U(F)^C(M)^U(F)U(M)U(F)C(M)U(F)U(M)C(F)U(M)G(F) C(F)A(M)A(F)A(M)A(F)A(M)U(F)A(M)A(F)G(M) | No. 6655 | C(F)^U(M)^U(F)A(M)U(F)U(M)U(F)U(M)U(F)G(M)C(M)A(F)G( M)A(F)A(M)G(F)A(M)A(F)A(M)G(F)A(M)^A(F)^A(M) |
| CH0953 | No. 5954 | C(F)^U(M)^U(F)C(M)U(F)G(M)C(F)A(M)A(F)A(M)A(F) A(F)U(M)A(F)A(M)G(F)G(M)A(F)A(M)A(F)A(M) | No. 6656 | U(F)^U(M)^U(F)U(M)C(F)C(M)U(F)U(M)A(F)U(M)U(M)U(F)U( M)U(F)G(M)C(F)A(M)G(F)A(M)A(F)G(M)^A(M)A(M) |
| CH0955 | No. 5955 | U(F)^C(M)^U(F)G(M)C(F)A(M)A(F)A(M)A(F)A(M)U(F) A(F)A(M)G(F)G(M)A(F)A(M)A(F)A(M)G(F)A(M) | No. 6657 | U(F)^C(M)^U(F)U(M)U(F)U(M)C(F)C(M)U(F)U(M)A(M)U(F)U( M)U(F)U(M)U(F)G(M)C(F)A(M)G(F)A(M)^A(F)^G(M) |
| CH0956 | No. 5956 | C(F)^U(M)^G(F)C(M)A(F)A(M)A(F)A(M)A(F)U(M)A(F) A(F)G(M)G(F)A(M)A(F)A(M)A(F)G(M)A(F)A(M) | No. 6658 | U(F)^U(M)^C(F)U(M)U(F)U(M)U(F)C(M)C(F)U(M)U(M)A(F)U( M)U(F)U(M)U(F)U(M)G(F)C(M)A(F)G(M)^A(F)^A(M) |
| CH0957 | No. 5957 | U(F)^G(M)^C(F)A(M)A(F)A(M)A(F)A(M)U(F)A(M)A(F) G(F)G(M)A(F)A(M)A(F)A(M)G(F)A(M)A(F)G(M) | No. 6659 | C(F)^U(M)^U(F)C(M)U(F)U(M)U(F)U(M)C(F)C(M)U(M)U(F)A M)U(F)U(M)U(F)U(M)U(F)G(M)C(F)A(M)^G(F)^A(M) |
| CH0964 | No. 5958 | A(F)^U(M)^A(F)A(M)G(F)G(M)A(F)A(M)A(F)A(M)G(F) A(F)A(M)G(F)U(M)G(F)U(M)A(F)G(M)C(F)U(M) | No. 6660 | A(F)^G(M)^C(F)U(M)A(F)C(M)A(F)C(M)U(F)U(M)C(M)U(F)U (M)U(F)U(M)C(F)C(M)U(F)U(M)A(F)U(M)^U(F)^U(M) |
| CH0965 | No. 5959 | U(F)^A(M)^A(F)G(M)G(F)A(M)A(F)A(M)A(F)G(M)A(F) A(F)G(M)U(F)G(M)U(F)A(M)G(F)C(M)U(F)A(M) | No. 6661 | U(F)^A(M)^G(F)C(M)U(F)A(M)C(F)A(M)C(F)U(M)U(M)C(F)U (M)U(F)U(M)U(F)C(M)C(F)U(M)U(F)A(M)^U(F)^U(M) |
| CH0966 | No. 5960 | A(F)^A(M)^G(F)G(M)A(F)A(M)A(F)A(M)G(F)A(M)A(F) G(F)U(M)G(F)U(M)A(F)G(M)C(F)U(M)A(F)U(M) | No 6662 | A(F)^U(M)^A(F)G(M)C(F)U(M)A(F)C(M)A(F)C(M)U(M)U(F)C (M)U(F)U(M)U(F)U(M)C(F)C(M)U(F)U(M)^A(F)^U(M) |
| CH0967 | No. 5961 | A(F)^G(M)^G(F)A(M)A(F)A(M)A(F)G(M)A(F)A(M)G(F) U(F)G(M)U(F)A(M)G(F)C(M)U(F)A(M)U(F)A(M) | No. 6663 | U(F)^A(M)^U(F)A(M)G(F)C(M)U(F)A(M)C(F)A(M)C(M)U(F)U (M)C(F)U(M)U(F)U(M)U(F)C(M)C(F)U(M)^U(F)^A(M) |
| CH0969 | No. 5962 | G(F)^A(M)^A(F)A(M)A(F)G(M)A(F)A(M)G(F)U(M)G(F) U(F)A(M)G(F)C(M)U(F)A(M)U(F)A(M)C(F)A(M) | No. 6664 | U(F)^G(M)^U(F)A(M)U(F)A(M)G(F)C(M)U(F)A(M)C(M)A(F)C (M)U(F)U(M)C(F)U(M)U(F)U(M)U(F)C(M)^C(F)^U(M) |
| CH0972 | No. 5963 | A(F)^A(M)^G(F)A(M)A(F)G(M)U(F)G(M)U(F)A(M)G(F) C(F)U(M)A(F)U(M)A(F)C(M)A(F)G(M)A(F)G(M) | No. 6665 | C(F)^U(M)^C(F)U(M)G(F)U(M)A(F)U(M)A(F)G(M)C(M)U(F) (M)C(F)A(M)C(F)U(M)U(F)C(M)U(F)U(M)^U(F)^U(M) |
| CH0974 | No. 5964 | G(F)^A(M)^A(F)G(M)U(F)G(M)U(F)A(M)G(F)C(M)U(F) A(F)U(M)A(F)C(M)A(F)G(M)A(F)G(M)G(F)A(M) | No. 6666 | U(F)^C(M)^C(F)U(M)C(F)U(M)G(F)U(M)A(F)U(M)A(M)G(F)C (M)U(F)A(M)C(F)A(M)C(F)U(M)U(F)C(M)^U(F)^U(M) |

### [Table 35]

**Table 3-15**

| Double stranded nucleic acid No. | SEQ ID NO | Sense strand sequence (5'→3') | SEQ ID NO | Antisence strand sequence (5'→3') |
|---|---|---|---|---|
| CH0975 | No. 5965 | A(F)^A(M)^G(F)U(M)G(F)U(M)A(F)G(M)C(F)U(M)A(F) U(F)A(M)C(F)A(M)G(F)A(M)G(F)G(M)A(F)U(M) | No. 6667 | A(F)^U(M)^C(F)C(M)U(F)C(M)U(F)G(M)U(F)A(M)U(M)A(F)G (M)C(F)U(M)A(F)C(M)A(F)C(M)U(F)U(M)^C(F)^U(M) |
| CH0977 | No. 5966 | G(F)^U(M)^G(F)U(M)A(F)G(M)C(F)U(M)A(F)U(M)A(F) C(F)A(M)G(F)A(M)G(F)G(M)A(F)U(M)G(F)C(M) | No. 6668 | G(F)^C(M)^A(F)U(M)C(F)C(M)U(F)C(M)U(F)G(M)U(M)A(F)U (M)A(F)G(M)C(F)U(M)A(F)C(M)A(F)C(M)^U(F)^U(M) |
| CH0978 | No. 5967 | U(F)^G(M)^U(F)A(M)G(F)C(M)U(F)A(M)U(F)A(M)C(F) A(F)G(M)A(F)G(M)G(F)A(M)U(F)G(M)C(F)U(M) | No. 6669 | A(F)^G(M)^C(F)A(M)U(F)C(M)C(F)U(M)C(F)U(M)G(M)U(F)A (M)U(F)A(M)G(F)C(M)U(F)A(M)C(F)A(M)^C(F)^U(M) |
| CH0980 | No. 5968 | U(F)^A(M)^G(F)C(M)U(F)A(M)U(F)A(M)C(F)A(M)G(F) A(F)G(M)G(F)A(M)U(F)G(M)C(F)U(M)C(F)A(M) | No. 6670 | U(F) ^G(M)^A(F)G(M)C(F)A(M)U(F)C(M)C(F)U(M)C(M)U(F)G (M)U(F)A(M)U(F)A(M)G(F)C(M)U(F)A(M)^C(F)^A(M) |
| CH0981 | No. 5969 | A(F)^G(M)^C(F)U(M)A(F)U(M)A(F)C(M)A(F)G(M)A(F) G(F)G(M)A(F)U(M)G(F)C(M)U(F)C(M)A(F)G(M) | No. 6671 | C(F)^U(M)^G(F)A(M)G(F)C(M)A(F)U(M)C(F)C(M)U(M)C(F)U (M)G(F)U(M)A(F)U(M)A(F)G(M)C(F)U(M)^A(F)^C(M) |
| CH0985 | No. 5970 | A(F)^U(M)^A(F)C(M)A(F)G(M)A(F)G(M)G(F)A(M)U(F) G(F)C(M)U(F)C(M)A(F)G(M)U(F)G(M)U(F)A(M) | No. 6672 | U(F)^A(M)^C(F)A(M)C(F)U(M)G(F)A(M)G(F)C(M)A(M)U(F)C (M)C(F)U(M)C(F)U(M)G(F)U(M)A(F)U(M)^A(F)^G(M) |
| CH0987 | No. 5971 | A(F)^C(M)^A(F)G(M)A(F)G(M)G(F)A(M)U(F)G(M)C(F) U(F)C(M A(F)G(M)U(F)G(M)U(F)A(M)U(F)A(M) | No. 6673 | U(F)^A(M)^U(F)A(M)C(F)A(M)C(F)U(M)G(F)A(M)G(M)C(F)A (M)U(F)C(M)C(F)U(M)C(F)U(M)G(F)U(M)^A(F)^U(M) |
| CH0988 | No. 5972 | C(F)^A(M)^G(F)A(M)G(F)G(M)A(F)U(M)G(F)C(M)U(F) C(F)A(M)G(F)U(M)G(F)U(M)A(F)U(M)A(F)G(M) | No. 6674 | C(F)^U(M)^A(F)U(M)A(F)C(M)A(F)C(M)U(F)A(M)G(F)C (M)A(F)U(M)C(F)C(M)U(F)C(M)U(F)G(M)^U(F)^A(M) |
| CH0989 | No. 5973 | A(F)^G(M)^A(F)G(M)G(F)A(M)U(F)G(M)C(F)U(M)G(F) A(F)G(M)U(F)G(M)U(F)A(M)U(F)(M)G(F)A(M) | No. 6675 | U(F)^C(M)^U(F)A(M)U(F)A(M)C(F)A(M)C(F)U(M)G(M)A(F)G (M)C(F)A(M)U(F)C(M)C(F)U(M)C(F)U(M)^G(F)^U(M) |
| CH0990 | No. 5974 | G(F)^A(M)^G(F)G(M)A(F)U(M)G(F)C(M)U(F)C(M)A(F) G(F)U(M)G(F)U(M)A(F)U(M)A(F)G(M)A(F)U(M) | No. 6676 | A(F)^U(M)^C(F)U(M)A(F)U(M)A(F)C(M)A(F)C(M)U(M)G(F)A( M)G(F)C(M)A(F)U(M)C(F)C(M)U(F)C(M)^U(F)^G(M) |
| CH0991 | NO. 5975 | A(F)^G(M)^G(F)A(M)U(F)G(M)C(F)U(M)C(F)A(M)G(F) U(F)G(M)U(F)A(M)U(F)A(M)G(F)A(M)U(F)G(M) | No. 6677 | C(F)^A(M)^U(F)C(M)U(F)A(M)U(F)A(M)C(F)A(M)C(M)U(F)G (M)A(F)G(M)C(F)A(M)U(F)C(M)C(F)U(M)^C(F)^U(M) |
| CH0996 | No. 5976 | G(F)^C(M)^U(F)C(M)A(F)G(M)U(F)G(M)U(F)A(M)U(F) A(F)G(M)A(F)U(M)G(F)G(M)C(F)A(M)C(F)U(M) | No. 6678 | A(F)^G(M)^U(F)G(M)C(F)C(M)A(F)U(M)C(F)U(M)A(M)U(F)A (M)C(F)A(M)C(F)U(M)G(F)A(M)G(F)C(M)^A(F)^U(M) |
| CH0997 | No. 5977 | C(F)^U(M)^C(F)A(M)G(F)U(M)G(F)U(M)A(F)U(M)A(F) G(F)A(M)U(F)G(M)G(F)C(M)A(F)C(M)U(F)A(M) | No. 6679 | U(F)^A(M)^G(F)U(M)G(F)C(M)C(F)A(M)U(F)C(M)U(M)A(F)U (M)A(F)C(M)A(F)C(M)U(F)G(M)A(F)G(M)^C(F)^A(M) |
| CH0998 | No. 5978 | U(F)^C(M)^A(F)G(M)U(F)G(M)U(F)A(M)U(F)A(M)G(F) A(F)U(M)G(F)G(M)C(F)A(M)C(F)U(M)A(F)U(M) | No. 6680 | A(F)^U(M)^A(F)G(M)U(F)G(M)C(F)C(M)A(F)U(M)C(M)U(F)A (M)U(F)A(M)C(F)A(M)C(F)U(M)G(F)A(M)^G(F)^C(M) |
| CH0999 | No. 5979 | C(F)^A(M)^G(F)U(M)G(F)U(M)A(F)U(M)A(F)G(M)A(F) U(F)G(M)G(F)C(M)A(F)C(M)U(F)A(M)U(F)C(F) | No. 6681 | G(F)^A(M)^U(F)A(M)G(F)U(M)G(F)C(M)C(F)A(M)U(M)C(F)U (M)A(F)U(M)A(F)C(M)A(F)C(M)U(F)G(M)^A(F)G(M) |
| CH1000 | No. 5980 | A(F)^ G(M)^U(F)G(M)U(F)A(M)U(F)A(M)G(F)A(M)U(F) G(F)G(M)C(F)A(M)C(F)U(M)A(F)U(M)C(F)G(M) | No. 6682 | C(F)^G(M)^A(F)U(M)A(F)G(M)U(F)G(M)C(F)C(M)A(M)U(F)C (M)U(F)A(M)U(F)A(M)C(F)A(M)C(F)U(M)^G(F)^A(M) |
| CH1001 | No. 5981 | G(F)^U(M)^G(F)U(M)A(F)U(M)A(F)G(M)A(F)U(M)G(F) G(F)C(M)A(F)C(M)U(F)A(M)U(F)C(M)C(F)A(M) | No. 6683 | U(F)^C(M)^G(F)A(M)U(F)A(M)G(F)U(M)G(F)C(M)C(M)A(F)U (M)C(F)U(M)A(F)U(M)A(F)C(M)A(F)C(M)^U(F)^G(M) |
| CH1002 | No. 5982 | U(F)^G(M)^U(F)A(M)U(F)A(M)G(F)A(M)U(F)G(M)G(F) C(F)A(M)C(F)U(M)A(F)U(M)C(F)G(M)A(F)A(M) | No. 6684 | U(F)^U(M)^ C(F)G(M)A(F)U(M)A(F)G(M)U(F)G(M)C(M)C(F)A (M)U(F)C(M)U(F)A(M)U(F)A(M)C(F)A(M)^C(F)^U(M) |
| CH1003 | No. 5983 | G(F)^U(M)^A(F)U(M)A(F)G(M)A(F)U(M)G(F)G(M)C(F) A(F)C(M)U(F)A(M)U(F)C(M)G(F)A(M)A(F)G(M) | No. 6685 | C(F)^U(M)^U(F)C(M)G(F)A(M)U(F)A(M)G(F)U(M)G(M)C(F)C (M)A(F)U(M)C(F)U(M)A(F)U(M)A(F)C(M)^A(F)^C(M) |
| CH1005 | No. 5984 | A(F)^U(M)^A(F)G(M)A(F)U(M)G(F)G(M)C(F)A(M)C(F) U(F)A(M)U(F)C(M)G(F)A(M)A(F)G(M)U(F)C(M) | No. 6686 | G(F)^A(M)^C(F)U(M)U(F)C(M)G(F)A(M)U(F)A(M)G(M)U(F)G (M)C(F)C(M)A(F)U(M)C(F)U(M)A(F)U(M)^A(F)^C(M) |
| CH1007 | No. 5985 | A(F)^G(M)^A(F)U(M)G(F)G(M)C(F)A(M)C(F)U(M)A(F) U(F)C(M)G(F)A(M)A(F)G(M)U(F)C(M)C(F)C(M) | No. 6687 | G(F)^G(M)^G(F)A(M)C(F)U(M)U(F)C(M)G(F)A(M)U(M)A(F)G (M)U(F)G(M)C(F)C(M)A(F)U(M)C(F)U(M)^A(F)^U(M) |
| CH1009 | No. 5986 | A(F)^U(M)^G(F)G(M)C(F)A(M)C(F)U(M)A(F)U(M)A(F) G(F)A(M)A(F)G(M)U(F)C(M)C(F)C(M)C(F)A(M) | No. 6688 | U(F)^G(M)^G(F)G(M)G(F)A(M)C(F)U(M)U(F)C(M)G(M)A(F)U (M)A(F)G(M)U(F)G(M)C(F)C(M)A(F)U(M)^C(F)^U(M) |
| CH1010 | No. 5987 | U(F)^G(M)^G(F)C(M)A(F)C(M)U(F)A(M)U(F)C(M)G(F) A(F)A(M)G(F)U(M)C(F)C(M)C(F)C(M)A(F)A(M) | No. 6689 | U(F)^U(M)^G(F)G(M)G(F)G(M)A(F)C(M)U(F)U(M)C(M)G(F)A (M)U(F)A(M)G(F)U(M)G(F)C(M)C(F)A(M)^U(F)^C(M) |
| CH1011 | No. 5988 | G(F)^G(M)^C(F)A(M)C(F)U(M)A(F)U(M)C(F)G(M)A(F) A(F)G(M)U(F)C(M)C(F)C(M)C(F)A(M)A(F)A(M) | No. 6690 | U(F)^U(M)^U(F)G(M)G(F)G(M)G(F)A(M)C(F)U(M)C(F)G (M)A(F)U(M)A(F)G(M)U(F)G(M)C(F)C(M)^A(F)^U(M) |
| CH1013 | No. 5989 | C(F)^A(M)^C(F)U(M)A(F)U(M)C(F)G(M)A(F)A(M)G(F) U(F)C(M)C(F)C(M)C(F)A(M)A(F)A(M)U(F)G(M) | No. 6691 | C(F)^A(M)^U(F)U(M)U(F)G(M)G(F)G(M)G(F)A(M)C(M)U(F)U (M)C(F)G(M)A(F)U(M)A(F)G(M)U(F)G(M)^C(F)^C(M) |
| CH1015 | No. 5990 | C(F)^U(M)^A(F)U(M)C(F)G(M)A(F)A(M)G(F)U(M)C(F) C(F)C(M)C(F)A(M)A(F)A(M)U(F)G(M)C(F)U(M) | No. 6692 | A(F)^G(M)^C(F)A(M)U(F)U(M)U(F)G(M)G(F)G(M)G(M)A(F)C (M)U(F)U(M)C(F)G(M)A(F)U(M)A(F)G(M)^U(F)^G(M) |
| CH1016 | No. 5991 | U(F)^A(M)^U(F)C(M)G(F)A(M)A(F)G(M)U(F)C(M)C(F) C(F)C(M)A(F)A(M)A(F)U(M)G(F)C(M)U(F)U(M) | No. 6693 | A(F)^A(M)^ G(F)C(M)A(F)U(M)U(F)U(M)G(F)G(M)G(M)G(F)A (M)C(F)U(M)U(F)C(M)G(F)A(M)U(F)A(M)^G(F)^U(M) |
| CH1017 | No. 5992 | A(F)^U(M)^C(F)G(M)A(F)A(M)G(F)U(M)C(F)C(M)C(F) C(F)A(M)A(F)A(M)U(F)G(M)C(F)U(M)U(F)C(M) | No. 6694 | G(F)^A(M)^A(F)G(M)C(F)A(M)U(F)U(M)U(F)G(M)G(M)G(F)G (M)A(F)C(M)U(F)U(M)C(F)G(M)A(F)U(M)^A(F)^G(M) |
| CH1018 | No. 5993 | U(F)^C(M)^G(F)A(M)A(F)G(M)U(F)C(M)C(F)C(M)C(F) A(F)A(M)A(F)U(M)G(F)C(M)U(F)U(M)C(F)A(M) | No. 6695 | U(F)^G(M)^A(F)A(M)G(F)C(M)A(F)U(M)U(F)U(M)G(M)G(F)G (M)G(F)A(M)C(F)U(M)U(F)C(M)G(F)A(M)^U(F)^A(M) |
| CH1021 | No. 5994 | A(F)^A(M)^G(F)U(M)C(F)C(M)C(F)C(M)A(F)A(M)A(F) U(F)G(M)C(F)U(M)U(F)C(M)A(F)A(M)G(F)G(M) | No. 6696 | C(F)^C(M)^U(F)U(M)G(F)A(M)A(F)G(M)C(F)A(M)U(M)U(F)U (M)G(F)G(M)G(F)G(M)A(F)C(M)U(F)U(M)^C(F)^G(M) |
| | No. 5995 | A(F)^G(M)^U(F)C(M)C(F)C(M)C(F)A(M)A(F)A(M)U(F) G(F)C(M)U(F)U(M)C(F)A(M)A(F)G(M)G(F)A(M) | No. 6697 | U(F)^C(M)^C(F)U(M)U(F)G(M)A(F)A(M)G(F)C(M)A(M)U(F)U (M)U(F)G(M)G(F)G(M)G(F)A(M)C(F)U(M)^U(F)^C(M) |
| CH1023 | No. 5996 | G(F)^U(M)^C(F)C(M)C(F)C(M)A(F)A(M)A(F)U(M)G(F) C(F)U(M)U(F)C(M)A(F)A(M)G(F)G(M)A(F)A(M) | No. 6698 | U(F)^U(M)^C(F)C(M)U(F)U(M)G(F)(M)A(F)G(M)C(M)A(F)U (M)U(F)U(M)G(F)G(M)G(F)G(M)A(F)C(M)^U(F)^U(M) |
| CH1024 | No. 5997 | U(F)^C(M)^C(F)C(M)C(F)A(M)A(F)A(M)U(F)G(M)C(F) U(F)U(M)C(F)A(M)A(F)G(M)G(F)A(M)A(F)C(M) | No. 6699 | G(F)^U(M)^U(F)C(M)C(F)U(M)U(F)G(M)A(F)A(M)C(F)A (M)U(F)U(M)U(F)G(M)G(F)G(M)G(F)A(M)^C(F)^U(M) |
| CH1025 | No. 5998 | C(F)^C(M)^C(F)C(M)A(F)A(M)A(F)U(M)G(F)C(M)U(F) U(F)C(M)A(F)A(M)G(F)G(M)A(F)A(M)C(F)A(M) | No. 6700 | U(F)^G(M)^U(F)U(M)C(F)C(M)U(F)U(M)G(F)A(M)A(M)G(F)C (M)A(F)U(M)U(F)U(M)G(F)G(M)G(F)G(M)^A(F)^C(M) |
| CH1026 | No. 5999 | C(F)^C(M)^C(F)A(M)A(F)A(M)U(F)G(M)C(F)U(M)U(F) C(F)A(M)A(F)G(M)G(F)A(M)A(F)C(M)A(F)C(M) | No. 6701 | G(F)^U(M)^G(F)U(M)U(F)C(M)C(F)U(M)U(F)G(M)A(M)A(F)G(M)C(F)A(M)U(F)U(M)U(F)G(M)G(F)G(M)^G(F)^A(M) |
| CH1027 | No. 6000 | C(F)^C(M)^A(F)A(M)A(F)U(M)G(F)C(M)U(F)U(M)C(F) A(F)A(M)G(F)G(M)A(F)A(M)C(F)A(M)C(F)A(M) | No. 6702 | U(F)^G(M)^U(F)G(M)U(F)U(M)C(F)C(M)U(F)U(M)G(M)A(F)A (M)G(F)C(M)A(F)U(M) U(F)U(M)G(F)G(M)^G(F)^G(M) |
| CH1028 | No. 6001 | C(F)^A(M)^A(F)A(M)U(F)G(M)C(F)U(M)U(F)C(M)A(F) A(F)G(M)G(F)A(M)A(F)C(M)A(F)C(M)A(F)G(M) | No. 6703 | C(F)^U(M)^G(F)U(M)G(F)U(M)U(F)C(M)C(F)U(M)U(M)G(F)A (M)A(F)G(M)C(F)A(M)U(F)U(M)U(F)G(M)^G(F)^G(M) |
| CH1029 | No. 6002 | A(F)^A(M)^A(F)U(M)G(F)C(M)U(F)U(M)C(F)A(M)A(F) G(F)G(M)A(F)A(M)C(F)A(M)C(F)A(M)G(F)U(M) | No. 6704 | A(F)^C(M)^U(F)G(M)U(F)G(M)U(F)U(M)G(F)C(M)U(M)U(F)G (M)A(F)A(M)G(F)C(M)A(F)U(M)U(F)U(M)^G(F)^G(M) |
| CH1030 | No. 6003 | A(F)^A(M)^U(F)G(M)C(F)U(M)U(F)C(M)A(F)A(M)G(F) G(F)A(M)A(F)C(M)A(F)C(M)A(F)G(M)U(F)U(M) | No. 6705 | A(F)^A(M)^C(F)U(M)G(F)U(M)G(F)U(M)U(F)C(M)C(M)U(F)U (M)G(F)A(M)A(F)G(M)C(F)A(M)U(F)U(M)^UF^G(M) |
| CH1031 | No. 6004 | A(F)^U(M)^G(F)C(M)U(F)U(M)C(F)A(M)A(F)G(M)G(F) A(F)A(M)C(F)A(M)C(F)A(M)G(F)U(M)U(F)C(M) | No. 6706 | G(F)^A(M)^A(F)C(M)U(F)G(M)U(F)G(M)U(F)U(M)C(M)C(F)U (M)U(F)G(M)A(F)A(M)G(F)C(M)A(F)U(M)^U(F)^U(M) |

### [Table 36]

**Table 3-16**

| Double stranded nucleic acid No. | SEQ ID NO. | Sense strand sequence (5'→3') | SEQ ID NO. | Antisense strand sequence (5'→3') |
|---|---|---|---|---|
| CH1032 | No. 6005 | U(F)^G(M)^C(F)U(M)U(F)C(M)A(F)A(M)G(F)G(M)A(F) A(F)C(M)A(F)C(M)A(F)G(M)U(F)U(M)C(F)U(M) | No. 6707 | A(F)^G(M)^A(F)A(M)C(F)U(M)G(F)U(M)G(F)U(M)U(M)C(F)C (M)U(F)U(M)G(F)A(M)A(F)G(M)C(F)A(M)^U(F)^U(M) |
| CH1034 | No. 6006 | C(F)^U(M)^U(F)C(M)A(F)A(M)G(F)G(M)A(F)A(M)C(F) A(F)C(M)A(F)G(M)U(F)U(M)C(F)U(M)C(F)U(M) | No. 6708 | A(F)^ G(M)^A(F)G(M)A(F)A(M)C(F)U(M)G(F)U(M)G(M)U(F)U (M)C(F)C(M)U(F)U(M)G(F)A(M)^C(F)^A(M) |
| CH1035 | No. 6007 | U(F)^U(M)^C(F)A(M)A(F)G(M)G(F)A(M)A(F)C(M)A(F) C(F)A(M)G(F)U(M)U(F)C(M)U(F)C(M)U(F)G(M) | No. 6709 | C(F)^A(M)^G(F)A(M)G(F)A(M)A(F)C(M)U(F)G(M)U(M)G(F)U (M)U(F)C(M)C(F)U(M)U(F)U(M)A(F)A(M)^G(F)^C(M) |
| CH1038 | No. 6008 | A(F)^A(M)^G(F)G(M)A(F)A(M)C(F)A(M)C(F)A(M)G(F) U(F)U(M)C(F)U(M)C(F)U(M)G(F)G(M)C(F)U(M) | No. 6710 | A(F)^G(M)^C(F)C(M)A(F)G(M)A(F)G(M)A(F)A(M)C(M)U(F)G (M)U(F)G(M)U(F)U(M)C(F)C(M)U(F)U(M)^G(F)^A(M) |
| CH1039 | No. 6009 | A(F)^G(M)^G(F)A(M)A(F)C(M)A(F)C(M)A(F)G(M)U(F) U(F)C(M)U(F)C(M)U(F)G(M)G(F)C(M)U(F)U(M) | No. 6711 | A(F)^A(M)^G(F)C(M)C(F)A(M)G(F)A(M)G(F)A(M)A(M)C(F)U (M)G(F)U(M)G(F)U(M)U(F)C(M)C(F)U(M)^U(F)^G(M) |
| CH1040 | No. 6010 | G(F)^G(M)^A(F)A(M)C(F)A(M)C(F)A(M)G(F)U(M)U(F) C(F)U(M)C(F)U(M)G(F)G(M)C(F)U(M)U(F)U(M) | No. 6712 | A(F)^A(M)^A(F)G(M)C(F)C(M)A(F)G(M)A(F)G(M)A(M)A(F)C (M)U(F)G(M)U(F)G(M)U(F)U(M)C(F)C(M)^U(F)^U(M) |
| CH1041 | No. 6011 | G(F)^A(M)^A(F)C(M)A(F)C(M)A(F)G(M)U(F)U(M)C(F) U(F)C(M)U(F)G(M)G(F)C(M)U(F)U(M)U(F)U(M) | No. 6713 | A(F)^A(M)^A(F)A(M)G(F)C(M)C(F)A(M)G(F)A(M)G(M)A(F)A (M)C(F)U(M)G(F)U(M)G(F)U(M)U(F)G(M)^C(F)^U(M) |
| CH1042 | No. 6012 | A(F)^A(M)^C(F)A(M)C(F)A(M)G(F)U(M)U(F)C(M)U(F) C(F)U(M)G(F)G(M)C(F)U(M)U(F)U(M)U(F)U(M) | No. 6714 | A(F)^A(M)^A(F)A(M)A(F)G(M)C(F)C(M)A(F)G(M)A(M)G(F)A (M)A(F)C(M)U(F)G(M)U(F)G(M)U(M)^C(F)^C(M) |
| CH1043 | No. 6013 | A(F)^C(M)^A(F)C(M)A(F)G(M)U(F)U(M)C(F)U(M)C(F) U(F)G(M)G(F)C(M)U(F)U(M)U(F)U(M)U(F)G(M) | No. 6715 | C(F)^A(M)^A(F)A(M)A(F)A(M)G(F)C(M)C(F)A(M)G(M)A(F)G (M)A(F)A(M)C(F)U(M)G(F)U(M)G(F)U(M)^U(F)^C(M) |
| CH1044 | No. 6014 | C(F)^A(M)^C(F)A(M)G(F)U(M)U(F)C(M)U(F)C(M)U(F) G(F)G(M)C(F)U(M)U(F)U(M)U(F)U(M)G(F)G(M) | No. 6716 | C(F)^C(M)^A(F)A(M)A(F)A(M)A(F)G(M)C(F)C(M)A(M)G(F)A (M)G(F)A(M)A(F)C(M)U(F)G(M)U(F)G(M)^U(F)^U(M) |
| CH1045 | No. 6015 | A(F)^C(M)^A(F)G(M)U(F)U(M)C(F)U(M)C(F)U(M)G(F) G(F)C(M)U(F)U(M)U(F)U(M)U(F)G(M)G(F)A(M) | No. 6717 | U(F)^C(M)^C(F)A(M)A(F)A(M)A(F)A(M)G(F)C (M)C (M)A(F)G (M)A(F)G(M)A(F)A(M)C(F)U(M)G(F)U(M)^G(F)^U(M) |
| CH1046 | No. 6016 | C(F)^A(M)^G(F)U(M)U(F)C(M)U(F)C(M)U(F)G(M)G(F) C(F)U(M)U(F)U(M)U(F)U(M)G(F)G(M)A(F)A(M) | No. 6718 | U(F)^U(M)^C(F)C(M)A(F)A(M)A(F)A(M)A(F)G(M)C(M)C(F)A (M)G(F)A(M)G(F)A(M)A(F)C(M)U(F)G(M)^U(F)^G(M) |
| CH1047 | No. 6017 | A(F)^G(M)^U(F)U(M)C(F)U(M)C(F)U(M)G(F)G(M)C(F) U(F)U(M)U(F)U(M)U(F)G(M)G(F)A(M)A(F)A(M) | No. 6719 | U(F)^U(M)^U(F)C(M)C(F)A(M)A(F)A(M)A(F)A(M)G(M)C(F)C (M)A(F)G(M)A(F)G(M)A(F)A(M)C(F)U(M)^G(F)^U(M) |
| CH1048 | No. 6018 | G(F)^U(M)^U(F)C(M)U(F)C(M)U(F)G(M)G(F)C(M)U(F) U(F)U(M)U(F)U(M)G(F)G(M)A(F)A(M)A(F)A(M) | No. 6720 | U(F)^U(M)^U(F)U(M)C(F)C(M)A(F)A(M)A(F)A(M)A(M)G(F)C( M)C(F)A(M)G(F)A(M)G(F)A(M)A(F)C(M)^U(F)^G(M) |
| CH1051 | No. 6019 | C(F)^U(M)^C(F)U(M)G(F)G(M)C(F)U(M)U(F)U(M)U(F) U(F)G(M)G(F)A(M)A(F)A(M)A(F)C(M)U(F)G(M) | No. 6721 | C(F)^A(M)^G(F)U(M)U(F)U(M)U(F)C(M)C(F)A(M)A(M)A(F)A( M)A(F)G(M) C(F)C(M)A(F)G(M)A(F)G(M)^A(F)^A(M) |
| CH1052 | No. 6020 | U(F)^C(M)^U(F)G(M)G(F)C(M)U(F)U(M)U(F)U(M)U(F) G(F)G(M)A(F)A(M)A(F)A(M)C(F)U(M)G(F)A(M) | No. 6722 | U(F)^C(M)^A(F)G(M)U(F)U(M)U(F)U(M)C(F)C(M)A(M)A(F)A( M)A(F)A(M)G(F)C(M)C(F)A(M)G(F)A(M)^G(F)^A(M) |
| CH1053 | No. 6021 | C(F)^U(M)^G(F)G(M)C(F)U(M)U(F)U(M)U(F)U(M)G(F) G(F)A(M)A(F)A(M)A(F)C(M)U(F)G(M)A(F)U(M) | No. 6723 | A(F)^U(M)^C(F)A(M)G(F)U(M)U(F)U(M)U(F)C(M)C(M)A(F)A( M)A(F)A(M)A(F)G(M)C(F)C(M)A(F)C(M)^A(F)^G(M) |
| CH1054 | No. 6022 | U(F)^G(M)^G(F)C(M)U(F)U(M)U(F)U(M)U(F)G(M)G(F) A(F)A(M)A(F)A(M)C(F)U(M)G(F)A(M)U(F)G(M) | No. 6724 | C(F)^A(M)^U(F)C(M)A(F)G(M)U(F)U(M)U(F)U(M)C(M)C(F)A (M)A(F)A(M)A(F)A(M)G(F)C(M)C(F)A(M)^G(F)^A(M) |
| CH1056 | No. 6023 | G(F)^C(M)^U(F)U(M)U(F)U(M)U(F)G(M)G(F)A(M)A(F) A(F)A(M)C(F)U(M)G(F)A(M)U(F)G(M)C(F)A(M) | No. 6725 | U(F)^G(M)^C(F)A(M)U(F)C(M)A(F)G(M)U(F)U(M)U(M)U(M)U(F)C (M)C(F)A(M)A(F)A(M)A(F)A(M)G(F)C(M)^C(F)^A(M) |
| CH1057 | No. 6024 | C(F)^U(M)^U(F)U(M)U(F)U(M)G(F)G(M)A(F)A(M)A(F) A(F)C(M)U(F)G(M)A(F)U(M)G(F)C(M)A(F)U(M) | No. 6726 | A(F)^U(M)^G(F)C(M)A(F)U(M)C(F)A(M)G(F)U(M)U(M)U(F)U( M)C(F)C(M)A(F)A(M)A(F)A(M)A(F)^G(M)^C(M) |
| CH1061 | No. 6025 | U(F)^U(M)^G(F)G(M)A(F)A(M)A(F)A(M)C(F)U(M)G(F) A(F)U(M)G(F)C(M)A(F)U(M)C(F)C(M)G(F)A(M) | No. 6727 | U(F)^C(M)^G(F)G(M)A(F)U(M)G(F)C(M)A(F)U(M)C(M)A(F)G (M)U(F)U(M)U(F)U(M)C(F)C(M)A(F)A(M)^A(F)^A(M) |
| CH1062 | No. 6026 | U(F)^G(M)^G(F)A(M)A(F)A(M)A(F)C(M)U(F)G(M)A(F) U(F)G(M)C(F)A(M)U(F)C(M)C(F)G(M)A( F)U(M) | No. 6728 | A(F)^U(M)^C(F)G(M)G(F)A(M)U(F)G(M)C(F)A(M)U(M)C(F)A (M)G(F)U(M)U(F)U(M)U(F)C(M)C(F)A(M)^A(F)^A(M) |
| CH1063 | No. 6027 | G(F)^G(M)^A(F)A(M)A(F)A(M)C(F)U(M)G(F)A(M)U(F) G(F)C(M)A(F)U(M)C(F)C(M)G(F)A(M)U(F)G(M) | No. 6729 | C(F)^A(M)^U(F)C(M)G(F)G(M)A(F)U(M)G(F)C(M)A(M)U(F)C (M)A(F)G(M)U(F)U(M)U(F)U(M)C(F)C(M)^A(F)^A(M) |
| CH1064 | No. 6028 | G(F)^A(M)^A(F)A(M)A(F)C(M)U(F)G(M)A(F)U(M)G(F) C(F)A(M)U(F)C(M)C(F)G(M)A(F)U(M)G(F)U(M) | No. 6730 | A(F)^C(M)^A(F)U(M)C(F)G(M)G(F)A(M)U(F)G(M)C(M)A(F)U(M)C(F)A(M)G(F)U(M)U(F)U(M)U(F)C(M)^C(F)^A(M) |
| CH1065 | No. 6029 | A(F)^A(M)^A(F)A(M)C(F)U(M)G(F)A(M)U(F)G(M)C(F) A(F)U(M)C(F)C(M)G(F)A(M)U(F)G(M)U(F)A(M) | No. 6731 | U(F)^A(M)^C(F)A(M)U(F)C(M)G(F)G(M)A(F)U(M)G(M)C(F)A (M)U(F)C(M)A(F)G(M)U(F)U(F)U(M)U(F)U(M)^C(F)^C(M) |
| CH1067 | No. 6030 | A(F)^A(M)^C(F)U(M)G(F)A(M)U(F)G(M)C(F)A(M)U(F) C(F)C(M)G(F)A(M)U(F)G(M)U(F)A(M)A(F)A(M) | No. 6732 | U(F)^U(M)^U(F)A(M)C(F)A(M)U(F)C(M)G(F)G(M)A(M)U(F)G (M)C(F)A(M)U(F)C(M)A(F)G(M)U(F)U(M)^U(F)^U(M) |
| CH1068 | No. 6031 | A(F)^C(M)^U(F)G(M)A(F)U(M)G(F)C(M)A(F)U(M)C(F) C(F)G(M)A(F)U(M)G(F)U(M)A(F)A(M)A(F)G(M) | No. 6733 | C(F)^U(M)^U(F)U(M)A(F)C(M)A(F)U(M)C(F)G(M)G(M)A(F)U (M)G(F)C(M)A(F)U(M)C(F)A(M)G(F)U(M)^U(F)^U(M) |
| CH1072 | No. 6032 | A(F)^U(M)^G(F)C(M)A(F)U(M)C(F)C(M)G(F)A(M)U(F) G(F)U(M)A(F)A(M)A(F)G(M)C(F)C(M)A(F)U(M) | No. 6734 | A(F)^U(M)^G(F)G(M)C(F)U(M)U(F)U(M)A(F)C(M)A(M)U(F)C (M)G(F)G(M)A(F)U(M)G(F)C(M)A(F)U(M)^C(F)^A(M) |
| CH1073 | No. 6033 | U(F)^G(M)^C(F)A(M)U(F)C(M)C(F)G(M)A(F)U(M)G(F) U(F)A(M)A(F)A(M)G(F)C(M)C( F)A(M)U(F)G(M) | No. 6735 | C(F)^A(M)^U(F)G(M)G(F)C(M)U(F)U(M)U(F)A(M)C(M)A(F)U (M)C(F)G(M)G(F)A(M)U(F)G(M)C(F)A(M)^U(F)^C(M) |
| CH1076 | No. 6034 | A(F)^U(M)^C(F)C(M)G(F)A(M)U(F)G(M)U(F)A(M)A(F) A(F)G(M)C(F)C(M)A(F)U(M)G(F)C(M)U(F)A(M) | No. 6736 | U(F)^A(M)^G(F)C(M)A(F)U(M)G(F)G(M)G(F)U(M)U(M)U(F)A (M)C(F)A(M)U(F)G(M)G(F)G(M)A(F)U(M)^G(F)^C(M) |
| CH1077 | No. 6035 | U(F)^C(M)^C(F)G(M)A(F)U(M)G(F)U(M)A(F)A(M)A(F) G(F)C(M)C(F)A(M)U(F)G(M)C(F)U(M)A(F)A(M) | No. 6737 | U(F)^U(M)^A(F)G(M)C(F)A(M)U(F)G(M)G(F)C(M)U(M)U(F)U (M)A(F)C(M)A(F)U(M)C(F)G(M)G(F)A(M)^UG(M) |
| CH1078 | No. 6036 | C(F)^C(M)^G(F)A(M)U(F)G(M)U(F)A(M)A(F)A(M)G(F) C(F)C(M)A(F)U(M)G(F)C(M)U(F)A(M)A(F)G(M) | No. 6738 | C(F)^U(M)^U(F)A(M)G(F)C(M)A(F)U(M)G(F)C(M)C(M)U(F)U (M)U(F)A(M)C(F)A(M)U(F)C(M)G(F)G(M)^A(F)^U(M) |
| CH1083 | No. 6037 | G(F)^(M)^A(F)A(M)A(F)G(M)C(F)C(M)A(F)U(M)G(F) C(F)U(M)A(F)A(M)G(F)G(M)U(F)G(M)G(F)U(M) | No. 6739 | A(F)^C(M)^C(F)A(M)C(F)C(M)U(F)U(M)A(F)G(M)C(M)A(F)U (M)G(F)G(M)C(F)U(M)U(F)U(M)A(F)C(M)^A(F)^U(M) |
| CH1084 | No. 6038 | U(F)^A(M)^A(F)A(M)G(F)C(M)C(F)A(M)U(F)G(M)C(F) U(F)A(M)A(F)G(M)G(F)U(M)G(F)G(M)U(F)U(M) | No. 6740 | A(F)^A(M)^C(F)C(M)A(F)C(M)C(F)U(M)U(F)A(M)G(M)C(F)A (M)U(F)G(M)G(F)C(M)U(F)U(M)U(F)A(M)^C(F)^A(M) |
| CH1085 | No. 6039 | A(F)^A(M)^A(F)G(M)C(F)C(M)A(F)U(M)G(F)C(M)U(F) A(F)A(M)G(F)G(M)U(F)G(M)G(F)U(M)U(F)U(M) | No. 6741 | A(F)^A(M)^A(F)C(M)C(F)A(M)C(F)C(M)U(F)U(M)A(M)G(F)C (M)A(F)U(M)G(F)G(M)C(F)U(M)U(F)U(M)^A(F)^C(M) |
| CN1086 | No. 6040 | A(F)^A(M)^G(F)C(M)C(F)A(M)U(F)G(M)C(F)U(M)A(F) A(F)G(M)G(F)U(M)G(F)G(M)U(F)U(M)U(F)U(M) | No. 6742 | A(F)^A(M)^A(F)A(M)C(F)C(M)A(F)C(M)C(F)U(M)U(M)A(F)G (M)C(F)A(M)U(F)G(M)G(F)C(M)U(F)U(M)^U(F)^A(M) |
| CH1087 | No. 6041 | A(F)^G(M)^C(F)C(M)A(F)U(M)G(F)C(M)U(F)A(M)A(F) G(F)G(M)U(F)G(M)G(F)U(M)U(F)U(M)U(F)C(M) | No. 6743 | G(F)^A(M)^A(F)A(M)A(F)C(M)C(F)A(M)C(F)C(M)U(M)U(F)A (M)G(F)C(M)A(F)U(M)G(F)G(M)C(F)U(M)^U(M) |
| CH1088 | No. 6042 | G(F)^C(M)^C(F)A(M)U(F)G(M)C(F)U(M)A(F)A(M)G(F) G(F)U(M)G(F)G(M)U(F)U(M)U(F)U(M)C(F)A(M) | No. 6744 | U(F)^G(M)^A(F)A(M)A(F)A(M)C(F)C(M)A(F)C(M)C(M)U(F)U (M)A(F)G(M)C(F)A(M)U(F)G(M)G(F)C(M)^U(F)^U(M) |
| CH1089 | No. 6043 | C(F)^C(M)^A(F)U(M)G(F)C(M)U(F)A(M)A(F)G(M)G(F) U(F)G(M)G(F)U(M)U(F)U(M)U(F)C(M)A(F)G(M) | No. 6745 | C(F)^U(M)^G(F)A(M)A(F)A(M)A(F)C(M)C(F)A(M)C(M)C(F)U (M)U(F)A(M)G(F)C(M)A(F)U(M)G(F)G(M)^C(F)^U(M) |
| CH1090 | No. 6044 | C(F)^A(M)^U(F)G(M)C(F)U(M)A(F)A(M)G(F)G(M)U(F) G(F)G(M)U(F)U(M)U(F)U(M)C(F)A(M )G(F)A(M) | No. 6746 | U(F)^C(M)^U(F)G(M)A(F)A(M)A(F)A(M)C(F)C(M)A(M)C(F)C (M)U(F)U(M)A(F)G(M)C(F)A(M)U(F)G(M)^G(F)^C(M) |

### [Table 37]

**Table 3-17**

| Double stranded nucleic acid No. GH1092 | SEQ ID NO. | Sense strand sequence (5'→3') | SEQ ID NO. | Antisense strand sequence (5'→3') |
|---|---|---|---|---|
| | No. 6045 | U(F)^G(M)^C(F)U(M)A(F)A(M)G(F)G(M)U(F)G(M)G(F) U(F)U(M)U(F)U(M)C(F)A(M)G(F)A(M)U(F)U(M) | No. 6747 | A(F)^A(M)^U(F)C(M)U(F)G(M)U(F)A(M)A(F)A(M)C(M)C(F)A( M)C(F)C(M)U(F)U(M)A(F)G(M)C(F)A(M)^U(F)^G(M) |
| CH1093 | No. 6046 | G(F)^C(M)^U(F)A(M)A(F)G(M)G(F)U(M)G(F)G(M)U(F) UF)U(M)U(F)C(M)A(F)G(M)A(F)U(M)U(F)C(M) | No. 6748 | G(F)^A(M)^A(F)U(M)C(F)U(M)G(F)A(M)A(F)A(M)A(M)C(F)C (M)A(F)C(M)C(F)U(M)U(F)A(M)G(F)C(M)^A(F)^U(M) |
| CH1095 | No. 6047 | U(F)^A(M)^A(F)G(M)G(F)U(M)G(F)G(M)U(F)U(M)U(F) U(F)C(M)A(F)G(M)A(F)U(M)U(F)C(M)C(F)A(M) | No. 6749 | U(F)^G(M)^G(F)A(M)A(F)U(M)C(F)U(M)G(F)A(M)A(M)A(F)A( M)C(F)C(M)A(F)C(M)C(F)U(M)U(F)A(M)^G(F)^C(M) |
| CH1096 | No. 6048 | A(F)^A(M)^G(F)G(M)U(F)G(M)G(F)U(M)U(F)U(M)U(F) C(F)A(M)G(F)A(M)U(F)U(M)C(F)C(M)A(F)C(M) | No. 6750 | G(F)^U(M)^G(F)G(M)A(F)A(M)U(F)C(M)U(F)G(M)A(M)A(F)A (M)A(F)C(M)C(F)A(M)C(F)C(M)U(F)U(M)^A(F)^G(M) |
| CH1097 | No. 6049 | A(F)^G(M)^G(F)U(M)G(F)G(M)U(F)U(M)U(F)U(M)C(F) A(F)G(M)A(F)U(M)U(F)C(M)C(F)A(M)C(F)A(M) | No. 6751 | U(F)^G(M)^U(F)G(M)G(F)A(M)A(F)U(M)C(F)U(M)G(M)A(F)A (M)A(F)A(M)C(F)C(M)A(F)C(M)C(F)U(M)^U(F)^A(M) |
| CH1099 | No. 6050 | G(F)^U(M)^G(F)G(M)U(F)U(M)U(F)U(M)C(F)A(M)G(F) A(F)U(M)U(F)C(M)C(F)A(M)C(F)A(M)C(F)A(M) | No. 6752 | U(F)^G(M)^U(F)G(M)U(F)G(M)G(F)A(M)A(F)U(M)C(M)U(F)G (M)A(F)A(M)A(F)A(M)C(F)C(M)A(F)C(M)^C(F)^U(M) |
| CH1100 | No. 6051 | U(F)^G(M)^G(F)U(M)U(F)U(M)U(F)C(M)A(F)G(M)A(F) U(F)U(M)C(F)C(M)A(F)C(M)A(F)C(M)A(F)A(M) | No. 6753 | U(F)^U(M)^G(F)U(M)G(F)U(M)G(F)G(M)A(F)A(M)U(M)C(F)U (M)G(F)A(M)A(F)A(M)A(F)C(M)C(F)A(M)^C(F)^C(M) |
| GH1101 | No. 6052 | G(F)^G(M)^U(F)U(M)U(F)U(M)C(F)A(M)G(F)A(M)U(F) U(F)C(M)C(F)A(M)C(F)A(M)C(F)A(M)A(F)A(M) | No. 6754 | U(F)^U(M)^U(F)G(M)U(F)G(M)U(F)G(M)G(F)A(M)A(M)U(F)C (M)U(F)G(M)A(F)A(M)A(F)A(M)C(F)C(M)^A(F)^C(M) |
| CH1102 | No. 6053 | G(F)^U(M)^U(F)U(M)U(F)C(M)A(F)G(M)A(F)U(M)U(F) C(F)C(M)A(F)C(M)A(F)C(M)A(F)A(M)A(F)A(M) | No. 6755 | U(F)^U(M)^U(F)U(M)G(F)U(M)G(F)U(M)G(F)G(M)A(M)A(F)U( M)C(F)U(M)G(F)A(M)A(F)A(M)A(F)C(M)^C(F)^A(M) |
| CH1103 | No. 6054 | U(F)^U(M)^U(F)U(M)C(F)A(M)G(F)A(M)U(F)U(M)C(F) C(F)A(M)C(F)A(M)C(F)A(M)A(F)A(M)A(F)U(M) | No. 6756 | A(F)^U(M)^U(F)U(M)U(F)G(M)U(F)G(M)U(F)G(M)G(M)A(F)A( M)U(F)C(M)U(F)G(M)A(F)A(M)A(F)A(M)^C(F)^C(M) |
| CH1104 | No. 6055 | U(F)^U(M)^U(F)C(M)A(F)G(M)A(F)U(M)U(F)C(M)C(F) A(F)C(M)A(F)C(M)A(F)A(M)A(F)A(M)U(F)G(M) | No. 6757 | C(F)^A(M)^U(F)U(M)U(F)U(M)G(F)U(M)G(F)U(M)G(M)G(F)A (M)A(F)U(M)C(F)U(M)G(F)A(M)A(F)A(M)^A( F)^C(M) |
| CH1105 | No. 6056 | U(F)^U(M)^C(F)A(M)G(F)A(M)U(F)U(M)C(F)C(M)A(F) C(F)A(M)C(F)A(M)A(F)A(M)A(F)U(M)G(F)U(M) | No. 6758 | A(F)^ C(M)^A(F)U(M)U(F)U(M)U(F)G(M)U(F)G(M)U(M)G(F)G (M)A(F)A(M)U(F)C(M)U(F)G(M)A(F)A(M)^A(F)^A(M) |
| CH1106 | No. 6057 | U(F)^C(M)^A(F)G(M)A(F)U(M)U(F)C(M)C(F)A(M)C(F) A(F)C(M)A(F)A(M)A(F)A(M)U(F)G(M)U(F)C(M) | No. 6759 | G(F)^A(M)^C(F)A(M)U(F)U(M)U(F)U(M)G(F)U(M)G(M)U(F)G (M)G(F)A(M)A(F)U(M)C(F)U(M)G(F)A(M)^A(F)^A(M) |
| CH1107 | No. 6058 | C(F)^A(M)^G(F)A(M)U(F)U(M)C(F)C(M)A(F)C(M)A(F) C(F)A(M)A(F)A(M)A(F)U(M)G(F)U(M)C(F)A(M) | No. 6760 | U(F)^G(M)^A(F)C(M)A(F)U(M)U(F)U(M)U(F)G(M)U(M)G(F)U( M)G(F)G(M)A(F)A(M)U( F)C(M)U(F)G(M)^A(F)^A(M) |
| CH1109 | No. 6059 | G(F)^A(M)^U(F)U(M)C(F)C(M)A(F)C(M)A(F)C(M)A(F) A(F)A(M)A(F)U(M)G(F)U(M)C(F)A(M)C(F)A(M) | No. 6761 | U(F)^G(M)^U(F)G(M)A(F)C(M)A(F)U(M)U(F)U(M)U(M)G(F)U( M)G(F)U(M)G(F)G(M)A(F)A(M)U(F)C(M)^U(F)^G(M) |
| CH1111 | No. 6060 | U(F)^U(M)^C(F)C(M)A(F)C(M)A(F)C(M)A(F)A(M)A(F) A(F)U(M)G(F)U(M)C(F)A(M)C(F)A(M)C(F)U(M) | No. 6762 | A(F)^G(M)^U(F)G(M)U(F)G(M)A(F)C(M)A(F)U(M)U(M)U(F)U( M)G(F)U(M)G(F)U(M)G(M)A(F)A(M)^U(F)^C(M) |
| CH1112 | No. 6061 | U(F)^C(M)^C(F)A(M)C(F)A(M)C(F)A(M)A(F)A(M)A(F) U(F)G(M)U(F)C(M)A(F)C(M)A(F)C(M)U(F)U(M) | No. 6763 | A(F)^A(M)^G(F)U(M)G(F)U(M)G(F)A(M)C(F)A(M)U(M)U(F)U( M)U(F)G(M)U(F)G(M)U(F)G(M)G(F)A(M)^A(F)^U(M) |
| CH1113 | No. 6062 | C(F)^C(M)^ A(F)C(M)A(F)C(M)A(F)A(M)A(F)A(M)U(F) G(F)U(M)C(F)A(M)C(F)A(M)C(F)U(M)U(F)G(M) | No. 6764 | C(F)^A(M)^A(F)G(M)U(F)G(M)U(F)G(M)A(F)C(M)A(M)U(F)U (M)U(F)U(M)G(F)U(M)G(F)U(M)G(F)G(M)^A(F)^A(M) |
| CH1114 | No. 6063 | C(F)^ A(M)^C(F)A(M)C(F)A(M)A(F)A(M)A(F)U(M)G(F) U(F)C(M)A(F)C(M)A(F)C(M)U(F)U(M)G(F)U(M) | No. 6765 | A(F)^C(M)^A(F)A(M)G(F)U(M)G(F)U(M)G(F)A(M)C(M)A(F)U (M)U(F)U(M)U(F)G(M)U(F)G(M)U(F)G(M)U(F)G(M)^G(F)^A(M) |
| CH1115 | No. 6064 | A(F)^C(M)^A(F)C(M)A(F)A(M)A(F)A(M)U(F)G(M)U(F) C(F)A(M)C(F)A(M)C(F)U(M)U(F)G(M)U(F)U(M) | No. 6766 | A(F)^A(M)^C(F)A(M)A(F)G(M)U(F)G(M)U(F)G(M)A(M)C(F)A (M)U(F)U(M)U(F)U(M)G(F)U(M)G(F)U(M)^G(F)^G(M) |
| CH1116 | No. 6065 | C(F)^A(M)^C(F)A(M)A(F)A(M)A(F)U(M)G(F)U(M)C(F) A(F)C(M)A(F)C(M)U(F)U(M)G(F)U(M)U(F)U(M) | No. 6767 | A(F)^A(M)^A(F)C(M)A(F)A(M)G(F)U(M)G(F)U(M)G(M)A(F)C (M)A(F)U(M)U(F)U(M)U(F)G(M)U(F)G(M)^U(F)^G(M) |
| CH1118 | No. 6066 | C(F)^A(M)^A(F)A(M)A(F)U(M)G(F)U(M)C(F)A(M)C(F) A(F)C(M)U(F)U(M)G(F)U(M)U(F)U(M)C(F)U(M) | No. 6768 | A(F)^G(M)^A(F)A(M)A(F)C(M)A(F)A(M)G(F)U(M)G(M)U(F)G (M)A(F)C(M)A(F)U(M)U(F)U(M)U(F)G(M)^U(F)^G(M) |
| CH1120 | No. 6067 | A(F)^A(M)^A(F)U(M)G(F)U(M)C(F)A(M)C(F)A(M)C(F) U(F)U(M)G(F)U(M)U(F)U(M)C(F)U(M)U(F)G(M) | No. 6769 | C(F)^A(M)^A(F)G(M)A(F)A(M)A(F)C(M)A(F)A(M)G(M)U(F)G (M)U(F)G(M)A(F)C(M)A(F)U(M)U(F)U(M)^U(F)^G(M) |
| CH1121 | No. 6068 | A(F)^A(M)^U(F) G(M)U(F)C(M)A(F)C(M)A(F)C(M)U(F) U(F)G(M)U(F)U(M)U(F)C(M)U(F)U(M)G(F)U(M) | No. 6770 | A(F)^C(M)^A(F)A(M)G(F)A(M)A(F)A(M)C(F)A(M)A(M)G(F)U( M)G(F)U(M)G(F)A(M)C(F)A(M)U(F)U(M)^U(F)^U(M) |
| CH1122 | No. 6069 | A(F)^U(M)^G(F)U(M)C(F)A(M)C(F)A(M)C(F)U(M)U(F) G(F)U(M)U(F)U(M)C(F)U(M)U(F)G(M)U(F)U(M) | No. 6771 | A(F)^A(M)^C(F)A(M)A(F)G(M)A(F)A(M)A(F)C(M)A(M)A(F)G( M)U(F)G(M)U(F)G(M)A(F)C(M)A(F)U(M)^U(F)^U(M) |
| CH1124 | No. 6070 | G(F)^U(M)^C(F)A(M)C(F)A(M)C(F)U(M)U(F)G(M)U(F) U(F)U(M)C(F)U(M)U(F)G(M)U(F)U(M)C(F)A(M) | No. 6772 | U(F)^G(M)^A(F)A(M)C(F)A(M)A(F)G(M)A(F)A(M)A(M)C(F)A( M)A(F)G(M)U(F)G(M)U(F)G(M)A(F)C(M)^A(F)^U(M) |
| CH1125 | No. 6071 | U(F)^C(M)^A(F)C(M)A(F)C(M)U(F)U(M)G(F)U(M)U(F) U(F)C(M)U(F)U(M)G(F)U(M)U(F)C(M)A(F)U(M) | No. 6773 | A(F)^U(M)^G(F)A(M)A(F)C(M)A(F)A(M)G(F)A(M)A(M)A(F)C( M)A(F)A(M)G(F)U(M)G(F)U(M)G(F)A(M)^C(F)^A(M) |
| CH1127 | No. 6072 | A(F)^C(M)^A(F)C(M)U(F)U(M)G(F)U(M)U(F)U(M)C(F) U(F)U(M)G(F)U(M)U(F)C(M)A(F)U(M)C(F)C(M) | No. 6774 | G(F)^G(M)^A(F)U(M)G(F)A(M)A(F)C(M)A(F)A(M)G(M)A(F)A (M)A(F)C(M )A(F)A(M)G(F)U(M)G(F)U(M)^G(F)^A(M) |
| CH1128 | No. 6073 | C(F)^A(M)^C(F)U(M)U(F)G(M)U(F)U(M)U(F)C(M)U(F) U(F)G(M)U(F)U(M)C(F)A(M)U(F)C(M)C(F)A(M) | No. 6775 | U(F)^G(M)^G(F)A(M)U(F)G(M)A(F)A(M)C(F)A(M)A(M)G(F)A (M)A(F)A(M)C(F)A(M)A(F)G(M)U(F)G(M)^U(F)^G(M) |
| CH1129 | No. 6074 | A(F)^C(M)^U(F)U(M)G(F)U(M)U(F)U(M)C(F)U(M)U(F) G(F)U(M)U(F)C(M)A(F)U(M)C(F)C(M)A(F)A(M) | No. 6776 | U(F)^U(M)^G(F)G(M)A(F)U(M)G(F)A(M)A(F)C(M)A(M)A(F)G (M)A(F)A(M)A(F)C(M)A(F)A(M)G(F)U(M)^G(F)^U(M) |
| CH1130 | No. 6075 | C(F)^U(M)^U(F)G(M)U(F)U(M)U(F)C(M)U(F)U(M)G(F) U(F)U(M)C(F)A(M)U(F)C(M)C(F)A(M)A(F)G(M) | No. 6777 | C(F)^U(M)^U(F)G(M)G(F)A(M)U(F)G(M)A(F)A(M)C(M)A(F)A (M)G(F)A(M)A(F)A(M)C(F)A(M)A(F)G(M)^U(F)^G(M) |
| CH1131 | No. 6076 | U(F)^U(M)^G(F)U(M)U(F)U(M)C(F)U(M)U(F)G(M)U(F) U(F)C(M)A(F)U(M)C(F)C(M)A(F)A(M)G(F)G(M) | No. 6778 | C(F)^C(M)^U(F)U(M)G(F)G(M)A(F)U(M)G(F)A(M)A(M)C(F)A (M)A(F)G(M)A(F)A(M)A(F)C(M)A(F)A(M)^G(F)^U(M) |
| CH1133 | No. 6077 | G(F)^U(M)^U(F)U(M)C(F)U(M)U(F)G(M)U(F)U(M)C(F) A(F)U(M)C(F)C(M)A(F)A(M)G(F)G(M)A(F)A(M) | No. 6779 | U(F)^U(M)^C(F)C(M)U(F)U(M)G(M)A(F)G(M)A(F)U(M)G(M)A(F)A (M)G(F)A(M)A(F)G(M)A(F)A(M)A(F)C(M)^A(F)^A(M) |
| CH1134 | No. 6078 | U(F)^U(M)^U(F)C(M)U(F)U(M)G(F)U(M)U(F)C(M)A(F) U(F)C(M)C(F)A(M)A(F)G(M)G(F)A(M)A(F)C(M) | No. 6780 | G(F)^U(M)^U(F)C(M)C(F)U(M)U(F)G(M)G(F)A(M)U(M)G(F)A (M)A(F)C(M)A(F)A(M)G(F)A(M)A(F)A(M)^C(F)^A(M) |
| CH1135 | No. 6079 | U(F)^U(M)^C(F)U(M)U(F)G(M)U(F)U(M)C(F)A(M)U(F) C(F)C(M)A(F)A(M)G(F)G(M)A(F)A(M)C(F)C(M) | No. 6781 | G(F)^G(M)^U(F)U(M)C(F)C(M)U(F)U(M)G(F)G(M)A(M)U(F)G (M)A(F)A(M)C(F)A(M)A(F)G(M)A(F)A(M)^A(F)^C(M) |
| CH1136 | No. 6080 | U(F)^C(M)^U(F)U(M)G(F)U(M)U(F)C(M)A(F)U(M)C(F) C(F)A(M)A(F)G(M)G(F)A(M)A(F)C(M)C(F)U(M) | No. 6782 | A(F)^G(M)^G(F)U(M)U(F)C(M)C(F)U(M)U(F)G(M)G(M)A(F)U (M)G(F)A(M)A(F)C(M)A(F)A(M)G(F)A(M)^A(F)^A(M) |
| CH1137 | No. 6081 | C(F)^U(M)^U(F)G(M)U(F)U(M)C(F)A(M)U(F)C(M)C(F) A(F)A(M)G(F)G(M)A(F)A(M)C(F)C(M)U(F)A(M) | No. 6783 | U(F)^A(M)^G(F)G(M)U(F)U(M)C(F)C(M)U(F)U(M)G(M)G(F)A (M)U(F)G(M)A(F)A(M)C(F)A(M)A(F)G(M)^A(F)^A(M) |
| CH1138 | No. 6082 | U(F)^U(M)^G(F)U(M)U(F)C(M)A(F)U(M)C(F)C(M)A(F) A(F)G(M)G(F)A(M)A(F)C(M)C(F)U(M)A(F)A(M) | No. 6784 | U(F)^U(M)^A(F)G(M)G(F)U(M)U(F)C(M)C(F)U(M)U(M)G(F)G (M)A(F)U(M)G(F)A(M)A(F)C(M)A(F)A(M)^G(F)^A(M) |
| CH1139 | No. 6083 | U(F)^G(M)^U(F)U(M)C(F)A(M)U(F)C(M)C(F)A(M)A(F) G(F)G(M)A(F)A(M)C(F)C(M)U(F)A(M)A(F)U(M) | No. 6785 | A(F)^U(M)^U(F)A(M)G(F)G(M)U(F)U(M)C(F)C(M)U(M)U(F)G (M)G(F)A(M)U(F)G(M)A(F)A(M)C(F)A(M)^A(F)^G(M) |
| CH1140 | No. 6084 | G(F)^U(M)^U(F)C(M)A(F)U(M)C(F)C(M)A(F)A(M)G(F) G(F)A(M)A(F)C(M)C(F)U(M)A(F)A(M)U(F)U(M) | No. 6786 | A(F)^A(M)^U(F)U(M)A(F)G(M)G(F)U(M)U(F)C(M)C(M)U(F)U( M)G(F)G(M)A(F)U(M)G(F)A(M)A(F)C(M)^A(F)^A(M) |

### [Table 38]

**Table 3-18**

| Double stranded nucleic acid No. | SEQ ID NO. | Sense strand sequence (5'→3') | SEQ ID NO. | Antisense strand sequence (5'→3') |
|---|---|---|---|---|
| CH1141 | No. 6085 | U(F)^U(M)^C(F)A(M)U(F)C(M)C(F)A(M)A(F)G(M)G(F) A(F)A(M)C(F)C(M)U(F)A(M)A(F)U(M)U(F)G(M) | No. 6787 | C(F)^A(M)^A(F)U(M)U(F)A(M)G(F)G(M)U(F)U(M)C(M)C(F)U (M)U(F)G(M)G(F)A(M)U(F)G(M)A(F)A(M)^C(F)^A(M) |
| CH1142 | No. 6086 | U(F)^C(M)^A(F)U(M)C(F)C(M)A(F)A(M)G(F)G(M)A(F) A(F)C(M)C(F)U(M)A(F)A(M)U(F)U(M)G(F)A(M) | No. 6788 | U(F)^C(M)^A(F)A(M)U(F)U(M)A(F)G(M)G(F)U(M)U(M)C(F)C (M)U(F)U(M)G(F)G(M)A(F)U(M)G(F)A(M)^A(F)^C(M) |
| CH1143 | No. 6087 | C(F)^A(M)^U(F)C(M)C(F)A(M)A(F)G(M)G(F)A(M)A(F) C(F)C(M)U(F)A(M)A(F)U(M)U(F)G(M)A(F)A(M) | No. 6789 | U(F)^U(M)^C(F)A(M)A(F)U(M)U(F)A(M)G(F)G(M)U(M)U(F)C( M)C(F)U(M)U(F)G(M)G(F)A(M)U(F)G(M)^A(F)^A(M) |
| CH1144 | No. 6088 | A(F)^U(M)^C(F)C(M)A(F)A(M)G(F)G(M)A(F)A(M)C(F) C(F)U(M)A(F)A(M)U(F)U(M)G(F)A(M)A(F)A(M) | No. 6790 | U(F)^U(M)^U(F)C(M)A(F)A(M)U(F)U(M)A(F)G(M)G(M)U(F)U( M)C(F)C(M)U(F)U(M)G(F)G(M)A(F)U(M)^G(F)^A(M) |
| CH1145 | No. 6089 | U(F)^C(M)^C(F)A(M)A(F)G(M)G(F)A(M)A(F)C(M)C(F) U(F)A(M)A(F)UM)U(F)G(M)A(F)A(M)A(F)U(M) | No. 6791 | A(F)^U(M)^U(F)U(M)C(F)A(M)A(F)U(M)U(F)A(M)G(M)G(F)U( M)U(F)C(M)C(F)U(M)U(F)G(M)G(F)A(M)^U(F)^G(M) |
| CH1146 | No. 6090 | C(F)^C(M)^A(F)A(M)G(F)G(M)A(F)A(M)C(F)C(M)U(F) A(F)A(M)U(F)U(M)G(F)A(M)A(F)A(M)U(F)U(M) | No. 6792 | A(F)^A(M)^U(F)U(M)U(F)C(M)A(F)A(M)U(F)U(M)A(M)G(F)G( M)U(F)U(M)C(F)C(M)U(F)U(M)G(F)G(M)^A(F)^U(M) |
| CH1147 | No. 6091 | C(F)^A(M)^A(F)G(M)G(F)A(M)A(F)C(M)C(F)U(M)A(F) A(F)U(M)U(F)G(M)A(F)A(M)A(F)U(M)U(F)U(M) | No. 6793 | A(F)^A(M)^A(F)U(M)U(F)U(M)C(F)A(M)A(F)U(M)U(M)A(F)G( (M)G(F)U(M)U(F)C(M)C(F)U(M)U(F)G(M)^G(F)^A(M) |
| CH1148 | No. 6092 | A(F)^A(M)^G(F)G(M)A(F)A(M)C(F)C(M)U(F)A(M)A(F) U(F)U(M)G(F)A(M)A(F)A(M)U(F)U(M)U(F)A(M) | No. 6794 | U(F)^A(M)^A(F)A(M)U(F)U(M)U(F)C(M)A(F)A(M)U(M)U(F)A( M)G(F)G(M)U(F)U(M)C(F)C(M)U(F)U(M)^G(F)^G(M) |
| CH1149 | No. 6093 | A(F)^G(M)^G(F)A(M)A(F)C(M)C(F)U(M)A(F)A(M)U(F) U(F)G(M)A(F)A(M)A(F)U(M)U(F)U(M)A(F)A(M) | No. 6795 | U(F)^U(M)^A(F)A(M)A(F)U(M)U(F)U(M)C(F)A(M)A(M)U(F)U( M)A(F)G(M)G(F)U(M)U(F)C(M)C(F)U(M)^U(F)^G(M) |
| CH1150 | No. 6094 | G(F)^G(M)^A(F)A(M)C(F)C(M)U(F)A(M)A(F)U(M)U(F) G(F)A(M)A(F)A(M)U(F)U(M)U(F)A(M)A(F)A(M) | No. 6796 | U(F)^U(M)^U(F)A(M)A(F)A(M)U(F)U(M)U(F)C(M)A(M)A(F)U( M)U(F)A(M)G(F)G(M)U(F)U(M)C(F)C(M)^U(F)^U(M) |
| CH1151 | No. 6095 | G(F)^A(M)^A(F)C(M)C(F)U(M)A(F)A(M)U(F)U(M)G(F) A(F)A(M)A(F)U(M)U(F)U(M)A(F)A(M)A(F)A(M) | No. 6797 | U(F)^U(M)^U(F)U(M)A(F)A(M)A(F)U(M)U(F)U(M)C(M)A(F)A( M)U(F)U(M)A(F)G(M)G(F)U(M)U(F)C(M)^C(F)^U(M) |
| CH1155 | No. 6096 | C(F)^U(M)^A(F)A(M)U(F)U(M)G(F)A(M)A(F)A(M)U(F)U (F)U(M)A(F)A(M)A(F)A(M)A(F)U(M)A(F)A(M) | No. 6798 | U(F)^U(M)^A(F)U(M)U(F)U(M)U(F)U(M)A(F)A(M)A(M)U(F)U M)U(F)C(M)A(F)A(M)U(F)U(M)A(F)G(M)^G(F)^U(M) |
| CH1167 | No. 6097 | U(F)^A(M)^A(F)A(M)A(F)A(M)U(F)A(M)A(F)A(M)G(F)G (F)U(M)A(F)C(M)U(F)G(M)A(F)A(M)U(F)U(M) | No. 6799 | A(F)^A(M)^U(F)U(M)C(F)A(M)G(F)U(M)A(F)G(M)C(M)U(F)U( M)U(F)A(M)U(F)U(M)U(F)U(M)U(F)A(M)^A(F)^A(M) |
| CH1168 | No. 6098 | A(F)^A(M)^A(F)A(M)A(F)U(M)A(F)A(M)A(F)G(M)C(F)U (F)A(M)C(F)U(M)G(F)A(M)A(F)U(M)U(F)U(M) | No. 6800 | A(F)^A(M)^A(F)U(M)U(F)C(M)A(F)G(M)U(F)A(M)G(M)C(F)U( M)U(F)U(M)A(F)U(M)U(F)U(M)U(F)U(M)^A(F)^A(M) |
| CH1169 | No. 6099 | A(F)^A(M)^A(F)A(M)U(F)A(M)A(F)A(M)G(F)C(M)U(F)A (F)C(M)U(F)G(M)A(F)A(M)U(F)U(M)U(F)A(M) | No. 6801 | U(F)^A(M)^A(F)A(M)U(F)U(M)C(F)A(M)G(F)U(M)A(M)G(F)C( M)U(F)U(M)U(F)A(M)U(F)U(M)U(F)U(M)^U(F)^A(M) |
| CH1171 | No. 6100 | A(F)^A(M)^U(F)A(M)A(F)A(M)G(F)C(M)U(F)A(M)C(F) U(F)G(M)A(F)A(M)U(F)U(M)U(F)A(M)U(F)U(M) | No. 6802 | A(F)^A(M)^U(F)A(M)A(F)A(M)U(F)U(M)C(F)A(M)G(M)U(F)A( M)G(F)C(M)U(F)U(M)U(F)A(M)U(F)U(M)^U(F)^U(M) |
| CH1175 | No. 6101 | A(F)^A(M)^G(F)C(M)U(F)A(M)C(F)U(M)G(F)A(M)A(F) U(F)U(M)U(F)A(M)U(F)U(M)G(F)C(M)C(F)G(M) | No. 6803 | C(F)^G(M)^G(F)C(M)A(F)A(M)U(F)A(M)A(F)A(M)U(M)U(F)C (M)A(F)G(M)U(F)A(M)G(F)C(M)U(F)U(M)^U(F)^A(M) |
| CH1176 | No. 6102 | A(F)^G(M)^C(F)U(M)A(F)C(M)U(F)G(M)A(F)A(M)U(F) U(F)U(M)A(F)U(M)U(F)G(M)C(F)C(M)G(F)C(M) | No. 6804 | G(F)^C(M)^G(F)G(M)C(F)A(M)A(F)U(M)A(F)A(M)A(M)U(F)U (M)C(F)A(M)G(F)U(M)A(F)G(M)C(F)U(M)^U(F)^U(M) |
| CH1177 | No. 6103 | G(F)^C(M)^U(F)A(M)C(F)U(M)G(F)A(M)A(F)U(M)U(F) U(F)A(M)U(F)U(M)G(F)C(M)C(F)G(M)C(F)A(M) | No. 6805 | U(F)^G(M)^C(F)G(M)G(F)C(M)A(F)A(M)U(F)A(M)A(M)A(F)U (M)U(F)C(M)A(F)G(M)U(F)A(M)G(F)C(M)^U(F)^U(M) |
| CH1178 | No. 6104 | C(F)^U(M)^A(F)C(M)U(F)G(M)A(F)A(M)U(F)U(M)U(F) A(F)U(M)U(F)G(M)C(F)C(M)G(F)C(M)A(F)C(M) | No. 6806 | G(F)^U(M)^G(F)C(M)G(F)G(M)C(F)A(M)A(F)U(M)A(M)A(F)A (M)U(F)U(M)C(F)A(M)G(F)U(M)A(F)G(M)^C(F)^U(M) |
| CH1179 | No. 6105 | C(F)^U(M)^C(F)U(M)G(F)C(M)C(F)A(M)U(F)G(M)U(F) U(F)G(M)C(F)U(M)A(F)U(M)U(F)G(M)C(F)A(M) | No. 6807 | U(F)^G(M)^C(F)A(M)A(F)U(M)A(F)G(M)C(F)A(M)A(M)C(F)A (M)U(F)G(M)G(F)C(M)A(F)G(M)A(F)G(M)^G(F)^A(M) |
| CH1180 | No. 6106 | G(F)^C(M)^U(F)G(M)G(F)A(M)A(F)U(M)C(F)U(M)U(F) A(F)G(M)A(F)A(M)A(F)A(M)U(F)G(M)G(F)A(M) | No. 6808 | U(F)^C(M)^C(F)A(M)U(F)U(M)U(F)U(M)C(F)U(M)A(M)A(F)G( M)A(F)U(M)U(F)C(M)C(F)A(M)G(F)C(M)^G(F)^A(M) |

The double-stranded nucleic acid in the composition of the present invention can be introduced into cells of a mammal by administering the composition of the present invention to the cells.

The nucleic acid conjugate of the present invention can be introduced into mammalian cells in vivo by known transfection procedures that can be implemented in vivo. By administering the composition of the present invention intravenously to mammals including humans, it is possible to deliver it to the blood vessels, liver, lungs, pancreas and/or kidneys for example, and thereby introduce the double-stranded nucleic acid in the composition of the present invention into cells at the organ or the site.

By introducing the double-stranded nucleic acid in the composition of the present invention into cells at the organ or the site, it is possible to reduce expression of the β2GPI gene in those cells, and thereby treat or prevent β2GPI-associated diseases such as systemic lupus erythematosus (SLE), antiphospholipid antibody syndrome, complications of blood dialysis in end-stage renal failure patients, and arteriosclerosis. The target of administration is a mammal, preferably a human.

The lipid particle-containing composition of the present invention may also be used as a tool to verify the effectiveness of β2GPI gene suppression in an in vivo efficacy evaluation model of a therapeutic or preventative agent for such diseases. The in vivo efficacy evaluation model may be a lupus anticoagulant (LA) test or the like. Like anti-β2GPI antibodies, LA is a kind of anti-phospholipid antibody, and inhibits phospholipid-dependent clotting reactions in collected blood in vitro. LA appears mainly in diseases such as SLE and APS. It has been reported a lot that like anti-β2GPI antibodies, LA is associated with the occurrence or pathology of thrombosis and infertility (Blood, 2003, Vol. 101, No. 5, pp. 1827-1832). Moreover, anti-β2GPI antibodies have also been reported to cause β2GPI-dependent LA activity because they acquire phospholipid-binding activity via β2GPI (Thrombosis and Haemostasis, 1998, Vol. 79, No. 1, pp. 79-86). Furthermore, it has been reported that this β2GPI-dependent LA is strongly correlated to the incidence of disease pathology (Blood, 2004, Vol. 104, No. 12, pp. 3598-3602), so it is thought that a novel and effective treatment method for β2GPI-associated disease could be provided if LA activity could be decreased by suppressing β2GPI expression in blood. In clinical testing, LA can be detected by measuring activated partial thromboplastin time, kaolin clotting time and/or dilute Russell viper venom time (dRVVT).

When the nucleic acid conjugate of the present invention is used as a therapeutic or preventive agent against β2GPI-associated disease, an administration route which is the most effective for the treatment is preferably selected. The route is preferably intravenous administration, subcutaneous administration or intramuscular administration, and more preferably subcutaneous administration.

The dosage differs depending on the condition and age of the subject and the administration route and the like. The dosage may be 0.1 µg to 1,000 mg per day, and preferably 1 to 100 mg per day (based on the amount of the double-stranded nucleic acid).

### Examples

Next, the present invention is explained in detail using Reference Examples, Examples and Test Examples. However, the present invention is not limited to these Examples and Test Examples. The proton nuclear magnetic resonance spectra (1H NMR) given in Reference Examples were measured at 270 MHz, 300 MHz or 400 MHz. Exchangeable protons may not be observed clearly depending on the compound and the measurement conditions. The multiplicity of the signal is shown in a conventional way. The term "br" represents an apparently broad signal.

### Reference Example 1

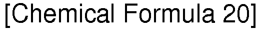

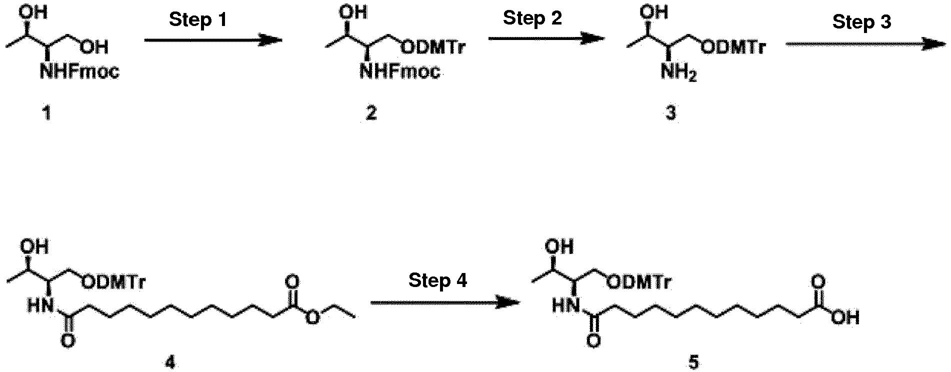

(wherein, Fmoc and DMTr are defined as above.)

### Step 1

(9H-fluoren-9-yl)methyl((2R,3R)-1,3-dihydroxybutan-2-yl)carbamate (Chem-Impex International, Inc., 1.50 g, 4.58 mmol) was dissolved in pyridine (20 mL), 4,4'-dimethoxytrityl chloride (Tokyo Chemical Industry Co., Ltd., 1.71 g, 5.04 mmol) was added under ice cooling, and the mixture was stirred for 2 hours at room temperature. The reaction solution was ice cooled, 10% aqueous citric acid solution was added, and the mixture was extracted with ethyl acetate. The organic layer was washed with saturated saline and dried with anhydrous magnesium sulfate. The solvent was distilled off under reduced pressure, and the residue was purified by silica gel column chromatography (hexane/ethyl acetate = 90/10) to obtain Compound 2 (1.07 g, yield 37%),
ESI-MS m/z: 630 (M + H)+;

### Step 2

Compound 2 obtained in step 1 (1.07 g, 1.699 mmol) was dissolved in N,N-dimethylformamide (10 mL), and piperidine (0.336 mL, 3.40 mmol) was added at room temperature, and the mixture was stirred for 3 hours. Water was added to the reaction solution, which was then extracted with ethyl acetate, and the organic layer was washed with saturated saline and dried with anhydrous magnesium sulfate. The solvent was distilled off under reduced pressure, and the residue was purified by amino silica gel column chromatography (chloroform/methanol = 90/10) to obtain Compound 3 (0.59 g, yield 85%).
ESI-MS m/z: 408 (M + H)+;

### Step 3

Compound 3 obtained in step 2 (0.590 g, 1.45 mmol) was dissolved in N,N-dimethylformamide (8 mL), 12-ethoxy-12-oxododecanoic acid (Tokyo Chemical Industry Co., Ltd., 0.374 g, 1.45 mmol), diisopropylethylamine (0.632 mL, 3.62 mmol) and 2-(1H-benzotriazol-1-yl)-1,1,3,3-tetramethyluronium hexafluorophosphate (Wako Pure Chemicals Industries, Ltd., 0.659 g, 1.74 mmol) were added, and the mixture was stirred overnight at room temperature. Water was added to the reaction solution, which was then extracted twice with ethyl acetate, and the organic layer was washed with saturated saline and dried with anhydrous magnesium sulfate. The solvent was distilled off under reduced pressure, and the residue was purified by amino silica gel column chromatography (hexane/ethyl acetate = 90/10 to 40/60) to obtain Compound 4 (0.430 g, yield 46%).
ESI-MS m/z: 648 (M + H)+;

### Step 4

Compound 4 obtained in step 3 (0.430 g, 0.664 mmol) was dissolved in ethanol (5 mL), and an aqueous lithium hydroxide solution (1.0 mL, 1.99 mol/L) was added, and the mixture was stirred overnight at room temperature. The reaction solution was ice cooled, 10% aqueous citric acid solution was added, the mixture was extracted twice with ethyl acetate. The organic layer was washed with saturated saline and dried with anhydrous magnesium sulfate. The solvent was then distilled off under reduced pressure to obtain Compound 5 as a crude product (0.420 g, crude yield 100%).
ESI-MS m/z: 618(M - H)-; 1H-NMR (CDCl3) δ: 1.12 (d, J = 6.3 Hz, 3H),1.26-1.35 (m, 12H), 1.57-1.67 (m, 4H), 2.23 (t, J = 7.7 Hz, 2H), 2.29 (t, J = 7.4 Hz, 2H), 3.25 (dd, J = 9.6, 3.9 Hz, 1H), 3.37 (dd, J = 9.6, 4.7 Hz, 1H), 3.39-3.41 (m, 1H), 3.79 (s, 6H), 3.91-3.94 (m, 1H), 4.04-4.09 (m, 1H), 6.43 (d, J = 8.8 Hz, 1H), 6.83 (d, J = 8.8 Hz, 4H), 7.21-7.24 (m, 1H), 7.26-7.30 (m, 6H), 7.37-7.39 (m, 2H).

### Reference Example 2

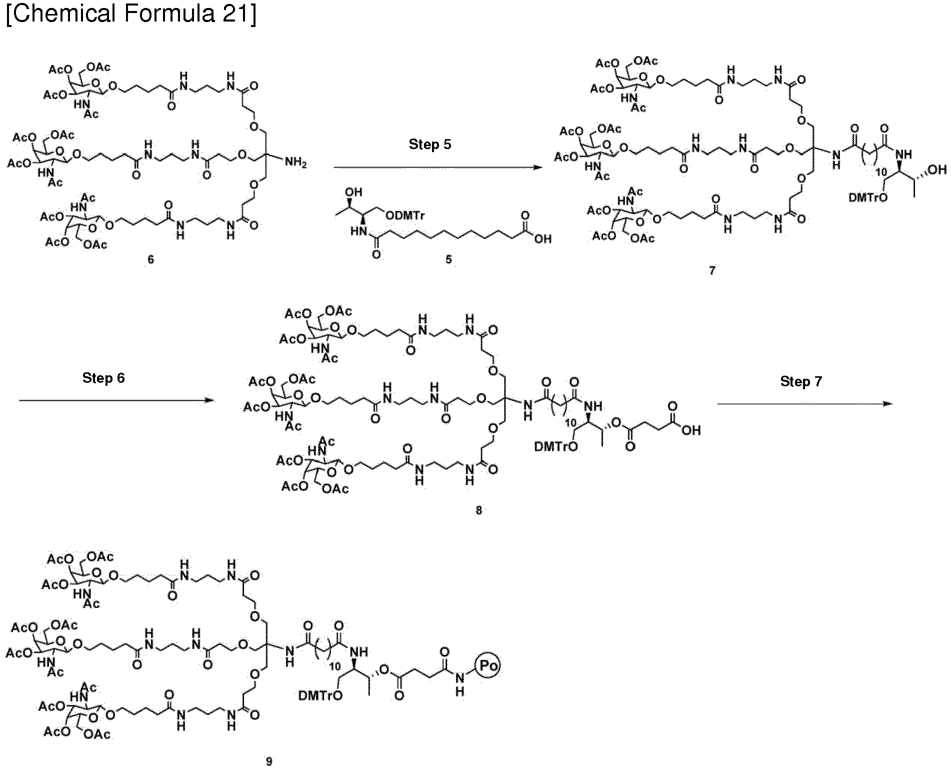

(wherein, Ac and DMTr are defined as above, and Po represents the solid-phase carrier LCAA-CPG.)

### Step 5

Compound 6 (0.119 g, 0.066 mmol) that had been synthesized by a known method (see Journal of the American Chemical Society, Vol. 136, pp. 16958-16961, 2014) was dissolved in THF (2 mL), to which Compound 5 (0.062 g, 0.100 mmol) obtained in step 4 of Example 1, diisopropylethylamine (0.116 mL, 0.663 mmol), 2-(1H-benzotriazol-1-yl)-1,1,3,3-tetramethyluronium hexafluorophosphate (0.050 g, 0.133 mmol) and N,N-dimethylaminopyridine (Nacalai Tesque, Inc., 8.1 mg, 0.133 mmol) was added. The mixture was stirred overnight at room temperature. The reaction solution was ice cooled, 10% aqueous citric acid solution was added, the mixture was extracted with chloroform. The organic layer was washed with saturated saline and dried with anhydrous magnesium sulfate. The solvent was distilled off under reduced pressure, and the residue was purified by amino silica gel column chromatography (methanol/chloroform) to obtain Compound 7 (0.035 g, yield 22%).
ESI-MS m/z: 1048 (M + 2H)2+

### Step 6

Compound 7 obtained in step 5 (0.135 g, 0.056 mmol) was dissolved in dichloromethane (2 mL), succinic acid anhydride (Tokyo Chemical Industry Co., Ltd., 0.011 g, 0.113 mmol) and N,N-dimethylaminopyridine (0.021 g, 0.169 mmol) were added, and the mixture was stirred overnight at room temperature. The reaction solution was ice cooled, 10% aqueous citric acid solution was added, and the mixture was extracted twice with ethyl acetate. The organic layer was washed with saturated saline and dried with anhydrous magnesium sulfate. The solvent was distilled off under reduced pressure to obtain Compound 8 as a crude product (0.110 g, crude yield 78%).
ESI-MS m/z: 1089 (M + 2H)2+;
1H-NMR (DMSO-d6) δ: 1.02 (d, J= 6.3 Hz, 3H), 1.15-1.25 (m, 12H), 1.39-1.55 (m, 22H), 1.77 (s, 9H), 1.88 (d, J = 7.9 Hz, 9H), 1.99 (s, 9H), 2.04 (t, J = 7.1 Hz, 6H), 2.10 (s, 9H), 2.11-2.15 (m, 2H), 2.28 (t, J = 6.5 Hz, 6H), 2.37-2.42 (m, 2H), 2.49-2.53 (m, 2H), 2.83-2.95 (m, 2H), 2.99-3.08 (m, 12H), 3.31-3.45 (m, 6H), 3.48-3.58 (m, 12H), 3.67-3.75 (m, 2H), 3.73 (s, 6H), 3.83-3.92 (m, 3H), 3.97-4.06 (m, 9H), 4.10-4.18 (m, 1H), 4.49 (d, J = 8.4 Hz, 3H), 4.97 (dd, J = 11.3, 3.4 Hz, 3H), 5.09-5.16 (m, 1H), 5.21 (d, J = 3.3 Hz, 3H), 6.84-6.91 (m, 4H), 6.99 (s, 1H), 7.12-7.40 (m, 9H), 7.71-7.89 (m, 10H).

### Step 7

Compound 8 obtained in step 6 (0.050 g, 0.014 mmol), O-(benzotriazol-1-yl)-N,N,N',N'-tetramethyluronium hexafluorophosphate (0.011 g, 0.028 mmol) and diisopropylethylamine (0.098 mL, 0.056 mmol) were dissolved in N,N-dimethylformamide (2 mL), and the solid-phase carrier LCAA-CPG (Sigma-Aldrich Corporation, 0.25 g, 0.014 mmol) was added, and the mixture was stirred overnight at room temperature. The mixture was filtered through frit, washed with dichloromethane, 10% methanol dichloromethane solution and diethyl ether, and reacted with an acetic anhydride/pyridine solution to obtain Compound 9 (17.7 µmol/g, yield 30%). The yield was calculated from an introduction rate onto the solid-phase carrier, which can be determined from the absorption derived from DMTr groups by adding a 1% trifluoroacetic acid/dichloromethane solution to the solid-phase carrier.

### Test Example 1: Measuring knockdown activity of β2GPI mRNA

HepG2 cells (obtained from ATCC, ATCC No. HB-8065), a cell strain derived from human liver cancer, were seeded on 96-well culture plates so as to be 5,000 cells/80 µL/well. MEM medium (Life Technologies Corporation, Catalog No. 11095-098) containing 10% fetal bovine serum (FBS) was used. The double-stranded nucleic acids described in Tables 4-1 to 4-16 and RNAiMax transfection reagent (Life Technologies Corporation, Catalog No. 1401251) were diluted with Opti-MEM medium (Life Technologies Corporation, Catalog No. 11058-021), and 20 µL of the siRNA/RNAiMax mixture was added to each 96-well culture plate so as to be a final concentration of the double-stranded nucleic acid of 100 pmol/L, and the plate was cultured for 24 hours under conditions of 37°C, 5% CO₂. The cells were then washed with PBS (phosphate-buffered saline), and cDNA was synthesized from each plate using a Cells-to-Ct kit (Applied Biosystems, Inc., Catalog No. AM1728) according to the methods described in the attached instruction. 5 µL of this cDNA was added to a MicroAmpOptical 96-well plate (Applied Biosystems, Inc., Catalog No. 4326659), to which 10 µL of TaqMan Gene Expression Master Mix (Applied Biosystems, Inc., Catalog No. 4369016), 3 µL of UltraPure Distilled Water (Life Technologies Corporation, Catalog No. 10977-015), 1 µL of a human β2GPI probe and 1 µL of a human GAPDH probe were further added. Using an ABI7900 HT real time PCR system, real time PCR was performed on the human β2GPI gene and human GAPDH (D-glyceraldehyde-3-phosphate dehydrogenase). GAPDH is a constitutively expressed gene, which was measured as an internal control and used to correct the expressed amount of β2GPI. The amount of β2GPI mRNA obtained when HepG2 cells were treated with the transfection reagent alone without adding siRNA being set to be 1.0, the relative expressed amount of β2GPI mRNA obtained when each siRNA was introduced was calculated. This test was performed three times, and the average values of the relative expressed amounts of β2GPI mRNA are described in Tables 4-1 to 4-16.

### [Table 39]

**Table 4-1**

| Double stranded nucleic acid No. | SEQ ID NO. | Sense strand sequence (5'→3') | SEQ ID NO. | Antisense strand sequence (5'→3') | SEQ ID NO. | Target β2GPI mRNA sequence | Relative expressed amount of β2GPI |
|---|---|---|---|---|---|---|---|
| AH0033 | No. 33 | GUAGUGCCAGUGUGACUCAUC | No. 1213 | UGAGUCACACUGGCACUACCA | No. 2393 | GTAGTGCCAGTGTGACTCA | 0.172 |
| AH0034 | No. 34 | UAGUGCCAGUGUGACUCAUCC | No. 1214 | AUGAGUCACACUGGCACUACC | No. 2394 | TAGTGCCAGTGTGACTCAT | 0. 185 |
| AH0035 | No. 35 | AGUGCCAGUGUGACUCAUCCA | No. 1215 | GAUGAGUCACACUGGCACUAC | No. 2395 | AGTGCCAGTGTGACTCATC | 0.178 |
| AH0036 | No. 36 | GUGCCAGUGUGACUCAUCCAC | No. 1216 | GGAUGAGUCACACUGGCACUA | No. 2396 | GTGCCAGTCTGACTCATCC | 0.191 |
| AH0037 | No. 37 | UGCCAGUGUGACUCAUCCACA | No. 1217 | UGGAUGAGUCACACUGGCACU | No. 2397 | TGCCAGTGTGACTCATCCA | 0.148 |
| AH0038 | No. 38 | GCCAGUGUGACUCAUCCACAA | No. 1218 | GUGGAUGAGUCACACUGGCAC | No. 2398 | GCCAGTGTGACTCATCCAC | 0.166 |
| AH0039 | No. 39 | CCAGUGUGACUCAUCCACAAU | No. 1219 | UGUGGAUGAGUCACACUGGCA | No. 2399 | CCAGTGTGACTCATCCACA | 0.179 |
| AH0040 | No. 40 | CAGUGUGACUCAUCCACAAUG | No. 1220 | UUGUGGAUGAGUCACACUGGC | No. 2400 | CAGTGTGACTCATCCACAA | 0.126 |
| AH0041 | No. 41 | AGUGUGACUCAUCCACAAUGA | No. 1221 | AUUGUGGAUGAGUCACACUGG | No. 2401 | AGTGTGACTCATCCACAAT | 0.081 |
| AH0042 | No. 42 | GUGUGACUCAUCCACAAUGAU | No. 1222 | CAUUGUGGAUGAGUCACACUG | No. 2402 | GTGTGACTCATCCACAATG | 0.123 |
| AH0043 | No. 43 | UGUGACUCAUCCACAAUGAUU | No. 1223 | UCAUUGUGGAUGAGUCACACU | No. 2403 | TGTGACTCATCCACAATGA | 0.144 |
| AH0044 | No. 44 | GUGACUCAUCCACAAUGAUUU | No. 1224 | AUCAUUGUGGAUGAGUCACAC | No. 2404 | GTGACTCATCCACAATGAT | 0.148 |
| AH0045 | No. 45 | UGACUCAUCCACAAUGAUUUC | No. 1225 | AAUCAUUGUGGAUGAGUCACA | No. 2405 | TGACTCATCCACAATGATT | 0.100 |
| AH0046 | No. 46 | GACUCAUCCACAAUGAUUUCU | No. 1226 | AAAUCAUUGUGGAUGAGUCAC | No. 2406 | GACTCATCCACAATGATTT | 0.114 |
| AH0047 | No. 47 | ACUCAUCCACAAUGAUUUCUC | No. 1227 | GAAAUCAUUGUGGAUGAGUCA | No. 2407 | ACTCATCCACAATGATTTC | 0.211 |
| AH0048 | No. 48 | CUCAUCCACAAUGAUUUCUCC | No. 1228 | AGAAAUCAUUGUGGAUGAGUC | No. 2408 | CTCATCCACAATGATTTCT | 0.136 |
| AH0050 | No. 50 | CAUCCACAAUGAUUUCUCCAG | No. 1230 | GGAGAAAUCAUUGUGGAUGAG | No. 2410 | CATCCACAATGATTTCTCC | 0.157 |
| AH0051 | No. 51 | AUCCACAAUGAUUUCUCCAGU | No. 1231 | UGGAGAAAUCAUUGUGGAUGA | No. 2411 | ATCCACAATGATTTCTCCA | 0.136 |
| AH0052 | No. 52 | UCCACAAUGAUUUCUCCAGUG | No. 1232 | CUGGAGAAAUCAUUGUGGAUG | No. 2412 | TCCACAATGATTTCTCCAG | 0.290 |
| AH0053 | No. 53 | CCACAAUGAUUUCUCCAGUGC | No. 1233 | ACUGGAGAAAUCAUUGUGGAU | No. 2413 | CCACAATGATTTCTCCAGT | 0.128 |
| AH0054 | No. 54 | CACAAUGAUUUCUCCAGUGCU | No. 1234 | CACUGGAGAAAUCAUUGUGGA | No. 2414 | CACAATGATTTCTCCAGTG | 0.130 |
| AH0055 | No. 55 | ACAAUGAUUUCUCCAGUGCUC | No. 1235 | GCACUGGAGAAAUCAUUGUGG | No. 2415 | ACAATGATTTCTCCAGTGC | 0.291 |
| AH0056 | No. 56 | CAAUGAUUUCUCCAGUGCUCA | No. 1236 | AGCACUGGAGAAAUCAUUGUG | No. 2416 | CAATGATTTCTCCAGTGCT | 0.139 |
| AH0057 | No. 57 | AAUGAUUUCUCCAGUGCUCAU | No. 1237 | GAGCACUGGAGAAAUCAUUGU | No. 2417 | AATGATTTCTCCAGTGCTC | 0.247 |
| AH0058 | No. 58 | AUGAUUUCUCCAGUGCUCAUC | No. 1238 | UGAGCACUGGAGAAAUCAUUG | No. 2418 | ATGATTTCTCCAGTGCTCA | 0.110 |
| AH0059 | No. 59 | UGAUUUCUCCAGUGCUCAUCU | No. 1239 | AUGAGCACUGGAGAAAUCAUU | No. 2419 | TGATTTCTCCAGTGCTCAT | 0.118 |
| AH0060 | No. 60 | GAUUUCUCCAGUGCUCAUCUU | No. 1240 | GAUGAGCAClJGGAGAAAUCAU | No. 2420 | GATTTCTCCAGTGCTCATC | 0.111 |
| AH0061 | No. 61 | AUUUCUCCAGUGCUCAUCUUG | No. 1241 | AGAUGAGCACUGGAGAAAUCA | No. 2421 | ATTTCTCCAGTGCTCATCT | 0.146 |
| AH0062 | No. 62 | UUUCUCCAGUGCUCAUCUUGU | No. 1242 | AAGAUGA6CACUGGAGAAAUC | No. 2422 | TTTCTCCAGTGCTCATCTT | 0.202 |
| AH0063 | No. 63 | UUCUCCAGUGCUCAUCUUGUU | No. 1243 | CAAGAUGAGCACUGGAGAAAU | No. 2423 | TTCTCCAGTGCTCATCTTG | 0.187 |
| AH0064 | No. 64 | UCUCCAGUGCUCAUCUUGUUC | No. 1244 | ACAAGAUGAGCACUGGAGAAA | No. 2424 | TCTCCAGTGCTCATCTTGT | 0.103 |
| AH0065 | No. 65 | CUCCAGUGCUCAUCUUGUUCU | No. 1245 | AACAAGAUGAGCACUGGAGAA | No. 2425 | CTCCAGTCGCTCATCTTGTT | 0.084 |
| AH0066 | No. 66 | UCCAGUGCUCAUCUUGUUCUC | No. 1246 | GAACAAGAUGAGCACUGGAGA | No. 2426 | TCCAGTGCTCATCTTGTTC | 0.128 |
| AH0067 | No. 67 | CCAGUGCUCAUCUUGUUCUCG | No. 1247 | AGAACAAGAUGAGCACUGGAG | No. 2427 | CCAGTGCTCATCTTGTTCT | 0.106 |
| AH0068 | No. 68 | CAGUGCUCAUCUUGUUCUCGA | No. 1248 | GAGAACAAGAUGAGCACUGGA | No. 2428 | CAGTGCTCATCTTGTTCTC | 0.103 |
| AH0069 | No. 69 | AGUGCUCAUCUUGUUCUCGAG | No. 1249 | CGAGAACAAGAUGAGCACUGG | No. 2429 | AGTGCTCATCTTGTTCTCG | 0.152 |
| AH0070 | No. 70 | GUGCUCAUCUUGUUCUCGAGU | No. 1250 | UCGAGAACAAGAUGAGCACUG | No. 2430 | GTGCTCATCTTGTTCTCGA | 0.078 |
| AH0071 | No. 71 | UGCUCAUCUUGUUCUCGAGUU | No. 1251 | CUCGAGAACCAAGAUGAGCACU | No. 2431 | TGCTCATCTTGTTCTCGAG | 0.091 |
| AH0072 | No. 72 | GCUCAUCUUGUUCUCGAGUUU | No. 1252 | ACUCGAGAACAAGAUGAGCAC | No. 2432 | GCTCATCTTGTTCTCGAGT | 0.111 |
| AH0073 | No. 73 | CUCAUCUUGUUCUCGAGUUUU | No. 1253 | AACUCGAGAACAAGAUGAGCA | No. 2433 | CTCATCTTGTTCTCGAGTT | 0.086 |
| AH0074 | No. 74 | UCAUCUUGUUCUCGAGUUUUC | No. 1254 | AAACUCGAGAACAAGAUGAGC | No. 2434 | TCATCTTGTTCTCGAGTTT | 0.073 |
| AH0075 | No. 75 | CAUCUUGUUCUCGAGUUUUCU | No. 1255 | AAAACUCGAGAACAAGAUGAG | No. 2435 | CATCTTGTTCTCGAGTTTT | 0.078 |
| AH0076 | No. 76 | AUCUUGUUCUCGAGUUUUCUC | No. 1256 | GAAAACUCGAGAACAUGAUGA | No. 2436 | ATCTTGTTTCGAGTTTTTC | 0.107 |
| AH0077 | No. 77 | UCUUGUUCUCGAGUUUUCUCU | No. 1257 | AGAAAACUCGAGAACAAGAUG | No. 2437 | TCTTGTTCTCGAGTTTTCT | 0.137 |
| AH0078 | No. 78 | CUUGUUCUCGAGUUUUCUCUG | No. 1258 | GAGAAAACUCGAGAACAAGAUG | No. 2438 | CTTGTTCTCGAGTTTTCTC | 0.123 |

### [Table 40]

**Table 4-2**

| Double stranded nucleic acid No. | SEQ ID NO. | Sense strand sequence (5'→3') | SEQ ID NO. | Antisense strand sequence (5'→3') | SEG ID NO. | Target β2GPI mRNA sequence | Relative expressed amount of β2GPI |
|---|---|---|---|---|---|---|---|
| AH0079 | No. 79 | UUGUUCUCGAGUUUCUCUGC | No. 1259 | AGAGAAAACUCGAGAACAAGA | No. 2439 | TTGTTCTCGAGTTTTCCTC | 0.163 |
| AH0080 | No. 80 | UGUUCUCGAGUUUUCUCUGCC | No. 1260 | CAGAGAAAACUCGAGAACAAG | No. 2440 | TGTTCTCGAGTTTTCTCTG | 0.291 |
| AH0081 | No. 81 | GUUCUCGAGUUUUCUCUGCCA | No. 1261 | GCAGACAAAACUCGAGAACAA | No. 2441 | GTTCTCGAGTTTTCTCTGC | 0.298 |
| AH0083 | No. 83 | UCUCGAGUUUUCUCUGCCAUG | No. 1263 | UGGCAGAGAAAACUCGAGAAC | No. 2443 | TCTCGAGTTTTCTCTGCCA | 0.148 |
| AH0084 | No. 84 | CUCGAGUUUUCUCUGCCAUGU | No. 1264 | AUGGCAGAGAAAACUCGAGAA | No. 2444 | CTCGAGTTTTCTCTGCCAT | 0.187 |
| AH0085 | No. 85 | UCGAGUUUUCUCUGCCAUGUU | No. 1265 | CAUGGCAGAGAAAACUCGAGA | No. 2445 | TCGAGTTTTCTCTGCCATG | 0.146 |
| AH0086 | No. 86 | CGAGUUUUCUCUGCCAUGUUG | No. 1266 | ACAUGCAGAGAAAACUCGAG | No. 2446 | CGAGTTTTCTCTGCCATGT | 0.149 |
| AH0087 | No. 87 | GAGUUUUCUCUGCCAUGUUGC | No. 1267 | AACAUGGCAGAGAAAACUCGA | No. 2447 | GAGTTTTCTCTGCCATGTT | 0.221 |
| AH0088 | No. 88 | AGUUUUCUCUGCCAUGUUGCU | No. 1268 | CAACAUGGCAGAGAAAACUCG | No. 2448 | AGTTTTCTCTGCCATGTTG | 0.088 |
| AH0089 | No. 89 | GUUUUCUCUGCCAUGUUGCUA | No. 1269 | GCAACAUGGCAGAGAAAACUC | No. 2449 | GTTTTCTCTGCCATGTTGC | 0.194 |
| AH0090 | No. 90 | UUUUCUCUGCCAUGUUGCUAU | No. 1270 | AGCAACAUGGCAGAGAAAACU | No. 2450 | TTTTCTCTGCCATGTTGCT | 0.200 |
| AH0091 | No. 91 | UUUCUCUGCCAUGUUGCUAUU | No. 1271 | UAGCAACAUGGCAGAGAAAAC | No. 2451 | TTTCTCTGCCATGTTGCTA | 0.296 |
| AH0092 | No. 92 | UUCUCUGCCAUGUUGCUAUUG | No. 1272 | AUAGCAACAUGGCAGAGAAAA | No. 2452 | TTCTCTGCCATGTTGCTAT | 0.203 |
| AH0093 | No. 93 | UCUCUGCCAUGUUGCUAUUGC | No. 1273 | AAUAGCAACAUGGCAGAGAAA | No. 2453 | TCTCTGCCATGTTGCTATT | 0.204 |
| AH0096 | No. 96 | CUGCCAUGUUGCUAUUGCAGG | No. 1276 | UGCAAUAGCAACAUGGCAGAG | No. 2456 | CTGCCATGTTGCTATTGCA | 0.051 |
| AH0097 | No. 97 | UGCCAUGUUGCUAUUGCAGGA | No. 1277 | CUGCAAUAGCAACAUGGCAGA | No. 2457 | TGCCATGTTGCTATTGCAG | 0.226 |
| AH0098 | No. 98 | GCCAUGUUGCUAUUGCAGGAC | No. 1278 | CCUGCAAUAGCAACAUGGCAG | No. 2458 | GCCATGTTGCTATTGCAGG | 0.184 |
| AH0099 | No. 99 | CCAUGUUGCUAUUGCAGGACG | No. 1279 | UCCUGCAAUAGCAACAUGGCA | No. 2459 | CCATGTTGCTATTGCAGGA | 0.066 |
| AH0103 | No. 103 | GUUGCUAUUGCAGGACGGACC | No. 1283 | UCCGUCCUGCAAUAGCAACAU | No. 2463 | GTTGCTATTGCAGGACGGA | 0.101 |
| AH0106 | No. 106 | GCUAUUGCAGGACGGACCUGU | No. 1286 | AGGUCCGUCCUGCAAUAGCAA | No. 2466 | GCTATTGCAGGACGGACCT | 0.118 |
| AH0107 | No. 107 | CUAUUGCAGGACGGACCUGUC | No. 1287 | CAGGUCCGUCCUGCAAUAGCA | No. 2467 | CTATTGCAGGACGGACCTG | 0.170 |
| AH0108 | No. 108 | UAUUGCAGGACGGACCUGUCC | No. 1288 | ACAGGUCCGUCCUGCAGAUAGC | No. 2468 | TATTGCAGGACGGACCTGT | 0.212 |
| AH0109 | No. 109 | AUUGCAGGACGGACCUGUCCC | No. 1289 | GACAGGUCCGUCCUGCAAUAG | No. 2469 | ATTGCAGGACGGACCTGTC | 0.210 |
| AH0112 | No. 112 | GCAGGACGGACCCUGUCCCAAG | No. 1292 | UGGGACAGGUCCGUCCUGCAA | No. 2472 | GCAGGACGGACCTGTCCCA | 0.225 |
| AH0114 | No. 114 | AGGACGGACCUGUCCCAAGCC | No. 1294 | CUUGGGACAGGUCCGUCCUGC | No. 2474 | AGGACGGACCTGTCCCAAG | 0.155 |
| AH0115 | No. 115 | GGACGGACCUGUCCCAAGCCA | No. 1295 | GCUUGGGACAGGUCCGUCCUG | No. 2475 | GGACGGACCTGTCCCAAGC | 0.073 |
| AH0117 | No. 117 | ACGGACCUGUCCCAAGCCAGA | No. 1297 | UGGCUUGGGACAGUUCCGUCC | No. 2477 | ACGGACCTGTCCCAAGCCA | 0.194 |
| AH0118 | No. 118 | CGGACCUGUCCCAAGCCAGAU | No. 1298 | CUGGCUUGGGACAGGUCCGUC | No. 2478 | UGGACCTGTCCCAAGCCAG | 0.111 |
| AH0119 | No. 119 | GGACCUGUCCCAAGCCAGAUG | No. 1299 | UCUGGCUUGGGACAGGUCCGU | No. 2479 | GGACCTGTCCCAAGCCAGA | 0.057 |
| AH0120 | No. 120 | GACCUGUCCCAAGCCAGAUGA | No. 1300 | AUCUGGCUUGGGACAGGUCCG | No. 2480 | GACCTGTCCCAAGCCAGAT | 0.136 |
| AH0121 | No. 121 | ACCUGUCCCAAGCCAGAUGAU | No. 1301 | CAUCUGGCUUGGGACAGGUCC | No. 2481 | ACCTGTCCCAAGCCAGATG | 0.298 |
| AH0122 | No. 122 | CCUGUCCCAAGCCAGAUGAUU | No. 1302 | UCAUCUGGCUUGGGACAGGUC | No. 2482 | CCTGTCCCAAGCCAGATGA | 0.111 |
| AH0123 | No. 123 | CUGUCCCAAGCCAGAUGAUUU | No. 1303 | AUCAUCUGGCUUGGGACAGGU | No. 2483 | CTGTCCCAAGCCAGATGAT | 0.156 |
| AH0124 | No. 124 | UGUCCCAAGCCAGAUGAUUUA | No. 1304 | AAUCAUCUGGCUUGGGACAGG | No. 2484 | TGTCCCAAGCCAGATGATT | 0.098 |
| AH0125 | No. 125 | GUCCCAAGCCAGAUGAUUUAC | No. 1305 | AAAUCAUCUGGCUUGGGACAG | No. 2485 | GTCCCAAGCCAGATGATTT | 0.050 |
| AH0126 | No. 126 | UCCCAAGCCAGAUGAUUUACC | No. 1306 | UAAAUCAUCUGGCUUGGGACA | No. 2486 | TCCCAAGCCAGATGATTTA | 0.063 |
| AH0127 | No. 127 | CCCAAGCCAGAUGAUUUACCA | No. 1307 | GUAAAUCAUCUGGCUUGGGAC | No. 2487 | CCCAAGCCAGATGATTTAC | 0.260 |
| AH0128 | No. 128 | CCAAGCCAGAUGAUUUACCAU | No. 1308 | GGUAAAUCAUCUGGCUUGGGA | No. 2488 | CCAAGCCAGATGATTTACC | 0.326 |
| AH0129 | No. 129 | CAAGCCAGAUGAUUUACCAUU | No. 1309 | UGGUAAAUCAUCUGCUUGGG | No. 2489 | CAAGCCAGATGATTTACCA | 0.092 |
| AH0130 | No. 130 | AAGCCAGAUGAUUUACCAUUU | No. 1310 | AUGGUAAAUCAUCUGGCUUGG | No. 2490 | AAGCCAGATGATTTACAT | 0.098 |
| AH0131 | No. 131 | AGCCAGAUGAUUUACCAUUUU | No. 1311 | AAUGGUAAAUCAUCUGGCUUG | No. 2491 | AGCCAGATGATTTACCATT | 0.147 |
| AH0132 | No. 132 | GCCAGAUGAUUUACCAUUUUC | No. 1312 | AAAUGGAAAUCAUCUGGCUU | No. 2492 | GCCAGATGATTTACCATTT | 0.116 |
| AH0133 | No. 133 | CCAGAUGAUUUACCAUUUUCC | No. 1313 | AAAAUGGUAAAUCAUCUGGCU | No. 2493 | CCAGATGATTTACCATTTT | 0.062 |
| AH0134 | No. 134 | CAGAUGAUUUACCAUUUUCCA | No. 1314 | GAAAAUGGUAAUCAUCUGGC | No. 2494 | CAGATGATTTACCATTTTC | 0.074 |
| AH0135 | No. 135 | AGAUGAUUUACCAUUUUCCAC | No. 1315 | GGAAAAUGGUAAAUCAUCUGG | No. 2495 | AGATGATTTACCATTTTCC | 0.122 |
| AH0136 | No. 136 | GAUGAUUUACCAUUUUCCACA | No. 1316 | UGGAAAAUGGUAAAUCAUCUG | No. 2496 | GATGATTTACCATTTTCCA | 0.114 |

### [Table 41]

**Table 4-3**

| Double stranded nucleic acid No. | SEQ ID NO | Sense strand sequence (5'-3') | SEQ ID NO. | Antisense strand sequence (5'→3) | SEQ ID NO. | Target β2GPI mRHA sequence | Relative expressed amount of β2GPI |
|---|---|---|---|---|---|---|---|
| AH0137 | No. 137 | AUGAUUUACCAUUUUCCACAG | No. 1317 | GUGGAAAAUGUAAAUCAUCU | Nο. 2497 | ATGATTTACCATTTTCCAC | 0.333 |
| AH0138 | No. 138 | UGAUUUACCAUUUUCCACAGU | No. 1318 | UGUGGAAAUGGUAAAUCAUC | Nο. 2498 | TGATTTACCATTTTCCACA | 0.210 |
| AH0139 | No. 139 | GAUUUACCAUUUUCCACAGUG | No. 1319 | CUGUGGAAAAUGGUAAAUCAU | No. 2499 | GATTTACCATTTTCCACAG | 0.303 |
| AH0143 | No. 143 | UACCAUUUUCCACAGUGGUCC | No. 1323 | ACCACUGUGGAAAAUGGUAAA | No. 2503 | TACCATTTTCCACAGTGGT | 0.298 |
| AH0144 | No. 144 | ACCAUUUUCCACAGUGGUCCC | No. 1324 | GACCACUGUGGAAAAUGGUAA | Nο. 2504 | ACCATTTTCCACAGTGGTC | 0.155 |
| AH0145 | No. 145 | CCAUUUUCCACAGUGGUCCG | No. 1326 | GGACCACUGUGGAAAAUGGUA | No. 2505 | CCATTTTCCACAGTGGTCC | 0.305 |
| AH0149 | No. 149 | UUUCCACAGUGGUCCCUUAA | No. 1329 | AACGGGACCACUGUGGAAAAU | No. 2509 | TTTCCACAGTGGTCCCGTT | 0.080 |
| AH0150 | No. 150 | UUCCACAGUGGUCCCGUAAA | No. 1330 | UAACGGGACCACUGUGGAAAA | No. 2510 | TTCCACAGTGGTCCCGTTA | 0.184 |
| AH0151 | No. 151 | UCCACAGUGGUCCCGUUAAAA | No. 1331 | UUAACGGGACCACUGUGGAAA | No. 2511 | CCACAAGTGGTCCCGTTAA | 0.164 |
| AH0152 | No. 152 | CCACAGUGGUCCCGUUAAAAA | No. 1332 | UUUAACGGGACCACUGUGGAA | No. 2512 | CCACAGTGGTCCCGTTAAA | 0.060 |
| AH0153 | No. 153 | CACAGUGGUCCCGUUAAAAAC | No. 1333 | UUUUAACGGGACCACUGUGGA | No. 2513 | CACAGTGGTCCCGTTAAAA | 0.198 |
| AH0154 | No. 154 | ACAGUGGUCCCGUUAAAAACA | No. 1334 | UUUUUAACGGGACCACUGUGG | No. 2514 | ACAGTGGTCCCGTTAAAAA | 0.077 |
| AH0155 | No. 155 | CAGUGGUCCCGUUAAAAACAU | No. 1335 | GUUUUUAACGGGACCACUGUG | No. 2515 | CAGTGGTCCCGTTAAAAAC | 0.062 |
| AH0156 | No. 156 | AGUGGUCCCGUUAAAACCAUU | No. 1336 | UGUUUUUAACGGGACCACUGU | No. 2516 | AGTGGTCCCGTTAAAAACA | 0.238 |
| AH0157 | No. 157 | GUGGUCCCGUUAAAAACAUUC | No. 1337 | AUGUUUUUAACGGGACCACUG | No. 2517 | GTGGTCCCGTTAAAAACAT | 0.130 |
| AH0158 | No. 158 | UGGUCCCGUUAAAAACAUUCU | No. 1338 | AAUGUUUUUAACGGGACCACU | No. 2518 | TGGTCCCGTTAAAAACATT | 0.145 |
| AH0159 | No. 159 | GGUCCCGUUAAAAACAUUCUA | No. 1339 | GAAUGUUUUUAACGGGACCAC | No. 2519 | GGTCCCGTTAAAAACATTC | 0.266 |
| AH0160 | No. 160 | GUCCCGUUAAAAACAUUCUAU | No. 1340 | AGAAUGUUUUUAACGGGACCA | No. 2520 | GTCCCGTTAAAAACATTCT | 0.265 |
| AH0161 | No. 161 | UCCCGUUAAAAACAUUCUAUG | No. 1341 | UAGAAUGUUUUUAACGGGACC | No. 2521 | TCCCGTTAAAAACATTCTA | 0.156 |
| AH0162 | No. 162 | CCCGUUAAAAACAUUCUAUGA | No. 1342 | AUAGAAUGUUUUUAACGGGAC | No. 2522 | CCCGTTAAAAACATTCTAT | 0.096 |
| AH0163 | No. 163 | CCGUUAAAAACAUUCUAUGAG | No. 1343 | CAUAGAAUGUUUUUAACGGGA | No. 2523 | CCGTTAAAAACATTCTATG | 0.116 |
| AH0164 | No. 164 | CGUUAAAAACAUUCUAUGAGC | No. 1344 | UCAUAGAAUGUUUUUAACGGG | Nο. 2524 | CGTTAAAAACATTCTATGA | 0.109 |
| AH0173 | No. 173 | CAUUCUAUGAGCCAGGAGAAG | No. 1353 | UCUCCUGGCUCAUAGAAUGUU | No. 2533 | CATTCTATGAGCCAGGAGA | 0.131 |
| AH0174 | No. 174 | AUUCUAUGAGCCAGGAGAAGA | No. 1354 | UUCUCCUGGCUCAUAGAAUGU | No. 2534 | ATTCTATGAGCCAGGAGAA | 0.255 |
| AH0177 | No. 177 | CUAUGAGCCAGGAGAAGAGAU | No. 1357 | CUCUUCUCCUGGCUCAUAGAA | No. 2537 | CTATGAGCCAGGAGAAGAG | 0.168 |
| AH0178 | No. 178 | UAUGAGCCAGGAGAAGAGAUU | No. 1358 | UCUCUUCUCCUGGCUCAUAGA | No. 2538 | TATGAGCCAGGAGAAGAGA | 0.319 |
| AH0179 | No. 179 | AUGAGCCAGGAGAAGAGAUUA | No. 1359 | AUCUCUUCUCCUGGCUCAUAG | No. 2539 | ATGAGCCAGGAGAAGAGAT | 0.260 |
| A H0180 | No. 180 | UGAGCCAGGAGAAGAGAUUAC | No. 1360 | AAUCUCUUCUCCUGGCUCAUA | No. 2540 | TGAGCCAGGAGAAGAGATT | 0.128 |
| AH0181 | No. 181 | GAGCCAGGAGAAGAGAUUACG | No. 1361 | UAAUCUCUUCUCCUGGCUCAU | No. 2541 | GAGCCAGGAGAAGAGATTA | 0.049 |
| AH0183 | No. 183 | GCCAGGAGAAGAGUUACGUA | No. 1363 | CGAAUCUCUUCUCCUGGCUC | No. 2543 | GCCAGGAGAAGAGATTACG | 0.083 |
| AH0184 | No. 184 | CCAGGAGAAGAGAUUACGUAU | No. 1364 | ACGUAAUCUCUUCUCCUGGCU | No. 2544 | CCAGGAGAAGAGATTACGT | 0.066 |
| AH0185 | No. 185 | CAGGAGAAGAGAUUACGUAUU | No. 1365 | UACGUAAUCUCUUCUCCUGGC | No. 2545 | CAGGAGAAGAGATTACGTA | 0.055 |
| AH0186 | No. 186 | AGGAGAAGAGAUUACGUAUUC | No. 1366 | AUACGUAAUCUCUUCUCCUGG | No. 2546 | AGGAGAAGATTAIACGTAT | 0.094 |
| AH0187 | No. 187 | GGAGAAGAGAUUACGUAUUCC | No. 1367 | AAUACGUAAUCUCUUCUCCUG | No. 2547 | GGAGAAGAGATTACGTATT | 0.071 |
| AH0188 | No. 188 | GAGAAGAGAUUACGUAUUCCU | No. 1368 | GAAUACGUAAUCUCUCUUCCU | No. 2548 | GAGAAGAGATTACGTATTC | 0.069 |
| AH0189 | No. 189 | AGAAGAGAUUACGUAUUCCUG | No. 1369 | GGAAUACGUAAUCUCUUCUCC | No. 2549 | AGAAGAGATTACGTATTCC | 0.168 |
| AH0190 | No. 190 | GAAGAGAUUACGUAUUCCUGC | No. 1370 | AGGAAUACGUAAUCUCUUCUC | No. 2550 | GAAGAGATTACGTATTCCT | 0.157 |
| AH0193 | No. 193 | GAGAUUACGUAUUCCUGCAAG | No. 1373 | UGCAGGAAUACGUAAUCUCUU | No. 2553 | GAGATTACGTATTCCTGCA | 0.070 |
| AH0194 | No. 194 | AGAUUACGUAUUCCUGCAAGC | No. 1374 | UUGCAGGAAUACGUAAUCUCU | No. 2554 | AGATTACGTATTCCTGCAA | 0.186 |
| AH0195 | No. 195 | GAUUACGUAUUCCUGCAAGCC | No. 1375 | CUUGCAGGAAUACGUAAUCUC | No. 2555 | GATTACGTATTCCTGCAAG | 0.095 |
| AH0199 | No. 199 | ACGUAUUCCUGCAAGCCGGGC | No. 1379 | CCGGCUUGCAGGAAUACGUAA | Nο. 2559 | ACGTATTCCTGCAAGCCGG | 0.294 |
| AH0203 | No. 203 | AUUCCUGCAAGCCGGGCUAUG | No. 1383 | UAGCCCGGCUUGCAGGAAUAC | No. 2563 | ATTCCTGCAAGCCGGGCTA | 0.232 |
| AH0207 | No. 207 | CUGCAAGCCGGGCUAUGUGUC | No. 1387 | CACAUAGCCCGGCUUGCAGGA | No. 2567 | CTGCAAGCCGGGCTATGTG | 0.341 |
| AH0225 | No. 225 | GUCCCGAGGAGGGAUGAGAAA | No. 1405 | UCUCAUCCCUCCUCGGGACAC | No. 2585 | GTCCCGAGGAGGGATGAGA | 0.212 |
| AH0226 | No. 226 | UCCCGAGGAGGGAUGAGAAAG | No. 1406 | UUCUCAUCCCUCCUCGGGACA | No. 2586 | TCCCGAGGAGGGATGAGAA | 0.133 |
| AH0229 | No. 229 | CGAGGAGGGAUGAGAAAGUUU | No. 1409 | ACUUUCUCAUCCCUCCUCGGG | No. 2589 | CGAGGAGGGATGAAAGT | 0.182 |

### [Table 42]

**Table 4-4**

| Double stranded nucleic acid No | SEQ ID NO. | Sense strand sequence (5'→3') | SEQ ID NO. | Antisense strand sequence (5'→3') | SEQ ID NO. | Target β2GPI mRNA sequence | Relative expressed amount of β2GPI |
|---|---|---|---|---|---|---|---|
| AH0230 | No. 230 | GAGGAGGGAUGAGAAAGUUUA | No. 1410 | AACUUUCUCAUCCCUCCUCGG | No. 2590 | GAGGAGGGATGAGAAAGTT | 0.290 |
| AH0231 | No. 231 | AGGAGGGAUGAGAAAGUUUAU | No. 1411 | AAACUUUCUCAUCCCUCGUCG | No. 2591 | AGGAGGGATGAGAAAGTTT | 0.149 |
| AH0237 | No. 232 | GGAGGGAUGAGAAAGUUUAUC | No. 1412 | UAAACUUUCUCAUCCCUCCUC | No. 2592 | GGAGGGATGAGAAAGTTTA | 0.080 |
| AH0233 | No. 233 | GAGGGAUGAGAAAGUUUAUCU | No. 1413 | AUAAACUUUCUCAUCCCUCCU | No. 2593 | GAGGGATGAGAAAGTTTAT | 0.096 |
| AH0234 | No. 234 | AGGGAUGAGAAAGUUUAUCUG | No. 1414 | GAUAAACUUUCUCAUCCCUCC | No. 2594 | AGGGATGAGAAAGTTTATC | 0.252 |
| AH0235 | No. 235 | GGGAUGAGAAAGUUUAUCUGC | No. 1415 | AGAUAAACUUUCUCAUCCCUC | No. 2595 | GGGATGAGAAAGTTTATCT | 0.134 |
| AH0236 | No. 236 | GGAUGAGAAAGUUUAUCUGCC | No. 1416 | CAGAUAAACUUUCUCAUCCCU | No. 2596 | GGATGAGAAAGTTTATCTG | 0.065 |
| AH0242 | No. 242 | GAAAGUUUAUCUGCCCUCUCA | No. 1422 | AGAGGGCAGAUAAACUUOCUC | No. 2602 | GAAAGTTTATCTGCCCTCT | 0.318 |
| AH0244 | No. 244 | AAGUUUAUCUGCCCUCUCACA | No. 1424 | UGAGAGGGCAGAUAAACUUUC | No. 2604 | AAGTTTATCTGCCCTCTCA | 0.192 |
| AH0255 | No. 255 | CCCUCUCACAGGACUGUGGCC | No. 1435 | CCACAGUCCUGUGAGAGGGCA | No. 2615 | CCCTCTCACAGGACTGTGTGG | 0.340 |
| AH0260 | No. 260 | UCACAGGACUGUGGCCCAUCA | No. 1440 | AUGGGCCACAGUCCUGUGAGA | No. 2620 | TCACAGGACTGTGGCCCAT | 0.295 |
| AH0262 | No. 262 | ACAGGACUGUGGCCCAUCAAC | No. 1442 | UGAUGGGCCACAGUCCUGUGA | No. 2622 | ACAGGACTGTGGCCCATCA | 0.305 |
| AH0265 | No. 265 | GGACUGUGGCCCAUCAACACU | No. 1445 | UGUUGAUGGGCCACAGUCCUG | No. 2625 | GGACTGTGGCCCATCAACA | 0.137 |
| AH0266 | No. 266 | GACUGUGGCCCAUCAACACUC | No. 1446 | GUGUUGAUGGGCCACAGUCCU | No. 2626 | GACTGTGGCCCATCAACAC | 0.169 |
| AH0272 | No. 272 | GGCCCAUCAACACUCUGAAAU | No. 1452 | UUCAGAGUGUUGAUGGGCCAC | No. 2632 | GGCCCATCAACACTCTGAA | 0.166 |
| AH0274 | No. 274 | CCCAUCAACACUCUGAAAUGU | No. 1454 | AUUUCAGAGUGUUGAUGGGCC | No. 2634 | CCCATCAACACTCTGAAAT | 0.253 |
| AH0275 | No. 275 | CCAUCAACACUCUGAAAUGUA | No. 1455 | CAUUUCAGAGUGUUGAUGGGC | No. 2635 | CCATCAACACTCTGAAATG | 0.068 |
| AH0276 | No. 276 | CAUCAACACUCUGAAAUGUAC | No. 1456 | ACAUUUCAGAGUGUUGAUGGG | No. 2636 | CATCAACACTCTGAAATGT | 0.165 |
| AH0277 | No. 277 | AUCAACACUCUGAAAUGUACA | No. 1457 | UACAUUUCAGAGUGUUGAUGG | No. 2637 | ATCAACACTCTGAAATGTA | 0.172 |
| AH0279 | No. 279 | CAACACUCUGAAAUGUACACC | No. 1459 | UGUAACAUUUCAGAGUGUUGAU | No. 2639 | CAACACTCTGAAATGTACA | 0.229 |
| AH0283 | No. 283 | ACUCUGAAAUGUACACCCAGA | No. 1463 | UGGGUGUACAUUUCAGAGUGU | No. 2643 | ACTCTGAAATGTACACCCA | 0.288 |
| AH0285 | No. 285 | UCUGAAAUGUACACCCAGAGU | No. 1465 | UCUGGGUGUACAUUUCAGAGU | No. 2645 | TCTGAAATGTACACCCAGA | 0.305 |
| AH0288 | No. 288 | GAAAUGUACACCCAGAGUAUG | No. 1468 | UACUCUGGGUGUACAUUUCAG | No. 2648 | GAAATGTACACCCAGAGTA | 0.221 |
| AH0289 | No. 289 | AAAUGUACACCCAGAGUAUGU | No. 1469 | AUACUCUGGGUGUACAUUUCA | No. 2649 | AAATGTACACCCAGAGTAT | 0.297 |
| AH0291 | No. 291 | AUGUACACCCAGAGUAUGUCC | No. 1471 | ACAUCUCUGGGUGUACAUUU | No. 2651 | ATGTACACCCAGAGTATGT | 0.209 |
| AH0294 | No. 294 | UACACCCAGAGUAUGUCCUUU | No. 1474 | AGGACAUACUCUGGUGUACA | No. 2654 | TACACCCAGAGTATGTCCT | 0.179 |
| AH0295 | No. 295 | ACACCCAGAGUAUGUCCUUUU | No. 1475 | AAGGACAUACUCUGGGUGUAC | No. 2655 | ACACCCAGAGTATGTCCTT | 0.125 |
| AH0296 | No. 296 | CACCCAGAGUAUGUCCUUUUUG | No. 1476 | AAAGGACAUACUCUGGGUGUA | No.2656 | CACCCAGAGATGTCCTTT | 0.076 |
| AH0297 | No. 297 | ACCCAGAGUAUGUCCUUUUGC | No. 1477 | AAGGACAUACUCUGGGUGU | No. 2657 | ACCCAGAGTATGTCCTTTT | 0.139 |
| AH0298 | No. 298 | CCCAGAGUAUGUCCUUUUGCU | No. 1478 | CAAAAGGACAUACUCUGGGUG | No. 2658 | CCCAGAGTATGTCCTTTTG | 0.134 |
| AH0300 | No. 300 | CAGAGUAUGUCCUUUUGCUGG | No. 1480 | AGCAAAAGGACAUACUCUGGG | No. 2660 | CAGAGTATGTCCTTTTGCT | 0.125 |
| 0301 | No. 301 | AGAGUAUGUCCUUUUGCUGGA | No. 1481 | CAGCAAAAGGACAUACUCUGG | No. 2661 | AGAGTATGTCCTTTTGCTG | 0.176 |
| AH0302 | No. 302 | GAGUAUGUCCUUUUGCUGGAA | No. 1482 | CCAGCAAAAGGACAUACUCUG | No. 2662 | GAGTATGTCCTTTTGCTGG | 0.076 |
| AH0303 | No. 303 | AGUAUGUCCUUUUGCUGGAAU | No. 1483 | UCCAGCAAAAGGACAUACUCU | No. 2663 | AGTATGTCCTTTTGCTGGA | 0.083 |
| AH0304 | No. 304 | GUAUGUCCUUUUGCUGGAAUC | No. 1484 | UUCCAGCAAAAGGACAUACUC | No. 2664 | GTATGTCCTTTTGCTGGAA | 0.156 |
| AH0306 | No. 306 | AUGUCCUUUUUGCUGGAAUCUU | No. 1486 | GAUUCCAGCAAAAGGACAUAC | No. 2666 | ATGTCCTTTTGCTGGAATC | 0.261 |
| AH0307 | No. 307 | UGUCCUUUUGCUGGAAUCUUA | No. 1487 | AGAUUCCAGCAAAAGGACAUA | No. 2667 | TGTCCTTTTGCTGGAATCT | 0.168 |
| AH0308 | No. 308 | GUCCUUUUGCUGGAAUCUUAG | No. 1488 | AAGAUUCCAGCAAAAGGACAU | No. 2668 | GTCCTTTTGCTGGAATCTT | 0.076 |
| AH0309 | No. 309 | UCCUUUUGCUGGAAUCUUAGA | No. 1489 | UAAGAUUCCAGCAAAAGGACA | No. 2669 | TCCTTTTGCTGGAATCTTA | 0.072 |
| AH0310 | No. 310 | CCUUUUGCUGGAAUCUUAGAA | No. 1490 | CUAAGAUUCCAGCAAAAGGAC | No. 2670 | CCTTTTGCTGGAATCTTAG | 0.097 |
| AH0311 | No. 311 | CUUUUGCUGGAAUCUUAGAAA | No. 1491 | UCUAAGAUUCCAGCAAAAGGA | No. 2671 | CTTTTGCTGGAATCTTAGA | 0.198 |
| AH0314 | No. 314 | UUGCUGGAAUCUUAGAAAAUG | No. 1494 | UUUUCUAAGAUUCCAGCAAAA | No. 2674 | TTGTTGGAATCTTAGAAAA | 0.139 |
| AH0315 | No. 315 | UGCUGGAAUCUUAGAAAAUGG | No. 1495 | AUUUUCUAAGAUUCCAGCAAA | No. 2675 | TGCTGGAATCTTAGAAAAT | 0.132 |
| AH0316 | No. 316 | GCUGGAAUCUUAGAAAAUGGA | No. 1496 | CAUUUUCUAAGAUUCCAGCAA | No. 2676 | GCTGGAATCTTAGAAAATG | 0.102 |
| AH0317 | No. 317 | CUGGAAUCUUAGAAAAUGGAG | No. 1497 | CCAUUUUCUAAGAUUCCAGCA | No. 2677 | CTGGAATCTTAGAAAATGG | 0.304 |
| AH0318 | No. 318 | UGGAAUCUUAGAAAAUGGAGC | No. 1498 | UCCAUUUUCUAAGAUUCCAGC | No. 2678 | TGGAATCTTAGAAAATGGA | 0.122 |

### [Table 43]

**Table 4-5**

| Double stranded nucleic acid No. | SEQ ID NO. | Sense strand sequence (5'-3') | SEQ ID NO. | Antisense strand sequence (5'→3') | SEQ ID NO. | Target β2GPI mRNA sequence | Relative expressed amount of β2GPI |
|---|---|---|---|---|---|---|---|
| AH0319 | No. 319 | GGAAUCUUAGAAAAUGGAGCC | No. 1499 | CUCCAUUUUCUAAGAUUCCAG | No. 2679 | GGAATCTTAGAAAATGGAG | 0.232 |
| AH0324 | No. 324 | CUUAGAAAAUGGAGCCGUACG | No. 1504 | UACGGCUCCAUUUUCUAAGAU | No. 2684 | CTTAGAAAATGGAGCCGTA | 0.125 |
| AH0327 | No. 327 | ALAAAAUGGAGCCGUACGCUA | No. 1507 | GCGUACGGCUCCAUUUUCUAA | No. 2687 | AGAAAATGGAGCCGTACGC | 0.334 |
| AH0328 | No. 328 | GAAAAUGGAGCCGUACGCUAU | No. 1508 | AGCGUACGGCUCCAUUUUCUA | No. 2688 | GAAAATGGAGCCGTACGCT | 0.153 |
| AH0329 | No. 329 | AAAAUGGAGCCGUACGCUAUA | No. 1509 | UAGCGUACGGCUCCAUUUUCU | No. 2689 | AAAATGGAGCCGTACGCTA | 0.063 |
| AH0330 | No. 330 | AAAUGGAGCCGUACGCUAUAC | No. 1510 | AUAGCGUACGGCUCCAUUUUC | No. 2690 | AAATGGAGCCGTACGCTAT | 0.182 |
| AH0331 | No. 331 | AAUGGAGCCGUACGCUAUACG | No. 1511 | UAUAGCGUACGGCUCCAUUUU | No. 2691 | AATGGAGCCGTACGCTATA | 0.149 |
| AH0334 | No. 334 | GGAGCCGUACGCUAUACGACU | No. 1514 | UCGUAUAGCGUACGGCUCCAU | No. 2694 | GGAGCCGTACGCTATACGA | 0.164 |
| AH0335 | No. 335 | GAGCCGUACGCUAUACGACUU | No. 1515 | GUCGUAUAGCGUACGGCUCCA | No. 2695 | GAGCCGTACGCTATACGAC | 0.181 |
| AH0336 | No. 336 | AGCCGUACGCUAUACGACUUU | No. 1516 | AGUCGUAUAGCGUACGGCUCC | No. 2696 | AGCCGTACGCTATACGACT | 0.114 |
| AH0337 | No. 337 | GCCGUACGCUAUACGACUUUU | No. 1517 | AAGUCGUAUAGCGUACGGCUC | No. 2697 | GCCGTACGCTATACGACTT | 0.106 |
| AH0338 | No. 338 | CCGUACGCUAUACGACUUUUG | No. 1518 | AAAGUCGUAUAGCGUACGGCU | No. 2698 | CCGTACGCTATACGACTTT | 0.017 |
| AH0339 | No. 339 | CGUACGCUAUACGACUUUUGA | No. 1519 | AAAAGUCGUAUAGCGUACGGC | No. 2699 | CGTACGCTATACGACTTTT | 0.102 |
| AH0340 | No. 340 | GUACGCUAUACGACUUUUGAA | No. 1520 | CAAAAGUCGUAUAGCGUACGG | No. 2700 | GTACGCTATACGACTTTTG | 0.224 |
| AH0341 | No. 341 | UACGCUAUACGACUUUUGAAU | No. 1521 | UCAAAAGUCGUAUAGCGUACG | No. 2701 | TACGCTATACGACTTTTGA | 0.233 |
| AH0342 | No. 342 | ACGCUAUACGACUUUUGAAUA | No. 1522 | UUCAAAAGUCGUAUAGCGUAC | No. 2702 | ACGCTATACGACTTTTGAA | 0.120 |
| AH0343 | No. 343 | CGCUAUACGACUUUUGAAUAU | No. 1523 | AUUCAAAAGUCGUAUAGCGUA | No. 2703 | CGCTATACGACTTTTGAAT | 0.102 |
| AH0345 | No. 345 | CUAUACGACUUUUGAAUAUCC | No. 1525 | AUAUUCAAAAGUCGUAUAGCG | No. 2705 | CTATACGACTTTTGAATAT | 0.086 |
| AH0346 | No. 346 | UAUACGACUUUUGAAUAUCCC | No. 1526 | GAUAUUCAAAAGUCGUAUAGC | No. 2706 | TATACGACTTTTGAATATC | 0.220 |
| AH0349 | No. 349 | ACGACUUUUGAAUAUCCCAAC | No. 1529 | UGGGAUAUUCAAAAGUCGUAU | No. 2709 | ACGACTTTTGAATATCCCA | 0.316 |
| AH0350 | No. 350 | CGACUUUUGAAUAUCCCAACA | No. 1530 | UUGGGAUAUUCAAAAGUCGUA | No. 2710 | CGACTTTTGAATATCCCAA | 0.120 |
| AH0351 | No. 351 | GACUUUUGAAUAUCCCAACAC | No. 1531 | GUUGGGAUAUUCAAAAGUCGU | No. 2711 | GACTTTTGAATATCCCAAC | 0.230 |
| AH0352 | No. 352 | ACUUUUGAAUAUCCCAACACG | No. 1532 | UGUUGGGAUAUUCAAAAGUCG | No. 2712 | ACTTTTGAATATCCCAACA | 0.114 |
| AH0355 | No. 355 | UUUGAAUAUCCCAACACGAUC | No. 1535 | UCGUGUUGGGAUAUUCAAAAG | No. 2715 | TTTGAATATCCCAACACGA | 0.293 |
| AH0356 | No. 356 | UUGAAUAUCCCAACACGAUCA | No. 1536 | AUCGUGUUGGGAUAUUCAAAA | No. 2716 | TTGAATATCCCAACACGAT | 0.137 |
| AH0357 | No. 357 | UGAAUAUCCCAACACGAUCAG | No. 1537 | GAUCGUGUUGGGAUAUUCAAA | No. 2717 | TGAATATCCCAACACGATC | 0.280 |
| AH0358 | No. 358 | GAAUAUCCCAACACGAUCAGU | No. 1538 | UGAUCGUGUUGGGAUAUUCAA | No. 2718 | GAATATCCCAACACGATCA | 0.113 |
| AH0361 | No. 361 | UAUCCCAACACGAUCAGUUUU | No. 1541 | AACUGAUCGUGUUGGGAUAUU | No. 2721 | TATCCCAACACGATCAGTT | 0.244 |
| AH0362 | No. 362 | AUCCCAACACGAUCAGUUUUU | No. 1542 | AAACUGAUCGUGUUGGGAUAU | No. 2722 | ATCCCAACACGATCAGTTT | 0.194 |
| AH0363 | No. 363 | UCCCAACACGAUCAGUUUUUC | No. 1543 | AAAACUGAUCGUGUUGGGAUA | No. 2723 | TCCCAACACGATCAGTTTT | 0.332 |
| AH0364 | No. 364 | CCCAACACGAUCAGUUUUUCU | No. 1544 | AAAAACUGAUCGUGUUGGGAU | No. 2724 | CCCAACACGATCAGTTTTT | 0.171 |
| AH0365 | No. 365 | CCAACACGAUCAGUUUUUCUU | No. 1545 | GAAAAACUGAUCGUGUUGGGA | No. 2725 | CCAACACGATCAGTTTTTC | 0.165 |
| AH0366 | No. 366 | CAACACGAUCAGUUUUUCUUG | No. 1546 | AGAAAAACUGAUCGUGUUGGG | No. 2726 | CAACACGATCAGTTTTTCT | 0.119 |
| AH0367 | No. 367 | AACACGAUCAGUUUUUCUUGU | No. 1547 | AAGAAAAACUGAUCGUGUUGG | No. 2727 | AACACGATCAGTTTTTCTT | 0.216 |
| AH0369 | No. 369 | CACGAUCAGUUUUUCUUGUAA | No. 1549 | ACAAGAAAAACUGAUCGUGUU | No. 2729 | CACGATCAGTTTTTCTTGT | 0.131 |
| AH0370 | No. 370 | ACGAUCAGUUUUUCUUGUAAC | No. 1550 | UACAAGAAAAACUGAUCGUGU | No. 2730 | ACGATCAGTTTTTCTTGTA | 0.152 |
| AH0371 | No. 371 | CGAUCAGUUUUUCUUGUAACA | No. 1551 | UUACAAGAAAAACUGAUCGUG | No. 2731 | CGATCAGTTTTTCTTGTAA | 0.077 |
| AH0372 | No. 37 2 | GAUCAGUUUUUCUUGUAACAC | No. 1552 | GUUACAAGAAAAACUGAUCGU | No. 2732 | GATCAGTTTTTCTTGTAAC | 0.185 |
| AH0373 | No. 373 | AUCAGUUUUUGUUGUAACACU | No. 1553 | UGUUACAAGAAAAACUGAUCG | No. 2733 | ATCAGTTTTTCTTGTAACA | 0.141 |
| AH0375 | No. 375 | CAGUUUUUCUUGUAACACUGG | No. 1555 | AGUGUUACAAGAAAAACUGAU | No. 2735 | CAGTTTTTCTTGTAACACT | 0.139 |
| AH0376 | No. 376 | AGUUUUUCUUGUAACACUGGG | No. 1556 | CAGUGUUACAAGAAAAACUGA | No. 2736 | AGTTTTTCTTGTAACACTG | 0.233 |
| AH0382 | No. 382 | UCUUGUAACACUGGGUUUUAU | No. 1562 | AAAACCCAGUGUUACAAGAAA | No. 2742 | TCTTGTAACACTGGGTTTT | 0.134 |
| AH0383 | No. 383 | CUUGUAACACUGGGUUUUAUC | No. 1563 | UAAAACCCAGUGUUACAAGAA | No. 2743 | CTTGTAACACTGGGTTTTA | 0.134 |
| AH0384 | No. 384 | UUGUAACACUGGGUUUUAUCU | No. 1564 | AUAAAACCCAGUGUUACAAGA | No. 2744 | TTGTAACACTGGGTTTTAT | 0.074 |
| AH0385 | No. 385 | UGUAACACUGGGUUUUAUCUG | No. 1565 | GAUAAAACCCAGUGUUACAAG | No. 2745 | TGTAACACTGGGTTTTATC | 0.246 |
| AH0386 | No. 386 | GUAACACUGGGUUUUAUCUGA | No. 1566 | AGAUAAAACCCAGUGUUACAA | No. 2746 | GTAACACTGGGTTTTATCT | 0.131 |

### [Table 44]

**Table 4-6**

| Double stranded nucleic acid No. | SEQ ID NO. | Sense strand sequence (5'-3') | SEQ ID NO. | Antisense strand sequence (5'-3') | SEQ ID NO. | Target β2GPI mRNA sequence | Relative expressed amount of β2GPI |
|---|---|---|---|---|---|---|---|
| AH0388 | No. 388 | AACACUGGGUUUUAUCUGAAU | No. 1568 | UCAGAUAAAACCCAGUGUUAC | No. 2748 | AACACTGGGTTTTATCTGA | 0.107 |
| AH0389 | No. 389 | ACACUGGGUUUUAUCUGAAUG | No. 1569 | UUCAGAUAAAACCCAGUGUUA | No. 2749 | ACACTGGGTTTTATCTGAA | 0.275 |
| AH0390 | No. 390 | CACUGGGUUUUAUCUGAAUGG | No. 1570 | AUUCAGAUAAAACCCAGUGUU | No. 2750 | CACTGGGTTTTATCTGAAT | 0.294 |
| AH0392 | No. 392 | CUGGGUUUUAUCUGAAUGGCG | No. 1572 | CCAUUCAGAUAAAACCCAGUG | No. 2752 | CTGGGTTTTATCTGAATGG | 0.254 |
| AH0394 | No. 394 | GGGUUUUAUCUGAAUGGCGCU | No. 1574 | CGCCAUUCAGAUAAAACCCAG | No. 2754 | GGGTTTTATCTGAATGGCG | 0.080 |
| AH0395 | No. 395 | GGUUUUAUCUGAAUGGCGCUG | No. 1575 | GCGCCAUUCAGAUAAAACCCA | No. 2755 | GGTTTTATCTGAATGGCGC | 0.139 |
| AH0396 | No. 396 | GUUUUAUCUGAAUGGCGCUGA | No. 1576 | AGCGCCAUUCAGAUAAAACCC | No. 2756 | GTTTTATCTGAATGGCGCT | 0.169 |
| AH0399 | No. 399 | UUAUCUGAAUGGCGCUGAUUC | No. 1579 | AUCAGCGCCAUUCAGAUAAAA | No. 2759 | TTATCTGAATGGCGCTGAT | 0.113 |
| AH0402 | No. 402 | UCUGAUGGCGCUGAUUCUGC | No. 1582 | AGAAUCAGCGCCAUUCAGAUA | No. 2762 | TCTGAATGGCGCTGATTCT | 0.335 |
| AH0406 | No. 406 | AAUGGCGCUGAUUCUGCCAAG | No. 1586 | UGGCAGAAUCAGCGCCAUUCA | No. 2766 | AATGGCGCTGATTCTGCCA | 0.177 |
| AH0409 | No. 409 | GGCGCUGAUUCUGCCAAGUGC | No. 1589 | ACUUGGCAGAAUCAGCGCCAU | No. 2769 | GGCGCTGATTCTGCCAAGT | 0.189 |
| AH0410 | No. 410 | GCGCUGAUUCUGCCAAGUGCA | No. 1590 | CACUUGGCAGAAUCAGCGCCA | No. 2770 | GCGCTGATTCTGCCAAGTG | 0.251 |
| AH0411 | No. 411 | CGCUGAUUCUGCCAAGUGCAC | No. 1591 | GCACUUGGCAGAAUCAGCGCC | No. 2771 | CGCTGATTCTGCCAAGTGC | 0.070 |
| AH0412 | No. 412 | GCUGAUUCUGCCAAGUGCACU | No. 1592 | UGCACUUGGCAGAAUCAGCGC | No. 2772 | GCTGATTCTGCCAAGTGCA | 0.104 |
| AH0413 | No. 413 | CUGAUUCUGCCAAGUGCACUG | No. 1593 | GUGCACUUGGCAGAAUCAGCG | No. 2773 | CTGATTCTGCCAAGTGCAC | 0.217 |
| AH0414 | No. 414 | UGAUUCUGCCAAGUGCACUGA | No. 1594 | AGUGCACUUGGCAGAAUCAGC | No. 2774 | TGATTCTGCCAAGTGCACT | 0.301 |
| AH0415 | No. 415 | GAUUCUGCCAAGUGCACUGAG | No. 1595 | CAGUGCACUUGGCAGAAUCAG | No. 2775 | GATTCTGCCAAGTGCACTG | 0.151 |
| AH0419 | No. 419 | CUGCCAAGUGCACUGAGGAAG | No. 1599 | UCCUCAGUGCACUUGGCAGAA | No. 2779 | CTGCCAAGTGCACTGAGGA | 0.097 |
| AH0420 | No. 420 | UGCCAAGUGCACUGAGGAAGG | No. 1600 | UUCCUCAGUGCACUUGGCAGA | No. 2789 | TGCCAAGTGCACTGAGGAA | 0.184 |
| AH0422 | No. 422 | CCAAGUGCACUGAGGAAGGAA | No. 1602 | CCUUCCUCAGUGCACUUGGCA | No. 2782 | CCAAGTGCACTGAGGAAGG | 0.243 |
| AH0423 | No. 423 | CAAGUGCACUGAGGAAGGAAA | No. 1603 | UCCUUCCUCAGUGCACUUGGC | No. 2783 | CAAGTGCACTGAGGAAGGA | 0.095 |
| AH0424 | No. 424 | AAGUGCACUGAGGAAGGAAAA | No. 1604 | UUCCUUCCUCAGUGCACUUGG | No. 2784 | AAGTGCACTGAGGAAGGAA | 0.166 |
| AH0425 | No. 425 | AGUGCACUGACGAAGGAAAAU | No. 1605 | UUUCCUUCCUCAGUGCACUUG | No. 2785 | AGTGCACTGAGGAAGGAAA | 0.084 |
| AH0426 | No. 426 | GUGCACUGAGGAAGGAAAAUG | No. 1606 | UUUUCCUUCCUCAGUGCACUU | No. 2786 | GTGCACTGAGGAAGGAAAA | 0.299 |
| AH0427 | No. 427 | UGCACUGAGGAAGGAAAAUGG | No. 1607 | AUUUUCCUUCCUCAGUGCACU | No. 2787 | TGCACTGAGGAAGGAAAAT | 0.236 |
| AH0436 | No. 436 | GAAGGAAAUGGAGCCCGGAG | No. 1616 | CCGGGGUCCAUUUUCCUUCCU | No. 2796 | GAAGGAAAATGGAGCCCGG | 0.337 |
| AH0449 | No. 449 | GCCCGGAGCUUCCUGUCUGUG | No. 1629 | CAGACAGGAAGCUCCGGGCUC | No. 2809 | GCCCGGAGCTTCCTGTCTG | 0.166 |
| AH0450 | No. 450 | CCCGGACCUUCCUGUCUGUGC | No. 1630 | ACAGACAGGAAGCUCCGGGCU | No. 2810 | CCCGGAGCTTCCTGTCTGT | 0.138 |
| AH0453 | No. 453 | GGAGCUUCCUGUCUGUGCUCC | No. 1633 | AGCACAGACAGGAAGCUCCGG | No. 2813 | GGAGCTTCCTGTCTGTGCT | 0.128 |
| AH0454 | No. 454 | GAGCUUCCUGUCUGUGCUCCC | No. 1634 | GAGCACAGACAGGAAGCUCCG | No 2814 | GAGCTTCCTGTCTGTGCTC | 0.203 |
| AH0457 | No. 457 | CUUCCUGUCUGUGCUCCCAUC | No. 1637 | UGGGAGCACAGACAGGAAGCU | No. 2817 | CTTCCTGTCTGTGCTCCCA | 0.244 |
| AH0461 | No. 461 | CUGUCUGUGCUCCCAUCAUCU | No. 1641 | AUGAUGGGAGCACAGACAGGA | No. 2821 | CTGTCTGTGCTCCCATCAT | 0.306 |
| AH0463 | No. 463 | GUCUGUGCUCCCAUCAUCUGC | No. 1643 | AGAUGAUGGGAGCACAGACAG | No. 2823 | GTCTGTGCTCCCATCATCT | 0.257 |
| AH0472 | No. 472 | CCCAUCAUCUGCCCUCCACCA | No. 1652 | GUGGAGGGCAGAUGAUGGGAG | No. 2832 | CCCATCATCTGCCCTCCAC | 0.196 |
| AH0475 | No. 475 | AUCAUCUGCCCUCCACCAUCC | No. 1656 | AUGGUGGAGGGCAGAUGAUGG | No. 2835 | ATCATCTGCCCTCCACCAT | 0.250 |
| AH0476 | No. 476 | UCAUCUGCCCUCCACCAUCCA | No. 1656 | GAUGGUGGAGGGCAGAUGAUG | No. 2836 | TCATCTGCCCTCCACCATC | 0.249 |
| AH0480 | No. 480 | CUGCCCUCCACCAUCCAUACC | No. 1660 | UAUGGAUGGUGGAGGGCAGAU | No. 2840 | CTGCCCTCCACCATCCATA | 0.291 |
| AH0484 | No. 484 | CCUCCACCAUCCAUACCUACG | No. 1664 | UAGGUAUGGAUGGUGGAGGGC | No. 2844 | CCTCCACCATCCATACCTA | 0.113 |
| AH0487 | No. 487 | CCACCAUCCAUACCUACGUUU | No. 1667 | ACGUAGGUAUGGAUGGUGGAG | No. 2847 | CCACCATCCATACCTACGT | 0.213 |
| AH0488 | No. 488 | CACCAUCCAUCCAUACGUUUG | No. 1668 | AACGUAGGUAUGGAUGGUGGA | No. 2848 | CACCATCCATAACTACGT | 0.210 |
| AH0489 | No. 489 | ACCAUCCAUACCUACGUUUGC | No. 1669 | AAACGUAGGUAUGGAUGGUGG | No. 2840 | ACCATCCATACCTACGTT | 0.160 |
| AH0490 | No. 490 | CCAUCCAUACCUACGUUUGCA | No. 1670 | CAAACGUAGGUAUGGAUGGUG | No. 2850 | CCATCCATACCTACGTTTG | 0.171 |
| AH0491 | No. 491 | CAUCCAUACCUACGUUUGCAA | No. 1671 | GCAAACGUAGGUAUGGAUGGU | No. 2851 | CATCCATACCTACGTTTGC | 0.078 |
| AH0492 | No. 492 | AUCCAUACCUACGUUUGCAAC | No. 1672 | UGCAAACGUAGGUAUGGAUGG | No. 2852 | ATCCATACCTACGTTTGCA | 0.153 |
| AH0493 | No. 493 | UCCAUACCUACGUUUGCAACA | No. 1673 | UUGCAAACGUAGGUAUGGAUG | No. 2853 | TCCATACCTACGTTTGCAA | 0.157 |
| AH0495 | No. 495 | CAUACCUACGUUUGCAACACU | No. 1675 | UGUUGCAAACGUAGGUAUGGA | No. 2855 | CATACCTACGTTTGCAACA | 0.161 |

### [Table 45]

**Table 4-7**

| Double stranded nucleic acid No. | SEQ ID NO. | Sense strand sequence (5'→3') | SEQ ID NO. | Antisense strand sequence (5'→3') | SEQ ID NO. | Target β2GPI mRNA sequence | Relative expressed amount of β2GPI |
|---|---|---|---|---|---|---|---|
| AH0498 | No. 498 | ACCUACGUUUGCAACACUUCG | No. 1678 | AAGUGUUGCAAACGUAGGUAU | No. 2858 | ACCTACGTTTGCAACACTT | 0.214 |
| AH0499 | No. 499 | CCUACGUUUGCAACACUUCGU | No. 1679 | GAAGUGUUGCAAACGUAGGUA | No. 2859 | CCTACGTTTGCAACACTTC | 0.213 |
| AH0500 | No. 500 | CUACGUUUGCAACACUUCGUG | No. 1680 | CGAAGUGUUGCAAACGUAGGU | No. 2860 | CTACGTTTGCAACACTTCG | 0.150 |
| AH0501 | No. 501 | UACGUUUGCAACACUUCGUGU | No. 1681 | ACGAAGUGUUGCAAACGUAGG | No. 2861 | TACGTTTGCAACACTTCGT | 0.201 |
| AH0502 | No. 502 | ACGUUUGCAACACUUCGUGUU | No. 1682 | CACGAAGUGUUGCAAACGUAG | No. 2862 | ACGTTTGCAACACTTCGTG | 0.159 |
| AH0503 | No. 503 | CGUUUGCAACACUUCGUGUUU | No. 1683 | ACACGAAGUGUUGCAAACGUA | No. 2863 | CGTTTGCAACACTTCGTGT | 0.126 |
| AH0504 | No. 504 | GUUUGCAACACUUCGUGUUUA | No. 1684 | AACACGAAGUGUUGCAAACGU | No. 2864 | GTTTGCAACACTTCGTGTT | 0.055 |
| AH0505 | No. 505 | UUUGCAACACUUUCGUGUUUAU | No. 1685 | AAACACGAAGUGUUGCAAACG | No. 2865 | TTTGCAACACTTCGTGTTT | 0.170 |
| AH0506 | No. 506 | UUGCAACACUUCGUGUUUAUA | No. 1686 | UAAACACGAAGUGUUGCAAAC | No. 2866 | TTGCAACACTTCGTGTTTA | 0.076 |
| AH0507 | No. 507 | UGCAACACUUCGUGUUUAUAA | No. 1687 | AUAAACACGAAGUGUUGCAAA | No. 2867 | TGCAACACTTCGTGTTTAT | 0.091 |
| AH0508 | No. 508 | GCAACACUUCGUGUUUAUAAG | No. 1688 | UAUAAACACGAAGUGUUGCAA | No. 2868 | GCAACACTTCGTGTTTATA | 0.146 |
| AH0509 | No. 509 | CAACACUUCGUGUUUAUAAGC | No. 1689 | UUAUAAACACGAAGUGUUGCA | No. 2869 | CAACACTTCGTGTTTATAA | 0.125 |
| AH0510 | No. 510 | AACACUUCGUGUUUAUAAGCC | No. 1690 | CUUAUAAACACGAAGUGUUGC | No. 2870 | AACACTTCGTGTTTATAAG | 0.330 |
| AH0513 | No. 513 | ACUUCGUGUUUAUAAGCCAUC | No. 1693 | UGGCUUAUAAACACGAAGUGU | No 2873 | ACTTCGTGTTTATAAGCCA | 0.119 |
| AH0514 | No. 514 | CUUCGUGUUUAUAAGCCAUCA | No. 1694 | AUGGCUUAUAAACACGAAGUG | No. 2874 | CTTCGTGTTTATAAGCCAT | 0.118 |
| AH0518 | No. 518 | GUGUUUAUAAGCCAUCAGCUG | No. 1698 | GCUGAUGGCUUAUAAACACGA | Nο. 2878 | GTGTTTATAAGCCATCAGC | 0.233 |
| AH0519 | No. 519 | UGUUUAUAAGCCAUCAGCUGG | No. 1699 | AGCUGAUGGCUUAUAAACACG | No. 2879 | TGTTTATAAGCCATCAGCT | 0.297 |
| AH0520 | No. 520 | GUUUAUAAGCCAUCAGCUGGA | No. 1700 | CAGCUGAUGGCUUAUAAACAC | No. 2880 | GTTTATAAGCCATCAGCTG | 0.216 |
| AH0526 | No. 526 | AAGCCAUCAGCUGGAAACAAU | No. 1706 | UGUUUCCAGCUGAUGGCUUAU | No. 2886 | AAGCCATCAGCTGGAAACA | 0.299 |
| AH0527 | No. 527 | AGCCAUCAGCUGGAAACAAUU | No. 1707 | UUGUUUCCAGCUGAUGGCUUA | No. 2887 | AGCCATCAGCTGGAAACAA | 0.258 |
| AH0528 | No. 528 | GCCAUCAGCUGGAAACAAUUC | No. 1708 | AUUGUUUCCAGCUGAUGGCUU | No. 2888 | GCCATCAGCTGGAAACAAT | 0.091 |
| AH0529 | No. 529 | CCAUCAGCUGGAAACAAUUCC | No. 1709 | AAUUGUUUCCAGCUGAUGGCU | No. 2889 | CCATCAGCTGGAAACAATT | 0.067 |
| AH0530 | No. 530 | CAUCAGCUGGAAACAAUUCCC | No. 1710 | GAAUUGUUUCCAGCUGAUGGC | No. 2890 | CATCAGCTGGAAACAATTC | 0.229 |
| AH0534 | No. 534 | AGCUGGAAACAAUUGCCUCUA | No. 1714 | GAGGGAAUUGUUUCCAGCUGA | No. 2894 | AGCTGGAAACAATTCCCTC | 0.183 |
| AH0535 | No. 535 | GCUGGAAACAAUUCCCUCUAU | No. 1715 | AGAGGGAAUUGUUUCCAGCUG | No. 2895 | GCTGGAAACAATTCCCTCT | 0.202 |
| AH0538 | No. 538 | GGAAACAAUUCCCUCUAUCGG | No. 1718 | GAUAGAGGGAAUUGUUUCCAG | No. 2898 | GGAAACAATTCCCTCTATC | 0.245 |
| AH0542 | No. 542 | ACAAUUCCCUCUAUCGGGACA | No. 1722 | UCCCGAUAGAGGGAAUUGUUU | No. 2902 | ACAATTCCCTCTATCGGGA | 0.102 |
| AH0549 | No. 549 | CCUCUAUCGGGACACAGCAGU | No. 1729 | UGCUGUGUCCCGAUAGAGGGA | No. 2909 | CCTCTATCGGGACACAGCA | 0.275 |
| AH0552 | No. 552 | CCUCUAUCGGGACACAGCAGU | No. 1732 | AACUGCUGUGUCCCGAUAGAG | No. 2912 | CTATCGGGACACAGCAGTT | 0.230 |
| AH0553 | No. 553 | UAUCGGGACACAGCAGUUUUU | No. 1733 | AAACUGCUGUGUCCCGAUAGA | No. 2913 | TATCGGGACACAGCAGTTT | 0.289 |
| AH0554 | No. 554 | AUCGGGACACAGCAGUUUUUG | No. 1734 | AAAACUGCUGUGUCCCGAUAG | No. 2914 | ATCGGGACACAGCAGTTTT | 0.324 |
| AH0555 | No. 555 | UCGGGACACAGCAGUUUUUGA | No. 1735 | AAAAACUGCUGUGUCCCGAUA | No. 2915 | TCGGGACACAGCAGTTTTT | 0.203 |
| AH0557 | No. 557 | GGGACACAGCAGUUUUUGAAU | No. 1737 | UCAAAAACUGCUGUGUCCCGA | No. 2917 | GGGACACAGCAGTTTTGA | 0.208 |
| AH0558 | No. 558 | GGACACAGCAGUUUUUGAAUG | No. 1738 | UUCAAAAACUGCUGUGUCCCG | No. 2918 | GGACACAGCAGTTTTTGAA | 0.237 |
| AH0559 | No. 559 | GACACAGCAGUUUUUGAAUGU | No. 1739 | AUUCAAAAACUGCUGUGUCCC | No. 2919 | GACACAGCAGTTTTTGAAT | 0.226 |
| AH0561 | No. 561 | CACAGCAGUUUUUGAAUGUUU | No. 1741 | ACAUUCAAAAACUGCUGUGUC | No. 2921 | CACAGCAGTTTTTGAATGT | 0.151 |
| AH0562 | No. 562 | ACAGCAGUUUUUGAAUGUUUG | No. 1742 | AACAUUCAAAAACUGCUGUGU | No. 2922 | ACAGCAGTTTTTGAATGTT | 0.096 |
| | No. 563 | CAGCAGUUUUUGAAUGUUUGC | No. 1743 | AAACAUUCAAAAACUGCUGUG | No. 2923 | CAGCAGTTTTTGAATGTTT | 0.077 |
| AH0564 | No. 564 | AGCAGUUUUUGAAUGUUUGCC | No. 1744 | CAAACAUUCAAAAACUGCUGU | No. 2924 | AGCAGTTTTTGAATGTTTG | 0.206 |
| AH0565 | No. 565 | GCAGUUUUUGAAUGUUUGCCA | No. 1745 | GCAAACAUUCAAAAACUGCUG | No. 2925 | GCAGTTTTTGAATGTTTGC | 0.080 |
| AH0567 | No. 567 | AGUUUUUGAAUGUUUGCCACA | No. 1747 | UGGCAAACAUUCAAAAACUGC | No. 2927 | AGTTTTTGAATGTTTGCCA | 0.078 |
| AH0568 | No. 568 | GUUUUUGAAUGUUUGCCACAA | No. 1748 | GUGGCAAACAUUCAAAAACUG | No. 2928 | GTTTTTGAATGTTTGCCAC | 0.250 |
| AH0570 | No. 570 | UUUUGAAUGUUUGCCACAACA | No. 1750 | UUGUGGCAAACAUUCAAAAAC | No. 2930 | TTTTGAATGTTTGCCACAA | 0.286 |
| AH0572 | No. 572 | UUGAAUGUUUGCCACAACAUG | No. 1752 | UGUUGUGGCAAACAUUCAAAA | No. 2932 | TTGAATGTTTGCCACAACA | 0.152 |
| AH0573 | No. 573 | UGAAUGUUUGCCACAACAUGC | No. 1753 | AUGUUGUGGCAAACAUUCAAA | No. 2933 | TGAATGTTTGCCACAACAT | 0.266 |
| AH0574 0574 | No. 574 | GAAUGUUUGCCACAACAUGCG | No. 1754 | CAUGUUGUGGCAAACAUUCAA | No. 2934 | GAATGTTTGCCACAACATG | 0.142 |

### [Table 46]

**Table 4-8**

| Double stranded nucleic acid No. | SEQ ID NO. | Sense strand sequence (5'→3') | SEQ ID NO. | Antisense strand sequence (5'→3') | SEQ ID NO. | Target β2GPI mRNA sequence | Relative expressed amount of β2GPI |
|---|---|---|---|---|---|---|---|
| AH0578 | No. 578 | GUUUGCCACAACAUGCGAUGU | No. 1758 | AUCGCAUGUUGUGGCAAACAU | No. 2938 | GTTTGCCACAACATGCGAT | 0.120 |
| AH0582 | No. 582 | GCCACAACAUGCGAUGUUUGG | No. 1762 | AAACAUCGCAUGUUGUGGCAA | No. 2942 | GCCACAACATGCGATGTTT | 0.208 |
| AH0583 | No. 583 | CCACAACAUGCGAUGUUUGGA | No. 1763 | CAAACAUCGCAUGUUGUGGCA | No. 2943 | CCACAACATGCGATGTTTG | 0.222 |
| AH0585 | No. 585 | ACAACAUGCGAUGUUUGGAAA | No. 1765 | UCCAAACAUCGCAUGUUGUGG | No. 2945 | ACAACATGCGATGTTTGGA | 0.188 |
| AH0586 | No. 586 | CAACAUGCGAUGUUUGGAAAU | No. 1766 | UUCCAAACAUCGCAUGUUGUG | No. 2946 | CAACATGCGATGTTTGGAA | 0.151 |
| AH0588 | No. 588 | ACAUGCGAUGUUUGGAAAUGA | No. 1768 | AUUUCCAAACAUCGCAUGUUG | No. 2948 | ACATGCGATGTTTGGAAAT | 0.261 |
| AH0590 | No. 590 | AUGCGAUGUUUGGAAAUGAUA | No. 1770 | UCAUUUCCAAACAUCGCAUGU | No. 2950 | ATGCGATGTTTGGAAATGA | 0.248 |
| AH0591 | No. 591 | UGCGAUGUUUGGAAAUGAUAC | No. 1771 | AUCAUUUCCAAACAUCGCAUG | No. 2951 | TGCGATGTTTGGAAATGAT | 0.183 |
| AH0592 | No. 592 | GCGAUGUUUGGAAAUGAUACA | No. 1772 | UAUCAUUUCCAAACAUCGCAU | No. 2952 | GCGATGTTTGGAAATGATA | 0.098 |
| AH0593 | No. 593 | CGAUGUUUGGAAAUGAUACAA | No. 1773 | GUAUCAUUUCCAAACAUCGCA | No. 2953 | CGATGTTTGGAAATGATAC | 0.135 |
| AH0594 | No. 594 | GAUGUUUGGAAAUGAUACAAU | No. 1774 | UGUAUCAUUUCCAAACAUCGC | No. 2954 | GATGTTTGGAAATGATACA | 0.114 |
| AH0595 | No. 595 | AUGUUUGGAAAUGAUACAAUU | No. 1775 | UUGUAUCAUUUCCAAACAUCG | No. 2955 | ATGTTTGGAAATGATACAA | 0.333 |
| AH0597 | No. 597 | GUUUGGAAAUGAUACAAUUAC | No. 1777 | AAUUGUAUCAUUUCCAAACAU | No. 2957 | GTTTGGAAATGATACAATT | 0.143 |
| AH0598 | No. 598 | UUUGGAAAUGAUACAAUUACC | No. 1778 | UAAUUGUAUCAUUUCCAAACA | No. 2958 | TTTGGAAATGATACAATTA | 0.166 |
| AH0601 | No. 601 | GGAAAUGAUACAAUUACCUGC | No. 1781 | AGGUAAUUGUAUCAUUUCCAA | No. 2961 | GGAAATGATACAATTACCT | 0.177 |
| AH0607 | No. 607 | GAUACAAUUACCUGCACGACA | No. 1787 | UCGUGCAGGUAAUUGUAUCAU | No. 2967 | GATACAATTACCTGCACGA | 0.093 |
| AH0609 | No. 609 | UACAAUUACCUGCACGACACA | No. 1789 | UGUCGUGCAGGUAAUUGUAUC | No. 2969 | TACAATTACCTGCACGACA | 0.159 |
| AH0610 | No. 610 | ACAAUUACCUGCACGACACAU | No. 1790 | GUGUCGUGCAGGUAAUUGUAU | No. 2970 | ACAATTACCTGCACGACAC | 0. 59 |
| AH0611 | No. 611 | CAAUUACCUGCACGACACAUG | No. 1791 | UGUGUCGUGCAGGUAAUUGUA | No. 2971 | CAATTACCTGCACGACACA | 0.156 |
| AH0612 | No. 612 | AAUUACCUGCACGACACAUGG | No. 1792 | AUGUGUCGUGCAGGUAAUUGU | No. 2972 | AATTACCTGCACGACACAT | 0.257 |
| AH0616 | No. 616 | ACCUGCACGACACAUGGAAAU | No. 1796 | UUCCAUGUGUCGUGCAGGUAA | No. 2976 | ACCTGCACGACACATGGAA | 0.292 |
| AH0617 | No. 617 | CCUGCACGACACAUGGAAAUU | No. 1797 | UUUCCAUGUGUCGUGCAGGUA | No. 2977 | CCTGCACGACACATGGAAA | 0.148 |
| AH0618 | No. 618 | CUGCACGACACAUGGAAAUUG | No. 1798 | AUUUCCAUGUGUCGUGCAGGU | Nο. 2978 | CTGCACGACACATGGAAAT | 0.118 |
| AH0622 | No. 622 | ACGACACAUGGAAAUUGGACU | No. 1802 | UCCAAUUUCCAUGUGUCGUGC | No. 2982 | ACGACACATGGAAATTGGA | 0.310 |
| AH0624 | No. 624 | GACACAUGGAAAUUGGACUAA | No. 1804 | AGUCCAAUUUCCAUGUGUCGU | No. 2984 | GACACATGGAAATTGGACT | 0.240 |
| AH0625 | No. 625 | ACACAUGGAAAUUGGACUAAA | No. 1805 | UAGUCCAAUUUCCAUGUGUCG | No. 2985 | ACACATGGAAATTGGACTA | 0.184 |
| AH0627 | No. 627 | ACAUGGAAAUUGGACUAAAUU | No. 1807 | UUUAGUCCAAUUUCCAUGUGU | No. 2987 | ACATGGAAATTGGACTAAA | 0.104 |
| AH0628 | No. 628 | CAUGGAAAUUGGACUAAAUUA | No. 1808 | AUUUAGUCCAAUUUCCAUGUG | No. 2988 | CATGGAAATTGGACTAAAT | 0.163 |
| AH0629 | No. 629 | AUGGAAAUUGGACUAAAUUAC | No. 1809 | AAUUUAGUCCAAUUUCCAUGU | No. 2989 | ATGGAAATTGGACTAAATT | 0.298 |
| AH0630 | No. 630 | UGGAAAUUGGACUAAAUUACC | No. 1810 | UAAUUUAGUCCAAUUUCCAUG | No. 2990 | TGGAAATTGGACTAAATTA | 0.215 |
| AH0631 | No. 631 | GGAAAUUGGACUAAAUUACCA | No. 1811 | GUAAUUUAGUCCAAUUUCCAU | No. 2991 | GGAAATTGGACTAAATTAC | 0.307 |
| AH0633 | No. 633 | AAAUUGGACUAAAUUACCAGA | No. 1813 | UGGUAAUUUAGUCCAAUUUCC | No. 2993 | AAATTGGACTAAATTACCA | 0.306 |
| AH0638 | No. 638 | GGACUAAAUUACCAGAAUGCA | No. 1818 | CAUUCUGGUAAUUUAGUCCAA | No. 2998 | GGACTAAATTACCAGAATG | 0.128 |
| AH0649 | No. 649 | CCAGAAUGCAGGGAAGUAAAA | No. 1829 | UUACUUCCCUGCAUUCUGGUA | No. 3009 | CCAGAATGCAGGGAAGTAA | 0.139 |
| AH0650 | No. 650 | CAGAAUGCAGGGAAGUAAAAU | No. 1830 | UUUACUUCCCUGCAUUCUGGU | No. 3010 | CAGAATGCAGGGAAGTAAA | 0.126 |
| AH0651 | No. 651 | AGAAUGCAGGGAAGUAAAAUG | No. 1831 | UUUUACUUCCCUGCAUUCUGG | No. 3011 | AGAATGCAGGGAAGTAAAA | 0.117 |
| AH0652 | No. 652 | GAAUGCAGGGAAGUAAAAUGC | No. 1832 | AUUUUACUUCCCUGCAUUCUG | No. 3012 | GAATGCAGGGAAGTAAAAT | 0.331 |
| AH0653 | No. 653 | AAUGCAGGGAAGUAAAAUGCC | No. 1833 | CAUUUUACUUCCCUGCAUUCU | No. 3013 | AATGCAGGGAAGTAAAATG | 0.198 |
| AH0657 | No. 657 | CAGGGAAGUAAAAUGCCCAUU | No. 1837 | UGGGCAUUUUACUUCCCUGCA | No. 3017 | CAGGGAAGTAAAATGCCCA | 0.139 |
| AH0661 | No. 661 | GAAGUAAAAUGCCCAUUCCCA | No. 1841 | GGAAUGGGCAUUUUACUUCCC | No. 3021 | GAAGTAAAATGCCCATTCC | 0.321 |
| AH0664 | No. 664 | GUAAAAUGCCCAUUCCCAUCA | No. 1844 | AUGGGAAUGGGCAUUUUACUU | No. 3024 | GTAAAATGCCCATTCCCAT | 0.091 |
| AH0669 | No. 669 | AUGCCCAUUCCCAUCAAGACC | No. 1849 | UCUUGAUGGGAAUGGGCAUUU | No. 3029 | ATGCCCATTCCCATCAAGA | 0.249 |
| AH0670 | No. 670 | UGCCCAUUCCCAUCAAGACCA | No. 1850 | GUCUUGAUGGGAAUGGGCAUU | No. 3030 | TGCCCATTCCCATCAAGAC | 0.160 |
| AH0672 | No. 672 | CCCAUUCCCAUCAAGACCAGA | No. 1852 | UGGUCUUGAGGGAAUGGGGCA | No. 3032 | CCCATTCCCATCAAGACCA | 0.097 |
| AH0673 | No. 673 | CCAUUCCCAUCAAGACCAGAC | No. 1853 | CUGGUCUUGAUGGGAAUGGGC | No. 3033 | CCATTCCCATCAAGACCAG | 0.137 |
| AH0674 | No. 674 | CAUUCCCAUCAAGACCAGACA | No. 1854 | UCUGGUCUUGAUGGGAAUGGG | No. 3034 | CATTCCCATCAAGACCAGA | 0.335 |

### [Table 47]

**Table 4-9**

| Double stranded nucleid acid No | SEQ ID NO. | Sense strand sequence (5→3) | SEQ ID NO. | Antisense strand sequence (5'-3') | SEQ ID No. | Target β2GPI mRNA sequence | Relative expressed amount of β2GPI |
|---|---|---|---|---|---|---|---|
| AH0678 | No. 678 | CCCUAAGACCAGACAAUGG | No. 1858 | AUUGUCUGGUCUUGAUGGGAA | No. 3038 | CCCATCAAGACCAGACAAT | 0.139 |
| AH0679 | No. 679 | CCAUCAAGACCAGACAAUGGA | No. 1859 | CAUUGUCUGGUCUUGAUGGGA | No. 3039 | CCATCAAGACCAGACAATG | 0.148 |
| AH0680 | No. 680 | CAUCAAGACCAGACAAUGGAU | No. 1860 | CCAUUGUCUGGUCUUGAUGGG | No. 3040 | CATCAAGACCAGACAATGG | 0.286 |
| AH0681 | No. 681 | AUCAAGACCAGACAAUGGAUU | No. 1861 | UCCAUUGUCUGGUCUUGAUGG | No. 3041 | ATCAAGACCAGACAATGGA | 0.159 |
| AH0682 | No. 682 | UCAAGACCAGACAAUGGAUUU | No. 1862 | AUCCAUUGUCUGGUCUUGAUG | No. 3042 | TCAAGACCAGACAATGGAT | 0.218 |
| AH0683 | No. 683 | CAAGACCAGACAAUGGAUUUG | No. 1863 | AAUCCAUUGUCUGGUCUUGAU | No. 3043 | CAAGACCAGACAATGGATT | 0.096 |
| AH0684 | No. 684 | AAGACCAGACAAUGGAUUUUGU | No. 1864 | AAAUCCAUUGUCUGGUCUUGA | No. 3044 | AAGACCAGACAATGGTTT | 0.220 |
| AH0685 | No. 685 | AGACCAGACAAUGGAUUUGUG | No. 1865 | CAAUCCAUUGUCUGGUCUUG | No. 3045 | AGACCAGACAATGGATTTG | 0.35 |
| AH0687 | No. 687 | ACCAGACAAUGGAUUUGUGAA | No. 1867 | CACAAAUCCAUUGUCUGGUCU | No. 3047 | ACCAGACAATGGATTTGTG 6 | 0.46 |
| AH0688 | No. 688 | CCAGACAAUGGAUUUGAAAC | No. 1868 | UCACAAAUCCAUUGUCUGGUC | No. 3048 | CCAGACAATGGATTTGTGA | 0.114 |
| AH0689 | No. 689 | CAGACAAUGGAUUUGAACU | No. 1869 | UUCACAAAUCCAUUGUCUGGU | No. 3049 | CAGACAATGGATTTGTGAA | 0.067 |
| AH0690 | No. 690 | AGACAAUGGAUUUGUGAACUA | No. 1870 | GUUCACAAAUCCAUUGUCUGG | No. 3060 | AGACAATGGATTTGTGAAC | 0.280 |
| AH0692 | No. 692 | ACAAUGGAUUUGUGAACUAUC | No. 1872 | UAGUUCACAAAUCCAUUGUCU | No. 3052 | ACAATGGATTTGTGAACTA | 0.296 |
| AH0693 | No. 693 | CAAUGGAUUUGUGAACUAUCC | No. 1873 | AUAGUUCACAAAUCCAUUGUC | No. 3053 | CAATGGATTTGTGAACTAT | 0.086 |
| AH0694 | No. 694 | AAUGGAUUUGUGAACUAUCCU | No. 1874 | GAUAGUUCACAAAUCCAUUGU | No. 3054 | AATGGATTTGTGAACTATC | 0.267 |
| AH0697 | No. 697 | GGAUUUGUGAACUAUCCUGCA | No. 1877 | CAGGAUAGUUCACAAAUCCAU | No. 3057 | GGATTTGTGAACATCCTG | 0.196 |
| AH0699 | No. 699 | AUUUGUGAACUAUCCUGCAAA | No. 1879 | UGCAGGAUAGUUCACAAAUCC | No. 3059 | ATTTGTGAACTACCTGCA | 0.218 |
| AH0700 | No. 700 | UUUGUGAACUAUCCUGCAAAA | No. 1880 | UUGCAGGAUGUUCACAAAUC | No. 3060 | TTTGTGAACTATCCTGCAA | 0.319 |
| AH0701 | No. 701 | UUGUGAACUAUCCUGCAAAAC | No. 1881 | UUUGCAGGAUAGUUCACAAAU | No. 3061 | TTGTGAACTATCCTGCAAA | 0.112 |
| AH0702 | No. 702 | UGUGAACUAUCCUGCAAAACC | No. 1882 | UUUUGCAGGAUAGUUCACAAA | No. 3062 | TGTGAACTATCCTGCAAAA | 0.231 |
| AH0705 | No. 705 | GAACUAUCCUGCAAAACCAAC | No. 1885 | UGGUUUUGCAGGAUAGUUCAC | No. 3066 | GAACTATCCTGCAAAACCA | 0.05 |
| HA0706 | No. 706 | AACUAUCCUGCAAAACCAACA | No. 1886 | UUGGUUUGCAGGAUAGUUCA | No. 3066 | AACATCCTGCAAAACCAA | 0.092 |
| AH0708 | No. 708 | CUAUCCUGCAAAACCAACACU | No. 1888 | UGUUGGUUUUGC2GGAGUU | No. 3068 | CTACCCTGCAAAACCAACA | 0.088 |
| AH0709 | No. 709 | UAUCCUGCAAAAGCAACACUU | No. 1889 | GUGUUGGUUUGCAGGAUAUGU | No. 3069 | TATCCTGCAAAACCAACAC | 0.340 |
| AH0711 | No. 711 | UCCUGCAAAACCAACACUUUA | No. 1891 | AAGUGUUGGUUUUGCAGGAUA | No. 3071 | TCCTGCAAAACCAACACTT | 0.146 |
| AH0712 | No. 712 | CUGCAAAACCAACACUUUAU | No. 1892 | AAAGUGUUGGUUUUGGAU | No. 3072 | CCTGCAAAACCAACACTTT | 0.060 |
| AH0713 | No. 713 | CUGCAAAACCAACACUUUAUU | No. 1893 | UAAAGUGUUGGUU UUGCAGGA | No. 3073 | CTGCAAAACCAACACTTTA | 0.059 |
| AH0714 | No. 714 | UGCAAAACCAACACUUUAUUA | No. 1894 | AUAAAGUGUUGGUUUUGCAGG | No. 3074 | TGCAAAACCAACACTTTAT | 0.092 |
| AH0715 | No. 715 | GCAAAACCAACACUUUAUUAC | No. 1895 | AAUAAAGUGUUGGUUUUGCAG | No. 3075 | GCAAAACCAACACTTTATT | 0.183 |
| AH0716 | No. 716 | CAAAACCAACACUUUAUUACA | No. 1896 | UAAAUAAGUGUUGGUUUUGCA | No. 3076 | CAAAACCAACACTTTATTA | 0.180 |
| AH0718 | No. 718 | AAACCAACACUUUAUUACAAG | No. 1898 | UGUAAUAAAGUGUUGGUUUUG | No. 3078 | AAACCAACACTTTATTACA | 0.133 |
| AH0719 | No. 719 | AACCAACACUUUAUUACAAGG | No. 1899 | UUGUAAUAAAGUGUUGGUUUU | No. 3079 | AACCAACACTTTATTACAA | 0.202 |
| AH0121 | No. 721 | CCAACACUUUAUUACAAGGAU | No. 1901 | CCUUGUAAUAAAGUGUUGGUU | No. 306 | CCAACACTTTATTACAAGG | 0.247 |
| AH0723 | No. 723 | AACACUUUAUUACAAGGAUAA | No. 1903 | AUCCUUG UAAUAAAGUGUUGG | No. 3083 | AACACTTTATTACAAGGAT | 0.174 |
| AH0724 | No. 724 | ACACUUUAUUACAAGGAUAAA | No. 1904 | UAUCCUUGUAAUAAAGUGUUG | No. 3084 | ACACTTTATTTACAAGGATA | 0.090 |
| AH0725 | No. 725 | CACUUUAUUACAAGGAUAAAG | No. 1905 | _{'}JUAUCCUUGUAAl A AAGWSJU | No. 3085 | CACTTTATTACAAGGATAA | 0.146 |
| AH0735 | Nο. 735 | CAAGGAUAAAGCCACAUUUGG | No. 1915 | AAAUGUGGCUUU..UCCUUGUA | No. 3095 | CAAGGATAAAGCCACATTT | 0.134 |
| AH0737 | No. 737 | AGGAUAAAGCCACAUUUGGCU | No. 1917 | CCAAAUGUGGCUUUAUCCUUG | No. 3097 | AGGATAAAGCCACATTTGG | 0.321 |
| AH0739 | Nο. 739 | GAUAAAGCCACAUUUGGCUGC | No. 1919 | AGCCAAAUGUGGUUUAUCCU | No. 3099 | GATAAAGCCACATTTGGCT | 0.304 |
| AH0743 | No. 743 | AAGCCACAUUUGGCUGCCAUG | No. 1923 | UGGCAGCCAAAUGUGGCUUUA | No. 3103 | AAGCCACATTGGCTGCCA | 0.216 |
| AH0744 | No. 744 | AGCCACAUUUGGCUGCCAUGA | No. 1924 | AUGGCAGCCAAAUGUGGCUUU | No. 3104 | AGCCACATTTGCTGCCAT | 0.306 |
| AH0745 | No. 745 | GCCACAUUUGGCUGCCAUGAU | No. 1925 | CAUGGCAGCCAAAUGUGGCUU | No. 3105 | GCCACATTTGGCTGCCATG | 0.112 |
| AH0746 | No. 746 | CCACAUUUGGCUGCCAUGAUG | No. 1926 | UCAUGGCAGCCAAAUGUGGCU | No. 3106 | CCACATTTGGCTGCCCATGA | 0.342 |
| AH0747 | No. 747 | CACAUUUGGCUGCCAUGAUGG | No. 1927 | AUCAUGGCAGCCAAAUGUGGC | No. 3107 | CACATTTGGCTGCATGAT | 0.185 |
| AH0748 | No. 748 | ACAUUUGGCUGCCAUGAUGGA | No. 1928 | CAUCAU GGCAGCCAAAUGUGG | No. 3108 | ACATTTGGCTGATGTG | 0.107 |
| AH0749 | No. 749 | CAUUUGGCUGCCAUGAUGGAU | No. 1929 | CCAUCAUGGCAGCCAAAUGUG GGCAGCCAAAUGUG | No. 3109 | CATTTGGCTGCCATGATGATGG | 0.214 |

### [Table 48]

**Table 4-10**

| Doublestranded nudec acif No. | SEQ ID NO | Sense strand sequence (5'→3') | SEQ ID No. | Antisense strand sequence (5→3) | SEQ ID NO. | Target β2GPI mRNA sequence | Relative exprssed armont of β2GPI |
|---|---|---|---|---|---|---|---|
| AH0750 | No. 750 | AUUUGGCUGCCAUGAUGGAUA | No. 1930 | UCCAUCAUGGCAGCCAAAUGU | No. 3110 | ATTTGGCTGCCATGATGGA | 0.128 |
| AH0753 | No. 753 | UGGCUGCCAUGAUGGAUAUUC | No. 1933 | AUAUCCAUCAUGGCAGCCAAA | No. 3113 | TGGCTGCCATGATGGATAT | 0.117 |
| AH0754 | No. 754 | GGCUGCCAUGAUGGAUAUUCU | No. 1934 | AAUAUCCAUCAUGGCAGCCAA | No. 3114 | GGCTGCCATGATGGATATT | 0.162 |
| AH0756 | No. 766 | UGCCAUGAUGGAUAUUCUCUG | No. 1936 | AGAAUAUCCAUCAUGGCAGCC | No. 3116 | CTGCCATGATGGATATTCT | 0.095 |
| AH0757 | No. 757 | CCAUGAUGGAUAUUCUCUGGA | No. 1937 | GAGAAUAUCCAUCAUGGCAGC | No. 3117 | TGCCATGATGGATA TTCTC | 0.226 |
| AH0759 | No. 769 | CCAUGAUGGAUAUUCUCUGGA | No. 1939 | CAGAGAAUAUCCAUCAUGGCA | No. 3119 | CCATGATGGATATTCTCTG | 0.283 |
| AH0760 | No. 760 | CAUGAUGGAUAUUCUCUGGAU | No. 1940 | CCAGAGAAUAUCCAUCAUGGC | No. 3120 | CATGATGGATATTCTCTGG | 0.337 |
| AH0762 | No. 762 | UGAUGGAUAUUCUCUGGAUGG | No. 1942 | AUCCAGAGAAUAUCCAUCAUG | No. 3122 | TGATGGATATTCTCTG GAT | 0.195 |
| AH0763 | No. 763 | GAUGGAUAUUCUCUGGAUGGC | No. 1943 | CAUCCAGAGAAUAUCCAUCAU | No. 3123 | GATGGATATTCTCTGGATG | 0.141 |
| AH0766 | No. 766 | GGAUAUUCUCUGGAUGGCCCG | No. 1946 | GGCCAUCCAGAGAAUAUCCAU | No. 3126 | GGATATTCTCTGGATGGCC | 0.199 |
| AH0771 | No. 771 | UUCUCUGGAUGGCCCGGAAGA | No. 1951 | UUCCGGGCCAUCCAGAGAAUA | No. 3131 | TTCTCTGGATGGCCCGGAA | 0.267 |
| AH0773 | No. 773 | CUCUGGAUGGCCCGGAAGAAA | No. 1953 | UCUUCCGGGCCAUCCAGAGAA | No. 3133 | CTCTGGATGGCCCGGAAGA | 0.167 |
| AH0774 | No. 774 | UCUGGAUGGCCCGGAAGAAAU | No. 1964 | UUCUUCCGGGCCAUCCAGAGA | No. 3134 | TCTGGATGGCCCGGAAGAA | 0.207 |
| AH0775 | No. 775 | CUGGAUGGCCOGGAAGAAAUA | No. 1955 | UUUCUUCCGGGCCAUCCAGAG | No. 3136 | CTGGATGGCCCGGAAGAAA | 0.310 |
| AH0776 | No. 776 | UGGAUGGCCCGGAAGAAAUAG UGGAUGGCCCGAAGAAAUAG | No. 1956 | AUUUCUUCCGGGCCAUCCAGA | No. 3136 | TGGATGGCCCGGAAGAAAT | 0.134 |
| AH0777 | No. 777 | GGAUGGCCCGGAAGAAAUAGA | No. 1957 | UAUUUCUUCCGGGCCAUCCAG | No. 3137 | GGATGGCCCGGAAGAAATA | 0.070 |
| AH0779 | No. 779 | AUGGCCCGGAAGAAAUAGAAU | No. 1969 | UCUAUUUCUUCCGGGCCAUCC | No. 3139 | ATGGCCCCGGAAGAAATAGA | 0.201 |
| AH0780 | No. 780 | UGGCCGGAAGAAAUAGAAUG | No. 1960 | UUCUAUUUCUUCCGGGCCAUC | No. 3140 | TGGCCCGGAAGAAATAGAA | 0.160 |
| AH0781 | No. 781 | GGCCCCGGAAGAAAUAAGAAUGU | No. 1961 | AUUCUAUUUCUUCCGGGCCAU | No. 3141 | GGCCCCGGAAGAAATAGAAT | 0.205 |
| AH0782 | No. 782 | GCCCGGAAGAAAUAGAAUGUA | No. 1962 | GAUUCUAUUUCUUCCGGGCCA | No. 3142 | GCCCGGAAGAAATAGAATG | 0.066 |
| AH0783 | No. 783 | CCCGGAAGAAAUAGAAUGUAC | No. 1963 | ACAUUCUAUUUCUUCCGGGCC | No. 3143 | CCCGGAAGAAATAGAATGT | 0.153 |
| AH0785 | No. 785 | CGGAAGAAAUAGAAUACCA | No. 1965 | GUACAUUCUAUUUCUUCCGGG | No. 3145 | CGGAAGAAATAGAATGTAC | 0.126 |
| AH0786 | No. 786 | GGAAGAAAUAGAAUGUACCAA | No. 1966 | GGUACAUUCUAUUUCUUCCGG | No. 3145 | GGAAGAAATAGAATGTACC | 0.295 |
| AH0787 | No. 787 | GAAGAAAUÅGAAUGUACCAAA | No. 1967 | UGGUACAUUCUAUUUCUUCCG | No. 3147 | GAAGAAATAGAATGTACCA | 0.244 |
| AH0790 | No. 790 | GAAAUAGAAUGUACCAAACUG | No. 1970 | GUUUGGUACAUUCUAUUUCUU | No. 3150 | GAAATAGAATGTACCAAAC | 0.196 |
| AH0795 | No. 795 | AGAAUGUACCAAACUGGGAAA | No. 1975 | UCCCAGUUUGGUACAUUCUAU | No. 3155 | AGAATGTACCAAACTGGGA | 0.172 |
| AH0796 | No. 796 | GAAUGUACCAACUGGGAAAC | No. 1976 | UUCCCAGUUUGGUACAUUCUA | No. 3156 | GAAGTACCAAACTGGAA | 0.082 |
| AH0797 | No. 797 | AAUGUACCAAACUGGGAAACU | No. 1977 | UUUCCCAGUUUGGUACAUUCU | No. 3157 | AATGTACCAAACTGGGAAA | 0.310 |
| AH0800 | No. 800 | GUACCAAACUGGGAAACUGGU | No. 1980 | CAGUUUCCCAGUUUGGUACAU | No. 3160 | GTACCAAACTGGGAAACTG | 0.173 |
| AH0804 | No. 804 | CAAACUGGGAAACUGGUCUGC | No. 1984 | AGACCAGUUUCCCAGUUUGGU | No. 3164 | CAACTGGGAAAACTGGTGGTCT | 0.115 |
| AH0805 | No. 805 | AAACUGGGAAACUGGUCUGCC | No. 1985 | CAGACCAGUUUCCCAGUUUGG | No. 3165 | AAACTGGGAAACTGGTCTG | 0.174 |
| AH0808 | No. 808 | CUGGGAAACUGGUCUGCCAUG | No. 1968 | UGGCAGACCAGUUUCCCAGUU | No. 3168 | CTGGGAAACTGGTCTGCCA | 0.025 |
| AH0809 | No. 809 | UGGAAACUGGUCUGCCAUGC | No. 1989 | AUGGCAGACCAGUUUCCCAGU | No. 3169 | TGGGAAACTGGTC GCCAT | 0.143 |
| AH0810 | No. 810 | GGGAAACUGGUCUGCCAUGCC | No. 1990 | CAUGGCAGACCAGUUUCCCAG | No. 3170 | GGGAAACTGGTCTGCCATG | 0.331 |
| AH0811 | No. 811 | GGAAACUGGUCUGCCAUGCCA | No. 1991 | GCAUGGCAGACCAGUUUCCCA | No. 3171 | GGAAACTGGTCTGCCATGC | 0.326 |
| AH0813 | No. 813 | AAACUGGUCUGCCAUGCCAAG | No. 1993 | UGGCAUGGCAGACCAGUUUCC | No. 3173 | AACTGGTCTGCCATGCCA | 0.126 |
| AH0814 | No. 814 | AACUGGUCUGCCAUGCCAAGU | No. 1994 | UUGGCAUGGCAGACCAGUUUC | No. 3174 | AACTGGTGCCATGCCAA | 0.127 |
| AH0815 | No. 815 | ACUGGUCUGCCAUGCCAAGUU | No. 1995 | CUUGGCAUGGCAGACCAGUUU | No. 3175 | ACTGGTCTGCCATGCCAAG | 0.280 |
| AH0816 | No. 816 | CUGGUCUGCCAUGCCAAGUUG | No. 1996 | ACUUGGCAUGGCAGACCAGUU | No. 3176 | CTGCTCTGCCATGCCAAfiT | 0.323 |
| HA0817 | No. 817 | UGGUCUGCCAUGCCAAGUUGU | No. 1997 | AACUUGGCAUGGCAGACCAGU | No. 3177 | TGGTCTGCCATGCCAAGTT | 0.167 |
| AH0818 | No. 818 | GGUCUGCCAUGCCAAGUUGUA | No. 1998 | CAACUUGGCAUGGCAGACCAG | No. 3178 | GGTCTGCCATGCCAGTTG | 0.156 |
| AH0819 | No. 819 | GUCUGCCAUGCCAAGUUGUAA | No. 1999 | ACAACUUGGCAUGGCAGACCA | No. 3179 | GTCTGCCATGCCAAGTTGT | 0.118 |
| AH0820 | No. 820 | UCUGCCAUGCCAAGUUGUAAA | No. 2000 | UACAACUUGGCAUGGCAGACC | No. 3180 | | 0.072 |
| AH0821 | No. 821 | CUGCCAUGCCAAGUUGUAAAG | No. 2001 | UUACAACUUGGCAUGGCAGAC | No. 3181 | CTGCCATGCCAGTTGTAA | 0.066 |
| AH0822 | No. 822 | UGCCAUGCC AAGUUGUAAAGC | No. 2002 | UUUACAACUWGCAUGGCAGA | No. 3182 | TGCCATGCCAAGTTGTAAA | 0.090 |
| AH0823 | No. 823 | GCCAUGCCAAGUUGUAAAGCA | No. 2003 | CUUUACAACUUGGCAUGGCAG | No. 3183 | GCCATGCCAAGTTGTAAAG | 0.128 |

### [Table 49]

**Table 4-11**

| Double stranded nucleic acid No. | SEQ ID NO. | Sense strand sequence (5'-3') | SEQ ID NO. | Antisense strand sequence (5'→3') | SEQ ID NO. | Target β2GPI mRNA sequence | Relative expressed amount of β2GPI |
|---|---|---|---|---|---|---|---|
| AH0825 | No. 825 | CAUGCCAAGUUGUAAAGCAUC | No. 2005 | UGCUUUACAACUUGGCAUGGC | No. 3185 | CATGCCAAGTTGTAAAGCA | 0.086 |
| AH0826 | No. 826 | AUGCCAAGUUGUAAAGCAUCU | No. 2006 | AUGCUUUACAACUUGGCAUGG | No. 3186 | ATGCCAAGTTGTAAAGCAT | 0.082 |
| AH0827 | No. 827 | UGCCAAGUUGUAAAGCAUCUU | No. 2007 | GAUGCUUUACAACUUGGCAUG | No. 3187 | TGCCAAGTTGTAAAGCATC | 0.342 |
| AH0828 | No. 828 | GCCAAGUUGUAAAGCAUCUUG | No. 2008 | AGAUGCUUUACAACUUGGCAU | No. 3188 | CATCT | 0.160 |
| AH0829 | No. 829 | CCAAGUUGUAAAGCAUCUUGU | No. 2009 | AAGAUGCUUUACAACUUGGCA | No. 3189 | GCCAAGTTGTAAAG CCAAGTTGTAAAGCATCTT | 0.159 |
| AH0830 | No. 830 | CAAGUUGUAAAGCAUCUUGUA | No. 2010 | CAAGAUGCUUUACAACUUGGC | No. 3190 | CAAGTTGTAAAGCATCTTG | 0.134 |
| AH0831 | No. 831 | AAGUUGUAAAGCAUCUUGUAAA | No. 2011 | ACAAGAUGGUUUUACAACUUGG | No. 3191 | AAGTTGTAAAGCATCTTGT | 0.110 |
| AH0832 | No. 832 | AGUUGUAAAGCAUCUUGUAA | No. 2012 | UACAAGAUGCUUUACAACUUG | No. 3192 | AGTTGTAAAGCATCTTGTA | 0.064 |
| AH0833 | No. 833 | GUUGUAAAGCAUCUUGUAAAG | No. 2013 | UUACAAGAUGCUUUACAACUU | No. 3193 | GTTGTAAAGCATCTTGTAA | 0.175 |
| AH0834 | No. 834 | UUGUAAAGCAUCUUGUAAAGU | No. 2014 | UUUACAAGAUGCUUUACAACU | No. 3194 | TTGTAAAGCATCTTGTAAA | 0.092 |
| AH0835 | No. 835 | UGUAAAGCAUCUUGUAAAGUA | No. 2015 | CUUUACAAGAUGCUUUACAAC | No. 3195 | TGTAAAGCATCTTGTAAAG | 0.129 |
| AH0837 | No. 837 | UAAAGCAUCUUGUAAGUACC | No. 2017 | UACUUUACAAGAU GCUUUACA | No. 3197 | TAAAGCATCTTGTAAAGTA | 0.087 |
| AH0838 | No. 838 | AAAGCAUCUUGUAAAGUACCU | No. 2018 | GUACUUUACAAGAUGCUUUAC | No. 3198 | AAAGCATCTTGTAAAGTAC | 0.174 |
| AH0840 | No. 840 | AGCAUCUUGUAAAGUACCUGU | No. 2020 | AGGUACUUUACAAGAUGCUUU | No. 3200 | AGCATCTT GTAAAGTACCT | 0.326 |
| AH0842 | No. 842 | CAUCUUGUAAAGUACCUGUGA | No. 2022 | ACAGGUACUUUACAAGAUGCU | No. 3202 | CATCTTGTAAAGTACCTGT | 0.260 |
| AH0845 | No. 845 | CUUGUAAAGUACCUGUGAAAA | No. 2025 | UUCACAGGUACUUUACAAGAU | No. 3205 | CTTGTAAAGTACCTGTGAA | 0.187 |
| AH0846 | No. 846 | UUGUAAAGUACCUGUGAAAAAA | No. 2026 | UUUCACAGGUACUUUACAAGA | No. 3206 | TTGTAAAGTACCTGTGAAA | 0.166 |
| AH0847 | No. 847 | UGUAAAGUACCUGUGAAAAAA | No. 2027 | UUUUCACAGGUACUUUACAAG | No. 3207 | TGTAAAGTACCTGTGAAAA | 0.210 |
| AH0852 | No. 852 | AGUACCUGUGAAAAAAGCCAC | No. 2032 | GGCUUUUUUCACAGGUACUUU | No. 3212 | AGTACCT GTGAAAAAAGCC | 0.247 |
| AH0853 | No. 853 | GUACCUGUGAAAAAAGCCACU | No. 2033 | UGGCUUUUUUCACAGGUACUU | No. 3213 | GTACCTGTGAAAAAAGCCA | 0.334 |
| AH0854 | No. 854 | UACCUGUGAAAAAAGCCACUG | No. 2034 | GUGGCUUUUUUCACAGGUACU | No. 3214 | TACCTGTGAAAAAAGCCAC | 0.302 |
| AH0855 | No. 855 | ACCUGUGAAAAAAGCCACUGU | No. 2035 | AGUGGCUUUUUUCACAGGUAC | No. 3215 | ACCTGTGAAAAAAGCCACT | 0.312 |
| AH0857 | No. 857 | CUGUGAAAAAAGCCACUGUGG | No. 2037 | ACAGUGGCUUUUUUCACAGGU | No. 3217 | CTGTGAAAAAAGCCACTGT | 0.196 |
| AH0858 | No. 858 | UGUGAAAAAAGCCACUGUGGU | No. 2038 | CACAGUGGCUUUUUUCACAGG | No. 3218 | TGTGAAAAAAGCCACTGTG | 0.091 |
| AH0859 | No. 859 | GUGAAAAAAGCCACUGUGGUG | No. 2039 | CCACAGUGGCUUUUUUCACAG | No. 3219 | GTGAAAAAAGCCACTGTGG | 0.287 |
| AH0860 | No. 860 | UGAAAAAAGCCACUGUGGUGU | No. 2040 | ACCACAGUGGCUUUUUUCACA | No. 3220 | TGAAAAAAGCCACTGTGGT | 0.278 |
| AH0861 | No. 861 | GAAAAAAGCCACUGUGGUGUA | No. 2041 | CACCACAGUGGCUUUUUUCAC | No. 3221 | GAAAAAAGCCACTGTGGTG | 0.170 |
| AH0863 | No. 863 | AAAAAGCCACUGUGGUGUACC | No. 2043 | UACACCACAGUGGCUUUUUUC | No. 3223 | AAAAAGCCACTGTGGTGTA | 0.269 |
| AH0864 | No. 864 | AAAAGCCACUGUGGUGUACCA | No. 2044 | GUACACCACAGUGGCUUUUUU | No. 3224 | AAAAGCCACTGTGGTGTAC | 0.185 |
| AH0866 | No. 866 | AAGCCACUGUGGUGUACCAAG | No. 2046 | UGGUACACCACAGUGGCUUUU | No. 3226 | AAGCCACTGTGGTGTACCA | 0.209 |
| AH0867 | No. 867 | AGCCACUGUGGUGUACCAAGG | No. 2047 | UUGGUACACCAC AGUGGCUUU | No. 3227 | AGCCACTGTGGTGTACCAA | 0.235 |
| AH0868 | No. 868 | GCCACUGUGGUGUACCAAGGA | No. 2048 | CUUGGUACACCACAGUGGCUU | No. 3228 | GCCACTGTGGTGTACCAAG | 0.106 |
| AH0870 | No. 870 | CACUGUGGUGUACCAAGGAGA | No. 2050 | UCCUUGGUACACCACAGUGGC | No. 3230 | CACTGTGGTGTACCAAGGA | 0.154 |
| AH0872 | No. 872 | CUGUGGUGUACCAAGGAGAGA | No. 2052 | UCUCCUUGGUACACCACAGUG | No. 3232 | CTGTGGTGTACCAAGGAGA | 0.230 |
| AH0873 | No. 873 | UGUGGUGUACCAAGGAGAGAG | No. 2053 | CUCUCCUUGGUACACCACAGU | No. 3233 | TGTGGTGTACCAAGGAGAG | 0.289 |
| AH0874 | No. 874 | GUGGUGUACCAAGGAGAGAGA | No. 2054 | UCUCUCCUUGGUACACCACAG | No. 3234 | GTGGTGTACCAAGGAGAGA | 0.174 |
| AH0875 | No. 875 | UGGUGUACCAAGGAGAGAGAG | No. 2055 | CUCUCUCCUUGGUACACCACA | No. 3235 | TGGTGTACCAAGGAGAGAG | 0.281 |
| AH0876 | No. 876 | GGUGUACCAAGGAGAGAGAGU | No. 2056 | UCUCUCUCCUUGGUACACCAC | No. 3236 | GGTGTACCAAGGAGAGAGA | 0.117 |
| AH0877 | No. 877 | GUGUACCAAGGAGAGAGAGUA | No. 2057 | CUCUCUCUCCUUGGUACACCA | No. 3237 | GTGTACCAAGGAGAGAGAG | 0.149 |
| AH0879 | No. 879 | GUACCAAGGAGAGAGAGUAAA | No. 2059 | UACUCUCUCUCCUUGGUACAC | No. 3239 | GTACCAAGGAGAGAGAGTA | 0.325 |
| AH0880 | No. 880 | UACCAAGGAGAGAGAGUAAAG | No. 2060 | UUACUCUCUCUCCUUGGUACA | No. 3240 | TACCAAGGAGAGAGAGTAA | 0.198 |
| AH0881 | No. 881 | ACCAAGGAGAGAGAGUAAAGA | No. 2061 | UUUACUCUCUCUCCUUGGUAC | No. 3241 | ACCAAGGAGAGAGAGTAAA | 0.159 |
| AH0882 | No. 882 | CCAAGGAGAGAGAGUAAAGAU | No. 2062 | CUUUACUCUCUCUCCUUGGUA | No. 3242 | CCAAGGAGAGAGAGTAAAG | 0.244 |
| AH0883 | No. 883 | CAAGGAGAGAGAGUAAAGAUU | No. 2063 | UCUUUACUCUCUCUCCUUGGU | No. 3243 | CAAGGAGAGAGAGTAAAGA | 0.046 |
| AH0884 | No. 884 | AAGGAGAGAGAGUAAAGAUUC | No. 2064 | AUCUUUACUCUCUCUCCUUGG | No. 3244 | AAGGAGAGAGAGTAAAGAT | 0.118 |
| AH0885 | No. 885 | AGGAGAGAGAGUAAAGAUUCA | No. 2065 | AAUCUUUACUCUCUCUCCUUG | No. 3245 | AGGAGAGAGAGTAAAGATT | 0.150 |

### [Table 50]

**Table 4-12**

| Double stranded nucleic acid No. | SEQ ID NO | Sense strand sequence (5'→3') | SEQ ID NO. | Antisense strand sequence (5'→3') | SEQ ID NO | Target β2GPI mRNA sequence | Relative expressed amount of β2GPI |
|---|---|---|---|---|---|---|---|
| AH0886 | No. 886 | GGAGAGAGAGUAAAGAUUCAG | No. 2066 | GAAUCUUUACUCUCUCUCCUU | No. 3246 | GGAGAGAGAGTAAAGATTC | 0.187 |
| AH0887 | No. 887 | GAGAGAGAGUAAAGAUUCAGG | No. 2067 | UGAAUCUUUACUCUCUCUCCU | No. 3247 | GAGAGAGAGTAAAGATTCA | 0.061 |
| AH0888 | No. 888 | AGAGAGAGUAAAGAJUUCAGGA | No. 2068 | CUGAAUCUUUACUCUCUCUCC | No. 3248 | AGAGAGAGTAAAGATTCAG | 0.230 |
| AH0890 | No. 890 | AGAGAGUAAAGAUUCAGGAAA | No. 2070 | UCCUGAAUCUUUACUCUCUCU | No. 3250 | AGAGAGTAAAGATTCAGGA | 0.270 |
| AH0891 | No. 891 | GAGAGUAAAGAUUCAGGAAAA | No. 2071 | UUCCUGAAUCUUUACUCUCUC | No. 3251 | GAGAGTAAAGATTCAGGAA | 0.190 |
| AH0892 | No. 892 | AGAGUAAAGAUUCAGGAAAA | No. 2072 | UUUCCUGAAUCUUUACUCUCU | No. 3252 | AGAGTAAAGATTCAGGAAA | 0.212 |
| AH0893 | No. 893 | GAGUAAAGAUUCAGGAAAAAU | No. 2073 | UUUUCCUGAAUCUUUACUCUC | No. 3253 | GAGTAAAGATTCAGGAAAA | 0.114 |
| AH0894 | No. 894 | AGUAAAGAUUCAGGAAAAAUU | No. 2074 | UUUUUCCUGAAUCUUUACUCU | No. 3254 | AGTAAAGATTCAGGAAAAAA | 0.185 |
| AH0895 | No. 895 | GUAAAGAUUCAGGAAAAAUUU | No. 2075 | AUUUUUCCUGAAUCUUUACUC | No. 3255 | GTAAAGATTCAGGAAAAAT | 0.203 |
| AH0896 | No. 896 | UAAAGAUUCAGGAAAAAUUUA | No. 2076 | AAUUUUUCCUGAAUCUUUACU | No. 3256 | TAAAGATTCAGGAAAAATT | 0.149 |
| AH0897 | No. 897 | AAAGAUUCAGGAAAAAUUUAA | No. 2077 | AAAUUUUUCCUGAAUCUUUAC | No. 3257 | AAAGATTCAGGAAAAATTT | 0.218 |
| AH0898 | No. 898 | AAGAUUCAGGAAAAAUUUAAG | No. 2078 | UAAAUUUUUCCUGAAUCUUUA | No. 3258 | AAGATTCAGGAAAAATTTA | 0.262 |
| AH0899 | No. 899 | AGAUUCAGGAAAAAUUUAAGA | No. 2079 | UUAAAUUUUUCCUGAAUCUUU | No. 3259 | AGATTCAGGAAAAATTTAA | 0.334 |
| AH0905 | No. 905 | AGGAAAAAUUUAAGAAUGGAA | No. 2085 | CCAUUCUUAAAUUUUUCCUGA | No. 3265 | AGGAAAAATTTAAGAATGG | 0.172 |
| AH0906 | No. 906 | GGAAAAAUUUAAGAAUGGAAU | No. 2086 | UCCAUUCUUAAAUUUUUCCUG | No. 3266 | GGAAAAATTTAAGAATGGA | 0. 171 |
| AH0907 | No. 907 | GAAAAAUUUAAGAAUGGAAUG | No. 2087 | UUCCAUUCUUAAAUUUUUCCU | No. 3267 | GAAAAATTTAAGAATGGAA | 0.260 |
| AH0912 | No. 912 | AUUUAAGAAUGGAAUGCUACA | No. 2092 | UAGCAUUCCAUUCUUAA AUUU | No. 3272 | ATTTAAGAATGGAATGCTA | 0.323 |
| AH0914 | No. 914 | UUAAGAAUGGAAUGCUACAUG | No. 2094 | UGUAGCAUUCCAUUCUUAAAU | No. 3274 | TTAAGAATGGAATGCTACA | 0.342 |
| AH0917 | No. 917 | AGAAUGGAAUGCUACAUGGUG | No. 2097 | CCAUGUAGCAUUCCAUUCUUA | No. 3277 | AGAATGGAATGCTACATGG | 0.221 |
| AH0918 | No. 918 | GAAUGGAAUGCUACAUGGUGA | No. 2098 | ACCAUGU AGCAUUCCAUUCUU | No. 3278 | GAATGGAAATGCTACATGGT | 0.162 |
| AH0919 | No. 919 | AAUGGAAUGCUACAUGGUGAU | No. 2099 | CACCAUGUAGCAUUCCAUUCU | No. 3279 | AATGGAATGCTACATGGTG | 0.287 |
| AH0920 | No. 920 | AUGGAAUGCUACAUGGUGAUA | No. 2100 | UCACCAUGUAGCAUUCCAUUC | No. 3280 | ATGGAATGCTACATGGTGA | 0.212 |
| AH0921 | No. 921 | UGGAAUGCUACAUGGUGAUAA | No. 2101 | AUCACCAUGUAGCAUUCCAUU | No. 3281 | TGGAATGCTACATGGTGAT | 0.127 |
| AH0922 | No. 922 | GGAAUGCUACAUGGUGAUAAA | No. 2102 | UAUCACCAUGUAGCAUUCCAU | No. 3282 | GGAATGCTACATGGTGATA | 0. 241 |
| AH0923 | No. 923 | GAAUGCUACAUGGUGAUAAAG | No. 2103 | UUAUCACCAUGUAGCAUUCCA | No. 3283 | GAATGCTACATGGTGATAA | 0.343 |
| AH0924 | No. 924 | AAUGCUACAUGGUGAUAAAGU | No. 2104 | UUUAUCACCAUGUAGCAUUCC | No. 3284 | AATGCTACATGGTGATAAA | 0.269 |
| AH0926 | No. 926 | UGCUACAUGGUGAUAAAGUUU | No. 2106 | ACU UUAUCACCAUGUAGCAUU | No. 3286 | TGCTACATGGTGATAAAGT | 0.259 |
| AH0927 | No. 927 | GCUACAUGGUGAUAAAGUUUC | No. 2107 | AACUUUAUCACCAUGUAGCAU | No. 3287 | GCTACATGGTGATAAAGTT | 0.240 |
| AH0928 | No. 928 | CUACAUGGUGAUAAAGUUUCU | No. 2108 | AAACUUUAUCACCAUGUAGCA | No. 3288 | CTACATGGTGATAAAGTTT | 0.080 |
| AH0930 | No. 930 | ACAUGGUGAUAAAGUUUCUUU | No. 2110 | AGAAACUUUAUCACCAUGUAG | No. 3290 | ACATGGTGATAAAGTTTCT | 0.119 |
| AH0932 | No. 932 | AUGGUGAUAAAGUUUCUUUCU | No. 2112 | AAAGAAACUUUAUCACCAUGU | No. 3292 | ATGGTGATAAAGTTTCTTT | 0.342 |
| AH0934 | No. 934 | GGUGAUAAAGUUUCUUUCUUC | No. 2114 | AGAAAGAAACUUUAUCACCAU | No. 3294 | GGTGATAAAGTTTCTTTCT | 0.145 |
| AH0935 | No. 935 | GUGAUAAAGUUUCUUUCUUCU | No. 2115 | AAGAAAGAAACUUUAUCACCA | No. 3295 | GTGATAAAGTTTCTTTCIT | 0.185 |
| AH0936 | No. 936 | UGAUAAAGUUUCUUUCUUCUG | No. 2116 | GAAGAAAGAAACUUUAUCACC | No. 3296 | TGATAAAGTTTCTTTCTTC | 0.132 |
| AH0937 | No. 937 | GAUAAAGUUUCUUUCUUCUGC | No. 2117 | AGAAGAAAGAAACUUUAUCAC | No. 3297 | GATAAAGTTTCTTTCTTCT | 0.145 |
| AH0940 | No. 940 | AAAGUUUCUUUCUUCUGCAAA | No. 2120 | UGCAGAAGAAAGAAACUUUAU | No. 3300 | AAAGTTTCTTTCTTCTGCA | 0.213 |
| AH0941 | No. 941 | AAGUUUCUUUCUUCUGCAAAA | No. 2121 | UUGCAGAAGAAAGAAACUUUA | No. 3301 | AAGTTTCTTTCTTCTGCAA | 0.162 |
| AH0942 | No. 942 | AGUUUCUUUCUUCUGCAAAAA | No. 2122 | UUUGCAGAAGAAAGAAACUUU | No. 3302 | AGTTTCTTTCTTCTGCAAA | 0.229 |
| AH0943 | No. 943 | GUUUCUUUCUUCUGCAAAAAU | No. 2123 | UUUUGCAGAAGAAAGAAACUU | No. 3303 | GTTTCTTTCTTCTGCAAAA | 0.081 |
| AH0944 | No. 944 | UUUCUUUCUUCUGCAAAAAUA | No. 2124 | UUUUUGCAGAAGAAAGAAACU | No. 3304 | TTTCTTTCTTCTGCAAAAA | 0.119 |
| AH0945 | No. 945 | UUCUUUCUUCUGCAAAAAUAA | No. 2125 | AUUUUUGCAGAAGAAAGAAAC | No. 3305 | TTCTTTCTTCTGCAAAAAT | 0.168 |
| AH0946 | No. 946 | UCUUUCUUCUGCAAAAAUAAG | No. 2126 | UAUUUUUGCAGAAGAAAGAAA | Nο. 3306 | TCTTTCTTCTGCAAAAATA | 0.106 |
| AH0947 | No. 947 | CUUUCUUCUGCAAAAAUAAGG | No. 2127 | UUAUUUUUGCAGAAGAAAGAA | No. 3307 | CTTTCTTCTGCAAAAATAA | 0.115 |
| AH0948 | No. 948 | UUUCUUCUGCAAAAAUAAGGA | No. 2128 | CUUAUUUUUGCAGAAGAAAGA | No. 3308 | TTTCTTCTGCAAAAATAAG | 0.299 |
| AH0953 | No. 953 | UCUGCAAAAAUAAGGAAAAGA | No. 2133 | UUUUCCUUAUUUUUGCAGAAG | No. 3313 | TCTGCAAAAATAAGGAAAA | 0.188 |
| AH0955 | No. 955 | UGCAAAAAUAAGGAAAAGAAG | No. 2135 | UCUUUUCCUUAUUUUUGCAGA | No. 3315 | TGCAAAAATAAGGAAAAGA | 0.305 |

### [Table 51]

**Table 4-13**

| Double stranded nucleic acid No | SEQ ID NO. | Sense strand sequence (5'→3') | SEQ ID NO. | Antisense strand sequence (5'→3') | SEQ ID NO. | Target 02GPI mRNA sequence | Relative expressed amount of β2GPI |
|---|---|---|---|---|---|---|---|
| AH0966 | No. 956 | GCAAAAAUAAGGAAAAGAAGU | No. 2136 | UUCUUUUCCUUAUUUUUGCAG | No. 3316 | GCAAAAATAAGGAAAAGAA | 0.185 |
| AH0957 | No. 957 | CAAAAAUAAGGAAAAGAAGUG | No. 2137 | CUUCUUUUCCUUAUUUUUGCA | No. 3317 | CAAAATAAGGAAAAGAAG | 0.335 |
| AH0964 | No. 964 | AAGGAAAAGAAGUGUAGCUAU | No. 2144 | AGCUACACUUCUUUUCCUUAU | No. 3324 | AAGGAAAAGAAGTAGTAGCT | 0.329 |
| AH0965 | No. 965 | AGGAAAAGAAGUGUAGCUAUA | No. 2145 | UAGCUACACUUCUUUUCCUUA | No. 3325 | AGGAAAAGAAGTGTAGCTA | 0.153 |
| AH0966 | No. 966 | GGAAAAGAAGUGUAGCUAUAC | No. 2146 | AUAGCUACACUUCUUUUCCUU | No. 3326 | GGAAAAGAAGTGTAGCTAT | 0.106 |
| AH967 | No. 967 | GAAAAGAAGUGUAGCUAUACA | No. 2147 | UAUGCUACACUUCUUUUCCU | No. 3327 | GAAAGAAGTGTAGCTATA | 0.097 |
| AH0969 | No. 969 | AAAGAAGUGUAGCUAUACAGA | No. 2149 | UGUAUAGCUACACUUCUUUUC | No. 3329 | AAAGAAGTGTAGCTATACA | 0.228 |
| AH0972 | No. 972 | GAAGUGUAGCUAUACAGAGGA | No. 2152 | CUCUGUAUAGCUACACUUCUU | No. 3332 | GAAGTGTAGCTATACAGAG | 0.293 |
| AH0974 | No. 974 | AGUGUAGCUAUACAGAGGAUG | No 2154 | UCCUCUGUAUAGCUACACUUC | No. 3334 | AGTGTAGCTATACAGA | 0.160 |
| AH0975 | No. 975 | GUGUAGCUAUACAGAGGAUGC | No. 2155 | AUCCUCUGUAUAGCUACACUU | No. 3335. | GTGTAGCTATACAGAGGAT | 0.227 |
| AH0977 | No. 977 | GUAGCUAUACAGAGGAUGCUC | No. 2157 | GCAUCCUUCUGUAUAGCUACAC | No. 3337 | GTAGCTATACAGAGGATGC | 0.285 |
| AH0978 | No. 978 | UAGCUAUACAGAGGAUGCUCA | No. 2158 | AGCAUCCUCUGUAUAGCUACA | No. 3338 | TACGTATACAGAGGATGCT | 0.245 |
| AH0980 | No. 980 | GCUAUACAGAGGAUGCUCAGU | No. 2160 | UGAGCAUCCUCUGUAUAGCUA | No. 3340 | GCTATACAGAGGATGCTCA | 0.090 |
| AH0981 | No. 981 | CUAUACAGAGGAUGCUACAGUG | No. 2161 | CUAGAGCAUCCUCUGUAUAGCU | No. 3341 | CTATACAGAGGATGCTCAG | 0.137 |
| AH0985 | No. 985 | ACAGAGGAUGCUCAGUGUAUA | No. 2165 | UACACUGAGCAUCCUCUGUAU | No. 3345 | ACAGAGGATGCTCAGTGTA | 0.308 |
| AH0987 | No. 987 | AGAGGAUGCUAGUGUAUAGA | No. 2167 | UAUACACUGAGCAUCCUCUGU | No. 3347 | AGAGGATGCTCAGTGTATA | 0.082 |
| AH0988 | No. 988 | GAGGAUGCUCAGUGUAUAGAU | No. 2168 | CUAUACACUGAGCAUCCUCUG | No. 3348 | GAGGATGCTCAGTGTATAG | 0.292 |
| AH0989 | No. 989 | AGGAUGCUCAGUGUAUAGAUG | No. 2169 | UCUAUACACUGAGCAUCCUCU | No. 3349 | AGGATGCTCAGTGTATAGA | 0.338 |
| AH0990 | No. 990 | GGAUGCUCAGUGUAUAGAUGG | No. 2170 | AUCUAUACACUGAGCAUCCUC | No. 3350 | GGATGCTCAGTGTATAGAT | 0.252 |
| AH0991 | No. 991 | GAUGCUCAGUGUAUAGAUGGC | No. 2171 | CAUCUAUACACUGAGCAUCCU | No. 3351 | GATGCTCAGTGTATAGATG | 0.111 |
| AH0996 | No. 996 | UCAGUGUAUGAUGGCACUAU | No. 2176 | AGUGCCAUCUAUACACUGAGC | No. 3356 | TCAGTGTATAGATGGCACT | 0.233 |
| AH0997 | No. 997 | CAGUGUAUAGAUGGCACUAUC | No. 2177 | UAGUGCCAUCUAUACACUGAG | No. 3357 | CAGTGTATAGATGGCACTA | 0.290 |
| AH0998 | No. 998 | AGUGUAUAGAUGGCACUAUCG | No. 2178 | AUAGUGCCAUCUAUACACUGA | No. 3358 | AGTGTATAGATGGCACTAT | 0.199 |
| AH0999 | No. 999 | GUGUAUAGAUGGCACUAUCGA | No. 2179 | GAUAGUGCCAUCUAUACACUG | No. 3359 | GTGTATAGATGGCACTATC | 0.101 |
| AH1000 | No. 1000 | UGUAUAGAUGGCACUAUCGAA | No. 2180 | CGAUAGUGCCAUCUACACU | No. 3360 | TGTATAGATGGACTATCG | 0.314 |
| AH1001 | No. 1001 | GUAUAGAUGGCACUAUCGAAG | No. 2181 | UCGAUAGUGCAUCUAUACAC | No. 3361 | GTATAGATGGCACTATCGA | 0.082 |
| AH1002 | No. 1002 | UAUGAUGGCACUAUCGAAGU | No. 2182 | UUCGAUAGUGCCAUCUAUACA | No. 3362 | TATAGATGGCACTATCGAA | 0.084 |
| AH1003 | No. 1003 | AUAGAUGGCACUAUCGAAGUC | No. 2183 | CUUCGAUAGUGCCAUCUAUAC | No. 3363 | ATAGATGGCACTATCGAAG | 0.265 |
| AH1005 | No. 1005 | AGAUGGCACUAUCGAAGUCCC | No. 2185 | GACUUCGAUAGUGCCAUCUAU | No. 3365 | AGTGGCACTATCGAAGTC | 0.100 |
| AH1007 | No. 1007 | AUGGCACUAUCGAAGUCCCCA | No. 2187 | GGGACUUCGAUAGUGCCAUCU | No. 3367 | ATGGCACTATACGAAGTCCC | 0.232 |
| AH1009 | No. 1009 | GGCAUCUAUCGAAGUCCCAAA | No. 2189 | UGGGGACUUCGAUAGUGCCAU | No. 3369 | GGCACTATCGAAGTCCCCA | 0.122 |
| AH1010 | No. 1010 | GCACUAUCGAAGUCCCCAAAU | No. 2190 | UUGGGGAUCUUCGAUAGUGCCA | No. 3370 | GCATATCGAAGTCCCCAA | 0.089 |
| AH1011 | No. 1011 | CACUAUCGAAGUCCCCAAAUG | No. 2191 | UUUGGGGGACUUCGAUAGUGCC | No. 3371 | CACTATCGAAGTCCCCAAA | 0.154 |
| AH1013 | No. 1013 | CUAUCGAAGUCCCCAAAUGCU | No. 2193 | CAUUUGGGGACUUCGAUAGUG | No. 3373 | CTATCGAAGTCCCCAATG | 0.139 |
| AH1015 | No. 1015 | AUCGAAGUCCCCAAUGCUUC | No. 2195 | AGCAUUUGGGGACUUCGAUAG | No. 3375 | ATCGAAGTCCCCAATGCT | 0.125 |
| AH1016 | No. 1016 | UCGAAGUCCCCAAAUGCUUCA | No. 2196 | AAGCAUUUGGGGACUUCGAUA | No. 3376 | TCGAAGTCCCCAAATGCTT | 0.112 |
| AH1017 | No. 1017 | CGAAGUCCCCAAAUGCUUCAA | No. 2197 | GAAGCAUUUGGGGACUUCGAU | No. 3377 | CGAAGTCCCCAAATGCTTC | 0.109 |
| AH1018 | No. 1018 | GAAGUCCCCAAAUGCUCAAG | No. 2198 | UGAAGCAUUUGGGGAUCUUCGA | No. 3378 | GAAGTCCCCAAATGCTTCA | 0.170 |
| AH1021 | No. 1021 | GUCCCCAAAUGCUUCAAGGAA | No. 2201 | CCUUGAAGCAUUUGGGGACUU | No. 3381 | GTCCCCAAATGCTTCAAGG | 0.261 |
| AH1022 | No. 1022 | UCCCCAAAUGCUUCAAGGAAC | No. 2202 | UCCUUGAAGCAUUUGGGGACU | No. 3382 | TCCCCAAATGCTTCAAGGA | 0.074 |
| AH1023 | No. 1023 | CCCAAAUGCUUCAAGGAACA | No. 2203 | UUCCUUGAAGCAUUUGGGGAC | No. 3383 | CCCCAAATGCTTAAGGAA | 0.154 |
| AH1024 | No. 1024 | CCAAAUGCUUCAAGGAACAC | No. 2204 | GUUCCUUGAAGCAUUUGGGGA | No. 3384 | CCCAAATGCTTCAAGGAAC | 0.108 |
| AH1025 | No. 1025 | CCAAAUGCUUCAAGGAACACA | No. 2205 | UGUUCCUUGAAGACAUUUGGGG | No. 3385 | CCAAATGCTTCAAGGAACA | 0.056 |
| AH1026 | No. 1026 | CAAAUGCUUCAAGGAACACAG | No. 2206 | GUGUUCCUUGAAGCAUUUGGG | No. 3386 | CAAATGCTTCAAGGAACAC | 0.325 |
| AH1027 | No. 1027 | AAAUGCUUCAAGGAACACAGU | No. 2207 | UGUGUUCCUUGAAGCAUUUGG | No. 3387 | AAATGCTTCAAGGAACACA | 0.158 |
| AH1028 | No. 1028 | AAUGUUCAAGGAACACAGUU | No. 2208 | CUGUGUUCCUUGAAGCAUUUG | No. 3388 | AATGCTTCAAGGAACACAG | 0.257 |

### [Table 52]

**Table 4-14**

| Double stranded nucleic acid No. | SEQ ID NO. | Sense strand sequence (5'→3') | SEQ ID NO. | Antisense strand sequence (5'→3') | SEQ ID NO. | Target β2GPI mRNA sequent | Relative expressed amount of β2GPI |
|---|---|---|---|---|---|---|---|
| AH1029 | No. 1029 | AUGCUUCAAGGAACACAGUUC | No. 2209 | ACUGUGUUCCUUGAAGCAUUU | No.3389 | ATGCTTCAAGGAACACAGT | 0.175 |
| AH1030 | No. 1030 | UGCUUCAAGGAACACAGUUCU | No. 2210 | AACUGUGUUCCUUGAAGCAUU | No. 3390 | TGCTTCAAGGAACACAGTT | 0.200 |
| AH1031 | No. 1031 | GCUUCAAGGAACACAGUUCUC | No. 2211 | GAACUGUGUUCCUUGAAGCAU | No. 3391 | GCTTCAAGGAACACAGTTC | 0.074 |
| AH1032 | No. 1032 | CUUCAAGGAACACAGUUCUCU | No. 2212 | AGAACUGUGUUCCUUGAAGCA | No. 3392 | CTTCAAGGAACACAGTTCT | 0.083 |
| AH1034 | No. 1034 | UCAAGGAACACAGUUCUCUGG | No. 2214 | AGAGAACUGUGUUCCUUGAAG | No. 3394 | TCAAGGAACACAGTTCTCT | 0.279 |
| AH1035 | No. 1035 | CAAGGAACACAGUUCUCUGGC | No. 2215 | CAGAGAACUGUGUUCCUUGAA | No. 3395 | CAAGGAACACAGTTCTCTG | 0.238 |
| AH1038 | No. 1038 | GGAACACAGUUCUCUGGCUUU | No. 2218 | AGCCAGAGAACUGUGUUCCUU | No. 3398 | GGAACACATTTCTCTGGCT | 0.067 |
| AH1039 | No. 1039 | GAACACAGUUCUCUGGCUUUU | No. 2219 | AAGCCAGAGAACUGUGUUCCU | No. 3399 | GAACACAGTTCTCTGGCTT | 0.072 |
| AH1040 | No. 1040 | AACACAGUUCUCUGGCUUUUU | No. 2220 | AAAGCCAGAGAACUGUGUUCC | No. 3400 | AACACAGTTCTCTGGCTT | 0.094 |
| AH1041 | No. 1041 | ACACAGUUCUCUGGCUUUUUG | No. 2221 | AAAAGCCAGAGAACUGUGUUC | No. 3401 | ACACAGTTCTCTGGCTTT | 0.071 |
| AH1042 | No. 1042 | CACAGUUCUCUGGCUUUUUGG | No. 2222 | AAAAAGCCAGAGAACUGUGUU | No. 3402 | CACAGTTCTGGCTTTTT | 0. 143 |
| AH 1043 | No. 1043 | ACAGUUCUCUGGCUUUUUGGA | No. 2223 | CAAAAAGCCAGAGAACUGUGU | No. 3403 | ACAGTTCTCTGGCTTTTTG | 0.082 |
| AH1044 | No 1044 | CAGUUCUCUGGCUUUUUGGAA | No. 2224 | CCAAAAAGCCAGAGAACUGUG | No. 3404 | CAGTTCTCTGGCTTTTTGG | 0.199 |
| AH1045 | No. 1045 | AGUUCUCUGCUUUUUGGAAA | No. 2225 | UCCAAAAAGCCAGAGAACUGU | No. 3405 | AGTTCTCTGGCTTTTTGGA | 0.108 |
| AH1046 | No. 1046 | GUUCUCUGGCUUUUUGGAAAA | No. 2226 | UUCCAAAAAGCCAGAGAACUG | No. 3406 | GTTCTCTGGCTTTTTGGAA | 0.156 |
| AH1047 | No. 1047 | UUCUCUGGCUUUUUGGAAAAC | No. 2227 | UUUCCAAAAAGCCAGAGAACU | No. 3407 | TTCTCTGGCTTTTTGGAAA | 0.111 |
| AH1048 | No. 1048 | UCUCUGGCUUUUUGGAAAACU | No. 2228 | UUUUCCAAAAAGCCAGAGAAC | No. 3408 | TCTCTGGCTTTTTGGAAA | 0.313 |
| AH1051 | No. 1051 | CUGGCUUUUUGGAAAACUGAU | No. 2231 | CAGUUUUCCAAAAAGCCAGAG | No. 3411 | CTGGCTTTTTGGAAAACTG | 0.152 |
| AH1052 | No. 1052 | UGGCUUUUUGGAAAACUGAUG | No. 2232 | UCAGUUUUCCAAAAAGCCAGA | No. 3412 | TGGCTTTTTGGAAAACTGA | 0.097 |
| AH1053 | No. 1053 | GGCUUUUUGGAAAACUGAUGC | No. 2233 | AUCAGUUUUCCAAAAAGCCAG | No. 3413 | GGCTTTTTGGAAAACTGAT | 0.162 |
| AH1054 | No. 1054 | GCUUUUUGGAAAACUGAUGCA | No. 2234 | CAUCAGUUUUCCAAAAAGCCA | No. 3414 | GCTTTTTGGAAAACTGATG | 0.200 |
| AH1056 | No. 1056 | UUUUUGGAAAACUGAUGCAUC | No. 2236 | UGCAUCAGUUUUCCAAAAAGC | No. 3416 | TTTTTGGAAAACTGATGCA | 0.342 |
| Ai 057 | No. 1057 | UUUUGGAAAACUGAUGCAUCC | No. 2237 | AUGCAUCAGUUUUCCAAAAAG | No. 3417 | TTTTGGAAAACTGATGCAT | 0.230 |
| AH1061 | No. 1061 | GGAAAACUGAUGCAUCCGAUG | No. 2241 | UCGGAUGCAUCAGUUUUCCAA | No. 3421 | GGAAAACTGATGCATCCGA | 0.128 |
| AH1062 | No. 1062 | GAAAACUGAUGCAUCCGAUGU | No. 2242 | AUCGGAUGCAUCAGUUUUCCA | No. 3422 | GAAAACTGATGCATCCGAT | 0.182 |
| AH1063 | No. 1063 | AAAACUGAUGCAUCCGAUGUA | No. 2243 | CAUCGGAUGCAUCAGUUUUCC | No. 3423 | AAAACTGATGCATCCGATG | 0. 229 |
| AH1064 | No. 1064 | AAACUGAGCAUCCGAUGUAA | No. 2244 | ACAUCGGAUGCAUCAGUUUUC | No. 3424 | AAACTGATGCATCCGATGT | 0.336 |
| AH1065 | No. 1065 | AACUGAUGCAUCCGAUGUAAA | No. 2245 | UACAUCGGAUGCAUCAGUUUU | No. 3425 | AACTGATGCATCCGATGTA | 0.216 |
| AH1067 | No. 1067 | CUGAUGCAUCCGAUGUAAAGC | No. 2247 | UUUACAUCGGAUGCAUCAGUU | No. 3427 | CTGATGCATCCGATGTAAA | 0.241 |
| AH1068 | No. 1068 | UGAUGCAUCCGAUGUAAAGCC | No. 2248 | CUUUACAUCGGAUGCAUCAGU | No. 3428 | TGATGCATCCGATGTAAAG | 0.329 |
| AH1072 | No. 1072 | GCAUCCGAUGUAAAGCCAUGC | No. 2252 | AUGGCUUUACAUCGGAUGCAU | No. 3432 | GCATCCGATGTAAGCCAT | 0.197 |
| AH1073 | No. 1073 | CAUCCGAUGUAAAGCCAUGCU | No. 2253 | CAUGGCUUUCAUCGGAUGCA | No. 3433 | CATCCGATGTAAAGCCATG | 0.120 |
| AH1076 | No. 1076 | CCGAUGUAAAGCCAUGCUAAG | No. 2256 | UAGCAUGGCUUUACAUGGAU | No. 3436 | CCGATGTAAAGCCATGCTA | 0.219 |
| AH1077 | No. 1077 | CGAUGUAAAGCCAUGCUAAGG | No. 2257 | UUAGCAUGGCUUUACAUCGGA | No. 3437 | CGATGTAAAGCCATGCTAA | 0.061 |
| AH1078 | No. 1078 | GAUGUAAAAGCCAUGCUAAGGU | No. 2258 | CUUAGCAUGGCUUUACAUCGG | No. 3438 | GATGTAAAGCCATGCTAAG | 0.131 |
| AH1083 | No. 1083 | AAAGCCAUGCUAAGGUGGUUU | No. 2263 | ACCACCUUAGCAUGGCUUUAC | No. 3443 | AAAGCCATGCTAAGGTGGT | 0.239 |
| AH1084 | No. 1084 | AAGCCAUGCUAAGGUGGUUUU | No. 2264 | AACCACCUUAGCAUGGCUUUA | No. 3444 | AAGCCATGCTAAGGTGGTT | 0.139 |
| AH1085 | No. 1085 | AGCCAUGCUAAGGUGGUUUUC | No. 2265 | AAACCACCUUAGCAUGGCUUU | No. 3445 | AGCCATGCTAAGGTGGTTT | 0.166 |
| AH1086 | No. 1086 | GCCAUGCUAAGGUGGUUUUCA | No. 2266 | AAAACCACCUUAGCAUGGCUU | No. 3446 | GCCATGCTAAGGTGGTTTT | 0.100 |
| AH1087 | No. 1087 | CCAUGCUAAGGUGGUUUUCAG | No. 2267 | GAAAACCAACCUUAGCAUGGCU | No. 3447 | CCATGCTAAGGTGGTTTTC | 0.233 |
| AH1088 | No. 1088 | CAUGCUAAGGUUGGUUUUCAGA | No. 2268 | UGAAAACCACCUUAGCAUGGC | No. 3448 | CATGCTAAGGTGGTTTTCA | 0.072 |
| AH1089 | No. 1089 | AUGCUAAGGUGGUUUUCAGAU | No. 2269 | CUGAAAACCACCUUAGCAUGG | No. 3449 | ATGCTAAGGTGGTTTTCAG | 0.175 |
| AH1090 | No 1090 | UGCUAAGGUGGUUUUCAGAUU | No. 2270 | UCUGAAAACCAACCUUAGCAUG | No. 3450 | TGCTAAGGTGGTTTTCAGA | 0.130 |
| AH1092 | No. 1092 | CUAAGGUGGUUUUCAGAUUCC | No. 2272 | AAUCUGAAACCAACCUUAGCA | No. 3452 | CTAAGGTGGTTTTCAGATT | 0.065 |
| AH1093 | No. 1093 | UAAGGUGGUUUUCAGAUUCCA | No. 2273 | GAAUCUGAAAACCACCUUAGC | No. 3453 | TAAGGTGGTTTTCAGATTC | 0. 141 |
| AH1095 | No. 1095 | AGGUGGUUUUCAGAUUCCACA | No. 2275 | UGGAAUCUGAAAACCACCUUA | No. 3455 | AGGTGGTTTTCAGATTCCA | 0.044 |

### [Table 53]

**Table 4-15**

| Double stranded nucleic acid No. | SEQ ID No. | Sense strand sequence (5'→3') | SEQ ID NO. | Antisense strand sequence (5'→3') | SEQ ID NO. | Target β2GPI mRMA sequence | Relative expressed amount of β2GPI |
|---|---|---|---|---|---|---|---|
| AH1096 1096 | No. 1096 | GGUGGUUUUCAGAUUCCACAC | No. 2276 | GUGGAAUCUGAAAACCACCUU | No. 3456 | GGTGGTTTTCAGATTCCAC | 0.076 |
| AH1097 | No. 1097 | GUGGUUUUCAGAUUCCACACA | No. 2277 | UGUGGAAUCUGAAAACCACCU | No. 3457 | GTGGTTTTCAGATTCCACA | 0.045 |
| AH1099 | No. 1099 | GGUUUUCAGAUUCCACACAAA | No. 2279 | UGUGUGGAAUCUGAAAACCAC | No. 3459 | GGTTTTCAGATTCCACACA | 0.140 |
| AH1100 | No. 1100 | GUUUUCAGAUUCCACACAAAA | No. 2280 | UUGUGUGGAAUCUGAAAACCA | No. 3460 | GTTTTCAGATTCCACACAA | 0.120 |
| AH1101 | No. 1101 | UUUUCAGAUUCCACACAAAAU | No. 2281 | UUUGUGUGGAAUCUGAAAACC | No. 3461 | TTTTCAGATTCCACACAAA | 0.121 |
| AH1102 | No. 1102 | UUUCAGAUUCCACACAAAAUG | No. 2282 | UUUUGUGUGGAAUCUGAAAAC | No. 3462 | TTTCAGATTCCACACAAAA | 0.281 |
| AH1103 | No 1103 | UUCAGAUUCCACACAAAAUGU | No. 2283 | AUUUUGUGUGGAAUCUGAAAA | No. 3463 | TTCAGATTCCACACAAAAT | 0.197 |
| AH1104 | No. 1104 | UCAGAUUCCACACAAAAUGUC | No. 2284 | CAUUUUGUGUGGAAUCUGAAA | No. 3464 | TCAGATTCCACACAAAATG | 0.274 |
| AH1105 | No. 1105 | CAGAUUCCACACAAAAUGUCA | No. 2285 | ACAUUUUGUGUGGAAUCUGAA | No. 3465 | CAGATTCCACACAAAATGT | 0.193 |
| AH1106 | No. 1106 | AGAUUCCACACAAAAUGUCAC | No. 2286 | GACAUUUUGUGUGGAAUCUGA | No. 3466 | AGATTCCACACAAAATGTC | 0.175 |
| AH1107 | No. 1107 | GAUUCCACACAAAAUGUCACA | No. 2287 | UGACAUUUUGUGUGGAAUCUG | No. 3467 | GATTCCACACAAAATGTCA | 0.095 |
| AH1109 | No. 1109 | UUCCACACAAAAUGUCACACU | No. 2289 | UGUGACAUUUUGUGUGGAAUC | No. 3469 | TTCCACACAAAATGTCACA | 0.193 |
| AH1111 | No. 1111 | CCACACAAAAUGUCACACUUG | No. 2291 | AGUGUGACAUUUUGUGUGGAA | No. 3471 | CCACACAAAATGTCACACT | 0.147 |
| AH1112 | No. 1112 | CACACAAGAAUGUCACACUUGU | No. 2292 | AAGUGUGACAUUUUGUGUGGA | No. 3472 | CACACAAAATGTCACACTT | 0.067 |
| AH1113 | No. 1113 | ACACAAAAUGUCACACUUGUU | No. 2293 | CAAGUGUGACAUUUUGUGUGG | No. 3473 | ACACAAAATGCACACTTG | 0.186 |
| AH1114 | No. 1114 | CACAAAAUGUCACACUUGUUU | No. 2294 | ACAAGUGUGACAUUUUGUGUG | No. 3474 | CACAAAATGTCACACTTG | 0.082 |
| AH1115 | No. 1115 | ACAAAAUGUCACACUUGUUUC | No. 2295 | AACAAGUGUGACAUUUUGUGU | No. 3475 | ACAAAATGTCACACTTGTT | 0.229 |
| AH1116 | No. 1116 | CAAAAUGUCACACUUGUUUUCU | No. 2296 | AAACAAGUGUGACAUUUUGUG | No. 3476 | CAAAATGTCACACTTGTTT | 0.074 |
| AH1118 | No. 1118 | AAAUGUCACACUUGUUUCUUG | No. 2298 | AGAAACAAGUGUGACAUUUUG | No. 3478 | AAATGTCACACTTGTTCT | 0.073 |
| AH1120 | No. 1120 | AUGUCACACUUGUUUCUUGUU | No. 2300 | CAAGAAACAAGUGUGACAUUU | No. 3480 | ATGTCACACTTGTTTCTTG | 0.140 |
| AH121 | No. 1121 | UGUCACACUUGUUUCUUGUUC | No. 2301 | ACAAGAAACAAGUGUGACAUU | No. 3481 | TGTCACACTTGTTTCTGT | 0.090 |
| AH1122 | No. 1122 | GUCACACUUGUUUCUUGUUCA | No. 2302 | AACAAGAAACAAGUGUGACAU | No. 3482 | GTCACACTTGTTTCTTGTT | 0.085 |
| AH1124 | No. 1124 | CACACUUGUUUCUUGUUCAUC | No. 2304 | UGAACAAGAAACAAGUGUGAC | No. 3484 | CACACTTGTTTCTTGTTCA | 0.050 |
| AH1125 | No. 1125 | ACACUUGUUUCUUGUUCAUCC | No. 2305 | AUGAACAAGAAACAAGUGUGA | No. 3485 | ACACTTGTTTCTTGTTCAT | 0.065 |
| AH1127 | No. 1127 | ACUUGUUUCUUGUUCAUCCAA | No. 2307 | GGAUGAACAAGAAACAAGUGU | No. 3487 | ACTTGTTTCTGTTCATCC | 0.169 |
| AH1128 | No. 1128 | CUUGUUUCUUGUUCAUCCAAG | No. 2308 | UGGAUGAACAAGAAACAAGUG | No. 3488 | CTTGTTTCTTGTTCATCCA | 0.029 |
| AH1129 | No. 1129 | UUGUUUCUUGUUCAUCCAAGG | No. 2309 | UUGGAUGAACAAGAAACAAGU | No. 3489 | TTGTTTCTTGTTCATCCAA | 0.065 |
| AH1130 | No. 1130 | UGUUUCUUGUUCAUCCAAGGA | No. 2310 | CUUGGAUGAACAAGAAACAAG | No. 3490 | TGTTTCTTGTTCATCCAAG | 0.307 |
| AH1131 | No. 1131 | GUUUCUUGUUCAUCCAAGGAA | No. 2311 | CCUUGGAUGAACAAGAACAA | No. 3491 | GTTTCTTGTTCATCCAAGG | 0.163 |
| AH1133 | No. 1133 | UUCUUGUUCAUCCAAGGAACC | No. 2313 | UUCCUUGGAUGAACAAGAAAC | No. 3493 | TTCTTGTTCATCCAAGGAA | 0.196 |
| AH1134 | No. 1134 | UCUUGUUCAUCCAAGGAACCU | No. 2314 | GUUCCUUGGAUGAACAAGAAA | No. 3494 | TCTTGTTCATCCAAGGAAC | 0.261 |
| AH1135 | No. 1135 | CUUGUUCAUCCAAGGAACCUA | No. 2315 | GGUUCCUUGGAUGAACAAGAA | No. 3495 | CTGTTCATCCAAGGAACC | 0.145 |
| AH1136 | No. 1136 | UUGUUCAUCCAAGGAACCUAA | No. 2316 | AGGUUCCUUGGAUGAACAAGA | No. 3496 | TTGTTCATCCAAGGAACCT | 0.219 |
| AH1137 | No. 1137 | UGUUCAUCCAAGGAACCUAAU | No. 2317 | UAGGUUCCUUGGAUGAACAAG | No. 3497 | TGTTCATCCAAGGAACCTA | 0.044 |
| AH1138 | No. 1138 | GUUCAUCCAAGGAACCUAAUU | No. 2318 | UUAGGUUCCUUGGAUGAACAA | No. 3498 | GTTCATCCAAGGAACCTAA | 0.036 |
| AH1139 | No. 1139 | UUCAUCCAAGGAACCUAAUUG | No. 2319 | AUUAGGUUCCUUGGAUGAACA | No. 3499 | TTCATCCAAGGAACCTAAT | 0.061 |
| AH1140 | No. 1140 | UCAUCCAAGGAACCUAAUUGA | No. 2320 | AAUUAGGUUCCUUGGAUGAAC | No. 3500 | TCATCCAAGGAACCTAATT | 0.110 |
| AH1141 | No. 1141 | CAUCCAAGGAACCUAAULGAA | No. 2321 | CAAUUAGGUUCCUUGGAUGAA | No. 3501 | CATCCAAGGAACCTAATTG | 0.063 |
| AH1142 | No. 1142 | AUCCAAGGAACCUAAUUGAAA | No. 2322 | UCAAUUAGGUUCCUUGGAUGA | No. 3502 | ATCCAAGGAACCT AATTGA | 0.045 |
| AH1143 | No. 1143 | UCCAAGGAACCUAAUUGAAAU | No. 2323 | UUCAAUUAGGUUCCUUGGAUG | No. 3503 | TCCAAGGAACCTAATTGAA | 0.042 |
| AH1144 | No. 1144 | CCAAGGAACCUAAUUGAAAUU | No. 2324 | UUUCAAUUAGGUUCCUUGGAU | No. 3504 | CCAAGGAACCTAATTGAAA | 0.036 |
| AH1145 | No. 1145 | CAAGGAACCUAAUUGAAAUUU | No. 2325 | AUUUCAAUUAGGUUCCUUGGA | No. 3505 | CAAGGAACCAATTGAAAT | 0.042 |
| AH1146 | No. 1146 | AAGGAACCUAAUUGAAAUUUA | No. 2326 | AAUUUCAAUUAGGUUCCUUGG | No. 3506 | AAGGAACCTAATTGAAATT | 0.056 |
| AH1147 | No. 1147 | AGGAACCUAAUUGAAAUUUAA | No. 2327 | AAAUUCAAUUAGGUUCCUUG | No. 3507 | AGGAACCTAATTGAAATTT | 0.244 |
| AH1148 | No. 1148 | GGAACCUAAUUGAAAUUUAAA | No. 2328 | UAAAUUUCAAUUAGGUUCCUU | No. 3508 | GGAACCTAATTGAAATTA | 0.037 |
| AH1149 | No. 1149 | GAACCUAAUUGAAAUUUAAAA | No. 2329 | UUAAAUUUCAAUUAGGUUCCU | No. 3509 | GAACCTAATTGAAA TTTAA | 0.034 |

### [Table 54]

**Table 4-16**

| Double stranded nucleic acid No. | SEQ ID NO. | Sense strand sequence (5'→3') | SEQ ID NO. | Antisense strand sequence (5'→3') | SEQ ID NO. | Target β2GPI mRNA sequence | Relative expressed amount of 02GPI |
|---|---|---|---|---|---|---|---|
| AH1150 | No. 1150 | AACCUAAUUGAAAUUUAAAAA | No. 2330 | UUUAAAUUUCAAUUAGGUUCC | No. 3510 | AACCTAATTGAAATTTTAAA | 0.097 |
| AH1151 | No. 1151 | ACCUAAUUGAAAUUUAAAAAU | No. 2331 | UUUUAAAUUUCAAUUAGGUUC | No. 3511 | ACCTAATTGAAATTTAAAA | 0.330 |
| AH1155 | No. 1155 | AAUUGAAAUUUAAAAUAAAG | No. 2335 | UUAUUUUUAAAUUUCAAUUAG | No. 3515 | AATTGAAATTTAAAAATAA | 0.318 |
| AH1167 | No. 1167 | AAAAUAAAGCUACUGAAUUUA | No. 2347 | AAUUCAGUAGCUUUAUUUUUA | No. 3527 | AAAATAAAGCACTGAATT | 0.313 |
| AH1168 | No. 1168 | AAAUAAAGCUACUGAAUUUAU | No. 2348 | AAAUUCAGUAGCUUUAUUUUU | No. 3528 | AAATAAAGCTACTGAATTT | 0.082 |
| 4H1169 | No. 1169 | AAUAAAGCUACUGAAUUUAUU | No. 2349 | UAAAUUCAGUAGCUUUAUUUU | No. 3529 | AATAAAGCTACTGAATTTA | 0.174 |
| AH1171 | No. 1171 | UAAAGCUACUGAAUUUAUUGC | No. 2351 | AAUAAAUUCAGUACCUUUAUU | No. 3531 | TAAAGCTACTGAATTTATT | 0.191 |
| AH1175 | No. 1175 | GCUACUGAAUUUAUUGCCGCA | No. 2355 | CGGCAAUAAAUUCAGUAGCUU | No. 3535 | GCTACTGAATTTATTGCCG | 0.156 |
| AH1176 | No. 1176 | CUACUGAAUUUAUUGCCGCAC | No. 2356 | GCGGCAAUAAAUUCAGUAGCU | No. 3536 | CTACTGAATTTATTGCCGC | 0.230 |
| 4H1177 | No. 1177 | UACUGAAUUUAUUGCCGCACC | No. 2357 | UGCGGCAAUAAAUUCAGUAGC | No. 3537 | TACTGAATTTATTGCCGCA | 0.155 |
| AH1178 | No. 1178 | ACUGAAUUUAUUGCCGCACCC | No. 2358 | GUGCGGCAAUAAAUUCAGUAG | No. 3538 | ACTGAATTTATTGCCGCAC | 0.309 |

In order to select double-stranded nucleic acids having high knockdown activity, the relative expressed amount of the β2GPI gene was calculated in the same experimental system with the final concetration of the double-stranded nucleic acid decreased to 10 pmol/L. The results are described in Table 5.

### [Table 55]

**Table 5**

| Double stranded nucleic acid No. | Relative expressed amount of β2GPI | Double stranded nucleic acid No. | Relative expressed amount of β2GPI |
|---|---|---|---|
| AH0075 | 0.206 | AH0808 | 0.283 |
| AH0096 | 0.204 | AH0821 | 0.257 |
| AH0099 | 0.159 | AH0825 | 0.230 |
| AH0119 | 0.170 | AH0832 | 0.277 |
| AH0125 | 0.108 | AH0876 | 0.246 |
| AH0126 | 0.143 | AH0879 | 0.168 |
| AH01 33 | 0.202 | AH0883 | 0.173 |
| AH0134 | 0.256 | AH0887 | 0.243 |
| AH0149 | 0.172 | AH0943 | 0.110 |
| AH0152 | 0.114 | AH1022 | 0.288 |
| AH0154 | 0.219 | AH1025 | 0.218 |
| AH0155 | 0.206 | AH1031 | 0264 |
| AH0181 | 0.240 | AH1038 | 0.277 |
| AH0184 | 0.170 | AH1039 | 0.212 |
| AH0185 | 0.104 | AH1077 | 0.292 |
| AH0187 | 0.117 | AH1092 | 0.240 |
| AH0188 | 0.180 | AH1095 | 0.282 |
| AH0193 | 0.254 | AH1097 | 0.201 |
| AH0236 | 0.127 | AH1112 | 0.156 |
| AH0275 | 0.192 | AH1116 | 0.291 |
| AH0296 | 0214 | AH1118 | 0.268 |
| AH0324 | 0.255 | AH1124 | 0.160 |
| AH0330 | 0.265 | AH1125 | 0.215 |
| AH0345 | 0.242 | AH1128 | 0.164 |
| AH0371 | 0.167 | AH1129 | 0.276 |
| AH0394 | 0.285 | AH1137 | 0.288 |
| AH0504 | 0.282 | AH1139 | 0.165 |
| AH0528 | 0.197 | AH1141 | 0.130 |
| AH0529 | 0.107 | AH1142 | 0.078 |
| AH0592 | 0.279 | AH1143 | 0.110 |
| AH0689 | 0.178 | AH1144 | 0.073 |
| AH0693 | 0.206 | AH1145 | 0.091 |
| AH0705 | 0.281 | AH1146 | 0.178 |
| AH0712 | 0.149 | AH1148 | 0.106 |
| AH0713 | 0.199 | AH1149 | 0.110 |

### Example 1: Preparation of nucleic acid conjugate containing β2GPI gene-suppressing double-stranded nucleic acid (siRNA) and a ligand part

### Step 1

β2GPI-targeting siRNAs (G-CH1179-s, G-CH0099-s and G-CH1180-s) having a ligand part at the 3' end of the antisense strand of CH1179, CH0099 or CH1180 were synthesized on a scale of 0.2 µmol with a nucleic acid synthesizer (Ultra Fast Parallel Synthesizer, Sigma, hereinafter UFPS). Compound 9 obtained in step 7 of Reference Example 2 was used as the solid-phase carrier. DMT-2'-O-methyl-rA((tert-butyl)phenoxyacetyl)amidite (SAFC-Proligo), DMT-2'-fluoro-dA(benzoyl)amidite (SAFC-Proligo), DMT-2'-O-methyl-rG((tert-butyl)phenoxyacetyl)amidite (SAFC-Proligo), DMT-2'-fluoro-dG(isobutyryl)amidite (SAFC-Proligo), DMT-2'-O-methyl-rC((tert-butyl)phenoxyacetyl)amidite (SAFC-Proligo), DMT-2'-fluoro-dC(acetyl)amidite (SAFC-Proligo), DMT-2'-O-methyl-rU amidite (SAFC-Proligo) and DMT-2'-fluoro-dU amidite (SAFC-Proligo) were prepared as 0.1 mol/L acetonitrile solutions, chemical phosphorylation reagent II (Glen Research Corporation) was prepared as a 0.06 mol/L acetonitrile solution, and sulfurization reagent II (Glen Research Corporation) was prepared as a 0.05 mol/L acetonitrile solution, and they were used for a condensation reaction. Deblocking solution l (3% trichloracetic acid dichloromethane solution, Wako Pure Chemical Industries, Ltd.) was used as a deprotection reagent. 5-benzylthio-1 H-tetrazole (SAFC-Proligo) was used as a phosphoramidite activator, and the condensation time was 10 minutes each. After synthesis with trityl-off, the product was immersed in 28% ammonia solution, and left standing for 4 hours at 55°C. This was concentrated under reduced pressure, and 1-butanol was added to stop the reaction. This was then purified by reverse-phase liquid chromatography (Shiseido Capsell Pak C18, SG300, 6.0 mm × 75 mm, 5% acetonitrile/0.1% triethyl ammonium acetate buffer, B solution: 50% acetonitrile/water gradient) to obtain the target oligonucleotide. The results are described in Table 6.

### [Table 56]

**Table 6**

| Compound | Sequence (5'→3') | Molecular weight (theoretical value) | Molecular weight (Measured value) |
|---|---|---|---|
| G-CH1179-s | C(F)^U(M)^C(F)U(M)G(F)C(M)C(F)A(M)U(F)G(M)U(F)U(F)G(M)C(F)U(M)A(F)U(M)U(F)G(M)C(F)A(M)X | 8558.3 | 8556.4 |
| G-CH0099-s | U(F)^G(M)^C(F)C(M)A(F)U(M)G(F)U(M)U(F)G(M)C(F)U(F)A(M)U(F)U(M)G(F)C(M)A(F)G(M)G(F)A(M)X | 8661.3 | 8659.4 |
| G-CH1180-s | G(F)^C(M)^U(F)G(M)G(F)A(M)A(F)U(M)C(F)U(M)U(F)A(F)G(M)A(F)A(M)A(F)A(M)U(F)G(M)G(F)A(M)X | 8755.5 | 8758.0 |

(wherein, N(M) represents 2'-O-methyl modified RNA, N(F) represents 2'-fluorine modified RNA, ^ represents phosphorothioate modification, and X represents the ligand part in the nucleic acid conjugate I in Table 1. The phrase "ligand part in the nucleic acid conjugate in Table 1" means a structure of the nucleic acid conjugate I excluding the oligonucleotide part.)

### Step 2

The resulting single-stranded oligonucleotide was dissolved in a mixed buffer [100 mmol/L potassium acetate, 30 mmol/L 2-[4-(2-hydroxyethyl)piperazin-1-yl]ethanesulfonic acid, HEPES)-KOH (pH 7.4), 2 mmol/L magnesium acetate] so as to be a concentration of 50 µmol/L. The sense strand and the antisense strand were mixed in equal quantities, and left standing for 10 minutes at 80°C. The sense strand sequences were as shown by CH1179, CH0099 and CH1180 in Tables 3-2 and 3-18. The temperature was lowered gradually, and it was left standing for 1 hour at 37°C to obtain a double-stranded oligonucleotide. The resulting nucleic acid conjugates in which a ligand part was attached to the double-stranded nucleic acid (siRNA) of CH1179, CH0099 or CH1180 were called G-CH1179, G-CH0099 and G-CH1180.

### Test Example 2: In vitro activity of nucleic acid conjugate in mouse primary hepatocytes

Each of the nucleic acid conjugates obtained in Example 1 was introduced into mouse primary hepatocytes from CD-1 (Life Technologies Corporation, Catalog No. MSCP10) by the method described below.

Each nucleic acid conjugate was diluted with Opti-MEM (Gibco, 31985) so as to be a final concentration of 10 or 3 nmol/L, and 20 µL of the dilution was dispensed into each well of a 96-well culture plate, to which mouse primary hepatocytes suspended in William's E Medium (Life Technologies Corporation, Catalog No. A12176-01) containing Primary Hepatocyte Thawing and Plating Supplements (Life Technologies Corporation, Catalog No. CM3000) were seeded so as to be 12,500 cells/80 µL/well. The plate was cultured for 6 hours under conditions of 37°C, 5% CO₂ The culture supernatant was carefully removed and William's E Medium containing Primary Hepatocyte Maintenance Supplements (Life Technologies Corporation, Catalog No. CM4000) was added. Untreated cells were also seeded as a negative control group.

The cells to which each preparation was introduced were cultured for 18 hours in an incubator under conditions of 37°C, 5% CO₂ and washed with ice-cooled phosphate-buffered saline. Total RNA was collected using a Cells-to-Ct kit (Applied Bioscience (ABI), Catalog No. AM1728) according to the method described in the attached instruction, and cDNA was prepared by a reverse transcription reaction using the resulting total RNA as a template.

Using the resulting cDNA as a template and a Universal Probe Library (Roche Applied Science, 04683633001) as a probe, a PCR reaction was performed with ABI7900HT Fast (ABI) according to the method described in the attached instruction to amplify the mRNAs of the β2GPI gene and D-glyceraldehyde-3-phosphate dehydrogenase (hereinafter referred to as gapdh) gene (which is a constitutively expressed gene). The amplified amounts of the mRNAs were measured, and the semi-quantitative value of the β2GPI mRNA was calculated using the amplified amount of the gapdh mRNA as an internal control. The semi-quantitative value of the β2GPI mRNA of the negative control measured in the same way being set to be 1.0, the expression rate of the β2GPI mRNA was determined from the semi-quantitative value of the β2GPI mRNA. The results for expression rate of the β2GPI mRNA are described in Table 7 as IC50 values.

### [Table 57]

**Table 7**

| Nucleic acid conjugate name | G-CH1179 | | G-CH0099 | | G-CH1180 | |
|---|---|---|---|---|---|---|
| Dosage (nmol/L) | 10 | 3 | 10 | 3 | 10 | 3 |
| Amount of β2GPI mRNA (suppression rate %) | 98.7 | 96.4 | 80.4 | 67.6 | 95.6 | 86. 1 |

It is clear from Table 7 that each nucleic acid conjugate obtained in Example 1 and introduced into mouse primary hepatocytes suppressed expression of β2GPI gene mRNA.

### Test Example 3: In vivo activity of nucleic acid conjugate in mice

Each nucleic acid conjugate obtained in Example 1 was evaluated in vivo by the following method. Each nucleic acid conjugate was diluted with phosphate-buffered saline (DPBS, Nacalai Tesque, Inc.) for testing purposes. Mice (Balb/c, obtained from CLEA Japan, Inc.) were tamed, and each nucleic acid conjugate was administered to the mice subcutaneously at dosages of 3 mg/kg or 20 mg/kg. 3 days after administration, the livers were harvested and stored frozen with liquid nitrogen. Total RNA was then collected from the frozen liver samples with Trizol® RNA Isolation Reagents (Life Technologies Corporation, Catalog No. 15596026) and an RNeasy Mini Kit ((Qiagen N.V.), Catalog No. 74106) according to the method described in the attached instruction. Using the resulting RNA as a template, cDNA was then prepared by a reverse transcription reaction with a Transcriptor First Strand cDNA Synthesis Kit (Roche, Catalog No. 04897030001) according to the method described in the attached instruction. Using the resulting cDNA as a template and a TaqMan® Gene Expression Assays Probe (Applied Biosystems, Inc.) as a probe, a PCR reaction was performed with ABI7900 HT Fast (ABI) according to the method described in the attached instruction to amplify the mRNAs of the β2GPI gene and D-glyceraldehyde-3-phosphate dehydrogenase (hereinafter referred to as gapdh) gene (which is a constitutively expressed gene). The amplified amounts of the mRNAs were measured, and the semi-quantitative value of the β2GPI mRNA was calculated using the amplified amount of the gapdh mRNA as an internal control. The semi-quantitative value of the β2GPI mRNA of the group receiving phosphate-buffered saline measured in the same way being set to be 1, the expression rate of the β2GPI mRNA was determined from the semi-quantitative value of the β2GPI mRNA. The results for expression rate of the β2GPI mRNA are described in Table 8.

### [Table 58]

**Table 8**

| Nucleic acid conjugate name | G-CH1179 | | G-CH0099 | | G-CH1180 | |
|---|---|---|---|---|---|---|
| Dosage (mg/kg) | 3 | 20 | 3 | 20 | 3 | 20 |
| Amount of β2GPI mRNA in liver (suppression rate %) | 88.2 | 98.6 | 80.2 | 98.6 | 57.1 | 95.8 |

It is clear from Table 8 that a β2GPI-associated disease can be treated by administering the nucleic acid conjugate of the present invention to a mammal and reducing expression of the β2GPI gene in vivo.

### Industrial Applicability

An administration of the nucleic acid conjugate of the present invention to a mammal can suppress β2GPI gene expression in vivo and thereby treat a β2GPI-associated disease.

### Sequence Listing Free Text

SEQ ID NOS: 1 to 1180 represent sense strand RNA base sequences of siRNA for a β2GPI gene.
SEQ ID NOS: 1181 to 2360 represent antisense strand RNA base sequences of siRNA for a β2GPI gene.
SEQ ID NOS: 2361 to 3540 represent DNA base sequences for a target β2GPI gene.
SEQ ID NO: 3541 represents the cDNA base sequence of a β2GPI gene.
SEQ ID NOS: 4001 to 4702 represent the sense strand RNA base sequences of double-stranded nucleic acids (BH033 to BH1180).
SEQ ID NOS: 4703 to 5404 represent the antisense strand RNA base sequences of double-stranded nucleic acids (BH033 to BH1180).
SEQ ID NOS: 5405 to 6106 represent the sense strand RNA base sequences of double-stranded nucleic acids (CH033 to CH1180).
SEQ ID NOS: 6107 to 6808 represent the antisense strand RNA base sequences of double-stranded nucleic acids (CH033 to CH1180).

## Claims

1. A nucleic acid conjugate comprising: a double-stranded nucleic acid consisting of a sense strand and an antisense strand and comprising a duplex region of at least 11 base pairs; and a ligand part, wherein
an oligonucleotide strand with a strand length of 17 to 30 nucleotides in the antisense strand is complementary to a target β2GPI mRNA sequence selected from the group described in Tables 4-1 to 4-16, and
the 3' end or 5' end of the sense strand has the ligand part represented by formula (I), formula (II), formula (III), formula (IV) or formula (V): 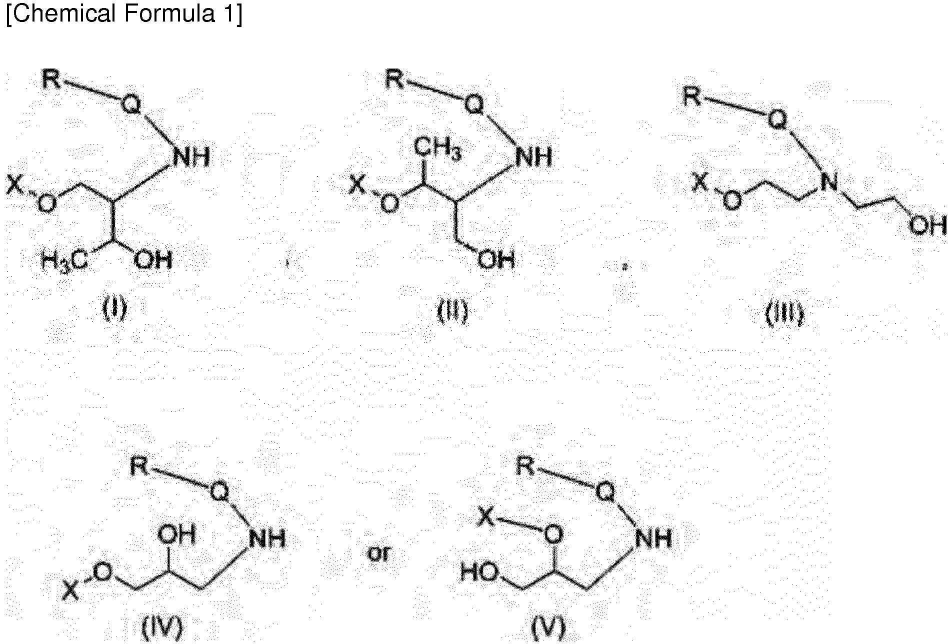 (wherein,
X is oligonucleotide-P(Z1)(Z2)-, where the oligonucleotide is the sense strand,
Q is absent or -T4-[Q4-P4]q4-,
R is the following structure 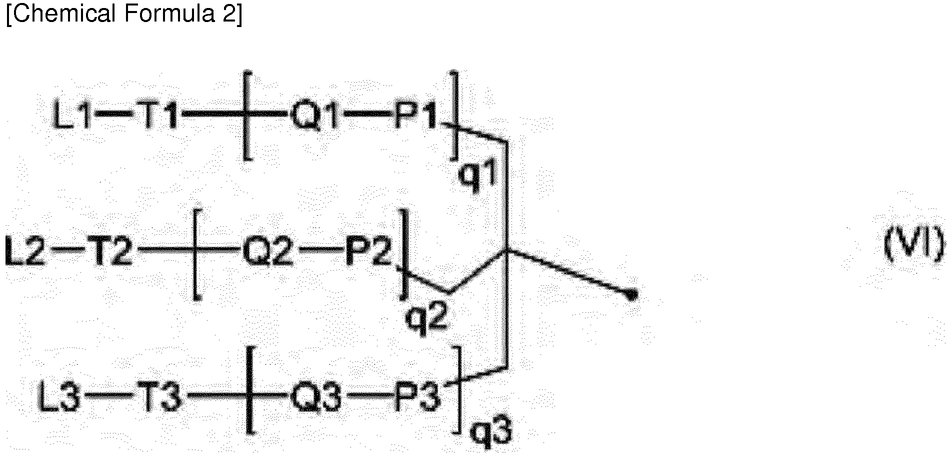
Z1 and Z2 are, independently of each other, O or S;
q1, q2, q3 and q4 are, independently of each other, an integer from 0 to 20;
P1, P2, P3 and P4 and T1, T2, T3 and T4 are, independently of each other, absent or -CO-, -NH-, -O-, -S-, -O-CO-, -NH-CO-, -CO-O- or CO-NH-, respectively, provided that when each of q1, q2, q3 and q4 is an integer from 2 to 20, each of P1, P2, P3 and P4 may be the same or different,
Q1, Q2, Q3 and Q4 are, independently of each other, absent or a substituted or unsubstituted C₁₋₁₄ alkylene, provided that when each of q1, q2, q3 and q4 is an integer from 2 to 20, each of Q1, Q2, Q3 and Q4 may be the same or different,
L1, L2 and L3 are, independently of each other, a sugar ligand.)

2. The nucleic acid conjugate according to Claim 1, wherein one or more of L1, L2 and L3 are a sugar ligand represented by the following structure 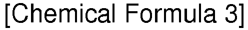 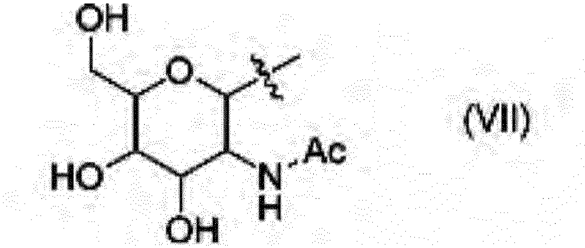 (wherein, Ac represents acetyl group).

3. The nucleic acid conjugate according to Claim 1 or 2, wherein L1, L2 and L3 are identical.

4. The nucleic acid conjugate according to any one of Claims 1 to 3, wherein
T1, T2 and T3 are identical,
q1, q2 and q3 are identical,
Q1, Q2 and Q3 are identical, and
P1, P2 and P3 are identical.

5. The nucleic acid conjugate according to any one of Claims 1 to 4, wherein R is 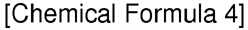 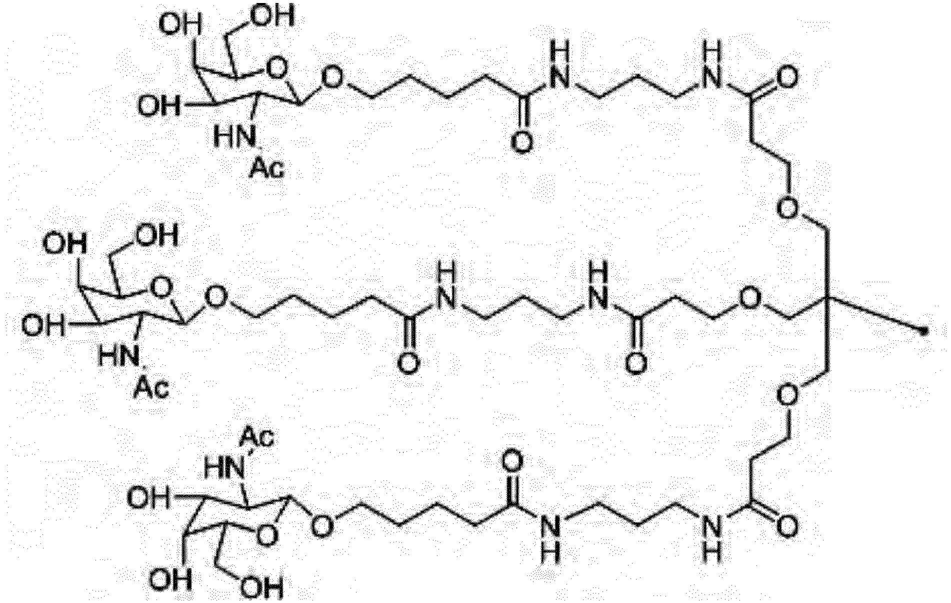 (wherein, Ac represents acetyl group).

6. The nucleic acid conjugate according to any one of Claims 1 to 5, wherein R-Q- is 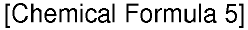 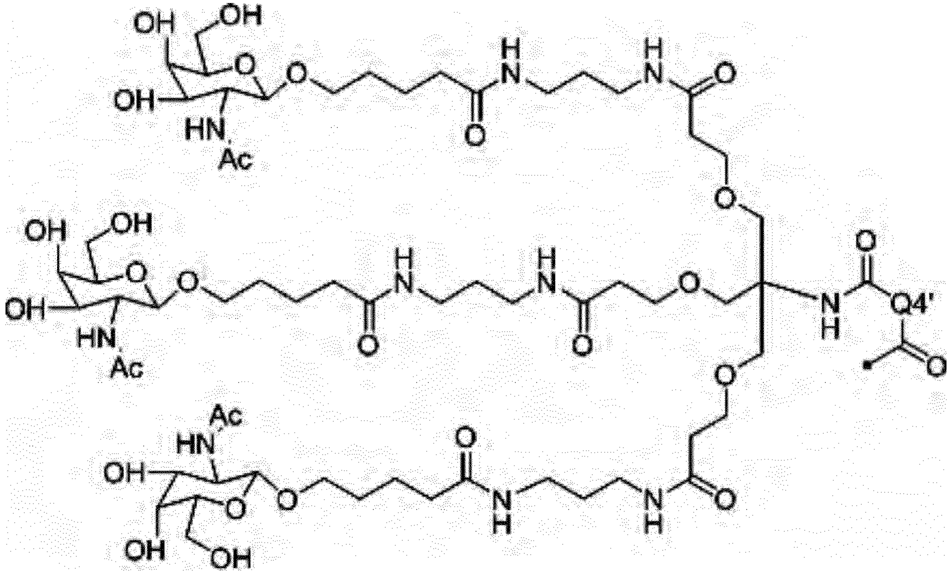 (wherein, Ac represents acetyl group, and Q4' is a substituted or unsubstituted C₁₋₁₄ alkylene).

7. The nucleic acid conjugate according to any one of Claims 1 to 6, comprising a ligand part represented by any of formulae (I) to (V) at the 3' end of the sense strand of the double-stranded nucleic acid.

8. The nucleic acid conjugate according to any one of Claims 1 to 6, comprising a ligand part represented by any of formulae (I) to (V) at the 5' end of the sense strand of the double-stranded nucleic acid.

9. The nucleic acid conjugate according to any one of Claims 1 to 8, wherein
the duplex region comprises 11 to 27 base pairs, and
the second nucleotide from the 5' end of the antisense strand, which is complementary to the target β2GPI mRNA sequence selected from the group described in Tables 4-1 to 4-16, is complementary to the second ribonucleotide from the 3' end of the target β2GPI mRNA sequence.

10. The nucleic acid conjugate according to any one of Claims 1 to 9, wherein the sense strand has a strand length of 21 to 25 nucleotides, and the antisense strand has a strand length of 21 to 25 nucleotides.

11. The nucleic acid conjugate according to any one of Claims 1 to 10, wherein the double-stranded nucleic acid in which the sense strand has a strand length of 21 to 25 nucleotides and the antisense strand has a strand length of 21 to 25 nucleotides comprises a duplex region of 19 to 21 base pairs.

12. The nucleic acid conjugate according to any one of Claims 1 to 9, wherein the 3' end of the sense strand and the 5' end of the antisense strand form a blunt end.

13. The nucleic acid conjugate according to any one of Claims 1 to 12, wherein the double-stranded nucleic acid comprises a 2'-modified nucleotide.

14. The nucleic acid conjugate according to Claim 13, wherein 50% to 100% of the nucleotides in the duplex region is the 2'-modified nucleotide.

15. The nucleic acid conjugate according to Claim 13 or 14, wherein the 2'-modified nucleotide is 2'-O-methyl modified nucleotide, 2'-F modified nucleotide or 2'-H modified nucleotide.

16. The nucleic acid conjugate according to any one of Claims 1 to 15, wherein the antisense strand comprises a sequence selected from the group of antisense strands described in Tables 2-1 to 2-18, Tables 3-1 to 3-18 or Tables 4-1 to 4-16.

17. The nucleic acid conjugate according to any one of Claims 1 to 15, wherein the sense strand comprises a sequence selected from the group of sense strands described in Tables 2-1 to 2-18, Tables 3-1 to 3-18 or Tables 4-1 to 4-16.

18. The nucleic acid conjugate according to any one of Claims 1 to 8, comprising sequences of a pair of sense/antisense strands selected from the group of sense/antisense strands described in Tables 2-1 to 2-18, Tables 3-1 to 3-18 or Tables 4-1 to 4-16.

19. A nucleic acid conjugate-comprising composition, comprising the nucleic acid conjugate according to any one of Claims 1 to 18.

20. A method for suppressing expression of a β2GPI gene, comprising introducing a double-stranded nucleic acid into cells by using the composition according to Claim 19.

21. The method according to Claim 20, wherein the cells are cells in the liver of a mammal.

22. The method according to Claim 20 or 21, wherein the method of introduction into cells is a method of introduction into cells by intravenous administration or subcutaneous administration.

23. A method for treating a β2GPI-associated disease, comprising administrating the composition according to Claim 19 to a mammal.

24. The method according to Claim 23, wherein the β2GPI-associated disease is an autoimmune disease or thrombosis.

25. The method according to Claim 23 or 24, wherein the method of administration is intravenous administration or subcutaneous administration.

26. A medicine for use in the treatment of a β2GPI-associated disease, comprising the composition according to Claim 19.

27. The medicine according to Claim 26, wherein the β2GPI-associated disease is an autoimmune disease or thrombosis.

28. The medicine according to Claim 26 or 27, for intravenous administration or subcutaneous administration.

29. An agent for treating an autoimmune disease or thrombosis, comprising the composition according to Claim 19.

30. The agent for treating an autoimmune disease or thrombosis according to Claim 29, for intravenous administration or subcutaneous administration.
